(19) Europäisches Patentamt
European Patent Office
Office européen des brevets

(11) EP 4 653 017 A1

(12) **EUROPEAN PATENT APPLICATION**
published in accordance with Art. 153(4) EPC

(43) Date of publication:
**26.11.2025 Bulletin 2025/48**

(21) Application number: **24744302.1**

(22) Date of filing: **17.01.2024**

(51) International Patent Classification (IPC):
***A61K 47/68*** *(2017.01)* ***A61K 31/7084*** *(2006.01)*
***A61K 35/00*** *(2006.01)*

(52) Cooperative Patent Classification (CPC):
**A61K 31/7084; A61K 35/00; A61K 47/68**

(86) International application number:
**PCT/CN2024/072762**

(87) International publication number:
**WO 2024/153127 (25.07.2024 Gazette 2024/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **18.01.2023 CN 202310074490**
**23.11.2023 CN 202311575575**
**12.01.2024 CN 202410046743**

(71) Applicant: **Tyligand Bioscience (Shanghai) Limited**
**Shanghai 201203 (CN)**

(72) Inventors:
- **ZHANG, Tony Yantao**
  **Fishers, IN 46037 (US)**
- **CHEN, Guangming**
  **Shanghai 201202 (CN)**
- **DONG, Chunlan**
  **Shanghai 201203 (CN)**
- **YANG, Anjiang**
  **Shanghai 200129 (CN)**
- **WANG, Xiaowen**
  **Shanghai 201299 (CN)**
- **LV, Fei**
  **Shanghai 200240 (CN)**
- **ZHENG, Puji**
  **Shanghai 201210 (CN)**

(74) Representative: **Vossius & Partner Patentanwälte Rechtsanwälte mbB**
**Siebertstraße 3**
**81675 München (DE)**

Remarks:
The complete document including Reference Table(s) and the Sequence Listing(s) can be downloaded from the EPO website

(54) **ANTIBODY-DRUG CONJUGATE AND USE THEREOF**

(57) The present invention relates to an antibody-drug conjugate, more particularly to an antibody-STING agonist conjugate (ADC), and a composition containing the ADC molecule, and the therapeutic use thereof.

EP 4 653 017 A1

## Description

### Cross Reference

This application claims priority to Chinese Invention Patent Application 2023100744909 filed January 18, 2023, Chinese Invention Patent Application 2023115755751 filed January 13, 2023, Chinese Invention Patent Application 2024100467436 filed January 12, 2024.

### Technical Field

The present invention relates to the use of antibody-drug conjugates (ADCs) for immunotherapy, and more specifically to the use of multifunctional cyclic dinucleotide (CDN) STING agonists in ADCs for immunotherapy; the present invention also relates to ADCs containing the STING agonists, methods for preparing the ADCs, compositions containing the ADCs, and their therapeutic applications.

### Background Technology

In addition to its primary function in host defense against invasion by DNA-containing microorganisms, cytosolic DNA sensing pathways play a critical role in initiating and maintaining immune responses against malignant tumors. Cytosolic DNA from damaged tumor cells trigger an enzyme called cyclic AMP-GMP synthase (cGAS), which synthesizes cyclic dinucleotide 2'3'-cyclic AMP-GMP (cGAMP). As an endogenous ligand, cGAMP binds and activates ER adaptor protein stimulator of interferon gene (STING) and leads to the induction of interferon and inflammatory cytokines, recruitment and maturation of antigen-presenting cells, and finally anti-tumor immunity by T cells and natural killer (NK) cells. Given that endogenous cGAMPs are critical mediators of the innate immune system in response to tumors, ultimately contributing to interferon or pro-inflammatory cytokine production and thereby achieving therapeutic benefit, a range of CDN-type STING agonists are being developed for cancer immunotherapy.

While existing CDN-type STING agonists hold promise for the treatment of a range of solid tumors, they have distinct limitations. Specifically, molecules of the CDN type are easily destroyed by ENPP phosphoester hydrolase in vivo and inactivated, so they have a very short half-life in the circulatory system, low bioavailability, and rely on intratumoral administration in clinical use; moreover, if injected in other ways, they have the potential to induce systemic cytokine responses, and even intratumoral injection may still induce unnecessary cytokine responses, because drug molecules leak rapidly into peripheral tissues. In addition, even when administered intratumorally, convenience and patient acceptability are limited, and some tumors are not suitable for intratumoral administration (e.g., bladder cancer). Moreover, transient exposure of such compounds to immune cells in the tumor environment also reduces their effects.

The inventor has previously developed bifunctional CDN-type STING agonists (the world's first bifunctional CDN compound that integrates cytotoxic molecules into STING agonists, WO2022083584, the entire content of which is incorporated herein) to first activate the STING innate immune pathway and kill tumor cells after entering the body; after drug decomposition, predesigned cytotoxic structure unit is released to further kill tumor cells, and provide tumor marker fragments for the acquired immune system, endow the body with immune memory, and consolidate long-term immune effects. So far, inventors have explored the activity of the bifunctional CDN-type STING agonists in multiple tumor cell lines (see Attached Table 3), all of which show more effective inhibition of tumor cell proliferation than the pure STING agonist marker compound ADU-S100, and also show significantly superior antitumor effects to ADU-S100 in the in vivo tumor pharmacological models (CT26, 4T1, A20, B16F10, H22, etc.). However, there are still common problems with the above-mentioned CDN drugs, including susceptibility to enzymatic degradation, short half-life, and restriction to intratumoral administration etc.

Therefore, there is an urgent need for novel therapies that can accurately deliver CDN STING agonists site-directed into the tumor environment, can replace intratumoral administration in a more friendly manner, and prolong efficacy and reduce toxic side effects.

Targeted conjugated drugs are a new drug delivery technology with high efficiency and low toxicity, which show strong advantages in the research and development of new drugs, particularly anticancer drugs with inherent toxic side effects. Cytotoxic molecules are linked to guide molecules, such as antibodies, peptides, GalNAc (N-acetylgalactosamine), or polymers, through suitable linkers, which can highly selectively deliver conjugated molecules to tumor tissues with highly expressed guide receptors, selectively bring anticancer drugs into cancer cells through internalization effects, and then cleave and release active anticancer molecules using differential functional molecules enriched in tumor tissues and cancer cells. Because active anticancer molecules can only be released and enriched inside tumor tissues with highly expressed guide receptors and specific "cutters", this dual selective drug delivery method greatly reduces the toxicity and improves the efficacy of anticancer drugs.

Although ADC development is in the ascendant, there are still many challenges, such as uncontrolled release or

insufficient release of payloads, off-target toxicity, prone to aggregation, resulting in some defects such as short half-life, rapid clearance and immunogenicity, complexity of pharmacokinetics, and drug resistance etc. Developing a specific cytotoxic molecule into an ideal ADC drug that remains stable in the blood circulation, precisely reaches the therapeutic target and eventually releases cytotoxic payloads within or near the target (e.g., cancer cells) is far from being accomplished overnight, but is influenced by many factors, including the choice of target, guide molecule, cytotoxic payload, linker, and coupling method, etc., each element will affect the final efficacy and safety of ADC.

**Summary of the Invention**

In order to meet the above needs in this field, the inventor has conducted in-depth study to couple the previously developed bifunctional CDN STING agonist with specific guide molecules through linker units to obtain the antibody drug conjugate as shown in the examples. The obtained conjugate has good physical properties, no obvious antibody aggregation phenomenon, can be rapidly and effectively endocytosed by tumor cells, and shows significant tumor growth inhibition activity and good tolerance compared with the antibody alone and bifunctional CDN STING agonist alone in animal models. It is expected that a more traditional, mature and friendly administration method (such as intravenous, subcutaneous, intramuscular, external application, etc.) can be used instead of intratumoral administration, prolonging the efficacy, reducing toxic side effects, and expanding the indications and beneficiary population.

Therefore, in the first aspect, the present invention provides antibody-drug conjugates (ADCs) with the following Formula (X) or pharmaceutically acceptable salts or solvates thereof :

$$Ab\text{-}[L\text{-}D]_q \qquad (X)$$

wherein,

Ab represents an antibody or antigen-binding fragment;

D represents a cyclic dinucleotide defined herein (e.g., compounds of Formula (II), (II-a), (II-a'), (II-b), (II-b');

L represents a linker unit linking D to Ab;

q represents the number of linker bases linked to Ab, e.g., an integer or non-integer for q=1 to 20, e.g., 1-10, 1-8, 3-8, 4-8, or 6-8.

In the second aspect, the present invention provides pharmaceutical compositions comprising the ADC of the present invention or a pharmaceutically acceptable salt or solvate thereof, optional at least one other therapeutic agent, and optional one or more pharmaceutically acceptable excipients.

In the third aspect, the present invention provides the use of the ADC or a pharmaceutically acceptable salt or solvate thereof or a pharmaceutical composition comprising the same for the prevention or treatment of diseases associated with or mediated by STING, more specifically for the treatment or prevention of inflammatory, allergic or autoimmune diseases, infectious diseases or hyperproliferative diseases, especially tumors or viral infections.

In the fourth aspect, the present invention provides ADCs or pharmaceutically acceptable salts or solvates thereof or pharmaceutical compositions comprising the same for use as therapeutic agents for the treatment or prevention of diseases associated with or mediated by STING, more specifically for the treatment or prevention of inflammatory, allergic or autoimmune diseases, infectious diseases or hyperproliferative diseases, in particular as antitumor or antiviral therapeutic agents, or as vaccine adjuvants; specifically, as cytotoxic agents for the treatment or prevention of hyperproliferative diseases, especially tumors, or as cytotoxic agents for the treatment or prevention of viral infections.

In the fifth aspect, the present invention provides ADCs or pharmaceutically acceptable salts or solvates thereof or a pharmaceutical composition comprising the same, which are used as multifunctional active agents, having both immunotherapeutic and cytotoxic therapeutic activities, including the ability to activate the immune system to exert the functions of antitumor and antiviral replication by activating the STING signaling pathway, cause tumor cell death or prevent viral replication by releasing cytotoxic agents, then continuously activate STING by releasing tumor DNA to kill tumor cells, and produce antibody-antigen responses by releasing tumor neoantigens to provide an "immunological memory" or long-lasting immunity to tumors. In this aspect, the present invention also provides the use of the ADC of the present invention or of a pharmaceutically acceptable salt or solvate thereof or a pharmaceutical composition comprising the same for achieving the various functions mentioned above.

In the sixth aspect, the present invention provides methods for treating or preventing diseases associated with or mediated by STING, more specifically inflammation, allergic or autoimmune diseases, infectious diseases or hyperproliferative diseases, especially tumors or viral infections, in subjects, including the application of the ADC of the present

invention or a pharmaceutically acceptable salt or solvate thereof or a pharmaceutical composition comprising the same to humans or animals; specifically, the present invention provides methods for treating or preventing hyperproliferative diseases, especially tumors, in subjects, or methods for treating or preventing viral infections in subjects, which include the application of the ADC of the present invention or a pharmaceutically acceptable salt or solvate thereof or a pharmaceutical composition comprising the same to humans or animals.

In the seventh aspect, the present invention provides the use of the ADC or a pharmaceutically acceptable salt or solvate thereof or a pharmaceutical composition comprising the same in the preparation of a medicament for the prevention or treatment of diseases associated with or mediated by STING, more specifically inflammatory, allergic or autoimmune diseases, infectious diseases or hyperproliferative diseases, especially tumors or viral infections, or in the preparation of vaccine adjuvants; specifically, the present invention provides the use of the ADC or a pharmaceutically acceptable salt or solvate thereof or a pharmaceutical composition comprising the same in the preparation of a medicament for the treatment or prevention of hyperproliferative diseases, especially tumors, and the use of the ADC or a pharmaceutically acceptable salt or solvate thereof or a pharmaceutical composition comprising the same in the preparation of a medicament for the treatment or prevention of viral infections.

In the eighth aspect, the present invention provides a pharmaceutical combination comprising the ADC of the present invention or a pharmaceutically acceptable salt or solvate thereof, and at least one other therapeutic agent; a combination intended for the prevention or treatment of STING related or mediated diseases, more specifically for the treatment or prevention of inflammatory, allergic or autoimmune diseases, infectious diseases or hyperproliferative diseases, especially tumors or viral infections; and a method for the treatment or prevention in subjects of STING related or mediated diseases, more specifically inflammation, allergic or autoimmune diseases, infectious diseases or hyperproliferative diseases, especially tumors or viral infections, which includes the application of the pharmaceutical combination of the present invention to humans or animals.

In the ninth aspect, the present invention also provides a method for preparing the ADC of the present invention or a pharmaceutically acceptable salt or solvate thereof by conjugating the CDNs (e.g. compounds of Formula (II), (II-a), (II-a'), (II-b), (II-b') as defined herein, via a linker unit to an antibody or antigen-binding fragment thereof. The CDNs defined herein can be conjugated to an antibody or antigen-binding fragment thereof via the cleavable or non-cleavable linker unit. In specific embodiments, CDNs are released into tumor cells, cancer-associated immune cells, or the tumor microenvironment upon cleavage of the linker unit.

The present invention is further described in the following drawings and specific embodiments. However, such drawings and specific embodiments shall not be deemed to limit the scope of the present invention, and alterations easily conceived by technicians in the field shall be covered by the spirit of the present invention and the protection of the appended claims.

## Description of the Drawings

FIG. 1A-1C show the tumor growth inhibitory activity of a representative CDN fragment of the present invention in a mouse xenograft CT26 colon cancer model. 1A: tumor volume change in treated tumors; 1B: tumor volume change in untreated tumors; 1C: body weight change in mice.

FIG. 2 shows the antitumor activity (by tumor volume) of representative ADC compounds (ADC-1 and ADC-2) of the present invention in the SK-OV-3 model.

FIG. 3 shows the antitumor safety (by weight change) of representative ADC compounds (ADC-1 and ADC-2) of the present invention, in the SK-OV-3 model.

FIG. 4 shows the antitumor activity (by tumor volume) of the representative ADC compounds (ADC-2, ADC-3, ADC-4, ADC-6, ADC-10, ADC-11, ADC-12, ADC-13) of the present invention in the SK-OV-3 model.

FIG. 5 shows the antitumor safety (by weight change) of representative ADC compounds (ADC-2, ADC-3, ADC-4, ADC-6, ADC-10, ADC-11, ADC-12, ADC-13) of the present invention in the SK-OV-3 model.

## Detailed Description of the Invention

### Definition

Unless otherwise defined, all technical and scientific terms used herein have the same meaning as those

generally understood by the general technicians in the field. For the purposes of the present invention, the following terms are defined below.

Where trade names are used herein, they include the product formulation, generic name of drug, and active pharmaceutical ingredient of the trade name product unless the context indicates otherwise.

In this context, the term "about" when used in combination with numeric values means to cover numeric values within a range having a lower bound that is 10% smaller, e.g. 5%, and an upper bound that is 10% larger, e.g. 5%, than a numeric value specified. For example, float ±5%, ±2% or ±1%. For example, as used herein, the statement " about 100" includes 90 and 110 and all values between them (e.g., 90.5, 95, 101, 105, 109.95 ..., etc.). For proportions, the term "about." is used to limit each number of proportions given, e.g., the ratio "about 1:1" refers to the ratio (0.9-1.1): (0.9-1.1), Another example is "about n-m" or "about n-about m" which ranges from 90% n-110% n to 90% m-110% m.

In this context, the term "essentially" refers to the vast majority, i.e., > 50% of the population, mixture or sample, preferably greater than 50%, 55%, 60%, 65%, 70%, 75%, 80%, 85%, 90%, 91%, 92%, 93%, 94%, 95%, 96%, 97%, 98%, or 99%.

In this context, the term "and/or" should be understood to mean any one of the options or any combination of two or more of the options.

In this context, the terms "contain", "contained" and "containing" ("comprise", "comprised" and "comprising") or "include", "included" and "including" mean including the element, integer or step as described, but not excluding any other element, integer or step. In this context, when the terms "contain", "contained" and "containing" ("comprise", "comprised" and "comprising") or "include", "included" and "including" are used, the circumstances comprising the element, integer or step described are covered unless otherwise specified. For example, when referring to an antibody variable region that "contain", "contained" and "containing" ("comprise", "comprised" and "comprising") a specific sequence, it is also intended to cover the antibody variable region that consists of that specific sequence.

In this context, the term "immune system" has the usual meaning as understood by technicians in the field and means the molecules, substances (such as body fluids), anatomical structures (such as cells, tissues or organs) and the whole or any one or more components of a physiological process that are involved in preventing infection in the body, protecting the body during infection or disease, and/or helping the body recover after infection or disease.

In this context, the term "disease associated with or mediated by an immune response" means a disease associated with or mediated by a defense response of the body’s immune system to a foreign component or a variant autologous component. For the present invention, "disease associated with or mediated by an immune response" refers specifically to the state of a person or animal in which the function of the immune system is weakened, inactivated, or otherwise compromised, or the function of one or more immune components is weakened, inactivated, or otherwise compromised or the disease state caused by that state, and especially to a disease in which the induction of an immune response by the STING pathway can be relieved.

In this context, the term "ADC" refers to antibody-drug conjugates.

In this context, the term "drug" refers to one or more substances administered to humans or animals to obtain a therapeutic effect, including substances that prevent, cure, or mitigate the effects of disease and improve health conditions. In this disclosure, "drugs" especially cover substances that have beneficial preventive or therapeutic effects on the diseases associated with or mediated by STING.

In this context, the term "STING" is an abbreviation for stimulator of interferon genes. STING is a transmembrane protein receptor in humans, and activation of STING by cyclic dinucleotides (CDNs) leads to activation of the IRF3 and NF-κB pathways, thus leading to induction of type I interferons and proinflammatory cytokines, respectively. The term "STING agonist" refers to any substance that activates STING in vitro or in vivo to elicit a physiological response.

In this context, the term "disease associated with or mediated by STING" refers to a disease in which induction of an immune response via the STING pathway can be relieved, that is, activation of STING will reduce the incidence of the disease and reduce or eliminate disease symptoms, including but not limited to inflammatory, allergic, or autoimmune diseases, infectious diseases, or cancer. For the presentinvention, "diseases associated with or mediated by STING" preferably select from tumors or cancers.

In this context, the terms "hyperproliferative disease", "tumor", or "cancer" refer to physiological conditions in subjects characterized by uncontrolled or dysregulated cell growth or death, including solid and hematogenous tumors, whether malignant or benign, including, but not limited to, brain cancer, skin cancer, bladder cancer, ovarian cancer, breast cancer, gastric cancer, pancreatic cancer, prostate cancer, colon cancer, blood cancer, lung cancer, and bone cancer, for example neuroblastoma, intestinal cancer such as rectal cancer, colon cancer, familial adenomatous polyposis carcinoma, and hereditary nonlymphocytic colorectal cancer, esophageal cancer, lip cancer, laryngeal cancer, nasopharyngeal carcinoma, oral cancer, salivary gland cancer, peritoneal cancer, soft tissue sarcoma, urothelial carcinoma, hidradenocarcinoma, gastric cancer, adenocarcinoma, medullary thyroid cancer, papillary thyroid cancer, renal carcinoma, renal parenchymal carcinoma, ovarian cancer, cervical cancer, uterine corpus cancer, endometrial cancer, pancreatic cancer, prostate cancer, testicular cancer, breast cancer (including HER2-negative breast cancer), urinary cancer, melanoma, brain tumors such as glioblastoma, astrocytoma, meningioma, medulloepithelioma, and peripheral

neuroectodermal tumor, Hodgkin lymphoma, non-Hodgkin lymphoma, Burkitt's lymphoma, acute lymphoblastic leukemia (ALL), chronic lymphocytic leukemia (CLL), chronic myelogenous leukemia (CLL), and lymphocytic carcinoma, acute myelogenous leukemia (AML), myelogenous leukemia (chronic myelogenous leukemia (CML), adult T-cell lymphoma, diffuse lymphoma (DLBCL), hepatoma, multiple myeloma, seminoma, osteosarcoma, chondrosarcoma, anal canal cancer, adrenocortical carcinoma, chordoma, fallopian tube cancer, gastrointestinal stromal tumor, myeloproliferative disorder, mesothelioma, biliary tract cancer, Ewing's sarcoma, and other rare tumor types.

In this context, the term "antitumor effect" refers to biological effects that can be demonstrated by a number of means, including, but not limited to, e.g., decreased tumor volume, decreased tumor cell number, decreased tumor cell proliferation, or decreased tumor cell survival.

In this context, the term "cytotoxic activity" refers to the cell killing effect of a drug or conjugate or its metabolites. Cytotoxic activity can be expressed as an IC50 value, which is the concentration per unit volume (molar or mass) at which half of the cells survive.

In this context, the term "cytotoxic agent" or equivalent refers to an active agent having cytotoxic activity and causing cell destruction and used to combat abnormal and uncontrolled development and growth of cells. The cytotoxic agents involved in the antibody-drug conjugate in this disclosure are bifunctional CDN STING agonists as defined herein and also include substances released in vivo with cytotoxic activity.

The term "multifunctional active agent" used herein refers to the portion of the drug carried in the conjugate of the present invention that, based on its unique structural design, is capable of multiple functions in subjects, combining immunotherapeutic activity and cytotoxic therapeutic activity, including, but not limited to, the ability to activate the immune system to exert antitumor and antiviral replication by activating the STING signaling pathway, to cause tumor cell death or prevent viral replication by releasing cytotoxic agents, to continuously activate STING by releasing tumor DNA to kill tumor cells, and to generate antibody-antigen responses by releasing tumor neoantigens to provide "immunological memory" or long-lasting immunity to tumors.

In this context, the term "antigen" refers to entities that specifically bind to an antibody.

In this context, the term "antibody" refers to peptides containing at least the variable regions of light or heavy immunoglobulin that specifically recognize and bind antigen. The term covers a variety of antibody structures including, but not limited to, monoclonal antibodies, polyclonal antibodies, single or multiple chain antibodies, monospecific or polyspecific antibodies (eg, bispecific antibodies), chimeric or humanized antibodies, full-length antibodies, and antibody fragments as long as they present the desired antigen-binding activity. Antibodies can be of any class (e.g., IgG, IgE, IgM, IgD, and IgA), type (e.g., IgG1, IgG2, IgG3, IgG4, IgA1, and IgA2), or subtype.

In this context, the terms "antibody fragment" and "antigen-binding fragment" are used interchangeably and refer to molecules that are not intact antibodies and that contain the portion of the intact antibody that binds the antigen to which that intact antibody binds. As technicians in the field understand, antibody fragments often contain amino acid residues from "complementarity determining regions" or "CDRs" for antigen binding purposes. Antibody fragments can be prepared by recombinant DNA technology, or by enzymatic or chemical cleavage of intact antibodies. Antigen-binding fragments include, but are not limited to, Fab, scFab, Fab', F(ab')2, Fab'-SH, Fv, single-chain Fv, double-chain antibody (diabody), triple-chain antibody (triabody), four-chain antibody (tetrabody), microantibody (minibody), single-domain antibody (sdAb); and polyspecific antibodies formed from antibody fragments. The Fab fragment is a monovalent fragment composed of VL, VH, CL, and CH1 domains, e.g., the Fab fragment can be obtained by complete antibody digestion with papain. The light chain (L chain) and heavy chain (H chain) of the Fab can be fused into a single polypeptide chain, the single chain Fab (scFab), by means of a linker (see e.g., US20070274985A1). In addition, complete antibody digestion by pepsin under the disulfide bond in the hinge region can produce F(ab')2, which is a dimer of Fab' and a bivalent antibody fragment. F(ab')2 can be reduced under neutral conditions by disrupting disulfide bonds in the hinge region and converted from F(ab')2 dimers to Fab' monomers. The Fab' monomer is essentially a Fab fragment with a hinge region. The Fv fragment consists of the VL and VH domains of a single arm of the antibody. In addition, the genes encoding VL and VH of two domains independently encoding Fv fragments can be linked together by nucleic acid sequences encoding linker peptides (linkerss) using recombination methods to recombine and express to form single-stranded Fv, where the VH and VL regions pair to provide antigen binding sites in this single protein chain. A double-chain antibody is an antibody fragment with two antigen-binding sites that contains VL and VH linked by a short linker in the same polypeptide chain. In a double-chain antibody, because the linker is too short, the two domains VH and VL on the same chain cannot pair, but are forced to pair with the complementary domain on the other chain and produce two antigen-binding sites. Double-chain antibodies can be bivalent or bispecific. More detailed descriptions of double-chain antibodies can be found, for example, EP 404,097; WO 1993/01161; Hudson et al., Nat.Med.9:129-134(2003); and Hollinger et al., PNAS USA 90:6444-6448(1993). Triplex and quadruplex antibodies and microantibodies are also described in Hudson et al, Nat.Med.9:129-134(2003), and Shao Rongguang et al. (editors), Antibody Drug Research and Application, People's Medical Publishing House (2013). Single-domain antibodies (sdAb) generally refer to antibodies in which a single variable domain (e.g., heavy chain variable domain (VH) or light chain variable domain (VL), heavy chain variable domain derived from camelid heavy chain antibodies, VH like single domain derived from fish IgNAR (v-NAR) confers antigen binding

without the need to interact with another variable domain to recognize the target antigen. (For a more detailed description of antibody fragments, see also: Fundamental Immunology, W. E. Paul editor, Raven Press, N.Y. (1993).

In this context, the term "immunoglobulin" refers to proteins with structures of naturally occurring antibodies. For example, IgG class immunoglobulins are heterotetrameric proteins of about 150,000 daltons composed of two light chains and two heavy chains bonded by disulfide bonds. Each immunoglobulin heavy chain has a heavy chain variable region (VH), also called the heavy chain variable domain, followed by three heavy chain constant domains (CH1, CH2, and CH3) from the N-to the C-terminus. Each immunoglobulin light chain has a variable light chain region (VL), also called the variable light chain domain, followed by a constant light chain domain (CL), from the N-terminus to the C-terminus. Accordingly, in this context, the mention of an antibody is IgG antibody, which refers to a heterotetrameric protein with IgG class immunoglobulin structure. In IgG antibodies, usually heavy chain VH-CH1 pairs with light chain VL-CL to form Fab fragments that specifically bind antigen. Thus, an IgG antibody essentially consists of two Fab molecules linked with the help of the immunoglobulin hinge region and two dimerized Fc regions. IgG immunoglobulins can be divided into subclasses based on the sequence of the constant region of the heavy chain, such as $\gamma$1(IgG1), $\gamma$2(IgG2), $\gamma$3(IgG3), and $\gamma$4(IgG4). The light chains of IgG immunoglobulins can also be divided into one of two types based on the amino acid sequence of their constant domain, called $\kappa$ and $\lambda$. In some embodiments, IgG antibodies are for example IgG1, IgG2, IgG3 or IgG4 antibodies. In other embodiments, IgG antibodies are IgG$\kappa$ or IgG$\lambda$ antibodies, for example, IgG1$\kappa$ or IgG1$\lambda$ antibodies.

In this context, the terms "complementarity determining region" or "CDR region" or "CDR" or "hypervariable region" are used interchangeably and refer to regions in the variable domain of an antibody that are highly variable in sequence and form loops identified on the structure ("supervariable loops") and/or contain antigen contact residues ("antigen contact points"). CDRs are primarily responsible for binding to antigenic epitopes. In this context, CDRs of antibody heavy and light chains are numbered sequentially starting at the N-terminus and are commonly called CDR1, CDR2, and CDR3. CDRs located in the variable domain of the antibody heavy chain are also called HCDR1, HCDR2, and HCDR3, while CDRs located in the variable domain of the antibody light chain are called LCDR1, LCDR2, and LCDR3. Within a given amino acid sequence of the variable region of the light chain or variable region of the heavy chain, the CDR sequence can be determined using a variety of protocols well-known in the field. For example, in http://www.abysis.org/abysis/, it is possible to obtain an annotation of CDRs in a given light chain variable region or heavy chain variable region, including CDR sequences defined based on Kabat, AbM, Chothia, Contact, IMGT. In addition, CDRs can also be identified based on having the same Kabat numbering position as the reference CDR sequence. Unless otherwise stated, in this invention, when referring to residue positions in the variable region of an antibody, including heavy chain variable region residues and light chain variable region residues, it refers to numbering positions according to the Kabat numbering system (Kabat et al., Sequences of Proteins of Immunological Interest, 5th Ed. Public Health Service, National Institutes of Health, Bethesda, Md. (1991)).

In this context, the "variable region" or "variable domain" is the domain of the heavy or light chain of an antibody involved in the binding of the antibody to its antigen. Heavy chain variable regions (VH) and light chain variable regions (VL) can be further divided into hypervariable regions (HVR, also known as complementarity determining regions (CDRs)), which are interspersed with more conserved regions (i.e., framework regions (FR)). Each VH and VL consists of three CDRs and four FRs in the following order from the amino to the carboxyl terminus: FR1, CDR1, FR2, CDR2, FR3, CDR3, FR4. In some aspects, one or more residues in one or both of the two variable regions (i.e. VH and/or VL) can be modified, e.g., one or more CDR regions and/or one or more framework regions can be modified, especially conservative residue substitutions, to obtain antibody variants that still essentially maintain at least one of the biological properties (e.g., antigen-binding capacity) of the previous antibody molecule. In other ways, antibody variable regions can be modified by CDR transplantation. Because the CDR sequence is responsible for most antibody-antigen interactions, recombinant antibody variants mimicking the properties of known antibodies can be constructed. In this antibody variant, CDR sequences from known antibodies are grafted onto the framework regions of different antibodies of different properties. The properties of mutated and/or modified antibodies or ADC conjugates containing them, such as target antigen binding properties or other desired functional properties, such as endocytosis, pharmacokinetics, and in vivo tumor killing activity of ADCs can be assessed in in vitro or in vivo assays.

In this context, the term "isotype" refers to the antibody type determined by the constant region of the antibody heavy chain. For example, the antibody portion of an ADC according to the present invention may be IgA (e.g., IgA1 or IgA2), IgG1, IgG2 (e.g., IgG2a or IgG2b), IgG3, IgG4, IgE, IgM, and IgD antibodies and have a heavy chain constant region of the immunoglobulin type described. In addition, the present invention considers not only antibodies employing constant regions of natural sequences but also antibodies containing constant regions of variant sequences.

In this context, the term "natural sequence Fc region" covers naturally occurring sequences of various immunoglobulin Fc regions, such as those of various Ig subtypes as well as their allotypes (Gestur Vidarsson et al., IgG subclasses and allotypes: from structure to effector functions, 20 October 2014, doi: 10.3389/fimmu.2014.00520.). In some embodiments, the Fc region of the heavy chain of human IgG has an amino acid sequence extending from Cys226 or Pro230 to the carboxyl terminus of the heavy chain. However, C-end lysine (Lys447) in the Fc region can be present or

absent. In additional embodiments, the Fc region of the heavy chain of human IgG carries a hinge sequence or part of the hinge sequence of innate immunoglobulin at the N-terminus, such as the sequence E216 to T225 or the sequence D221 to T225 according to EU numbers.

In this context, the term "variant sequence Fc region" refers to peptides containing modified Fc region relative to natural sequence Fc region peptides. The modifications described can be addition, deletion or substitution of amino acid residues. Substitutions can include naturally occurring amino acids and non-naturally occurring amino acids. Modifications can be aimed at altering the binding of the Fc region to its receptor and its resulting effector function, optimizing half-life, and/or introducing coupling sites required for coupling chemistry.

In this context, the term "epitope" includes any protein determinant that can specifically bind to immunoglobulins or otherwise interact with molecules. Epitope determinants typically consist of chemically active surface groups of molecules, such as amino acids or carbohydrates or sugar side chains, and can have specific three-dimensional structural features as well as specific charge profiles. Epitopes can be 'linear' or 'conformational'. Conformational and linear epitopes differ by loss of binding to the former but not the latter in the presence of denaturing solvent.

In this context, the term "receptor-mediated endocytosis" refers to the process whereby ligand/receptor complexes are internalized and delivered into the cytosol or transferred to the appropriate intracellular compartment, triggered by ligand binding to the corresponding receptor on the cell surface. The receptor-mediated endocytosis activity of antibodies can be characterized by measuring the endocytosis rate.

In this context, "sequence identity" refers to the degree to which sequences based on nucleotide by nucleotide or amino acid by amino acid are identical in the comparison window. The "percent sequence identity" can be calculated by comparing the two best aligned sequences in a comparison window and determining the number of positions in the two sequences that have the same nucleic acid base (e.g., A, T, C, G, I) or the same amino acid residue (e.g., Ala, Pro, Ser, Thr, Gly, Val, Leu, Ile, Phe, Tyr, Trp, Lys, Arg, His, Asp, Glu, Asn, Gln, Cys, and Met) to obtain the number of matching positions, dividing the number of matching positions by the total number of positions in the comparison window (i.e., window size), and multiplying the result by 100 to yield the percent sequence identity. Optimal alignment to determine percent sequence identity can be achieved in a variety of ways known in the field, for example, using publicly available computer software such as BLAST, BLAST-2, ALIGN, or Megalign (DNASTAR) software. Technicians in this field can identify suitable parameters for aligning sequences, including any algorithms required to achieve maximum alignment within the full length sequence range or target sequence region being compared.

In the present invention, as far as an antibody sequence is concerned, the percent amino acid sequence identity is determined by optimal alignment of a candidate antibody sequence to a given antibody sequence, followed by optimal alignment according to the Kabat numbering rule in a preferred scheme. In this context, without specifying the comparison window (i.e., the target antibody region to be compared), it will be suitable for alignment over the full length of a given antibody sequence. In some embodiments, in terms of antibody, sequence identity may be spread over the entire heavy chain variable region and/or over the entire light chain variable region, or sequence percent identity may be restricted to the framework region only, while sequences corresponding to the CDR region remain 100% identical.

In this context, the term "isotype" refers to the antibody type determined by the constant region of the antibody heavy chain. For example, an antibody according to the present invention may be IgA (e.g., IgA1 or IgA2), IgG1, IgG2 (e.g., IgG2a or IgG2b), IgG3, IgG4, IgE, IgM, and IgD antibodies and have a heavy chain constant region of the immunoglobulin type described. For example, the antibody of the present invention may be an IgG1 antibody with a constant region of human IgG1. In addition, the present invention considers not only antibodies employing constant regions of natural sequences but also antibodies containing constant regions of variant sequences.

In this context, the term "whole antibody" (used interchangeably with "full-length antibody", "complete antibody", and "intact antibody" in this context) refers to immunoglobulin molecules containing at least two heavy chains (H) and two light chains (L). Each heavy chain consists of a variable region (abbreviated as VH in this context) and a constant region of the heavy chain. Each light chain consists of a variable light chain region (abbreviated as VL in this context) and a constant light chain region. Variable regions are domains in the heavy or light chains of an antibody that are involved in the binding of the antibody to its antigen. The constant region is not directly involved in antibody binding to antigen but shows multiple effector functions. The light chain of an antibody can be assigned to one of two types, called kappa (κ) and lambda (λ), based on the amino acid sequence of its constant region. The heavy chains of antibodies can be divided into five major classes based on the amino acid sequence of their constant regions: IgA, IgD, IgE, IgG, and IgM, and several of these classes can be further divided into subclasses, such as IgG1, IgG2, IgG3, and IgG4, IgA1, and IgA2.

In this context, the term "chimeric antibody" refers to an antibody in which the variable region sequence originates from one species and the constant region sequence originates from another species, e.g., where the variable region sequence originates from a mouse antibody and the constant region sequence originates from a human antibody.

In this context, the term "monoclonal antibody" or "monoclonal antibody composition" refers to peptides, including antibodies, bispecific antibodies, etc., which have essentially the same amino acid sequence or are derived from the same genetic origin. The term also includes antibody molecular products of a single molecular composition. Monoclonal antibody compositions exhibit a single binding specificity and affinity for a specific phenotype.

In this context, the term "humanized antibody" refers to antibodies that graft CDR sequences derived from other mammalian species such as mouse germline onto human frameworke sequences. Additional modifications to the framework region within the human framework sequence, and/or additional amino acid modifications in the CDR sequence, such as for affinity maturation of the antibody, may be performed. In this context, in some embodiments, the humanized antibody of the present invention has a sequence of the framework region "derived" from a particular germline sequence. "Derived" herein means that the amino acid sequence of the framework region of the antibody has at least 90%, preferably at least 95%, or even preferably at least 96%, 97%, 98%, or 99% identity to the amino acid sequence of the corresponding framework region encoded by the immunoglobulin gene of the human germline and that the antibody retains antigen-binding activity.

In this context, the term "human antibody" includes antibodies with variable regions, where both the framework and CDR regions are derived from human sequences. In addition, if an antibody contains a constant region, the constant region is also derived from such a human sequence, e.g., a human germline sequence or a mutated form of a human germline sequence or an antibody containing a consensus framework sequence derived from the analysis of the human framework sequence, as described by Knappik et al. (2000. J Mol Biol 296, 57-86). A well-known numbering scheme can be used to define the structure and location of immunoglobulin variable domains such as CDRs, such as the Kabat numbering scheme, the Chothia numbering scheme, or a combination of Kabat and Chothia. The human antibody of the present invention may include amino acid residues not encoded by the human sequence (e.g., mutations introduced by random or site-specific mutagenesis in vitro or somatic mutation or conservative substitution in vivo to promote stability or manufacture). However, the term "human antibody" as used herein is not intended to include such an antibody, where CDR sequences derived from a germline of another mammalian species such as mouse have been grafted onto the human framework sequence.

In this context, the term "recombinant human antibody" includes all human antibodies prepared, expressed, produced or isolated by recombinant means, such as antibodies isolated from animals (e.g., mice) that are transgenic or chromosome-transferred for the human immunoglobulin gene, hybridomas prepared by them, host cells that express the human antibody from transformation, such as antibodies isolated from transfectoma, antibodies isolated from recombinant combined human antibody libraries, and antibodies prepared, expressed, produced or isolated by any other means, any other means involving splicing of all or part of the human immunoglobulin gene and sequence to other DNA sequences. Such recombinant human antibodies have variable regions in which the framework and CDR region are derived from the human germline immunoglobulin sequence. However, in some embodiments, such recombinant human antibodies can be subjected to in vitro mutagenesis (or, in vivo somatic mutagenesis when animals transgenic for the human Ig sequence are used), and therefore, it is possible that the regions of the amino acid sequences in the VH and VL regions of the recombinant antibodies are not naturally present in the human antibody germline library in vivo, but are derived from or associated with the VH and VL sequences of the human race.

In this context, the term "isolated" antibody is an antibody that has been isolated from components in its natural environment. In some embodiments, antibodies are purified to greater than 90%, 95%, or 99% purity, which can be determined by e.g., electrophoresis (e.g., SDS-PAGE, isoelectric focusing (IEF), capillary electrophoresis) or chromatography (e.g., ion-exchange or reversed-phase HPLC).

In this context, the term "affinity" or "binding affinity" refers to the inherent binding affinity that reflects the interaction between members of the bound pair, e.g. the strength of the interaction between an antibody and an antigen at a single antigenic site, and the stronger the interaction, the stronger the affinity. The affinity of a molecule for its partner can generally be represented by the equilibrium dissociation constant ($K_D$), which is the ratio of the dissociation rate constant and the association rate constant ($k_{dis}$ and $k_{on}$, respectively). Affinity can be measured by common methods known in the field, such as antigen-based protein or cell-based ELISA assays, flow cytometry assays, biological film-based layer optical interference (BLI) techniques, etc.

In this context, the term "$K_D$"(M) refers in this context to the dissociation equilibrium constant for a particular antibody-antigen interaction. Affinity is inversely correlated with $K_D$ values, i.e., the higher the affinity, the smaller the $K_D$ value; conversely, the larger the affinity, the higher the $K_D$ value. Generally, $K_D$ values depend on the dissociation rate constant (Kd or Kdis, $sec^{-1}$) and association rate constant (Ka, $M^{-1}$ x $sec^{-1}$) between interacting antibody-antigen pairs.

In this context, the term "binding" or "specific binding" refers to the ability of a single antibody binding site to react with one antigenic determinant but not with different antigenic determinants. In the context of antibody binding to an associated antigen, it refers to binding with an affinity of approximately $10^{-6}$ M or less for $K_D$ values, e.g., $K_D$ values of approximately $10^{-7}$ M or less, or approximately $10^{-8}$ M or less. Binding $K_D$ values for antibodies to their associated antigens are preferably at least 100-fold lower or at least 1000-fold lower compared to binding $K_D$ values for nonspecific antigens (e.g., unrelated antigens such as BSA). The measurement of $K_D$ values is known in the field, for example based on biological film-based layer optical interference (BLI) technology using antibodies as ligands and antigens as analytes in, for example, ForteBio Octet ® instruments.

In this context, the term "effector function" refers to those biological activities that can be attributed to the Fc-region of an antibody, which change depending on the antibody class. The Fc region of IgG has been known to mediate

several important effector functions such as cytokine induction, ADCC, phagocytosis, complement-dependent cytotoxicity (CDC), and half-life/clearance rates of antibodies and antigen-antibody complexes. In some cases, depending on the therapeutic purpose, these effector functions are ideal for therapeutic antibodies, but may be unnecessary in other cases. Thus, in one embodiment, the present invention provides antibodies with Fc regions that elicit effector functions such as ADCC or CDC, thereby inducing apoptosis, cell lysis, and/or inhibiting proliferation, dissemination, and/or metastasis of tumor cells bearing TF antigens in tumor cells bearing TF antigens. In other embodiments, the present invention also provides antibodies to Fc regions with altered effector functions. Effector function can be altered by sequence changes to the Fc region of the antibody. Alternatively, antibodies with altered types of glycosylation in the Fc region can be prepared. Alterations in glycosylation patterns in the Fc region can be conveniently achieved by altering the amino acid sequence of the Fc region in order to create or remove one or more glycosylation sites.

In this context, the term "HER2" (also called ERBB2; NEU; NGL; TKR1; CD340; p185; MLN19; HER-2/neu) refers to transmembrane tyrosine kinase receptors of the epidermal growth factor (EGF) receptor family. HER2 contains an extracellular binding domain, a transmembrane domain, and an intracellular tyrosine kinase domain. HER2 does not possess its own ligand-binding domain and therefore cannot bind growth factors, however, HER2 tightly binds to other ligand-bound EGF receptor family members such as HER1 or HER3 to form heterodimers, thereby stabilizing ligand binding and enhancing kinase-mediated activation of downstream signaling pathways. The human HER2/NEU gene is localized at chromosome position 17q12, and the genome sequence of the HER2/NEU gene can be found in NG_007503.1 in GenBank. In humans, there are five HER2 isotypes: A, B, C, D, and E; the term "HER2" is used throughout this text to collectively represent all HER2 isotypes. As used herein, the human HER2 protein also includes proteins that share at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with HER2 isotypes A, B, C, D, and E over their entire length, where such proteins still have at least one function of HER2.

In this context, the term "HER2-positive cancer" or "HER2-expressing cancer" is cancer containing cells in which the HER2 protein is present on their cell surface. Many methods are known in this field for detecting or determining the presence of HER2 on cancer cells. For example, in some embodiments, the presence of HER2 on the cell surface can be determined by immunohistochemistry (IHC), flow cytometry, Western blotting, immunofluorescence assay, radioimmunoassay (RIA), enzyme-linked immunosorbent assay (ELISA), homogeneous time-resolved fluorescence (HTRF), or positron emission tomography (PET).

As used herein, the term "induction" refers to an increase or enhancement of function, such as an increase or enhancement of STING expression. In some embodiments, induction of STING expression refers to induction of transcription of STING RNA (mRNA, e.g., increased or enhanced, or translation of STING protein (e.g., increased or enhanced). In some embodiments, induction of STING expression refers to increasing or enhancing concentrations of STING RNA (e.g., mRNA) or STING protein such as in cells. In some embodiments, induction of STING expression refers to increasing the copy number of STING RNA (e.g., mRNA) or STIING protein (e.g., cell). In some embodiments, induction of STING expression can refer to triggering STING RNA (e.g., mRNA) or transcription or STING protein translation. In some embodiments, induction of STING expression can refer to increasing the rate of transcription of STING RNA (e.g., mRNA) or increasing the rate of expression of STING protein.

In this context, the term "linker unit" or "linker" refers to the bi-functional part that links the drug to the antibody in the antibody-drug conjugate. The linker unit of the present invention has multiple components, such as an autolysis linker; a cleavable linker; an optional property regulating unit; and an antibody connector.

In this context, the term "autolysis linker" refers to a temporary extender, spacer, or placeholder unit that joins two or more molecules together by a chemical bond that breaks to release both molecules under defined conditions. Typically, the autolysis linker units can be straight or branched, and two or more identical molecules can be linked together or two or more distinct molecules can be linked together. An autolysis unit may be defined as a bifunctional chemical group capable of covalently joining two interspaced chemical moieties together to form a generally stable molecule that releases one of the interspaced chemical moieties from the molecule by enzymatic cleavage; and, following enzymatic cleavage, spontaneously cleaves the remaining portion of the bifunctional chemical group to release the other of the interspaced chemical moieties. In some examples, the self-immolative unit refers to the heterocyclic self-immolative part. Typical self-immolative linker units include His Ala, p-aminobenzyloxycarbonyl (PABC), p-hydroxybenzyloxycarbonyl, 2,4-bis (hydroxymethyl) aniline, $-NH-(CH_2)_4-C(O)-$ and $-NH-(CH_2)_3-C(O)-$, etc.

In this context, the term "cleavable linker" refers to the part of the linker unit of ADC that is unstable in vivo. Preferably, the "cleavable linker" allows activation of markers or therapeutic agents by cutting markers or reagents from the rest of the conjugate. Operationally defined, the linkers that can be cleaved and lysed are self-preferred to be cleavable by the biological environment in the body. Cleavage can come from any process without restriction, such as enzymatic, reductive, pH, etc. Preferably, cleavable groups are selected so that activation occurs at the desired site of action, which can be sites in or near target cells (e.g., cancer cells) or tissues, e.g., therapeutic effects or marker active sites. Such cleavage can be enzymatic, and exemplary digestion groups include natural amino acids or peptide sequences ending with natural amino acids and are linked to linker units or self-immolative linkers at their carboxyl terminal.

In this context, the term "antibody linker" refers to any chemical group designed to facilitate attachment of a drug conjugate to an antibody, for example to overcome stereoscopic hindrance.

In this context, the term "property regulatory unit" refers to the functional part linked in series or branched in the linker unit of ADC, which aims to modulate the properties of ADC, such as stability in blood circulation, improving hydrophilicity, etc. Commonly used ADC property regulating units include, but are not limited to, polyethylene glycol (PEG), hydrophilic peptide, cyclodextrin unit, polyamine, polyamide, polysaccharide, dendrimer and bifunctional hydrocarbon chain, etc.

In this context, the term "PEG unit" refers to an organic part containing repeated ethylene oxy subunits (PEG or PEG subunits), which can be polydisperse, monodisperse, or discrete (i.e., with a discrete number of ethylene oxy subunits). Polydisperse PEG is a heterogeneous mixture of size and molecular weight, whereas monodisperse PEG is typically purified from a heterogeneous mixture and therefore has a single chain length and molecular weight. The preferred PEG units contain discrete PEG, which are compounds synthesized in a stepwise manner rather than via a polymerization process. Discrete PEG provides a single molecule with limited and designated chain lengths.

The PEG units presented herein include one or more polyethylene glycol chains, each consisting of one or more enylidene ethoxy subunits covalently linked to each other. Polyethylene glycol chains can be linked together, for example, in linear, branched, or star configurations. Typically, at least one polyethylene glycol chain is derivatized at one end with an alkyl moiety substituted by an electrophilic group covalently linked to the carbamate nitrogen of the methylene carbamate unit prior to incorporation into the CDN conjugate. Typically, the terminal ethoxylidene groups in each polyethylene glycol chain that are not involved in covalent attachment to the rest of the linker unit are modified with PEG capping units, usually optional substituted alkyl groups such as $-CH_3$, $CH_2CH_3$, or $CH_2CH_2CO_2H$. The preferred PEG units have a single polyethylene glycol chain with tandemly covalently linked 2 to 24 $-CH_2CH_2O$-subunits.

In this context, the term "base" refers to nitrogen-containing biological compounds within nucleosides linked to sugars, i.e., the basic building units of deoxyribonucleic acid (DNA) and ribonucleic acid (RNA). Naturally occurring nucleobases are cytosine (DNA and RNA), guanine (DNA and RNA), adenine (DNA and RNA), thymine (DNA), and uracil (RNA), which are abbreviated C, G, A, T, and U, respectively.

In this context, the term "drug: antibody ratio" or "DAR" refers to the ratio of the drug portion (D) coupled to the Ab portion described herein to the Ab portion in ADC conjugates. In some of the embodiments described herein, the DAR may be determined by q in Formula I, e.g., the DAR may be an integer or non-integer of at least 1, such as about 1 to 20, such as about 2-18, 4-16, 5-12, 6-10, 2-8, 3-8, 2-6, 4-6, 6-10, such as 2, 3, 4, 5, 6, 7, 8, 9, 10, 11, 12, 13, 14, or 15. DAR may also be calculated as the average DAR of the molecular population in the product, i.e., the overall proportion of the small molecule drug fraction (D) coupled to the Ab portion described herein in the product to the Ab portion as determined by the test method (e.g., by conventional methods such as mass spectrometry, ELISA assay, electrophoresis, and/or HPLC), and this DAR is called the average DAR in the text. In some embodiments, the average DAR value of the drug conjugate of the present invention is about 1 to 20, e.g., about 2-18, 4-16, 5-12, 6-10, 2-8, 3-8, 2-6, 4-6, 6-10, e.g., 1.0-8.0, 2.0-6.0, e.g., about 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.5, 7.6, 7.7, 7.8.0, 7.9, 8, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, or 10.0, use two of these values as the range of endpoints.

In this context, the term "halogen" generally refers to fluorine, chlorine, bromine, iodine, for example, it can be fluorine, chlorine.

In this context, the term "alkyl" refers to straight or branched aliphatic saturated hydrocarbon groups having a specified number of carbon atoms. Specifically, the alkyl group can have 1 to 14, 1 to 12, 1 to 10, 1 to 8, 1 to 7, 1 to 6, 1 to 5, 1 to 4, 1 to 3, or 1 to 2 carbon atoms. Examples of suitable $C_{1-14}$ alkyl groups include, but are not limited to, methyl, ethyl, n-propyl, isopropyl, n-butyl, isobutyl, sec-butyl, tert-butyl, n-pentyl, isopentyl, neo-pentyl, n-hexyl, isohexyl, dimethyl methyl, dipropyl methyl, ethyl butyl methyl, diethyl methyl, methyl ethyl methyl, ethyl propyl methyl, diethyl ethyl, diethyl propyl, dipropyl ethyl, etc. Specific alkyl groups have 1 to 7 carbon atoms, e.g., 1 to 6 carbon atoms, 1 to 4 carbon atoms.

In this context, the term "alkenyl" refers to straight or branched unsaturated hydrocarbon groups consisting of carbon and hydrogen atoms containing at least one double bond. Specifically, the alkenyl group has 2-8, e.g., 2 to 6, 2 to 5, 2 to 4 or 2 to 3 carbon atoms. For example, as used herein, the term "$C_2$-$C_6$ alkenyl" refers to an alkenyl group with a straight or branched chain of two to six carbon atoms, e.g., ethenyl, propenyl, allyl, 1-butenyl, 2-butenyl, 1,3-butadienyl, 1-pentenyl, 2-pentenyl, 3-pentenyl, 1,3-pentadienyl, 1,4-pentadienyl, 1-hexenyl, 2-hexenyl, 3-hexenyl, 1,4-hexadienyl, etc.

In this context, the term "alkynyl" refers to straight or branched unsaturated hydrocarbon groups consisting of carbon and hydrogen atoms containing at least one triple bond. Specifically, the alkynyl group has 2-8, e.g., 2 to 6, 2 to 5, 2 to 4, or 2 to 3 carbon atoms. For example, as used herein, the term "$C_2$-$C_6$ alkynyl" refers to an alkynyl group with a straight or branched chain of two to six carbon atoms, e.g., ethynyl, propynyl, propargyl, 1-butynyl, 2-butynyl, 1-pentynyl, 2-pentynyl, 3-pentynyl, 4-methyl-1-pentynyl, 1-hexynyl, 2-hexynyl, 3-hexynyl, 5-methyl-2-hexynyl, etc.

In this context, the term " alkylene" refers to divalent groups obtained by removing two hydrogen atoms from the same or two different carbon atoms of a saturated alkane of a straight or branched chain. Specifically, the alkylene group

has 1-10 carbon atoms, e.g., 1 to 6, 1 to 5, 1 to 4, 1 to 3, or 1 to 2 carbon atoms. For example, as used herein, the term "$C_1$-$C_6$ alkylene " refers to an alkylene group with straight or branched chains of 1 to 6 carbon atoms, including, but not limited to, methylene, ethylidene, propylidene, butylidene, etc.

In this context, the term "alkenylene" refers to divalent groups resulting from the removal of two hydrogen atoms from the same or two different carbon atoms of a straight or branched, unsaturated olefin containing at least one double bond. Specifically, the alkenylene group has 2-8, e.g., 2 to 6, 2 to 5, 2 to 4, or 2 to 3 carbon atoms. For example, as used herein, the term "$C_2$-$C_6$ alkenylene" refers to an alkenylene group having a straight or branched chain of two to six carbon atoms, such as ethenylene, propenylene, allylene, butenylene, pentenylene, and hexenylene.

In this context, the term "alkynylene" refers to divalent groups resulting from the removal of two hydrogen atoms from the same or two different carbon atoms of a straight or branched, unsaturated alkynyl containing at least one triple bond. Specifically, the alkynyl group has 2-8, e.g., 2 to 6, 2 to 5, 2 to 4, or 2 to 3 carbon atoms. For example, as used herein, the term "$C_2$-$C_6$ alkynylene " refers to an alkynylene group having a straight or branched chain of two to six carbon atoms, e.g., ethynylene, propynylene, propargylene, butynylene, pentynylene, and hexynylene.

In this context, the term "cycloalkyl" refers to monocyclic, fused polycyclic, bridged polycyclic, or spiro non-aromatic monovalent hydrocarbon ring structures with a specified number of ring atoms, which can be saturated or unsaturated, e.g., containing one or more double bonds. The cycloalkyl group may contain three or more carbon atoms in the ring, e.g., 3-18, 3-10, or 3-8 carbon atoms, e.g., $C_{3-10}$ cycloalkyl, $C_{3-8}$ cycloalkyl, $C_{3-6}$ cycloalkyl, $C_{5-6}$ cycloalkyl. Examples of cycloalkyl groups include, but are not limited to, cyclopropyl, cyclobutyl, cyclopentyl, cyclohexyl, cycloheptyl.

In this context, the term "heterocyclic ring" or "heterocyclyl" refers to a 5-20 membered (e.g., 5-14 membered, 5-8 membered, 5-6 membered) aromatic or non-aromatic monocyclic, bicyclic, or polycyclic ring system with 1 to 4 independently selected members of the heteroatomic ring of N, O, or S. One or more N, C, or S atoms in the heterocyclic ring can be oxidized. Preferably, the heterocyclic ring is a 5-10 membered ring system, which is a single ring or a fused double ring. Representative examples include, but are not limited to, pyrrolidine, azetidine, piperidine, morpholine, tetrahydrofuran, tetrahydropyran, benzofuran, benzothiophene, indole, benzopyrazole, pyrrole, thiophene (thiofuran), furan, thiazole, imidazole, pyrazole, pyrimidine, pyridine, pyrazine, pyridazine, isothiazole, and isoxazole. It should be understood that this term contains heteroaryl groups as defined herein.

In this context, the term "aryl" refers to monocyclic or polycyclic aromatic hydrocarbon groups having 6-20, e.g., 6-12 carbon atoms in the ring portion. Preferably, the aryl group is the ($C_6$-$C_{10}$) aryl. Non-limiting examples include phenyl, biphenyl, naphthyl, or tetrahydronaphthyl groups, each of which can be substituted optionally by 1 to 4 substituents such as alkyl, trifluoromethyl, cycloalkyl, halogen, hydroxyl, alkoxy, acyl, alkyl-C(O)-O-, aryl-O-, heteroaryl-O-, amino, thiol, alkyl-S-, aryl-S-, nitro, cyano, carboxyl, alkyl-O-C(O)-, carbamoyl, alkyl-S(O)-, sulfonyl, sulfonyl amino, heterocyclyl, etc.

In this context, the term "heteroaryl" refers to a 5-20 membered (e.g., 5-14 membered, 5-8 membered, 5-6 membered) aromatic monocyclic or polycyclic ring system with 1-4 heteroatoms selected from N, O, or S, which can be substituted or unsubstituted. Preferably, the heteroaryl group is a 5-10 membered ring system, which is either a single ring or a fused double ring. Representative heteroaryl groups include 2- or 3-thiophenyl, 2- or 3-furyl, 2- or 3-pyrrolyl, 2-, 4- or 5-imidazolyl, 3-, 4- or 5-pyrazolyl, 2-, 4- or 5-thiazolyl, 3-, 4- or 5-isothiazolyl, 2-, 4- or 5-oxazolyl, 3-, 4- or 5-isoxazolyl, 3- or 5-1,2,4-triazolyl, 4- or 5-1,2,3-triazolyl, tetrazolyl, 2-, 3- or 4-pyridyl, 3- or 4-pyridazinyl, 3-, 4- or 5-pyrazinyl, 2-pyrazinyl, 2-, 4-, or 5-pyrimidinyl.

In this context, the term "heteroalkyl" refers to stable straight or branched hydrocarbons that are fully saturated or contain 1 to 3 degrees of unsaturation, consist of the indicated number of carbon atoms and one to ten, preferably one to three heteroatoms selected from O, N, Si, and S, wherein nitrogen and sulfur atoms are optionally oxidized and nitrogen heteroatoms are optionally quaternized. The heteroatoms O, N, Si and S may be located at any internal position of the heteroalkyl group or at a position where the heteroalkyl group is attached to the rest of the molecule. Representative examples of heteroalkyl groups include $-CH_2-CH_2-O-CH_3$, $-CH_2-CH_2-NH-CH_3$, - $CH_2-CH_2-N(CH_3)-CH_3$, $-CH_2-S-CH_2-CH_3$, $-CH_2-CH_2-S(O)-CH_3$, $-NH-CH_2-CH_2-NH-C(O)-CH_2-CH_3$, $-CH_2-CH_2-S(O)_2-CH_3$, $-CH=CH-O-CH_3$, $-Si(CH_3)_3$, $-CH_2-CH=N-O-CH_3$, and $-CH=CH-N(CH_3)-CH_3$. At most two heteroatoms can be continuous, such as $-CH_2-NH-OCH_3$ and $-CH_2-O-Si(CH_3)_3$. Typically, C1 to C4 heteroalkyl or thionyl groups have 1 to 4 carbon atoms and 1 or 2 heteroatoms, and C1 to C3 heteroalkyl or heteroalkylene has 1 to 3 carbon atoms and 1 or 2 heteroatoms. In some aspects, heteroalkyl and heteroalkylene are saturated.

For clarity, the term "guanine

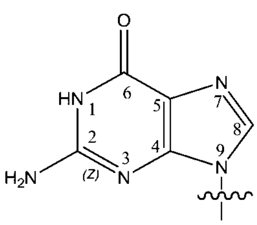

" as used herein may also be represented as "

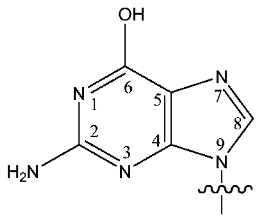

". The phosphorothioate groups used in the structural formula of the compound of the present invention may be drawn as

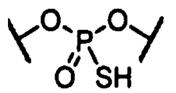 or 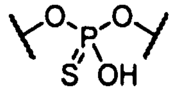 .

Unless otherwise indicated, the term "substituted" is used herein when defining each group, means that the corresponding group can be substituted by, for example, but not limited to, alkyl, alkenyl, alkynyl, cycloalkyl, aryl, heteroaryl, heterocyclyl, halogen, cyano, nitro, azide, carboxyl, hydroxyl, thiol, amino, mono- or dialkylamino, mono- or dicycloalkylamino, mono- or diarylamino, mono- or diheterocyclic amino, mono- or diheteroaryl amino, alkyl- or cycloalkyl- or heterocyclyl- or heteroaryl- or aryl-oxy, alkyl- or cycloalkyl- or heterocyclyl- or heteroaryl- or aryl- or aryl-thio, alkyl- or cycloalkyl- or heterocyclyl- or heteroaryl- or aryl-acyl, alkyl- or cycloalkyl- or heterocyclyl- or heteroaryl- or aryl-acylamino, alkyl- or cycloalkyl- or heterocyclyl- or heteroaryl- or aryl- or aryl- acyloxy, alkyl- or cycloalkyl- or heterocyclyl- or heteroaryl- or aryl-sulfonyl, alkyl- or cycloalkyl- or heterocyclyl- or heteroaryl- or aryl-sulfonyloxy, alkyl- or cycloalkyl- or heterocyclyl- or heteroaryl- or aryl-sulfonylamino, or the above mentioned optionally substituted amino-formyl group, and the group each of which is further substituted by the remaining optional substituents, are defined herein. Examples of substituents include, but are not limited to, one or more independently selected groups from the following: halogen, OH, SH, CN, $NH_2$, $NHCH_3$, $N(CH_3)_2$, $NO_2$, $N_3$, $C(O)CH_3$, COOH, C(O)-amino, $OCOCH_3$, methyl, ethyl, propyl, isopropyl, butyl, isobutyl, sec-butyl, tert-butyl, cyclopropyl, methoxy, ethoxy, propoxy, oxo, trifluoromethyl, difluoromethyl, sulfonyl amino, methanesulfonyl amino, SO, $SO_2$, phenyl, piperidinyl, piperazinyl, and pyrimidinyl.

In this context, the term "substitute" or "substituted" refers to one or more (e.g., 1, 2, 3, or 4) hydrogens on the designated atom are replaced by the designated group, provided that the normal valence bond of the designated atom is not exceeded and a stable compound is formed, and the combination of substituents and variables is allowed only if such combination forms a stable compound.

In this context, the term "pharmaceutically acceptable salt" refers to a salt that maintains the biological effects and properties of the ADC conjugate of the present invention and that the salt is not biologically or otherwise undesirable. ADC conjugates of the present invention can exist in their pharmaceutically acceptable salt forms, including acid addition salts and base addition salts. In the present invention, pharmaceutically acceptable non-toxic acid addition salts represent salts formed by ADC conjugates in the present invention with organic or inorganic acids, which include, but are not limited to, hydrochloric acid, sulfuric acid, hydrobromic acid, phosphoric acid, nitric acid, perchloric acid, acetic acid, oxalic acid, maleic acid, fumaric acid, tartaric acid, benzenesulfonic acid, methanesulfonic acid, salicylic acid, succinic acid, citric acid, lactic acid, propionic acid, benzoic acid, p-toluenesulfonic acid, malic acid, etc. Pharmaceutically acceptable non-toxic base addition salts represent salts formed by ADC conjugates of the present invention with organic or inorganic bases, including but not limited to base metal salts, such as lithium, sodium, or potassium salts; alkaline earth metal salts, such as calcium or magnesium salts; organic base salts, such as ammonium salts formed by organic bases containing N groups.

In this context, the term "solvate" refers to an association complex formed by one or more solvent molecules coupled to an ADC in the present invention. Solvents forming solvate include, but are not limited to, water, methanol, ethanol, isopropanol, ethyl acetate, tetrahydrofuran, N,N-dimethylformamide, dimethyl sulfoxide, etc. It should be understood that such solvate of the compound of the present invention also include solvate of pharmaceutically acceptable salts of the compound of the present invention.

In this context, the term "isotopic variants" refers to compounds in which one or more atoms constituting the compound are substituted by atoms having atomic masses or masses that differ from those typically found in nature. Examples of isotopes that can be incorporated into one or more atoms of the compound of the present invention include e.g., $^{2}$**H** , $^{3}$**H**, $^{13}$**C**, $^{14}$**C**, $^{15}$**N**, $^{17}$**O**, $^{18}$**O**, $^{31}$**P**, $^{32}$**P**, $^{35}$**S and** $^{18}$**F**, thereby forming isotopic variations of the compounds of the present invention, whether radioactive or not, are intended to cover the scope of the present invention. In some embodiments, the isotope incorporated is 2H (deuterium); in other embodiments, the isotype incorporated is 3H (tritium).

In this context, the term "stereoisomer" refers to an isomer formed due to at least one asymmetric center. In

compounds with one or more (e.g., one, two, three, or four) asymmetric centers, they can give rise to racemic mixtures, single enantiomers, diastereomeric mixtures, and separate diastereomers. Similarly, a compound of the present invention may exist as a mixture of two or more different structural forms (often called tautomers) in rapid equilibrium. It is understood that the scope of this application covers all such isomers or mixtures thereof in any proportion (e.g., 60%, 65%, 70%, 75%, 80%, 85%, 90%, 95%, 96%, 97%, 98%, 99%).

The compound of the present invention may have one or more centers of asymmetry and may therefore be prepared as (R)- or (S)-stereoisomers, respectively, or as a mixture thereof. The use of " 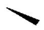 " or "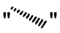" in the structural formula or structural fragment of a compound herein represents the configuration of the asymmetric center, that is, the chiral center. Accordingly, the configurations regarding this chiral center are indicated by R and S in the nomenclature of the compounds or intermediates provided by the present invention. Technicians in this field will realize that the phosphorothioate bonds in the compound of the present invention are inherently chiral and can each exist in an R or S configuration, so it is possible that the two phosphorothioate bonds are in the form of R,R, S,S, S,R, and R,S. The present invention covers the compound in basically pure form or in a mixture form and the specific embodiment thereof, and the compound containing two phosphorothioate bonds is preferred to have R,R, S,S, S,R, and R,S stereoisomers in basically pure form, particularly to have basically pure R,R stereoisomers, that is, both phosphorus atoms have R configuration. Their absolute configuration assignment can be performed according to methods in literature (Zhao et al. Nucleosides, Nucleotides and Nucleic Acids 2009, 289, 352-378*;* Knouse et al. Science 2018, 361, 1234*).* It should be pointed out that, errors in the designation of configurations caused by errors in literature-based methods do not affect the actual configuration of the compound of the present invention.

It should be understood that, when technicians in the field are able to judge the existence of a chiral isomer for a compound based on the structure of the compound shown herein and judge that it can be easily separated based on conventional methods in the field, the disclosure (either structural formula or chemical name) of the racemate of the compound herein should be regarded as having disclosed each isomer of the compound respectively.

In this context, the term "essentially pure" used for CDNs refers to a stereoconfiguration form that is at least 75% pure relative to other possible stereochemical configurations at the chiral center. In the preferred embodiment, essentially pure CDNs are at least 85% pure, at least 90% pure, at least 95% pure, at least 97% pure, and at least 99% pure. The essentially pure CDN formulation of the present invention is "stereochemically pure" and means that all CDNs in the formulation have specific stereochemical configurations at these chiral centers, rather than being intended to indicate that all CDNs in the formulation have specific stereochemical configurations at these chiral centers are otherwise identical. For example, essentially pure CDN R,R cGAMP phosphorothioate formulations can contain combinations of R,R c-di-GMP phosphorothioate and R,R c-di-AMP phosphorothioate and remain essentially pure cyclic purine dinucleotide formulations.

In this context, the number of groups attached to each atom in a compound definition, compound structural formula, or structural fragment depends on the chemical valence of that atom and does not have to be fully represented. In general, groups defined, structural formulae or structural fragments show only non-hydrogen groups, and groups not shown generally represent H, and technicians in the field can easily determine whether and how many groups not shown exist.

The "〰" used in the structural fragment described herein represents that the bond crossing with it is the bond that the structural fragment attached to the rest of the molecule.

In the absence of contradictions according to context, "pharmaceutically acceptable" and "medicinal" are used herein interchangeably.

In this context, the term "drug composition" refers to such a composition that exists in a form that allows the biological activity of the active ingredient contained therein to be effective and does not contain additional ingredients that have unacceptable toxicity to a subject administered the said composition.

Throughout this text, the terms "pharmaceutically acceptable excipient", "pharmaceutically acceptable carrier", and "therapeutic inert excipient " are used interchangeably and represent any pharmaceutically acceptable ingredient in a drug composition that is not therapeutically active and is not toxic to the administered subject, e.g., disintegrants, adhesives, fillers, solvents, buffers, tonicity agents, stabilizers, antioxidants, surfactants, carriers, diluents, or lubricants used to formulate a drug product.

In this context, the term "drug combination" refers to an unfixed combination product or fixed combination product, including but not limited to, a kit, drug combination. The term "unfixed combinations" means active ingredients, such as (i) ADC conjugates of the present invention, and (ii) other therapeutic agents applied simultaneously, without specific time constraints, or sequentially to patients at the same or different time intervals in separate entities, where such administration provides two or more active agents at preventive or therapeutic effective levels in the patient. In some embodiments, ADC conjugates and other therapeutic agents of the present invention used in drug combinations are administrated at levels not exceeding their levels when used alone. The term "fixed combination" refers to the simultaneous administration of two or more active agents to a patient as a single entity. It is preferred that the dose and/or time

interval between two or more active agents be selected so that the combined use of each component produces a greater effect than any component alone in the treatment of a disease or condition. Each component may be presented as a separate formulation, which may be the same or different.

In this context, the term "combination therapy" refers to the administration of two or more therapeutic agents or treatment methods, such as radiation therapy or surgery, to treat the diseases described herein. Such administration includes the co-administration of these therapeutic agents in an essentially simultaneous manner, such as a single capsule with a fixed proportion of the active ingredient. Alternatively, such administration includes co-administration of each active ingredient in multiple or separate containers (e.g., tablets, capsules, powders, liquids). Powder and/or liquid may be reconstituted or diluted to the desired dose prior to administration. In addition, such administration includes the use of each type of therapeutic agent at approximately the same time or in a sequential manner at different times. In either case, the treatment regimen will provide the beneficial effect of the drug combination in the treatment of the conditions or symptoms described herein.

In this context, the term "individual" or "subject" is used interchangeably and refers to mammals. Mammals include but are not limited to domesticated animals (e.g., dairy cows, sheep, cats, dogs, and horses), primates (e.g., human and nonhuman primates such as monkeys), rabbits, and rodents (e.g., mice and rats). In particular, the subjects are human.

In this context, the terms "treatment" and "treating" refer to slowing, interrupting, blocking, relieving, stopping, reducing, or reversing the progression or severity of an existing symptom, condition, disorder, or disease.

In this context, the terms "prevention" and "preventing" include inhibition of the onset or development of a disease or condition or symptoms of a particular disease or condition. In some embodiments, subjects with a family history of cancer are candidates for preventative regimens. Generally, in the context of cancer, the term "prevention" refers to the administration of a drug before the onset of signs or symptoms of cancer, particularly in subjects at risk of cancer.

In this context, the term "effective amount" refers to such an amount or dose of the antibody drug conjugate of the present invention, or of its composition or combination, which, when administered to a patient in single or multiple doses, produces the desired effect in a patient who requires treatment or prevention.

In this context, the term "therapeutically effective amount" refers to the amount that effectively achieves the desired treatment outcome at the required dose and for the required period of time. A therapeutically effective amount is also such an amount in which any toxic or harmful effect of the antibody drug conjugate of the present invention or its composition or combination is less than the therapeutically beneficial effect. Relative to untreated individuals, "therapeutically effective amount" is preferred to achieve inhibition of measurable parameters (e.g., tumor volume) by at least about 30%, or even more preferably by at least 40%, 45%, 50%, 55%, 60%, 65%, 70%, 75%, 80%, 85%, 90%, or even 100%.

In this context, the term "preventative effective amount" refers to the amount that effectively achieves the desired preventative outcome at the required dose and for the required period of time. Generally, since preventative doses are used in subjects before or at earlier stages of disease, the preventive effective amount will be less than the therapeutically effective amount.

**I: Antibody-drug conjugate**

In one aspect, the present invention provides antibody-drug conjugates (ADCs) with the following Formula (X) or pharmaceutically acceptable salts or solvates thereof :

$$Ab\text{-}[L\text{-}D]_q \qquad (X)$$

wherein,

Ab represents an antibody or antigen-binding fragment that binds to the target antigen;
L represents a linker unit linking D to Ab;
D represents a cyclic dinucleotide defined below in this text (e.g., compounds of Formula (II), (II-a), (II-a'), (II-b), (II-b');
q represents the number of D linked to Ab via the linker unit L as an integer or non-integer from 1 to 20.

In some embodiments of Formula X, q represents integers selected from 1 to 20, including 1, 2, 3, 4, 5, 6, 7, 8, 9, 10, 11, 12, 13, 14, 15, 16, 17, 18, 19, or 20. In some cases, q ranges from 1 to 2, 1 to 3, 1 to 4, 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, or 1 to 20, or is a range consisting of any two values between 1 and 20, e.g., 2 to 10, 2 to 8, 2 to 6, 2 to 4, 4 to 6, 4 to 8, 4 to 10, 6 to 8, or 6 to 10. In other embodiments, Formula A describes ADCs in ADC mixtures that exhibit q-values ranging from 1 to 2, 1 to 3, 1 to 4, 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, or 1 to 20, or a range consisting of any two values between 1 and 20, e.g., 2 to 10, 2 to 8, 2 to 6, 2 to 4, 4 to 6, 4 to 8, 4 to 10, 6 to 8, or 6 to 10.

In some embodiments, Formula X describes ADCs in ADC mixtures such that more than 60%, 65%, 70%, 75%, 80%, 85%, 90%, or 95% of ADCs in the mixture have q-values of 1, 2, 3, 4, 5, 6, 7, 8, 9, 10, 11, 12, 13, 14, 15, 16, 17, 18, 19, or 20. In some embodiments, Formula A describes ADCs in ADC mixtures such that more than 60%, 65%, 70%, 75%, 80%, 85%, 90%, or 95% of ADCs in the mixtures have q-values ranging from 1 to 2, 1 to 3, 1 to 4, 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, or 1 to 20, or a range consisting of any two values between 1 and 20, e.g., 2 to 10, 2 to 8, 2 to 6, 2 to 4, 4 to 6, 4 to 8, 4 to 10, 6 to 8, or 6 to 10.

In other embodiments, Formula X describes the ADC mixture, where q is "$q_{average}$", which represents the average of q values for the mixture, i.e., the average number of linker units (L) linked to a given antibody (Ab) in the mixture. In such an embodiment, $q_{average}$ represents an integer or non-integer value from 1 to 20, e.g., about 1 to 2, 1 to 3, 1 to 4, 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, or 1 to 20, or is an integer or decimal in a range consisting of any two values between 1 and 20, e.g., about 2 to 10, 2 to 8, 2 to 6, 2 to 4, 4 to 6, 4 to 8, 4 to 10, 6 to 8, or 6 to 10.

In the preferred embodiment, q in the Formula (X) of the present invention is an integer or non-integer of 1 to 10 or a range consisting of any two values between 1 and 10, e.g. about 1, 2, 3, 4, 5, 6, 7, 8, 9, 10, 1 to 2, 1 to 3, 1 to 4, 1 to 5, 1 to 6, 1 to 7, 1 to 8, 1 to 9, 1 to 10, 2 to 10, 2 to 8, 2 to 6, 2 to 4, 4 to 6, 4 to 8, 4 to 10, 6 to 8, or 6 to 10.

In some embodiments, q represents an average DAR of about 3. In some embodiments, q represents an average DAR of about 6. In some embodiment, q represents an average DAR of about 8.

Technicians in the field should also understand that the various ADCs described herein can be salts or solvates and are pharmaceutically acceptable salts in some specific embodiments.

Each component of the ADC conjugate of the present invention and the ADC conjugate comprising it are described in detail below. Technicians in this field understand that the ADCs disclosed in this text are essentially "modular" because each has the modular components Ab, L, and D. Throughout this text, a variety of specific unrestricted implementation examples and examples of these modular components are described, and the present invention covers specific combinations of specific embodiments for all modules, as each specific combination is explicitly described separately.

## Ab - antibody or antigen binding fragment

In the CDN-ADC of the present invention, an antibody is an antibody or antigen-binding fragment that specifically binds to a target antigen and acts to target and present the CDN drug component to a specific target cell population, which interacts with the specific target cell population due to the presence of its targeting component or molecule and subsequently releases the free drug within the target cell (intracellular mode) or near the target cell (extracellular mode).

In one set of embodiments, an antibody or antigen-binding fragment is bonded to a linker unit containing a releasable peptide component. As mentioned above, other connected components can also be present in the conjugates described herein to serve to provide additional space between the CDN compound and the antibody unit, or to provide the compositions with properties that increase solubility. In some of these embodiments, an antibody or antigen-binding fragment is bonded to a linker unit through its heteroatom. The heteroatoms available for this binding on the antibody or antigen-binding fragment include sulfur (in one embodiment, sulfhydryl groups from the targeted ligand), oxygen (in one embodiment, carboxyl or hydroxyl groups from the targeted ligand), and optionally substituted nitrogen (in one embodiment, primary or secondary amine functional groups from the targeted ligand, or optionally substituted amide nitrogen in another embodiment). These heteroatoms can be present on the antibody unit in the natural state of the ligand, such as naturally occurring antibodies, or can be introduced into the antibody unit by chemical modification or bioengineering.

The conjugation site on the antibody impacts ADC stability, pharmacokinetics, and pharmacodynamic properties. Excessive drug loading may sometimes result in rapid plasma clearance, whereas ADCs with low DARs (drug-antibody ratio) may exhibit weak activity. The drug loading of the antibody can be controlled by selecting the conjugation strategy and the conjugation site on the antibody while maintaining the structural integrity and homogeneity of the antibody.

In one embodiment, an antibody or antigen-binding fragment has a thiol functional group that binds to the linker unit via the sulfur atom of the thiol functional group.

In another embodiment, an antibody or antigen-binding fragment has one or more lysine residues capable of reacting with an activating ester (including, but not limited to, N-hydroxysuccinimide, pentafluorophenyl, and p-nitrophenyl ester) in a linker unit, thereby providing an amide bond consisting of the nitrogen atom of the antibody or antigen-binding fragment and C=O of the linker unit.

In other embodiments, artificial attachment sites are introduced in the antibody to achieve more site-directed conjugation.

In another embodiment, an antibody or antigen-binding fragment has one or more lysine residues that can be chemically modified to introduce one or more thiols. In these embodiments, an antibody or antigen-binding fragment is covalently linked to the linker unit via the sulfur atom of the thiol functional group. Reagents available to modify lysine in this manner include, but are not limited to, N-succinimidyl S-acetyl thioacetate (SATA) and 2-iminothiacyclopentane hydrochloride (Traut reagent).

In another embodiment, an antibody or antigen-binding fragment has one or more carbohydrate groups that can be modified to provide one or more thiol functional groups. Chemically modified antibodies or antigen-binding fragments in CDN conjugates are bonded to the linker unit via the sulfur atom of the thiol functional group.

In another embodiment, an antibody or antigen-binding fragment has one or more carbohydrate groups that can be oxidized to provide an aldehyde (-CHO) functional group. In these embodiments, the corresponding aldehyde interacts with the reactive site on the linker unit to form a chemical bond between the linker unit and the antibody unit.

Additional protocols for modifying proteins to link to linker units or related substances can be found in Coligan et al., Current Protocols in Protein Science, Vol. 2, John Wiley & Sons (2002), which are incorporated herein by reference.

In some embodiments, an antibody or antigen-binding fragment is able to form covalent bonds between the linker unit and the antibody or antigen-binding fragment corresponding to the antibody unit by interacting with reactive functional groups on the linker unit. Functional groups with the ability to interact with antibody units will depend on the property of the antibody or antigen-binding fragment. In some embodiments, this reactive group is maleimide. Covalent attachment of the antibody or antigen-binding fragment to a linker unit is achieved by the interaction of the sulfhydryl functional group of the antibody or antigen-binding fragment with the maleimide functional group of the linker unit, forming a sulfur-substituted succinimide. Thiol functional groups can be present in the natural state of an antibody or antigen-binding fragment, such as naturally occurring residues, or can be introduced into an antibody or antigen-binding fragment by chemical modification or bioengineering.

In other embodiments, thiols are created by reducing the interchain disulfide of the antibody. Thus, in some embodiments, the linker unit is conjugated to cysteine residues from the reduced interchain disulfide.

In other embodiments, thiol groups are chemically introduced into antibodies, such as cysteine residues. Accordingly, in some embodiments, linker units are conjugated to an antibody or antigen-binding fragment via cysteine residues introduced in an antibody or antigen-binding fragment.

Antibodies constituting the ADC of the present invention may be polyclonal, monoclonal, genetically engineered, and/or otherwise modified and are suitable for administration to human, such as humanized or fully human antibodies.

In some embodiments, the Ab unit of the ADC of the present invention is a monospecific antibody. In some embodiments, the Ab unit, upon binding to antigen receptors expressed on the surface of tumor cells, elicits endocytosis mediated by antigen receptors, which effectively delivers antitumor drugs of ADCs into tumor cells.

In some embodiments, the Ab units of the ADC of the present invention can be bispecific antibodies, bivariable domain antibodies, multi- or single-chain antibodies, single-domain antibodies, camelid antibodies, scFv-Fc antibodies, alternative antibodies, etc.

The present invention considers not only the use of monospecific antibody molecules as Ab units but also the use of bispecific antibodies as Ab units. One specificity of the antibody can target tumor-associated antigens to promote the specific binding of ADC to tumor cells; while the other specificity of the antibody can target tumor cell surface receptors to further promote the endocytosis and degradation of ADC. Examples of such combinations of bispecific targets that can be mentioned include, but are not limited to, dual HER2 and PRLR target combinations on breast cancer cells. The two specificities of antibodies can also target different epitopes of the same tumor-associated antigen to increase the selection of the antibody for cancer cells and/or enhance internalization and delivery to lysosomes by inducing clustering and cross-linking of antigens on the surface of tumor cells. Examples of such tumor-associated antigens that could be mentioned include, but are not limited to, HER2 on breast cancer cells.

The antibody portion constituting the ADC of the present invention may be in the form of a full-length antibody, which may contain or be derived from any antibody isotype, including, for example, IgA, IgD, IgE, IgG, IgM, or IgY. In some embodiments, the antibodies that make up the ADC are IgG (e.g., IgG1, IgG2, IgG3, or IgG4). In some embodiments, the antibody contains all or part of the constant region of the antibody.

The antibody portion constituting the ADC of the present invention may be a functionally active fragment, derivative, or analogue of an antibody that specifically binds target cells (e.g., cancer cell antigens, viral antigens, or microbial antigens). In this aspect, "functional activity" means that the fragment, derivative or analogue is capable of immunologically binding specifically to target cells.

Useful antibody fragments such as, but not limited to, $F(ab')_2$ fragment, Fab fragment, Fvs, single-chain antibody, double-antibody, triple-chain antibody, four-chain antibody, scFv, scFv-FV, or any other molecule with the same specificity as the antibody. The fragment may be obtained by molecular engineering or chemical or enzymatic treatment of an intact antibody or antibody chain or by recombination.

Useful modified antibody analogues and derivatives such as, but not limited to, derivatives and analogues of antibodies obtained by glycosylation, acetylation, pegylation, phosphorylation, amidation, derivatization by known protecting/blocking groups, proteolytic cleavage, attachment to cellular antibody units or other proteins. Any of the numerous chemical modifications can be performed by known techniques, including but not limited to specific chemical cleavage, acetylation, formylation, metabolic synthesis in the presence of tunicamycin, etc.

## Target Antigens and Antibodies

The antibody portion of the ADC of the present invention can target any suitable target molecule presented on the surface of the target cell, such as polypeptide, protein, polysaccharide, or lipid molecule. The binding of antibodies to target molecules should be highly specific to ensure specific binding of ADCs to target cells and reduce off-target toxicity. In some embodiments, binding affinity of antibody to target molecules can be selected at nanomolar or subnanomolar levels.

Appropriate target antigens can be selected by searching for cell surface proteins that are higher expressed in tumors but lower or even barely expressed in non-malignant tissues. In some embodiments, such target molecules are membrane antigens expressed on the surface of target tumor cells, such as tumor-specific antigens or tumor-associated antigens, the tumors include hematological and solid tumors, including primary and metastatic tumors. In a particularly preferred embodiment, an antibody or antigen-binding fragment of the CDN-ADC of the present invention specifically binds one or more tumor-specific antigens or tumor-associated antigens, or immune cell-associated antigens.

In some embodiments, tumor specific antigens or tumor associated antigens targeted by the antibody portion of the ADC of the present invention are selected from HER2, Her3, HER1 (ErbB1), HER4 (ErbB4), TROP2, Nectin4, Tissue Factor, PD-L1, PD-1, MET, CLDN18.2, KIT, CTLA-4, RPR1, Adrenaline A2 Receptor (EphA2), Folate Receptor (FRa), Mesothelin, Endothelin Receptor, GCPII, IL-13Ra, BCMA, GD2, CLL-1, CA-IX, MUC1, 5T4, AOC3, ALK, AXL, C242, CA-125, CCL11, CCR5, CD138, CD2, CD3, CD4, CDS, CD15, CA15-3, CD18, CD19, CD20, CD21, CD22, CD30, CD33, CD37, CD38, CD52, CD56, CD66e, CD70, CD72, CD79a, CD79b, CD123, CD174, CD276, CD79b, CA19-9, AGS-16, IGF1R, IGF2R, VEGFR1, VEGFR2, VEGFR3, PDGFR-$\alpha$, PDGFR-$\beta$, EGFR, EGFRvIII, SLTRK6, BMPR1B, E16, TOP1, STEAP1, 0772P, MUC16, Napi3b, Sema 5b, PSCA hIg, ETBR, RNF124, prostate cancer-related gene 1, TrpM4, teratoma-derived growth factor 1, C3DR, FcRH2, NCA, MDP, IL20R-$\alpha$, Brevican, EphB2R, ASLG659, prostate stem cell antigen precursor, GEDA, BAFF-R, CXCR5, HLA-DOB, P2X5, LY64, FcRH1, IRTA2, TENB2, integrin $\alpha5\beta6$, integrin $\alpha4\beta7$, FGF2, FGFR1, FGFR2, FGFR3, FGFR4, PSMA, Somatostatin receptor, RANK, SLAMF7, ITGB6, CEACAM5, MUC1, CA9, EGFRvIII, IL2RA, AXL receptor tyrosine kinase, TGF-$\beta$R, TNFRSF8, cancer/testis associated antigens, CLEC14A, GRP78, stem cell specific antigens, ASG-5, PRR4, GUCY2C, SLC39A6, TPBG, middle tumor-associated antigen CA242, FOLR1, GPNMB, HAVCR1, prostate tumor target Mindin, VTCN1, PTK7 protein tyrosine kinase 7, macrophage stimulating 1 receptor, TACSTD2, CA6, DLL3, DLL4, EpCAM, FAP, DKK-1, Endoglin, VCAM1, GPC3, DR5, ASCT2, B7H4.

In some embodiments, tumor-associated antigens and immune cell antigens are T-cell co-inhibitory molecules. In some embodiments, antibodies or antigen-binding fragments thereof specifically bind to tumor-associated antigens selected from PD-L1, PD-L2, CD47, CD80, CD86, HVEM, UL144, CD155, CD112, CD113, galectin-1, galectin-3, galectin-9, CD48, LIGHT, BTLA, and CD160. In some embodiments, tumor-associated antigens are molecules that bind to T-cell molecules selected from BTLA, Tim-3, PD-1, CTLA-4, TIGIT, CD244, and CD223.

In some embodiments, antibodies are anti-PD-L1 antibodies, such as atezolizumab, durvalumab, avelumab, or antigen-binding fragments thereof or antibodies with amino sequences equivalent to them or antigen-binding fragments thereof.

In some embodiments, antibodies are anti-PD-1 antibodies, such as nivolumab, pembrolizumab, cemiplimab, anti-mouse PD-1 antibody clone J43, anti-mouse PD-1 antibody clone RMP1-14, mouse anti-PD-1 antibody clone EH12, ANB011, MDX-1106, AMP-514, AMP-224, or pidilizumab. In some embodiments, anti-PD-1 antibodies are pembrolizumab or nivolumab.

In some embodiments, antibodies are anti-CTLA-4 antibodies, such as ipilimumab, clone 9H10, tremelimumab, or clone BNI3.

In some embodiments, antibodies are anti-CD47 antibodies, such as antibodies to Hu5F9-G4, IBI188, CC-90002, ZL1201, TTI-621, AO-176, SGN-CD47M, ALX148 antigen-binding domain, or antigen-binding fragments thereof, or antibodies with amino acid sequences equivalent to them or antigen-binding fragments thereof.

In other embodiments, antibodies or antigen-binding fragments thereof specifically bind tumor-associated antigens, which are growth factor receptors (GFRs). In some embodiments, the tumor-associated antigens are GFRs of the EGFR/ErbB/HER family. In some embodiments, tumor-associated antigens are selected from EGFR/HER1 (ErbB1), HER2/c-Neu (ErbB2), Her3 (ErbB3), and Her4 (ErbB4) receptors. In some embodiments, tumor-associated antigens are GFRs of the IGFR family. In some embodiments, cancer-associated tumor antigens are IGF1R or IGF2R receptors. In some embodiments, tumor-associated antigens are GFRs of the TGF-$\beta$R (T$\beta$R) family. In some embodiments, cancer-associated tumor antigens are either T$\beta$R I or T$\beta$R II receptors. In some embodiments, tumor-associated antigens are GFRs of the VEGFR family. In some embodiments, cancer-associated tumor antigens are VEGFR1, VEGFR2, or VEGFR3 receptors. In some embodiments, tumor-associated antigens are GFRs of the PDGFR family. In some embodiments, cancer-associated tumor antigens are PDGFR-$\alpha$ or PDGFR-$\beta$ receptors. In some embodiments, tumor-associated antigens are GFRs of the FGFR family. In some embodiments, cancer-associated tumor antigens are FGFR1, FGFR2, FGFR3, or FGFR4 receptors.

In some embodiments, antibodies are anti-EGFR/HER1 (ErbBl) antibodies, such as cetuximab, panitumumab,

necitumumab, or antigen-binding fragments thereof, or antibodies with amino acid sequences equivalent to them or antigen-binding fragments thereof. In some embodiments, antibodies are anti-HER2 (ErbB2) antibodies, such as trastuzumab, pertuzumab, or antigen-binding fragments thereof, or antibodies with amino acid sequences equivalent to them or antigen-binding fragments thereof. In some embodiments, antibodies are anti-VEGFR2 antibodies, such as ramucirumab or antigen-binding fragments thereof or antibodies with amino acid sequences equivalent to them or antigen-binding fragments thereof. In some embodiments, antibodies are anti-PDGFR-α antibodies, such as olaratumab or antigen-binding fragments thereof or antibodies with amino acid sequences equivalent to them or antigen-binding fragments thereof.

In other embodiments, antibodies or antigen-binding fragments thereof specifically bind lymphoma-associated antigens. In some embodiments, lymphoma-associated antigens are CD20, CD30, CD19/CD3, CD22, or CD33. In some embodiments, antibodies are anti-CD20 antibodies, such as rituximab, ibritumomab, ofatumumab, obinutuzumab, or antigen-binding fragments thereof or antibodies with amino acid sequences equivalent to them or antigen-binding fragments thereof. In some embodiments, antibodies are anti-CD30 antibodies, such as brentuximab or antigen-binding fragments thereof or antibodies with amino acid sequences equivalent to them or antigen-binding fragments thereof. In some embodiments, antibodies are anti-CD19/CD3 antibodies, such as blinatumomab or antigen-binding fragments thereof or antibodies with amino acid sequences equivalent to them or antigen-binding fragments thereof. In some embodiments, antibodies are anti-CD22 antibodies, such as inotuzumab or antigen-binding fragments thereof or antibodies with amino acid sequences equivalent to them or antigen-binding fragments thereof. In some embodiments, antibodies are anti-CD33 antibodies, such as gemtuzumab or antigen-binding fragments thereof or antibodies with amino acid sequences equivalent to them or antigen-binding fragments thereof.

In other embodiments, antibodies or antigen-binding fragments thereof specifically bind myeloma-associated antigens. In some embodiments, myeloma-associated antigens are SLAMF7 or CD38. In some embodiments, antibodies are anti-SLAMF7 antibodies, such as elotuzumab or antigen-binding fragments thereof or antibodies with amino acid sequences equivalent to them or antigen-binding fragments thereof. In some embodiments, antibodies are anti-CD38 antibodies, such as daratumumab or antigen-binding fragments thereof or antibodies with amino acid sequences equivalent to them or antigen-binding fragments thereof.

In other embodiments, antibodies or antigen-binding fragments thereof specifically bind to blastoma associated antigens. In some embodiments, the blastoma associated antigen is GD2. In some embodiments, antibodies are anti-GD2 antibodies, such as dinutuximab or antigen-binding fragments thereof or antibodies with amino acid sequences equivalent to them or antigen-binding fragments thereof.

In other embodiments, antibodies or antigen-binding fragments thereof specifically bind RANK ligands. In some embodiments, antibodies are antibodies against RANK ligands, such as denosumab or antigen-binding fragments thereof or antibodies with amino acid sequences equivalent to them or antigen-binding fragments thereof.

In other embodiments, antibodies or antigen-binding fragments thereof specifically bind TROP2 (a cell surface protein encoded and expressed by the TACSTD2 gene, also known as tumor-associated calcium signal transducer). TROP2 shows significantly increased expression in a variety of tumors and plays a key role in tumor growth and is also associated with more aggressive diseases and poor prognosis, involving tumors including breast cancer, lung cancer, gastric cancer, colorectal cancer, pancreatic cancer, prostate cancer, cervical cancer, head and neck cancer, and ovarian cancer. In some embodiments, antibodies are anti-TROP2 antibodies, such as Sacituzumab and Datopotamab, or antigen-binding fragments thereof or antibodies with amino acid sequences equivalent to them or antigen-binding fragments thereof.

In other embodiments, antibodies or antigen-binding fragments thereof specifically bind Claudin 18.2 (tight junction membrane proteins belonging to the Claudin family). Abnormal expression of Claudins can lead to structural destruction and functional impairment of epithelial cells and endothelial cells and play an important pathogenic role in the invasion and metastasis of a variety of tumors of epithelial origin. At the same time, its expression is highly specific and only expressed in gastric epithelial cells in healthy tissues, while it shows abnormal overexpression in a series of malignant tumors, especially in malignant tumors of the digestive system, such as gastric (70-80%), pancreas (60%), ovary, lung, and other tissues, so it has become a potential effective molecular target of anti-tumor drugs. Tumors involved include breast cancer, lung cancer, gastric cancer, colorectal cancer, pancreatic cancer, prostate cancer, cervical cancer, head and neck cancer, and ovarian cancer. In some embodiments, antibodies are anti-Claudin18.2 antibodies, such as zolbetuximab, Osemitamab (TST001), CMG901, ASKB589, ZL-1211, or antigen-binding fragments thereof or antibodies with amino acid sequences equivalent to them or antigen-binding fragments thereof.

In other embodiments, antibodies or antigen-binding fragments thereof specifically bind Met (also called c-Met, proto-oncogene), and the disease-causing mutated Met gene will encode an abnormal Met receptor, which transmits abnormal signals and has multifaceted effects, including cell growth, survival, invasion, metastasis, angiogenesis, etc., and continuous signal transmission can cause excessive cell proliferation, leading to the development and progression of tumors, including non-small cell lung cancer, colorectal cancer, gastric cancer, esophageal cancer, and glioma. In some embodiments, antibodies are anti-Met antibodies, such as onartuzumab or antigen-binding fragments thereof or antibodies with amino acid sequences equivalent to them or antigen-binding fragments thereof.

In other embodiments, antibodies or antigen-binding fragments thereof specifically bind to the adrenoceptor Eph, particularly the EphA2 receptor, which is not only a biomarker of malignant characteristics, but also an active participant in malignant progression, and has demonstrated an important role in the regulation of cancer development and tumor progression, and human EphA2 is abundantly expressed in prostate, lung, esophageal, colorectal, cervical, ovarian, breast, and skin cancers, which are associated with poor prognosis, increased metastatic potential, and decreased survival in cancer patients. In some embodiments, antibodies are anti-EphA2 antibodies or antigen-binding fragments thereof or antibodies with amino acid sequences equivalent to them or antigen-binding fragments thereof.

In other embodiments, tumor-associated antigens specifically bound by antibodies or antigen-binding fragments thereof are selected from KIT, FOLR1, CD276, PD-L1, NECTIN4, Mesothelin, MUC1, GCPII, BCMA, cMet, RPR1, CD22, CD19, TOP1, Claudin 18.2, EGFR, TROP2, HER2, and the corresponding antibodies were commercially available or could be prepared by techniques known in the field, such as hybridoma technology, recombinant technology, phage display technology, transgenic animals, or their combinations.

In preferred embodiments, antibodies are those that bind antigens preferentially expressed or overexpressed in cancer cells, the antigens, for example, are HER2 (ErbB2), PD-1, PD-L1, EGFR, TROP2, Claudin 18.2, EphA-2, and Met.

Referring to the tumor-associated antigens mentioned above, the ADC compound of the present invention may treat tumors associated with the expression of the antigens mentioned above, with a specific tumor list that is known or determined by technicians in the field based on existing technology.

Antibodies that are immune-specific for tumor-associated antigens may be commercially available or produced by any method known to the technicians in the field, such as recombinant expression technology. Nucleotide sequences encoding immune-specific antibodies for cancer cell antigens can be obtained, for example, from GenBank databases or similar databases, literature publications, or by conventional cloning and sequencing.

## Ab unit targeting HER2

In a particularly preferred embodiment, the antibody conjugates provided herein include antibodies that specifically bind to human HER2 or antigen-binding fragment thereof (anti-HER2 antibody), i.e., the antibody moiety specifically targets the tumor-associated antigen HER2. Thus, in some aspects, the present invention provides antibody-drug conjugates (ADCs) containing antibodies specifically binding to HER2 or antigen-binding fragments thereof as Ab units of ADCs.

HER2 is the receptor tyrosine protein kinase ErbB2, and HER2 overexpression or gene amplification is present in approximately 20-30% of breast cancers. Increased activation of HER2 triggers multiple downstream pathways, leading to aberrant proliferation of cancer cells (Treish I, Schwartz R, Lindley C: Pharmacology and therapeutic use of trastuzumab in breast cancer. Am J Health Syst Pharm. 2000 Nov 15; 57 (22): 2063-76; quiz 2077-9). HER2 is also overexpressed in many other types of cancer, such as gastric cancer, esophageal cancer, colon cancer, rectal cancer, breast cancer, ovarian cancer, cervical cancer, uterine cancer, endometrial cancer, bladder cancer, pancreatic cancer, lung cancer, prostate cancer, osteosarcoma, neuroblastoma, or head and neck cancer.

In some embodiments, antibodies that specifically bind to human HER2 or antibody fragments thereof (e.g., antigen-binding fragments) can be selected from trastuzumab, pertuzumab, margetuximab or HT-19 or its antibody fragments or its site-specific mutants, or other anti-human HER2 antibodies that recognize the same epitope or competitively bind human HER2.

Trastuzumab (brand name Herceptin or Herclon) is a humanized monoclonal antibody against human epidermal growth factor 2 that is used to treat HER2-positive breast cancer, gastrointestinal cancer, and gastric cancer. It binds the juxtamembrane portion of the extracellular domain of the HER2 receptor, and its amino acid sequences of the heavy and light chain variable regions are described in U.S. patents 5,821,337 interacting with the three loop regions formed by human HER2 residues 557 - 561, 570 - 573, and 593 - 603 (Cho et al., Nature 421:756-760, 2003), and may interfere with HER2 signaling by preventing HER2 receptor dimerization, promoting HER2 endocytic destruction, and inhibiting shedding of the extracellular domain (Hudis CA, N Engl J Med. 2007; 357(1): 39-51). Another important mechanism of action of anti-HER2 antibodies is mediated by antibody-dependent cellular cytotoxicity (ADCC). In ADCC, anti-HER2 antibodies bind tumor cells and then recruit immune cells, such as macrophages, through Fcε receptor (FcεR) interaction. Trastuzumab was approved by the US FDA in September 1998 for the treatment of patients with metastatic breast cancer.

Pertuzumab (also called 2C4, Omnitarg, Perjeta) is a humanized monoclonal antibody that binds to the extracellular domain of the HER2 receptor and inhibits dimerization of HER2 with other HER receptors, and its heavy and light chain amino acid sequences are described in U.S. patents 7,560,111. Pertuzumab predominantly interacts with residues within the 245 - 333 regions of human HER2, particularly residues His 245, Val 286, Ser 288, Leu 295, His 296, or Lys 311 (Franklin et al, Cancer Cell 5:317 - 328, 2004). Pertuzumab has been shown to be more effective than trastuzumab in disrupting HER1-HER2 and HER3-HER2 complex formation in breast and prostate cancer cell lines (Agus et al., J Clin Oncol. 2005; 23(11): 2534-43. Epub Feb 7, 2005). For potency, antibody-dependent cytotoxicity is not required for

pertuzumab because an intact Fc region is not required for its activity (Agus et al., J Clin Oncol. 2005; 23(11): 2534-43. Epub Feb 7, 2005). Pertuzumab is approved by the U.S. FDA in combination with trastuzumab and docetaxel for the treatment of patients with HER2-positive metastatic breast cancer.

Margetuximab (also called MGAH22) is another anti-HER2 monoclonal antibody (see http://www.macrogenics.com/products-margetuximab.html). The Fc region of margetuximab was optimized to allow increased binding to activated FcεRs. Margetuximab is currently being tested in clinical trials for the treatment of patients with recurrent or refractory advanced breast cancer who have been found to have tumor HER2 expression at 2+ levels by immunohistochemistry and lack evidence of HER2 gene amplification by FISH.

HT-19 is another anti-HER2 monoclonal antibody that binds epitopes in human HER2 that differ from trastuzumab or pertuzumab epitopes and has been shown to inhibit HER2 signaling as little as trastuzumab, and in combination with trastuzumab and pertuzumab promotes HER2 degradation (Bergstrom D. A. et al., Cancer Res. 2015; 75: LB-231).

An exemplary antibody targeting HER2 that can be used for the ADC of the present invention can be an antibody or antigen-binding fragment containing all six CDR sequences (preferably the heavy chain variable region and the light chain variable region sequences of the antibodies selected from the below group) of the antibodies selected from the below group: trastuzumab (Herceptin, Genentech, US6,054,297); ATCC accession numbers PTA-10355, PTA-10356, PTA-10357, PTA 10358 (US20100119511); ATCC accession numbers CRL-10463 (Genentech); ATCC accession numbers HB-12215, HB-12216, CRL 10463, HB-12697; pertuzumab (Perjeta, Genentech, US20110117097); ATCC accession numbers HB-12215, HB-12216, CRL 10463, HB-12698 (US20090202546); ATCC accession numbers HB-12215, HB-12216 (US20060088523); ATCC accession numbers (7C2) HB-12215, (7F3) HB-12216, (4D5) CRL-10463, (2C4) HB-12697 (US20060018899); TrasGEX (Glycotope: http://www.glycotope.conn/pipeline). The present invention also considers anti-HER2 antibodies disclosed in US201 10177095, US20100119511, US20110117097, US20090285837, US20090202546, US20060088523, US20060018899, US2011/0159014, US20090187007, US20110217305.

> Trastuzumab Light Chain Amino Acid Sequence (SEQ ID NO: 1)

DIQMTQSPSSLSASVGDRVTITCRASQDVNTAVAWYQQKPGKAPKLLIYSASFLYSGVPS
RFSGSRSGTDFTLTISSLQPEDFATYYCQQHYTTPPTFGQGTKVEIKRTVAAPSVFIFPP
SDEQLKSGTASVVCLLNNFYPREAKVQWKVDNALQSGNSQESVTEQDSKDSTYSLSSTLT
LSKADYEKHKVYACEVTHQGLSSPVTKSFNRGEC

> Trastuzumab Heavy Chain Amino Acid Sequence (SEQ ID NO: 2)

EVQLVESGGGLVQPGGSLRLSCAASGFNIKDTYIHWVRQAPGKGLEWVARIYPTNGYTRY
ADSVKGRFTISADTSKNTAYLQMNSLRAEDTAVYYCSRWGGDGFYAMDYWGQGTLVTV
SS
ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGG
PSVFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRW
QQGNVFSCSVMHEALHNHYTQKSLSLSPGK.

> Trastuzumab Light Chain LCDR1 sequence (SEQ ID NO: 3)
RASQDVNTAVA

> Trastuzumab Light Chain LCDR2 sequence (SEQ ID NO: 4)
SASFLYS

> Trastuzumab Light Chain LCDR3 sequence (SEQ ID NO: 5)
QQHYTTPPT

> Trastuzumab Heavy Chain HCDR1 Sequence (SEQ ID NO: 6)
DTYIH

> Trastuzumab Heavy Chain HCDR2 Sequence (SEQ ID NO: 7)
RIYPTNGYTRYADSVKG

> Trastuzumab Heavy Chain HCDR3 Sequence (SEQ ID NO: 8)
WGGDGFYAMDY

> Trastuzumab Light Chain Variable Region VL (SEQ ID NO: 9)

DIQMTQSPSSLSASVGDRVTITCRASQDVNTAVAWYQQKPGKAPKLLIYSASFLYSGVPS
RFSGSRSGTDFTLTISSLQPEDFATYYCQQHYTTPPTFGQGTKVEIK

> Trastuzumab Heavy Chain Variable Region VH (SEQ ID NO: 10)

EVQLVESGGGLVQPGGSLRLSCAASGFNIKDTYIHWVRQAPGKGLEWVARIYPTNGYTRY
ADSVKGRFTISADTSKNTAYLQMNSLRAEDTAVYYCSRWGGDGFYAMDYWGQGTLVTV
SS

In one embodiment, the antibody portion used for the ADC of the present invention contains all 6 CDR sequences of trastuzumab. In another embodiment, the antibody portion used for the ADC of the present invention comprises a heavy chain variable region sequence and a light chain variable region sequence of trastuzumab. In a further embodiment, the antibody portion used for the ADC of the present invention comprises a heavy chain sequence and a light chain sequence of trastuzumab.

In some embodiments, the Ab unit of the ADC of the present invention contains 3 CDRs of the heavy chain variable region (VH) sequence of SEQ ID NO: 10 and 3 CDRs of the light chain variable region (VL) sequence of SEQ ID NO: 9, and preferably, wherein the CDRs are defined by Kabat or IMGT or a combination thereof.

In some embodiment, the Ab unit of the ADC of the present invention contains 3 heavy chain complementarity determining regions (HCDRs) and 3 light chain complementarity determining regions (LCDRs), wherein:
As defined by Kabat, HCDR1 contains or consists of the amino acid sequence of SEQ ID NO: 6, HCDR2 contains or consists of the amino acid sequence of SEQ ID NO: 7, HCDR3 contains or consists of the amino acid sequence of SEQ ID NO: 8, LCDR1 contains or consists of the amino acid sequence of SEQ ID NO: 3, LCDR2 contains or consists of the amino acid sequence of SEQ ID NO: 4, and LCDR3 contains or consists of the amino acid sequence of SEQ ID NO: 5.

In one embodiment, the Ab unit of the ADC of the present invention contains a heavy chain variable region, wherein the heavy chain variable region contains the amino acid sequence shown in SEQ ID NO: 10, or an amino acid sequence with at least 80%, 85%, 90%, 95%, 96%, 97%, 98%, or 99% sequence identity to it.

In one embodiment, the Ab unit of the ADC of the present invention contains a light chain variable region, wherein the light chain variable region contains the amino acid sequence shown in SEQ ID NO: 9, or an amino acid sequence with at least 80%, 85%, 90%, 95%, 96%, 97%, 98%, or 99% sequence identity to it.

In some preferred embodiments, the Ab unit of the ADC of the present invention contains a heavy chain variable region and a light chain variable region, wherein the heavy chain variable region contains the amino acid sequence shown in SEQ ID NO: 10 and the light chain variable region contains the amino acid sequence shown in SEQ ID NO: 9.

In some embodiments, Ab units of the ADC of the present invention preferably also contain the heavy chain constant region and/or the light chain constant region of the antibody. Preferably, the heavy chain constant region is a heavy chain constant region derived from human immunoglobulin. Preferably, the light chain constant region is a light chain constant region derived from human immunoglobulin. In some aspects, the constant region of the heavy chain contained in the Ab unit can be any isotype or subtype, such as the constant region of the heavy chain for IgG1, IgG2, IgG3, or IgG4 isotypes, and preferably the constant region of the heavy chain for IgG1, IgG2, or IgG4, especially the constant region of the heavy chain for human IgG1. In other aspects, the constant region of the light chain contained in the Ab unit can be either the constant region of the κ light chain or the constant region of the λ light chain, particularly the constant region of the human κ light chain.

In some embodiments, the Ab unit of the ADC of the present invention contains a human IgG1 heavy chain constant region. Preferably, the heavy chain constant region contains the amino acid sequence of SEQ ID NO: 11, or an amino acid sequence with at least one, two, or three, but not more than 20, 10, or 5 amino acid changes relative to the amino acid sequence of SEQ ID NO: 11, or a sequence with at least 95-99% identity to the amino acid sequence of SEQ ID NO: 11.

An exemplary amino acid sequence of the constant region of the human IgG1 heavy chain (SEQ ID NO: 11)

ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGG
PSVFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRW
QQGNVFSCSVMHEALHNHYTQKSLSLSPGK

In some embodiments, the Ab unit of the ADC of the present invention contains a constant region of the human κ light chain. Preferably, the constant region of the light chain contains the amino acid sequence of SEQ ID NO: 12, or an amino acid sequence with at least one, two, or three amino acid sequences relative to SEQ ID NO: 12, but not more than 20, 10, or 5 amino acid changes, or a sequence with at least 95-99% identity to the amino acid sequence of SEQ ID NO: 12.
An exemplary amino acid sequence of the constant region of the human κ light chain (SEQ ID NO: 12)

RTVAAPSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC

In some embodiments, the Ab unit of the ADC of the present invention is a full-length antibody containing the constant regions of the heavy chain and light chain. In some embodiments, the Ab unit is a tetrameric structure consisting of two light chains and two heavy chains. In further embodiments, the Ab unit is an IgG antibody, especially an IgG1 antibody.
In some preferred embodiments, the Ab unit of the ADC of the present invention contains a heavy chain and a light chain, wherein: the heavy chain contains the amino acid sequence shown in SEQ ID NO: 2, or an amino acid sequence with at least 80%, 85%, 90%, 95%, 96%, 97%, 98%, or 99% sequence identity to it, or is composed of it. In other preferred embodiments, the Ab unit of the ADC of the present invention contains a heavy chain and a light chain, wherein: the light chain contains the amino acid sequence shown in SEQ ID NO: 1, or an amino acid sequence with at least 80%, 85%, 90%, 95%, 96%, 97%, 98%, or 99% sequence identity to it.
In some more preferred embodiments, the Ab unit of the ADC of the present invention contains:

(a) a heavy chain containing the amino acid sequence of SEQ ID NO: 2, and

(b) a light chain containing the amino acid sequence of SEQ ID NO: 1.

**Ab unit targeting TROP2**

In a particularly preferred embodiment, the antibody conjugates provided herein include antibodies that specifically bind to human TROP2 or antigen-binding fragments thereof (anti-TROP2 antibody), i.e., the antibody moiety specifically targets the tumor-associated antigen TROP2. Thus, in some aspects, the present invention provides antibody-drug conjugates (ADCs) containing antibodies specifically binding to TROP2 or antigen-binding fragments thereof as Ab units of ADCs.
Trophoblast cell surface antigen 2 (TROP2), also known as tumor-associated calcium signal transducer 2 (TACSTD2), is a type I transmembrane cell surface glycoprotein. The sequence of human TROP2 is available from UniProtKB accession number P09758. TROP2 has been shown to be overexpressed in many solid tumors, including, but not limited to, a variety of human epithelial cancers such as cervical cancer, endometrial cancer, breast cancer, urothelial cancer, lung cancer, gastric cancer, prostate cancer, colorectal cancer, and pancreatic cancer, etc. In addition, TROP2 may play a role in tumor cell proliferation, invasion, migration, apoptosis, and resistance to therapy by binding or interacting with a variety of molecules. These characteristics make TROP2 has become an attractive pan-cancer target for cancer therapy. Ying Wen et al, A literature review of the promising future of TROP2: a potential drug therapy target, Ann Transl Med. 2022 Dec; 10(24): 1403, doi: 10.21037/atm-22-5976.
Antibodies targeting human TROP2 that can be used for the ADC of the present invention can be prepared using an antibody preparation process known in the field. For example, antibodies against TROP2 can be obtained by the following methods: immunizing animals with human TROP2 (UniProtKB accession number P09758) or peptides containing the amino acid sequence of TROP2 extracellular domain, harvesting antibodies from immunized animals, and purifying and preferably humanizing. In addition, complete human sequence anti-human TROP2 antibodies may be obtained using yeast display libraries expressing human immunoglobulin sequences or transgenic animals.

In some embodiments, antibodies that specifically bind to human TROP2 or its antibody fragments (e.g. antigen binding fragments) can be selected from Sacituzumab, Datopotamab, or its antibody fragments, or other anti-human TROP2 antibodies that recognize the same epitope or competitively bind to human TROP2.

Sacituzumab is a humanized form of murine monoclonal antibody RS7 developed by Immunomedics as a humanized IgG1κ monoclonal antibody targeting TROP2. The antibody binds directly to TROP2-expressing cancer cells and triggers antibody endocytosis. The sequence of Sacituzumab can be found in US10179171B2.

Datopotamab is an IgG1-type antibody against TROP2. The antibody is generated by humanizing a mouse mAb that specifically binds to human TROP2. The sequence of Datopotamab can be found in Daisuke Okajima et al, Datopotamab Deruxtecan, a Novel TROP2-directed Antibody-drug Conjugate, Demonstrates Potent Antitumor Activity by Efficient Drug Delivery to Tumor Cells, Mol Cancer Ther (2021) 20 (12): 2329 - 2340.

An exemplary antibody targeting human TROP2 available for the ADC of the present invention can be an antibody or antigen-binding fragment containing all 6 CDR sequences (preferably containing the heavy chain variable region and light chain variable region sequences of the antibodies selected from the below group) of the antibodies selected from the below group: Sacituzumab and Datopotamab. The present invention also considers anti-human TROP2 antibodies disclosed in WO2010089782A1; US 2021/0393792 A1; WO2008/144891, WO2011/145744, WO2011/155579, WO2013/077458, WO2003/074566, WO2011/068845, WO2013/068946, US 2023/0270870 A1.

> Sacituzumab Light Chain Amino Acid Sequence (SEQ ID NO: 13)

DIQLTQSPSSLSASVGDRVSITCKASQDVSIAVAWYQQKPGKAPKLLIYSASYRYTGVPDRFS
GSGSGTDFTLTISSLQPEDFAVYYCQQHYITPLTFGAGTKVEIK

RTVAAPSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC

> Sacituzumab Heavy Chain Amino Acid Sequence (SEQ ID NO: 14)

QVQLQQSGSELKKPGASVKVSCKASGYTFTNYGMNWVKQAPGQGLKWMG<u>WINTYTGEP
TYTDDFKG</u>RFAFSLDTSVSTAYLQISSLKADDTAVYFCARGGFGSSYWYFDVWGQGSLVTV
SS

ASTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTVDKSRWQQGNVF
SCSVMHEALHNHYTQKSLSLSPGK

> Sacituzumab Light Chain LCDR1 Sequence (SEQ ID NO: 15)
KASQDVSIAVA
> Sacituzumab Light Chain LCDR2 Sequence (SEQ ID NO: 16)
SASYRYT
> Sacituzumab Light Chain LCDR3 Sequence (SEQ ID NO: 17)
QQHYITPLT
> Sacituzumab Heavy Chain HCDR1 Sequence (SEQ ID NO: 18)
NYGMN
> Sacituzumab Heavy Chain HCDR2 Sequence (SEQ ID NO: 19)
WINTYTGEPTYTDDFKG
> Sacituzumab Heavy Chain HCDR3 Sequence (SEQ ID NO: 20)
GGFGSSYWYFDV
> Sacituzumab Light Chain Variable Region VL (SEQ ID NO: 21)

DIQLTQSPSSLSASVGDRVSITCKASQDVSIAVAWYQQKPGKAPKLLIYSASYRYTGVPDRFS GSGSGTDFTLTISSLQPEDFAVYYCQQHYITPLTFGAGTKVEIK

> Sacituzumab Heavy Chain Variable Region VH (SEQ ID NO: 22)

QVQLQQSGSELKKPGASVKVSCKASGYTFTNYGMNWVKQAPGQGLKWMGWINTYTGEP TYTDDFKGRFAFSLDTSVSTAYLQISSLKADDTAVYFCARGGFGSSYWYFDVWGQGSLVTV SS

In one embodiment, the antibody portion used for the ADC of the present invention contains all 6 CDR sequences of Sacituzumab or Datopotamab. In another embodiment, the antibody portion used for the ADC of the present invention contains a heavy chain variable region sequence and a light chain variable region sequence of Sacituzumab or Datopotamab. In a further embodiment, the antibody portion used for the ADC of the present invention contains a heavy sequence and a light chain sequence of Sacituzumab or Datopotamab.

In some embodiments, the Ab unit of the ADC of the present invention contains 3 CDRs of the heavy chain variable region (VH) sequence of SEQ ID NO: 22 and 3 CDRs of the light chain variable region (VL) sequence of SEQ ID NO: 21, and preferably, wherein the CDRs are defined by Kabat or IMGT or a combination thereof.

In some embodiments, the Ab unit of the ADC of the present invention contains 3 heavy chain complementarity determining regions (HCDR) and 3 light chain complementarity determining regions (LCDR), wherein:

As defined by Kabat, HCDR1 contains or consists of the amino acid sequence of SEQ ID NO: 18, HCDR2 contains or consists of the amino acid sequence of SEQ ID NO: 19, HCDR3 contains or consists of the amino acid sequence of SEQ ID NO: 20, LCDR1 contains or consists of the amino acid sequence of SEQ ID NO: 15, LCDR2 contains or consists of the amino acid sequence of SEQ ID NO: 16, and LCDR3 contains or consists of the amino acid sequence of SEQ ID NO: 17.

In one embodiment, the Ab unit of the ADC of the present invention contains a heavy chain variable region, wherein the heavy chain variable region contains the amino acid sequence shown in SEQ ID NO: 22, or an amino acid sequence with at least 80%, 85%, 90%, 95%, 96%, 97%, 98%, or 99% sequence identity to it.

In one embodiment, the Ab unit of the ADC of the present invention contains a light chain variable region, wherein the light chain variable region contains the amino acid sequences shown in SEQ ID NO: 21, or an amino acid sequence with at least 80%, 85%, 90%, 95%, 96%, 97%, 98%, or 99% sequence identity to it.

In some preferred embodiments, the Ab unit of the ADC of the present invention contains a heavy chain variable region and a light chain variable region, wherein the heavy chain variable region contains the amino acid sequence shown in SEQ ID NO: 22, and the light chain variable region contains the amino acid sequence shown in SEQ ID NO: 21.

In some embodiments, the Ab unit of the ADC of the present invention preferably also contains a heavy chain constant region and/or a light chain constant region of the antibody. Preferably, the heavy chain constant region is derived from the heavy chain constant region of human immunoglobulin. Preferably, the light chain constant region is derived from the light chain constant region of human immunoglobulin. In some aspects, the constant region of the heavy chain contained in the Ab unit can be any isotype or subtype, such as the constant region of the heavy chain of IgG1, IgG2, IgG3, or IgG4 isotypes, and preferably the constant region of the heavy chain of IgG1, IgG2 or IgG4, especially the constant region of the heavy chain of human IgG1. In other aspects, the constant region of the light chain contained in Ab units can be either the constant region of the κ light chain or the constant region of the λ light chain, especially the constant region of the human κ light chain.

In some embodiments, the Ab unit of the ADC of the present invention contains a human IgG1 heavy chain constant region. Preferably, the heavy chain constant region contains the amino acid sequence of SEQ ID NO: 23 or an amino acid sequence with at least one, two, or three, but not more than 20, 10, or 5 amino acid changes relative to the amino acid sequence of SEQ ID NO: 23 or a sequence with at least 95-99% identity to the amino acid sequence of SEQ ID NO: 23.

An exemplary amino acid sequence of the constant region of the human IgG1 heavy chain (SEQ ID NO: 23)

ASTKGPSVFPLAPSSKSTSGGTAALGCLVKDYFPEPVTVSWNSGALTSGVHTFPAVLQ SSGLYSLSSVVTVPSSSLGTQTYICNVNHKPSNTKVDKRVEPKSCDKTHTCPPCPAPELLGG PSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSREEM TKNQVSLTCLVKGFYPSDIAVEWESNGQPENNYKTTPPVLDSDGSFFLYSKLTVDKSRWQ QGNVFSCSVMHEALHNHYTQKSLSLSPGK

In some embodiments, the Ab unit of the ADC of the present invention contains a constant region of the human κ

light chain. Preferably, the constant region of the light chain contains the amino acid sequence of SEQ ID NO: 12, or an amino acid sequence with at least one, two, or three amino acid sequences relative to SEQ ID NO: 12, but not more than 20, 10, or 5 amino acid changes or a sequence with at least 95-99% identity to the amino acid sequence of SEQ ID NO: 12.

In some embodiments, the Ab unit of the ADC of the present invention is a full-length antibody containing both the heavy chain constant region and the light chain constant region. In some embodiments, the Ab unit is a tetrameric structure consisting of two light chains and two heavy chains. In additional embodiments, the Ab unit is an IgG antibody, especially an IgG1 antibody.

In some preferred embodiments, the Ab unit of the ADC of the present invention contains both heavy and light chains, wherein the heavy chain contains the amino acid sequence shown in SEQ ID NO:14, or an amino acid sequence with at least 80%, 85%, 90%, 95%, 96%, 97%, 98%, or 99% sequence identity to it, or is composed of it. In other preferred embodiments, the Ab unit of the ADC of the present invention contains both heavy and light chains, wherein the light chain contains the amino acid sequence shown in SEQ ID NO: 13 or an amino acid sequence with at least 80%, 85%, 90%, 95%, 96%, 97%, 98%, or 99% sequence identity to it.

In some more preferred embodiments, the Ab unit of the ADC of the present invention contains:

(c) a heavy chain containing the amino acid sequence of SEQ ID NO: 14, and
(d) a light chain containing the amino acid sequence of SEQ ID NO: 13.

Cancers that can be treated with the ADC of the present invention targeting TROP2 include, but are not limited to, adenocarcinoma, squamous cell carcinoma, non-small cell lung cancer (NSCLC), small cell lung cancer (SCLC), colorectal cancer, gastric adenocarcinoma, esophageal cancer, hepatocellular carcinoma, epithelial ovarian cancer, breast cancer, metastatic breast cancer, triple-negative breast cancer (TNBC), prostate cancer, hormone-refractory prostate cancer, pancreatic ductal adenocarcinoma, head and neck cancer, renal cell carcinoma, bladder tumor, cervical cancer, endometrial cancer, uterine cancer, follicular thyroid cancer, glioblastoma multiforme tumor.

## Ab unit targeting Claudin 18.2

In a particularly preferred embodiment, the antibody conjugates provided herein include antibodies specifically binding to human Claudin18.2 or antigen-binding fragments thereof (anti-Claudin18.2 antibody), i.e., the antibody moiety specifically targets the tumor-associated antigen Claudin18.2. Thus, in some aspects, the present invention provides antibody-drug conjugates (ADCs) containing antibodies specifically binding to Claudin 18.2 or antigen-binding fragments thereof as Ab units of ADCs.

Compact protein 18.2 (Claudin18.2, also abbreviated CLDN18.2) is a tight junction membrane protein belonging to the Claudin family. Claudin18.2 sequences for humans and a variety of mammals can be found at UniProtKB. For example, the human Claudin18.2 sequence can be found in UniProtKB accession number P56856-2. The expression of this protein in healthy tissues is mainly confined to differentiated gastric epithelial cells, but it shows abnormal overexpression in a range of malignancies, particularly digestive system malignancies. Claudin18.2 is therefore proposed as a promising target for the development of antibody-based ADC cancer therapeutics. Daisuke Kyuno et al, Claudin-18.2 as a therapeutic target in cancers: cumulative findings from basic research and clinical trials, Tissue Barriers. 2022; 10(1): 1967080. doi: 10.1080/21688370.2021.1967080; Jinxia Chen, Targeting CLDN18.2 in cancers of the gastrointestinal tract: New drugs and new indications, Front Oncol. 2023; 13: 1132319, doi: 10.3389/fonc.2023.1132319.

Antibodies targeting human Claudin18.2 that can be used for the ADC of the present invention can be prepared using an antibody preparation process known in the field. For example, antibodies against Claudin18.2 can be obtained by the following methods: immunizing animals with human Claudin18.2 (UniProtKB accession number P56856-2) or peptides containing the amino acid sequence of Claudin18.2 extracellular domain, harvesting antibodies from immunized animals, and purifying and preferably humanizing. In addition, complete human sequence antibodies against human Claudin 18.2 can be obtained using yeast display libraries expressing human immunoglobulin sequences or transgenic animals.

In some embodiments, antibodies that specifically bind to human Claudin18.2 or its antibody fragments (e.g. antigen binding fragments) can be selected from Zolbetuximab, Osemitamab (TST001), CMG901, ASKB589, ZL-1211, or its antibody fragments, or other anti-human Claudin18.2 antibodies that recognize the same epitope or competitively bind to human Claudin18.2.

Zolbetuximab (also called GC-182, IMAB-362, IMAB362, claudiximab), is an IgG1 antibody derived from a murine monoclonal antibody and has been chimeric antibody-ized to display the human IgG1 constant region for clinical use. The antibody binds directly to CLDN18.2-expressing cancer cells and triggers antibody endocytosis. The sequence of Zolbetuximab can be found in WO2007059997 and WO2016/165762.

Osemitamab (TST001) is a high affinity humanized anti-Claudin18.2 antibody. The antibody has enhanced antibody-dependent cellular cytotoxicity (ADCC) and complement-dependent cytotoxicity (CDC) and demonstrated

strong antitumor activity in tumor xenograft models. The sequences of this antibody can be found in Inxight Drugs and INN (code 11927).

CMG901, ASKB589, ZL-1211 are exemplary antibodies against human CLDN18.2 in clinical trials. See DOI: 10.1200/JCO.2023.41.4_suppl.352; DOI: 10.1200/JCO.2023.41.4_suppl.397; DOI: 10.1200/JCO.2023.41.16_supp1.2537.

An exemplary antibody targeting human CLDN18.2 available for the ADC of the present invention can be an antibody or antigen-binding fragment containing all 6 CDR sequences (preferably containing the heavy chain variable region and light chain variable region sequences of the antibodies selected from the below group) of the antibodies selected from the below group: Zolbetuximab, Osemitamab (TST001), and CMG901.

The present invention also considers the anti-human Claudin18.2 antibodies disclosed in the following literature: WO2007059997A1, CN107667118A, WO2016/166122, US11555070B2, WO2020/135674, WO2018/006882, CN109762067, WO2019/242505, WO2020/038404, WO2020/043044, WO2020/063988, WO2020/082209, WO2020/018852, WO2020/023679, WO2020/135674, WO2020/135201, WO2020/139956, WO2020/025792, WO2020160560, CN111808194, and WO2020200196.

> Zolbetuximab Light Chain Amino Acid Sequence (SEQ ID NO: 24)

DIVMTQSPSSLTVTAGEKVTMSCKSSQSLLNSGNQKNYLTWYQQKPGQPPKLLIYWASTRE
SGVPDRFTGSGSGTDFTLTISSVQAEDLAVYYCQNDYSYPFTFGSGTKLEIK

RTVAAPSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC

> Zolbetuximab Heavy Chain Amino Acid Sequence (SEQ ID NO: 25)

QVQLQQPGAELVRPGASVKLSCKASGYTFTSYWINWVKQRPGQGLEWIGNIYPSDSYTNY
NQKFKDKATLTVDKSSSTAYMQLSSPTSEDSAVYYCTRSWRGNSFDYWGQGTTLTVSS

ASTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTVDKSRWQQGNVF
SCSVMHEALHNHYTQKSLSLSPGK

> Zolbetuximab Light Chain LCDR1 Sequence (SEQ ID NO: 26)
KSSQSLLNSGNQKNYLT
> Zolbetuximab Light Chain LCDR2 Sequence (SEQ ID NO: 27)
WASTRES
> Zolbetuximab Light Chain LCDR3 Sequence (SEQ ID NO: 28)
QNDYSYPFT
> Zolbetuximab Heavy Chain HCDR1 Sequence (SEQ ID NO: 29)
SYWIN
> Zolbetuximab Heavy Chain HCDR2 Sequence (SEQ ID NO: 30)
NIYPSDSYTNYNQKFKD
> Zolbetuximab Heavy Chain HCDR3 Sequence (SEQ ID NO: 31)
SWRGNSFDY
> Zolbetuximab Light Chain Variable Region VL (SEQ ID NO: 32)
DIVMTQSPSSLTVTAGEKVTMSCKSSQSLLNSGNOKNYLTWYQQKPGQPPKLLIYWASTRE SGVPDRFTGSGS
GTDFTLTISSVQAEDLAVYYCQNDYSYPFTFGSGTKLEIK
> Zolbetuximab Heavy Chain Variable Region VH (SEQ ID NO: 33)

QVQLQQPGAELVRPGASVKLSCKASGYTFTSYWINWVKQRPGQGLEWIGNIYPSDSYTNY
NQKFKDKATLTVDKSSSTAYMQLSSPTSEDSAVYYCTRSWRGNSFDYWGQGTTLTVSS

In one embodiment, the antibody portion used for the ADC of the present invention contains all 6 CDR sequences of Zolbetuximab. In another embodiment, the antibody portion used for the ADC of the present invention contains a heavy chain variable region sequence and a light chain variable region sequence of Zolbetuximab. In a further embodiment, the antibody portion used for the ADC of the present invention contains a heavy chain sequence and a light chain sequence of Zolbetuximab.

In some embodiments, the Ab unit of the ADC of the present invention contains three CDRs of the heavy chain variable region (VH) sequence of SEQ ID NO: 33 and three CDRs of the light chain variable region (VL) sequence of SEQ ID NO: 32, and preferably, wherein the CDRs are defined by Kabat or IMGT or a combination thereof.

In some embodiments, the Ab unit of the ADC of the present invention contains 3 heavy chain complementarity determining regions (HCDR) and 3 light chain complementarity determining regions (LCDR), wherein:

As defined by Kabat, HCDR1 contains or consists of the amino acid sequence of SEQ ID NO: 29, HCDR2 contains or consists of the amino acid sequence of SEQ ID NO: 30, HCDR3 contains or consists of the amino acid sequence of SEQ ID NO: 31, LCDR1 contains or consists of the amino acid sequence of SEQ ID NO: 26, LCDR2 contains or consists of the amino acid sequence of SEQ ID NO: 27, and LCDR3 contains or consists of the amino acid sequence of SEQ ID NO: 28.

In one embodiment, the Ab unit of the ADC of the present invention contains a heavy chain variable region, wherein the heavy chain variable region contains the amino acid sequence shown in SEQ ID NO: 33, or an amino acid sequence with at least 80%, 85%, 90%, 95%, 96%, 97%, 98%, or 99% sequence identity to it .

In one embodiment, the Ab unit of the ADC of the present invention contains a light chain variable region, wherein the light chain variable region contains the amino acid sequence shown in SEQ ID NO: 32, or an amino acid sequence with at least 80%, 85%, 90%, 95%, 96%, 97%, 98%, or 99% sequence identity to it.

In some preferred embodiments, the Ab unit of the ADC of the present invention contains a heavy chain variable region and a light chain variable region, wherein the heavy chain variable region contains the amino acid sequence shown in SEQ ID NO: 33 and the light chain variable region contains the amino acid sequence shown in SEQ ID NO: 32.

In some embodiments, the Ab unit of the ADC of the present invention preferably also contains a heavy chain constant region and/or a light chain constant region of the antibody. Preferably, the heavy chain constant region is derived from the heavy chain constant region from human immunoglobulin. Preferably, the light chain constant region is derived from the light chain constant region from human immunoglobulin. In some aspects, the constant region of the heavy chain contained in the Ab unit can be any isotype or subtype, such as the constant region of the heavy chain of IgG1, IgG2, IgG3, or IgG4 isotypes, and preferably the constant region of the heavy chain of IgG1, IgG2, or IgG4, especially the constant region of the heavy chain of human IgG1. In other aspects, the constant region of the light chain contained in Ab units can be either the constant region of the κ light chain or the constant region of the λ light chain, especially the constant region of the human κ light chain.

In some embodiments, the Ab unit of the ADC of the present invention contains a human IgG1 heavy chain constant region. Preferably, the heavy chain constant region contains the amino acid sequence of SEQ ID NO: 34 or an amino acid sequence with at least one, two, or three, but not more than 20, 10, or 5 amino acid changes relative to SEQ ID NO: 34 amino acid sequence or a sequence with at least 95-99% identity to the amino acid sequence of SEQ ID NO: 34.

An exemplary amino acid sequence of the constant region of the human IgG1 heavy chain SEQ ID NO: 34

ASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKRVEPKSCDKTHTCPPCPAPELLGGPS
VFLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSREEMTK
NQVSLTCLVKGFYPSDIAVEWESNGQPENNYKTTPPVLDSDGSFFLYSKLTVDKSRWQQGN
VFSCSVMHEALHNHYTQKSLSLSPGK

In some embodiments, the Ab unit of the ADC of the present invention contains a constant region of the human κ light chain. Preferably, the constant region of the light chain contains the amino acid sequence of SEQ ID NO: 12, or an amino acid sequence with at least one, two, or three amino acid sequences relative to SEQ ID NO: 12, but not more than 20, 10, or 5 amino acid changes or a sequence with at least 95-99% identity to the amino acid sequence of SEQ ID NO: 12.

In some embodiments, the Ab unit of the ADC of the present invention is a full-length antibody containing both the heavy chain constant region and the light chain constant region. In some embodiments, the Ab unit is a tetrameric structure consisting of two light chains and two heavy chains. In additional embodiments, the Ab unit is an IgG antibody, especially an IgG1 antibody.

In some preferred embodiments, the Ab unit of the ADC of the present invention contains both heavy and light chains, wherein the heavy chain contains the amino acid sequence shown in SEQ ID NO: 25, or an amino acid sequence with at least 80%, 85%, 90%, 95%, 96%, 97%, 98%, or 99% sequence identity to it. In other preferred embodiments, the Ab

unit of the ADC of the present invention contains both heavy and light chains, wherein the light chain contains the amino acid sequence shown in SEQ ID NO: 24 or an amino acid sequence with at least 80%, 85%, 90%, 95%, 96%, 97%, 98%, or 99% sequence identity to it.

In some more preferred embodiments, the Ab unit of the ADC of the present invention contains:

(e) a heavy chain containing the amino acid sequence of SEQ ID NO: 25, and
(f) a light chain containing the amino acid sequence of SEQ ID NO: 24.

Cancers that can be treated with the ADC of the present invention targeting Claudin18.2 include, but are not limited to, various cancers of the digestive tract, such as gastric cancer (GC), gastroesophageal junction (GEJ) cancer, esophageal cancer, and pancreatic cancer.

**Ab unit targeting EGFR**

In a particularly preferred embodiment, the antibody conjugates provided herein include antibodies specifically binding to human EGFR or antigen binding fragment (anti-EGFR antibody), i.e., the antibody moiety specifically targets the tumor-associated antigen EGFR. Thus, in some aspects, the present invention provides antibody-drug conjugates (ADCs) containing antibodies specifically binding to EGFR or antigen-binding fragments thereof as Ab units of ADCs.

Epidermal growth factor receptor, also abbreviated EGFR throughout this text, is a member of the family of epidermal growth factor receptors (HER) and is encoded by the c-erbB proto-oncogene (also known as HER-1 or Erb-B1). EGFR sequences for humans and many other mammals can be found at UniProtKB. For example, the human EGFR sequence can be found in UniProtKB accession number P00533. EGFR is overexpressed in many solid tumors, including lung cancer, head and neck cancer, breast cancer, renal cancer, gastric cancer, colon cancer, pancreatic cancer, ovarian cancer, prostate cancer, and bladder cancer; and EGFR can induce tumor proliferation through homodimerization.

EGFR has now been proposed as a promising target for antibody-based cancer therapeutics. To this date, five EGFR-targeted monoclonal antibodies have been approved for clinical cancer therapy, cetuximab (Erbitux®), panitumumab (Vectibix®), nimotuzumab (BIOMAB-EGFR®), necitumuma (Portrazza®), and amivantamab (amivantamab-vmjw; Rybrevant®). In addition, three EGFR-based ADC agents are in clinical trials. However, no EGFR-based ADC is currently approved for treatment. See Jinfeng Yu et al, Antibody-Drug Conjugates Targeting the Human Epidermal Growth Factor Receptor Family in Cancers, Front Mol Biosci. 2022; 9: 847835, doi: 10.3389/fmolb.2022.847835.

Several monoclonal antibodies against EGFR have been developed to bind to the extracellular domain of this receptor to block the interaction or dimerization between the receptor and ligand. These antibodies are suitable for the present invention. In addition, antibodies targeting human EGFR that can be used for the ADC of the present invention can also be prepared using an antibody preparation process known in the field. For example, anti-EGFR antibodies can be obtained by the following methods: immunizing animals with human EGFR (UniProtKB accession number P00533) or peptides containing the amino acid sequence of EGFR extracellular domain, harvesting antibodies from immunized animals, and purifying and preferably humanizing. In addition, complete human sequence antibodies against human EGFR can be obtained using yeast display libraries expressing human immunoglobulin sequences or transgenic animals.

Antibodies targeting human EGFR used for the ADC of the present invention can be monospecific antibodies that bind to EGFR. In some cases, antibodies targeting human EGFR for the ADC of the present invention can also be polyspecific antibodies, especially bispecific antibodies, such as those targeting EGFR and MET; or those targeting MUC1 and EGFR.

In some embodiment, the anti-human EGFR antibody or its antibody fragment (e.g. antigen binding fragment) used for the ADC of the present invention can be selected from cetuximab (Erbitux®), panitumumab (Vectibix®), and nimotuzumab (BIOMAB-EGFR®), necitumuma (Portrazza®), amivantamab (amivantamab-vmjw; Rybrevant®), depatuxizumab (ABT-806), NECITUMUMAB, IZALONTAMAB, BAFISONTAMAB, Petosemtamab, PIMURUTAMAB, FUTUXIMAB, MODOTUXIMAB (Zatuximab), or its antibody fragments, or other anti-human EGFR antibodies that recognize the same epitope or competitively bind to human EGFR. The sequences of the above antibodies can be found in Inxight Drugs and International Nonproprietary Names (INN) codes 7906, 8499, 8545, 9083, 11030, 10263, 9083, 12022, 11851, 11136, 11309, 9612, and 9613.

Cetuximab (Erbitux®) is a recombinant chimeric human/murine IgG1 monoclonal antibody. The antibody binds to the extracellular domain of non-activated EGFR with much higher affinity than endogenous ligands, competitively blocks the binding of ligand to the receptor, blocks the agonism of ligand on the receptor, and can promote endocytosis of EGFR, downregulating EGFR expression levels in the membrane. In addition, the antibody activates antibody-dependent cellular cytotoxicity (ADCC), resulting in further cell killing. The sequence of cetuximab can be found in WO2007092453 and INN code 7906.

Panitumumab (Vectibix®) is a recombinant humanized IgG2 monoclonal antibody. Panitumumab binds specifically to EGFR on tumor cells and competitively inhibits the binding of EGFR ligands. Preclinical studies have shown that

binding of panitumumab to EGFR prevents ligand-induced receptor autophosphorylation and activation of receptor-associated kinases, thereby inhibiting cell growth, inducing apoptosis, reducing the production of pro-inflammatory cytokines and vascular growth factor, and inducing EGFR internalization. The sequence of panitumumab can be found in INN code 8499.

Amivantamab (aka JNJ-61186372) is an anti-EGFR-MET bispecific antibody derived from Chinese hamster ovary cells and approved for the treatment of adult patients with locally advanced or metastatic non-small cell lung cancer (NSCLC) with epidermal growth. The antibody binds EGFR and MET and blocks the binding of these receptors to their ligands and induces internalization of EGFR and MET on the cell surface. Amivantamab can significantly downregulate the expression of EGFR and MET on the surface of NSCLC cells, further reducing downstream signaling. The sequence of Amivantamab can be found in INN code 9083.

An exemplary antibody targeting human EGFR that can be used for the ADC of the present invention can be an antibody or antigen-binding fragment containing all 6 CDR sequences (preferably containing the heavy chain variable region and light chain variable region sequences of the antibodies selected from the below group) of the antibodies selected from the below group: cetuximab, panitumumab, nimotuzumab necitumuma, amivantamab, depatuxizumab, NECITUMUMAB, IZALONTAMAB, BAFISONTAMAB, Petosemtamab, PIMURUTAMAB, FUTUXIMAB and MODOTUXIMAB.

The present invention also considers the anti-human EGFR antibodies disclosed in the following literature: WO2023040941A1, WO2022271722A1, WO2022159576A1, WO2022128716A1, WO2022105878A1, WO2021247798A1, WO2022104697A1, WO2021066869A1, WO2020233534A1, WO2020130125A1, WO2019046858A1, WO2019046859A1, WO2019035630A2, WO2019035630A3, WO2018098035A1, WO2017214233A1, WO2017214282A1, WO2017214301A1, WO2017161206A1, WO2017139623A1, WO2017136581A1, WO2017076492A1, WO2017060322A3, WO2017025458A1, WO2017008169A1, WO2016065456A1, WO2015143382A1, WO2014143765A1, WO2014143765A8, WO2014152199A1, WO2014094355A1, WO2012143495A2, and WO2012143495A3.

> Cetuximab Light Chain Amino Acid Sequence (SEQ ID NO: 35)

DILLTQSPVILSVSPGERVSFSCRASQSIGTNIHWYQQRTNGSPRLLIKYASESISGIPSRFSGSG
SGTDFTLSINSVESEDIADYYCQQNNNWPTTFGAGTKLELK
RTVAAPSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC

> Cetuximab Heavy Chain Amino Acid Sequence (SEQ ID NO: 36)

QVQLKQSGPGLVQPSQSLSITCTVSGFSLTNYGVHWVRQSPGKGLEWLGVIWSGGNTDYN
TPFTSRLSINKDNSKSQVFFKMNSLQSNDTAIYYCARALTYYDYEFAYWGQGTLVTVSA
ASTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTVDKSRWQQGNVF
SCSVMHEALHNHYTQKSLSLSPGK

> Cetuximab Light Chain LCDR1 Sequence (SEQ ID NO: 37)
RASQSIGTNIH

> Cetuximab Light Chain LCDR2 Sequence (SEQ ID NO: 38)
YASESIS

> Cetuximab Light Chain LCDR3 Sequence (SEQ ID NO: 39)
QQNNNWPTT

> Cetuximab Heavy Chain HCDR1 Sequence (SEQ ID NO: 40)

NYGVH

> Cetuximab Heavy Chain HCDR2 Sequence (SEQ ID NO: 41)
VIWSGGNTDYNTPFTS

> Cetuximab Heavy Chain HCDR3 Sequence (SEQ ID NO: 42)
ALTYYDYEFAY

> Cetuximab Light Chain Variable Region VL (SEQ ID NO: 43)

DILLTQSPVILSVSPGERVSFSCRASQSIGTNIHWYQQRTNGSPRLLIKYASESISGIPSRFSGSG
SGTDFTLSINSVESEDIADYYCQQNNNWPTTFGAGTKLELK

> Cetuximab Heavy Chain Variable Region VH (SEQ ID NO: 44)

QVQLKQSGPGLVQPSQSLSITCTVSGFSLTNYGVHWVRQSPGKGLEWLGVIWSGGNTDYN
TPFTSRLSINKDNSKSQVFFKMNSLQSNDTAIYYCARALTYYDYEFAYWGQGTLVTVSA

In one embodiment, the antibody portion used for the ADC of the present invention contains all 6 CDR sequences of cetuximab. In another embodiment, the antibody portion used for the ADC of the present invention contains a heavy chain variable region sequence and a light chain variable region sequence of cetuximab. In a further embodiment, the antibody portion used for the ADC of the present invention contains a heavy chain sequence and a light chain sequence of cetuximab.

In some embodiments, the Ab unit of the ADC of the present invention contains 3 CDRs of the heavy chain variable region (VH) sequence of SEQ ID NO: 44 and 3 CDRs of the light chain variable region (VL) sequence of SEQ ID NO: 43, and preferably, wherein the CDRs are defined by Kabat or IMGT or a combination thereof.

In some embodiments, the Ab unit of the ADC of the present invention contains 3 heavy chain complementarity determining regions (HCDR) and 3 light chain complementarity determining regions (LCDR), wherein:
As defined by Kabat, HCDR1 contains or consists of the amino acid sequence of SEQ ID NO: 40, HCDR2 contains or consists of the amino acid sequence of SEQ ID NO: 41, HCDR3 contains or consists of the amino acid sequence of SEQ ID NO: 42, LCDR1 contains or consists of the amino acid sequence of SEQ ID NO: 37, LCDR2 contains or consists of the amino acid sequence of SEQ ID NO: 38, and LCDR3 contains or consists of the amino acid sequence of SEQ ID NO: 39.

In one embodiment, the Ab unit of the ADC of the present invention contains a heavy chain variable region, wherein the heavy chain variable region contains the amino acid sequence shown in SEQ ID NO: 44, or an amino acid sequence with at least 80%, 85%, 90%, 95%, 96%, 97%, 98%, or 99% sequence identity to it.

In one embodiment, the Ab unit of the ADC of the present invention contains a light chain variable region, wherein the light chain variable region contains the amino acid sequences shown in SEQ ID NO: 43, or an amino acid sequence with at least 80%, 85%, 90%, 95%, 96%, 97%, 98%, or 99% sequence identity to it.

In some preferred embodiments, the Ab unit of the ADC of the present invention contains a heavy chain variable region and a light chain variable region, wherein the heavy chain variable region contains the amino acid sequence shown in SEQ ID NO: 44 and the light chain variable region contains the amino acid sequence shown in SEQ ID NO: 43.

In some embodiments, the Ab unit of the ADC of the present invention preferably also contains a heavy chain constant region and/or a light chain constant region of the antibody. Preferably, the heavy chain constant region is derived from the heavy chain constant region of human immunoglobulin. Preferably, the light chain constant region is derived from the light chain of human immunoglobulin. In some aspects, the constant region of the heavy chain contained in the Ab unit can be any isotype or subtype, such as the constant region of the heavy chain of IgG1, IgG2, IgG3, or IgG4 isotypes, and preferably the constant region of the heavy chain of IgG1, IgG2, or IgG4, especially the constant region of the heavy chain of human IgG1. In other aspects, the constant region of the light chain contained in Ab unit can be either the constant region of the κ light chain or the constant region of the λ light chain, especially the constant region of the human κ light chain.

In some embodiments, the Ab unit of the ADC of the present invention contains a human IgG1 heavy chain constant region. Preferably, the heavy chain constant region contains the amino acid sequence of SEQ ID NO: 34 or an amino acid sequence with at least one, two, or three, but not more than 20, 10, or 5 amino acid changes relative to the amino acid sequence of SEQ ID NO: 34 or a sequence with at least 95-99% identity to the amino acid sequence of SEQ ID NO: 34.

In some embodiments, the Ab unit of the ADC of the present invention contains a constant region of the human κ light chain. Preferably, the constant region of the light chain contains the amino acid sequence of SEQ ID NO: 12, or an amino acid sequence with at least one, two, or three, but not more than 20, 10 or 5 amino acid changes relative to the amino acid sequence of SEQ ID NO: 12 or a sequence with at least 95-99% identity to the amino acid sequence of SEQ ID NO: 12.

In some embodiments, the Ab unit of the ADC of the present invention is a full-length antibody containing both the heavy chain constant region and the light chain constant region. In some embodiments, the Ab unit is a tetrameric structure consisting of two light chains and two heavy chains. In further embodiments, the Ab unit is an IgG antibody, especially an IgG1 antibody.

In some preferred embodiments, the Ab unit of the ADC of the present invention contains both heavy and light chains, wherein the heavy chain contains the amino acid sequence shown in SEQ ID NO: 36 or an amino acid sequence with at least 80%, 85%, 90%, 95%, 96%, 97%, 98%, or 99% sequence identity to it, or is composed of it. In other preferred embodiments, the Ab unit of the ADC of the present invention contains both heavy and light chains, where the light chain contains the amino acid sequence shown in SEQ ID NO: 35 or an amino acid sequence with at least 80%, 85%, 90%, 95%, 96%, 97%, 98%, or 99% sequence identity to it.

In some more preferred embodiments, the Ab unit of the ADC of the present invention contains:

(g) a heavy chain containing the amino acid sequence of SEQ ID NO: 36, and

(h) a light chain containing the amino acid sequence of SEQ ID NO: 35.

Cancers that can be treated with the ADC of the present disclosuretargeting EGFR include, but are not limited to, a variety of primary and metastatic solid tumors, such as lung cancer (such as lung adenocarcinoma, lung squamous cell carcinoma, and non-small cell lung cancer), head and neck cancer (such as head and neck squamous cell carcinoma), nasopharyngeal carcinoma, esophageal cancer, biliary tract cancer, colon cancer, colorectal cancer, pancreatic cancer, gastric cancer, and glioblastoma.

<u>**Drug D Unit**</u>

The drug D unit of the antibody-drug conjugate is also called the payload of ADC drugs in this text. Drug D that can be used for the ADC of the present invention is a bifunctional cyclic dinucleotide (CDN) having the following formula:

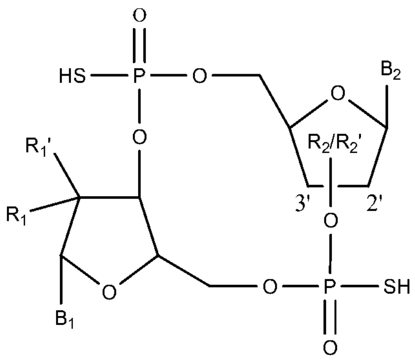

(II)

wherein

B $_1$ is adenine

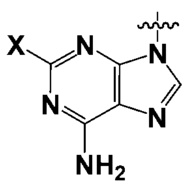

optionally substituted with X, wherein X is selected from an H, Cl, F or -NHC$_{1-6}$ alkyl; or guanine

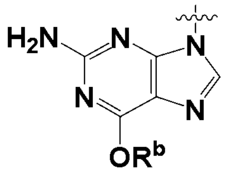

optionally substituted with $R^b$, wherein $R^b$ is selected from H or $-C_{1-6}$ alkyl;
$R_1$ and $R_1$' are independently selected from H, F or -OH, respectively;
$B_2$ is guanine

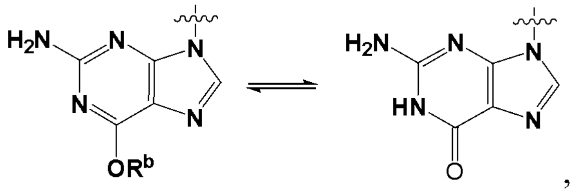

,

wherein $R^b$ is selected from H or $-C_{1-6}$ alkyl;

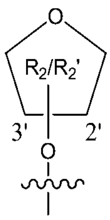

represents that the phosphate bond can be attached to either the 2' or 3' position of the pentose, where the site not looped to the phosphate is substituted with $R_2$ and $R_2$'; and
$R_2$ and $R_2$' are each independently selected from H, -OH or F;
stereoisomers, tautomers, stable isotopic variants, pharmaceutically acceptable salts, prodrugs or solvates thereof.

It should be noted that when CDN (D) of Formula (II) is covalently bonded to the linker unit (L) in the ADC of Formula (X), it is bonded by any of the following sites:

(1) Any -SH; or
(2) Amino group on the base of $B_1$ or $B_2$; or
(3) -OH on sugar ring.

In a preferred embodiment, CDN (D) of Formula (II) is covalently bonded via any -SH to the linker unit (L) in ADC of Formula (X).

In a specific embodiment, two phosphorothioate bonds in the compound of the present invention exist in R,R, S,S, S,R or R,S configurations or mixtures thereof. In the preferred embodiment, two phosphorothioate bonds in the compound of the present invention exist in R,R, S,S, S,R or R,S configurations in essentially pure form, particularly preferably exist in R,R configuration that is essentially pure.

In an embodiment of a Formula (II) compound, $B_1$ is an adenine

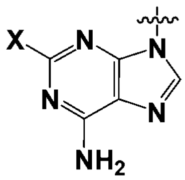

optionally substituted with X, wherein X is selected from H.

In an embodiment of a Formula (II) compound, $B_1$ is an adenine

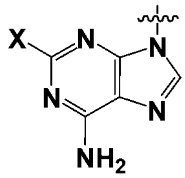

optionally substituted with X, wherein X is selected from Cl or F.

In an embodiment of a Formula (II) compound, $B_1$ is adenine

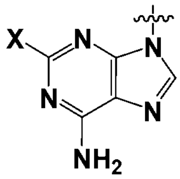

optionally substituted with X, wherein X is $-NHC_{1-6}$ alkyl, e.g. $-NHCH_3$, $-NHCH_2CH_3$, $-NHCH_2CH_2CH_3$, preferably $-NHCH_3$.

In an embodiment of a Formula (II) compound, $B_1$ is guanine

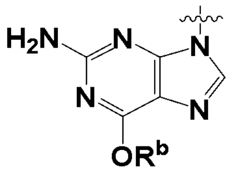

optionally substituted with $R^b$, wherein $R^b$ is selected from H or $-C_{1-6}$ alkyl, for example, $R^b$ is $-CH_3$, $-CH_2CH_3$, $-CH_2CH_2CH_3$, preferably $-CH_3$.

In an embodiment of a Formula (II) compound, both $R_1$ and $R_1$' are H, or one is H and the other is F, or one is H and the other is OH, or both are F.

In an embodiment of a Formula (II) compound, $B_2$ is guanine

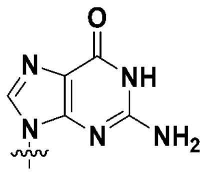

.

In an embodiment of a Formula (II) compound, both $R_2$ and $R_2$' are H, or one is H and the other is F, or one is H and the other is OH, or both are F; preferably one is H and the other is OH.

In an embodiment of a Formula (II) compound, $B_1$ is adenine

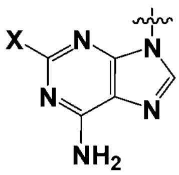

,

wherein X is selected from H, Cl, F or $-NHC_{1-6}$ alkyl; or guanine

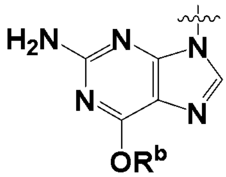

,

wherein $R^b$ is $-C_{1-6}$ alkyl; $B_2$ is guanine

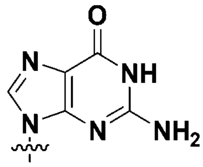

;

$R_1$ and $R_1$' are both H, or one is H and the other is F, or one is H and the other is OH; one of $R_2$ and $R_2$' is H and the other is OH.

The compound of Formula (II) of the present invention also covers any combination between each of the embodiments and their preferred or exemplary means.

In a further specific embodiment, Drug D in the ADC of the present invention is a bifunctional cyclic dinucleotide (CDN) having the following formula:

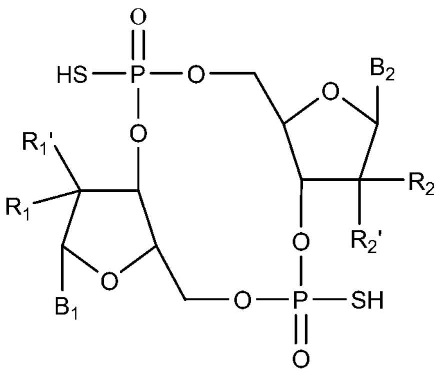

(II-a)

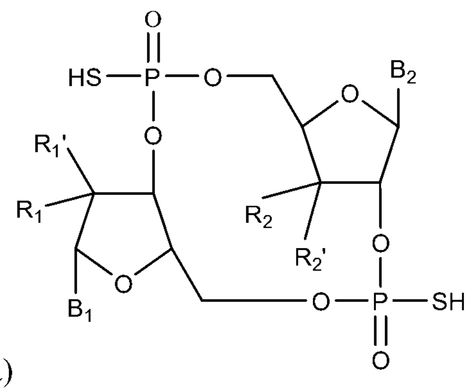

(II-b)

Wherein $B_1$, $B_2$, $R_1$, $R_1$', $R_2$ and $R_2$' have the meanings defined above for the compound of Formula (II) and each specific embodiment.

In an embodiment of a compound of Formula (II-a) or Formula (II-b), $B_1$ is adenine

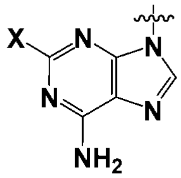

optionally substituted with X, wherein X is selected from H, Cl, F or $-NHC_{1-6}$ alkyl, or guanine

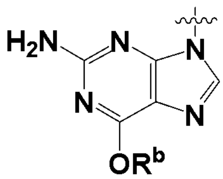

optionally substituted with $R^b$, wherein $R^b$ is $-C_{1-6}$ alkyl; for example

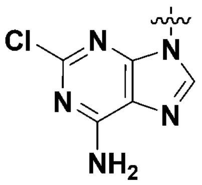, 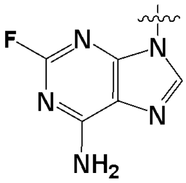, 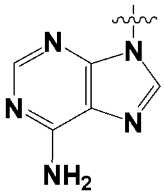, 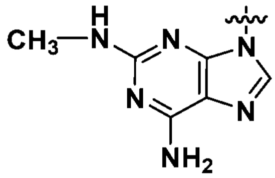 and 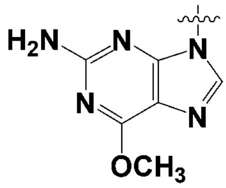.

In this specific embodiment, both $R_1$ and $R_1$' are H, or one is H and the other is F, or one is H and the other is OH; in this specific embodiment, F or OH and $B_1$ are located on the same side of the ribose.

In an embodiment of a compound of Formula (II-a) or Formula (II-b), $B_2$ is guanine

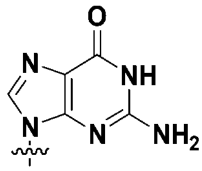.

In this specific embodiment, both $R_2$ and $R_2$' are H, or one is H, the other is selected from -OH or F, preferably one is H and the other is -OH; in this specific embodiment, F or OH and $B_2$ are located on the other side of the ribose.

In an embodiment of a compound of Formula (II-a) or Formula (II-b), $B_1$ is adenine

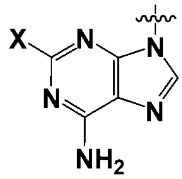

optionally substituted with X, wherein X is selected from H, Cl, F or -NHC$_{1-6}$ alkyl, or guanine

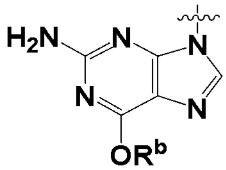

optionally substituted with $R^b$, wherein $R^b$ is -C$_{1-6}$ alkyl; $B_2$ is guanine

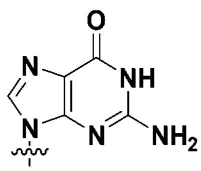;

$R_1$ and $R_1$' are both H, or one is H and the other is F, or one is H and the other is OH; $R_2$ and $R_2$' are both H, or one is H and the other is OH, preferably one is H and the other is -OH.

In an embodiment of a compound of Formula (II-a), $B_1$ is

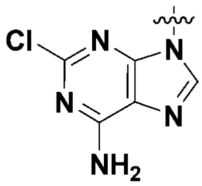,

and $B_2$ is

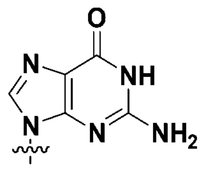

;

in a specific embodiment, both $R_1$ and $R_1$' are H; in another specific embodiment, one of $R_1$ and $R_1$' is H and the other is F. Further, in each specific embodiment described, $R_2$ and $R_2$ 'are both H, or one is H and the other is OH, preferably one is H and the other is -OH.

In an embodiment of a compound of Formula (II-a), $B_1$ is

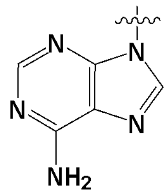

,

and $B_2$ is

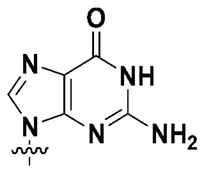

;

in a specific embodiment, both $R_1$ and $R_1$' are H; in another specific embodiment, one of $R_1$ and $R_1$' is H and the other is F. Further, in each specific embodiment described, $R_2$ and $R_2$' are both H, or one is H and the other is OH, preferably one is H and the other is -OH.

In an embodiment of a compound of Formula (II-b), $B_1$ is

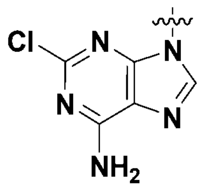

,

and $B_2$ is

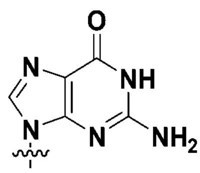

;

in a specific embodiment, both $R_1$ and $R_1$' are H; in another specific embodiment, one of $R_1$ and $R_1$' is H and the other is F; in another specific embodiment, one of $R_1$ and $R_1$' is H and the other is OH. Further, in each specific embodiment described, $R_2$ and $R_2$' are both H, or one is H and the other is OH, preferably one is H and the other is -OH.

In an embodiment of a compound of Formula (II-b), $B_1$ is

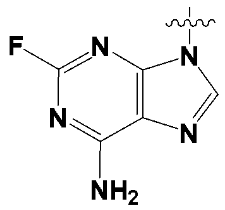

,

and $B_2$ is

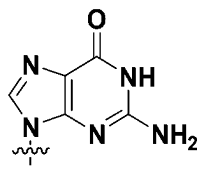

;

in a specific embodiment, both $R_1$ and $R_1$' are H; in another specific embodiment, one of $R_1$ and $R_1$' is H and the other is F; and in another specific embodiment, one of $R_1$ and $R_1$' is H and the other is OH. Further, in each specific embodiment example described, $R_2$ and $R_2$' are both H, or one is H and the other is OH, preferably one is H and the other is -OH.

In an embodiment of a compound of Formula (II-b), $B_1$ is

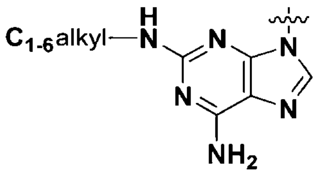

,

preferably

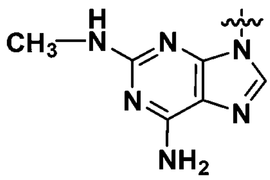

,

and $B_2$ is

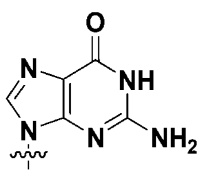

;

in a specific embodiment, both $R_1$ and $R_1$' are H; in another specific embodiment, one of $R_1$ and $R_1$' is H and the other is F; in another specific embodiment, one of $R_1$ and $R_1$' is H and the other is OH. Further, in each specific embodiment described, $R_2$ and $R_2$' are both H, or one is H and the other is OH, preferably one is H and the other is -OH.

In an embodiment of a compound of Formula (II-b), $B_1$ is

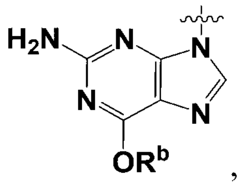

,

wherein $R^b$ is $-C_{1-6}$ alkyl, e.g. $-CH_3$, $-CH_2CH_3$, $-CH_2CH_2CH_3$, preferably $-CH_3$; and $B_2$ is

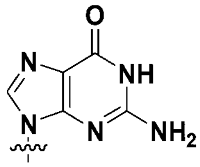

;

in a specific embodiment, both $R_1$ and $R_1$' are H; in another specific embodiment, one of $R_1$ and $R_1$' is H and the other is F; in another specific embodiment, one of $R_1$ and $R_1$' is H and the other is OH. Further, in each specific embodiment described, $R_2$ and $R_2$' are both H, or one is H and the other is OH, preferably one is H and the other is -OH.

In an embodiment of a compound of Formula (II-b), $B_1$ is

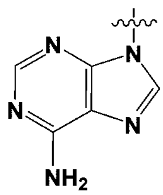

,

and $B_2$ is

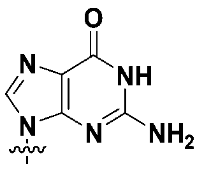

;

in a specific embodiment, both $R_1$ and $R_1$' are H; in another specific embodiment, one of $R_1$ and $R_1$' is H and the other is F; and in another specific embodiment, one of $R_1$ and $R_1$' is H and the other is OH. Further, in each specific embodiment described, $R_2$ and $R_2$' are both H, or one is H and the other is OH, preferably one is H and the other is -OH.

In the above mentioned embodiments of compounds of Formula (II-a) or Formula (II-b), the two phosphorothioate bonds exist in R,R, S,S, S,R or R,S configurations or mixtures thereof; preferably exist in R,R, S,S, S,R or R,S configurations in essentially pure form, particularly exist in R,R configuration that is essentially pure.

The compound of Formula (II-a)/(II-b) compounds of the present invention also cover any combination between each of the above embodiments and their preferred or exemplary means.

In a further specific embodiment, Drug D in ADC of the present invention is a CDN having the following sub-general formula,

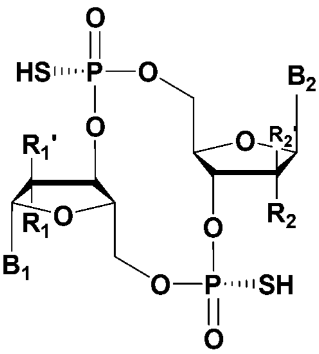
(II-a’)

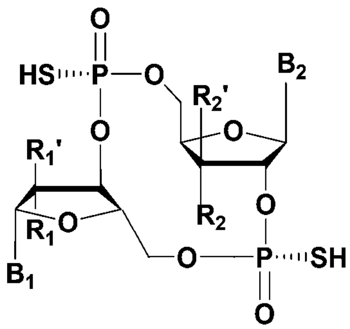
(II-b’)

wherein $B_1$, $B_2$, $R_1$, $R_1$’, $R_2$, $R_2$’ have the meanings defined above for the compound of Formula (II) or each of its specific embodiments; and more specifically, for the compound of Formula (II-a)/(II-b) or each of its specific embodiments.

In an embodiment of a compound of Formula (II-a’) or Formula (II-b’), $B_1$ is adenine

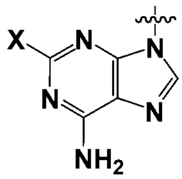

optionally substituted with X, wherein X is selected from H, Cl, F or $-NHC_{1-6}$ alkyl, or guanine

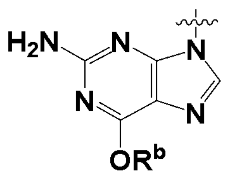

optionally substituted with $R^b$, wherein $R^b$ is selected from a $-C_{1-6}$ alkyl; examples include

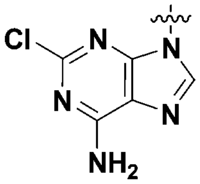
,
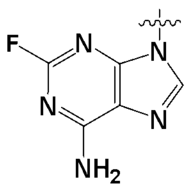
,
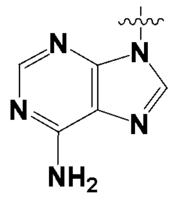
,
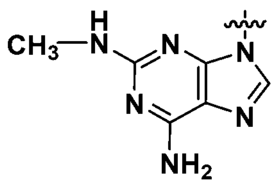

and

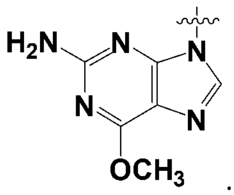
.

In this specific embodiment, both $R_1$ and $R_1$’ are H, or wherein $R_1$’ is H and $R_1$ is F, or $R_1$’ is H and $R_1$ is OH.

In an embodiment of a compound of Formula (II-a’) or Formula (II-b’), when $B_1$ is

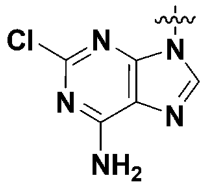

and both $R_1$ and $R_1$' are H, this $B_1$ forms the nucleoside antineoplastic cladribine together with its attached ribose; or when $R_1$ is F and $R_1$' is H, that is, F and $B_1$ are located on the same side of the ribose, i.e. this $B_1$, together with its attached ribose, thus forms the nucleoside antineoplastic clofarabine.

In an embodiment of a compound of Formula (II-a') or Formula (II-b'), when $B_1$ is

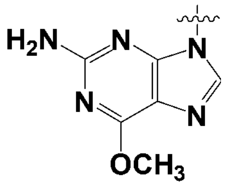

and $R_1$ is OH, and $R_1$' is H, this $B_1$ forms the nucleoside antineoplastic nelarabine together with its attached ribose.

In an embodiment of a compound of Formula (II-a') or Formula (II-b'), when $B_1$ is

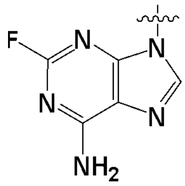

and $R_1$ is OH, and $R_1$' is H, this $B_1$ together with its attached ribose forms a nucleoside antineoplastic Fludarabine.

In an embodiment of a compound with Formula (II-a') or Formula (II-b'), $B_2$ is guanine

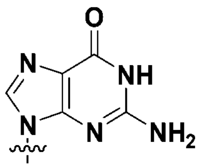

,

$R_2$ and $R_2$' are both H, or $R_2$' is H and $R_2$ is selected from -OH or F, preferably $R_2$' is H and $R_2$ is -OH.

In an embodiment of a compound of Formula (II-a') or Formula (II-b'), $B_1$ is adenine

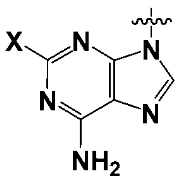

optionally substituted with X, wherein X is selected from H, Cl, F, or -NHC$_{1-6}$ alkyl; or guanine

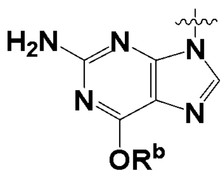

optionally substituted with $R^b$, wherein $R^b$ is selected from -$C_{1-6}$ alkyl; $B_2$ is guanine

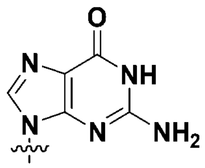

;

$R_1$ and $R_1$' are both H, or wherein $R_1$' is H and $R_1$ is F, or wherein $R_1$' is H and $R_1$ is OH; $R_2$ and $R_2$' are both H, or wherein $R_2$' is H and $R_2$ is OH, preferably $R_2$' is H and $R_2$ is OH.

In an embodiment of a compound of Formula (II-a'), $B_1$ is

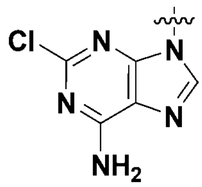

,

and $B_2$ is

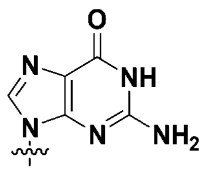

;

in a specific embodiment, both $R_1$ and $R_1$' are H; in another specific embodiment, $R_1$ is F and $R_1$' is H. Further, in each specific example described, $R_2$ and $R_2$' are both H, or $R_2$' is H and $R_2$ is OH, preferably $R_2$' is H and $R_2$ is OH.

In an embodiment of a compound of Formula (II-a'), $B_1$ is

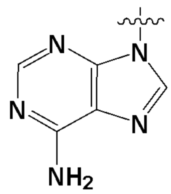

,

and $B_2$ is

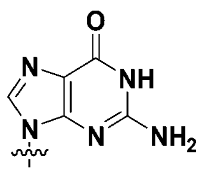

;

in a specific embodiment, both $R_1$ and $R_1$' are H; in another specific embodiment, $R_1$ is F and $R_1$' is H. Further, in each specific embodiment described, $R_2$ and $R_2$' are both H, or $R_2$' is H and $R_2$ is OH, preferably $R_2$' is H and $R_2$ is OH.

In an embodiment of a compound of Formula (II-b'), $B_1$ is

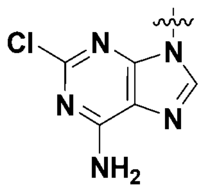

and $B_2$ is

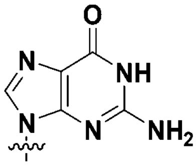

;

in a specific embodiment, both $R_1$ and $R_1$' are H; in another specific embodiment, $R_1$ is F and $R_1$' is H; and in another specific embodiment, $R_1$ is OH and $R_1$' is H. Further, in each specific embodiment described, $R_2$ and $R_2$' are both H, or $R_2$ is OH and $R_2$' is H, preferably $R_2$ is OH and $R_2$' is H.

In an embodiment of a compound of Formula (II-b'), $B_1$ is

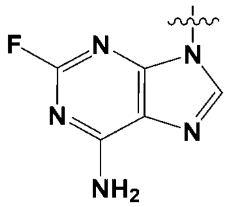

,

and $B_2$ is

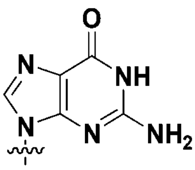

;

in a specific embodiment, both $R_1$ and $R_1$' are H; in another specific embodiment, $R_1$ is F and $R_1$' is H; and in another specific embodiment, $R_1$ is OH and $R_1$' is H. Further, in each specific embodiment described, $R_2$ and $R_2$' are both H, or $R_2$ is OH and $R_2$' is H, preferably $R_2$ is OH and $R_2$' is H.

In an embodiment of a compound of Formula (II-b'), $B_1$ is

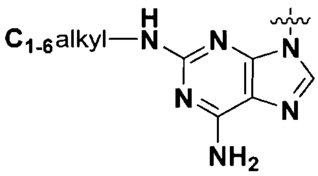

,

preferably

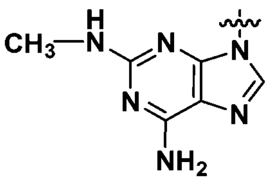

,

and $B_2$ is

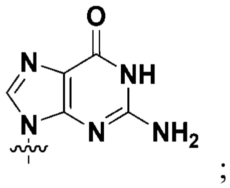

in a specific embodiment, both $R_1$ and $R_1$' are H; in another specific embodiment, both $R_1$ and $R_1$' are H; and in another specific embodiment, $R_1$ is F and $R_1$' is H; in another specific embodiment, $R_1$ is OH and $R_1$' is H. Further, in each specific embodiment described, $R_2$ and $R_2$' are both H, or $R_2$ is OH and $R_2$' is H, preferably $R_2$ is OH and $R_2$' is H.

In an embodiment of a compound of Formula (II-b'), $B_1$ is

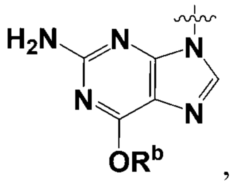

,

wherein $R^b$ is $-C_{1-6}$ alkyl, e.g., $-CH_3$, $-CH_2CH_3$, $-CH_2CH_2CH_3$, preferably $-CH_3$; and $B_2$ is

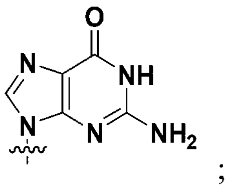

in this specific embodiment, both $R_1$ and $R_1$' are H; in another specific embodiment, $R_1$ is F and $R_1$' is H; in another specific embodiment, $R_1$ is OH and $R_1$' is H. Further, in each specific embodiment described, $R_2$ and $R_2$' are both H, or $R_2$ is OH and $R_2$' is H, preferably $R_2$ is OH and $R_2$' is H.

In an embodiment of a compound of Formula (II-b'), $B_1$ is

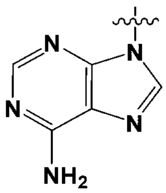

,

and $B_2$ is

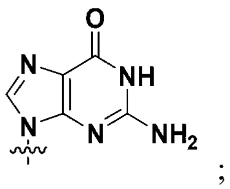

in a specific embodiment, both $R_1$ and $R_1$' are H; in another specific embodiment, $R_1$ is F and $R_1$' is H; and in another specific embodiment, $R_1$ is OH and $R_1$' is H. Further, in each specific embodiment described, $R_2$ and $R_2$' are both H, or $R_2$ is OH and $R_2$' is H, preferably $R_2$ is OH and $R_2$' is H.

It should be mentioned that Part D of the drug in the antibody-drug conjugate of the present invention covers each of the above specific embodiments, and also covers the embodiments composed of any combination or subcombination of each of the above specific embodiments, and also covers the embodiments composed of any combination of any of the above preferred or exemplified.

It should be noted that when CDN (D) of Formula (II), (II-a), (II-b), (II-a'), (II-b') is covalently bonded to a linker unit

(L) in the ADC of Formula (X), it is bonded through any of the following sites of the CDN:

(1) Any -SH; or

(2) Amino group on the base of $B_1$ or $B_2$; or

(3) OH on sugar ring.

In the preferred embodiment, the CDN (D) of each of the above formula is covalently bonded to the linker unit (L) in the ADC of Formula (X) via the free thiol group of either phosphate ester.

Part D of the drug in the antibody-drug conjugate of the present invention preferably comprises the following compounds, stereoisomers, tautomers, stable isotopic variants, pharmaceutically acceptable salts or solvates thereof,

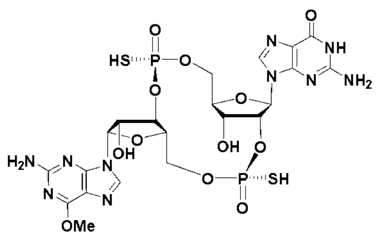

CDN-1

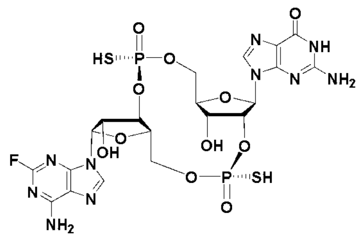

CDN-2

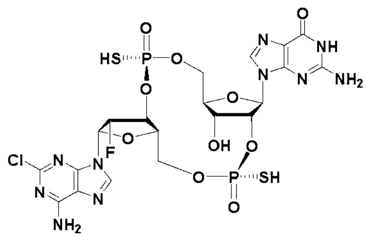

CDN-3

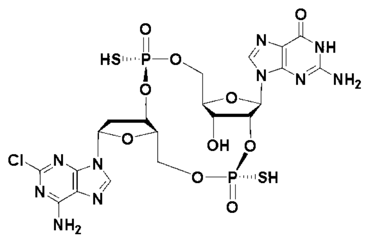

CDN-4

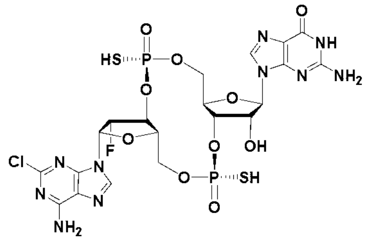

CDN-5

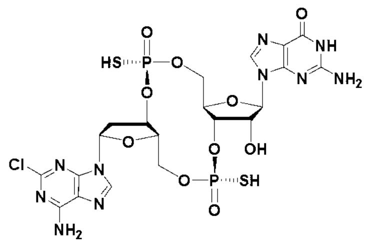

CDN-6

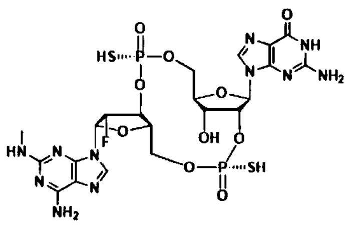

CDN-8

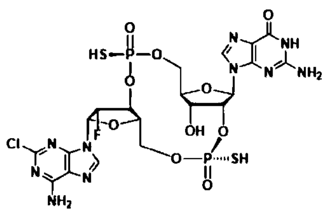

CDN-7

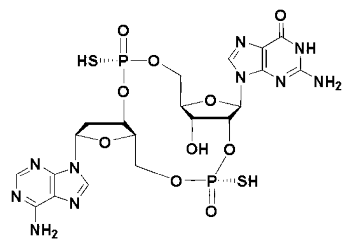

CDN-9

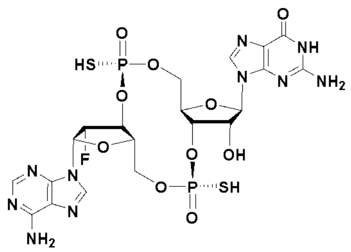

CDN-10

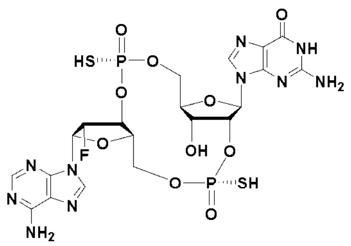

CDN-11

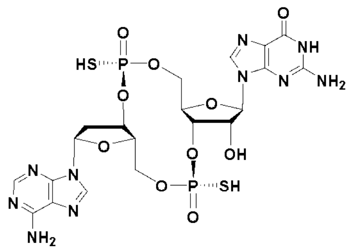

CDN-12

It should be mentioned that the present invention covers ADC compounds formed by any of the above general, specific, or preferred CDN compounds with linker units and antibody drugs as defined herein.

## Linker unit L

In the ADCs described herein, CDNs are linked to antibodies or antigen-binding fragments thereof via linker units. The linker unit links the CDN to an antibody or antigen-binding fragment by forming a covalent bond with the CDN at one of its positions and a covalent bond with the antibody or antigen-binding fragment at the other. Linker units can be univalent relative to CDNs, such that they covalently link a single CDN to a single site on an antibody or its fragment, or multivalent relative to a CDN, such that they covalently link more than one CDN to a single site on an antibody or its fragment. As used herein, the expression "linker unit" is intended to include unconjugated, partially conjugated (i.e., conjugated only to CDN or only to Ab), and fully conjugated forms (i.e., conjugated to CDN and Ab) of linker units.

The number of CDNs linked to an antibody or antigen-binding fragment thereof to an ADC can vary (called the "drug-antibody ratio" or "DAR") and will be limited by the number of available attachment sites on the antibody or antigen-binding fragment thereof and the number of CDNs linked to a single linker. In ADCs containing multiple CDNs, each CDN may be the same or different. As long as CDNs do not exhibit unacceptable aggregation levels under usage and/or storage conditions, ADCs with DARs of 10 or even higher may be considered. In some embodiments, ADCs described herein can have DARs in the range of about 1 to 10, 1 to 9, 1 to 8, 1 to 7, 1 to 6, 1 to 5, or 1 to 4. In some embodiments, ADCs described herein can have DARs in the range of about 2 to 10, 2 to 9, 2 to 8, 2 to 7, 2 to 6, 2 to 5, or 2 to 4. In some specific embodiments, the DAR of ADC can be about 1, 2, 3, or 4. In other specific embodiments, the DARs of ADCs can be about 5, 6, 7, or 8. In some specific embodiments, the DAR of ADC can be about 1.

A linker unit L applicable to the ADC of the present invention may be any linker capable of conjugating the drug of the present invention to an antibody. Appropriately, the addition of the linker shall ensure sufficient stability of the ADC of the present invention in the circulatory system, shall not trigger off-target toxicity by premature cleavage in the circulation, and can provide rapid and effective CDN release at target sites (e.g., tumor cells or tumor environment). For example, linker units can be chemically stable to the extracellular environment and serum or can include intentionally unstable linker units and can release CDNs in the extracellular environment or tumor microenvironment.

Ideal Linker Units

In some embodiments, linker units include bonds designed to release CDNs following intracellular ADC internalization. In some specific embodiments, linker units include bonds designed to cleave and/or digest within cells or otherwise degrade specifically or non-specifically.

In some embodiments, the linker unit in the ADC of Formula (X) of the present invention is a non-degradable linker. Examples of non-degradable linker units include, but are not limited to, thioether linkers, N-succinimidyl-4-(N-maleimide methyl) cyclohexane-1-carboxylate (SMCC), and maleimide hexanoyl (MC). Typically, such linkers are more stable, and ADCs containing such linkers must be internalized by the cell, where antibody moieties of ADCs are degraded by intracellular lysosomal proteases to release drug active molecules.

In a further embodiment, the linker unit in the ADC of Formula (X) of the present invention is a degradable linker unit that contains one or more chemically or enzymatically degradable chemical bonds. Drug release from ADCs containing such linkers is triggered by the nature of the cleavage site in the linker. Therefore, cleavage sites for such linkers can be designed based on the characteristics of target treatment sites (e.g., tumor cell lysosomes and/or tumor environment).

In some embodiments, degradable linker units contain chemically unstable groups that take advantage of the differential properties between plasma and some cytoplasmic compartments, such as the acidic environment of endosomes or lysosomes or high thiol concentrations in the cytosol (e.g., glutathione); in some cases, the plasma stability of linkers containing chemically unstable groups can be increased or decreased by changing the steric hindrance near the group using substituents.

In some embodiments, chemically unstable moieties of degradable linker units are acid-unstable groups that remain intact during the neutral pH cycle of blood and hydrolyze to release CDNs under acidic conditions, such as in acidic tumor environments or when internalized into endosomal (pH 5.0-6.5) and lysosomal (pH 4.5-5.0) cellular compartments, this pH-dependent release mechanism can be optimized by chemical modifications to finely modulate CDN release against specific pH. Examples of such acid unstable groups include hydrazone, hydrazine, acetal, orthoester or imine groups.

In some embodiments, degradable linker units contain reducible groups such as disulfide groups. This group is reduced at the time ADC is internalized into the cell, as the cytosol in the cell provides a more reductive environment (e.g., reduced glutathione), thereby releasing the drug. Tumor cells can induce hypoxia due to irregular blood flow, resulting in enhanced reductase activity, as well as increased concentrations of glutathione, which facilitates the selective release of drugs from disulfide-bond-containing linkers in tumor cells.

In some embodiments, degradable linker units are enzymatic degradable linker units and are more stable in plasma and extracellular environments than chemically unstable linker groups. Such linkages can be based on peptides or

include peptide regions, or non-peptide linkages such as peptide mimics, sugars, esters, and amides. Such linkages can be cleaved by tumor-specific enzymes, such as tumor-specific proteases with increased abundance in tumors and/or tumor environments, including but not limited to, lysosomal proteases, such as cathepsins (e.g., cathepsin B), legumain, MMP-2/9, plasmin, esterases, or amidases.

Generally, an enzyme-degradable linker unit may consist of a self-immolative linker, a cleavable linker, an optional property regulator unit, and an antibody linker part. The autolysis linker connects drug D to the cleavable linker and promotes the release of the rest of the self-conjugate of the drug active molecule, such as p-aminobenzyl, p-hydroxybenzyl, p-aminobenzyloxyacyl, p-hydroxybenzyloxyacyl, etc.; the cleavable linker will contain peptides or peptide analogues, esters (e.g., carbamates, sulfates), amides, disulfide-containing parts, sugars, etc. that can be recognized by the enzyme under the enzyme-based release mechanism; the addition of the property regulatory unit may be beneficial in improving the properties of the ADC, such as stability in the blood circulation, efficacy of the ADC at the target site, and optimizing the hydrophilicity of the ADC, for example, when the drug is highly hydrophobic, it can be considered (but not necessary) to add PEG units to optimize the hydrophilicity of the ADC, such as reducing precipitation and aggregation; the antibody linker is to connect the antibody or antigen-binding fragment thereof of the target antigen to the rest of the conjugate, which has the functional group that can form bonds with the functional groups on the antibody.

The linker unit L in the ADC of Formula (X) of the present invention has the following Formula (III) with the structure:

-Z-A-P-Y-M- (III),

wherein

M is a self-immolative linker that is linked to drug D;

Y is a cleavable linker selected from peptides of 2-8 amino acids preferably dipeptide, tripeptide or tetrapeptide; disulfide; ester; amide; and sugar;

P is a property regulating unit, which is absent or selected from polyethylene glycol (PEG), hydrophilic peptide, cyclodextrin unit, polyamine, polyamide, polysaccharide, dendrimer, and bifunctional hydrocarbon chain;

A is a linker unit;

Z is an antibody connector linked to Ab.

Accordingly, the ADC composition of the present invention can be expressed as: Ab-[Z-A-P-Y-M-D]$_q$.

The following are general, specific or preferred embodiments of each possible component of the linker unit L. It should be mentioned that the present invention covers linker units L obtained by any combination of the general, specific, or preferred embodiments of each of the components together with the general, specific, or preferred embodiment of any one or more of the remaining components; accordingly, the present invention covers ADC compounds obtained by any combination of linker units obtained by any combination described together with the general, specific, or preferred embodiment of antibody and drug parts are defined as in the present invention.

## Z-Antibody Connector

The antibody linker acts to connect an antibody or antigen-binding fragment thereof targeting an antigen to the rest of the conjugate, which has functional groups that form bonds with functional groups on the antibody.

In some embodiments, the antibody linker has nucleophilic groups that interact with reactive electrophilic groups on the antibody to form covalent bonds between the antibody and the linker unit. The electrophilic groups on the antibody include, but are not limited to aldehyde and ketocarbonyl groups, and the nucleophilic groups on the antibody linker include, but are not limited to hydrazide, hydroxylamine, amino, hydrazine, thiosemicarbazone, hydrazine carboxylate, and arylhydrazide.

In other embodiments, antibody linkers have electrophilic groups that interact with reactive nucleophiles on the antibody to form covalent bonds between the antibody and the linker unit. The nucleophilic groups on the antibody include, but are not limited to sulfhydryl, hydroxyl, or amino functional groups, and the electrophilic groups on the antibody linker include, but are not limited to maleimide, haloacetamide groups, activated disulfides, active esters such as NHS esters or HOBt esters, haloformates, acyl halides, alkyl halides, or benzyl halides such as haloacetamide. In some embodiments, the bonds created between the antibody linker and the antibody are thioether, amide, ester, carbamate, carbonate, urea, disulfide, or ether.

In some embodiments, Z in Formula (III) has the following structure:

$-Z_1-Z_2-Z_3-$,

wherein:

$Z_1$ is bonded atoms in Ab, such as S, N or C atoms;
$Z_2$ is selected from:

- 5-10 membered heterocyclic group preferably containing 1 or 2 heteroatoms selected from N, S and O in which the cyclic carbon atoms are optionally oxidized;
- 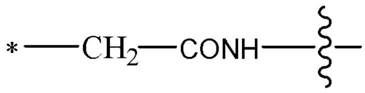 ;
- 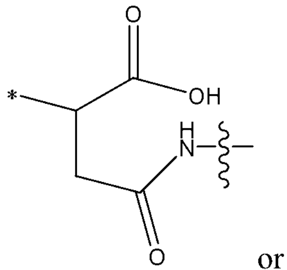 or 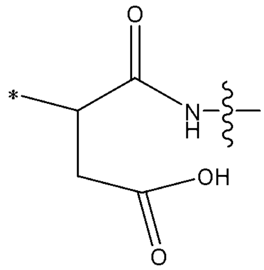 ;
- 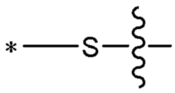 ;
- 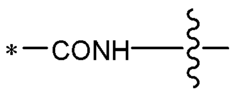 ; 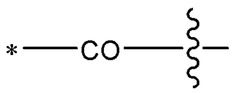 ; 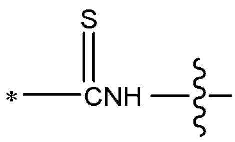 ;
- 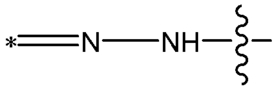 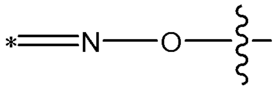 ; 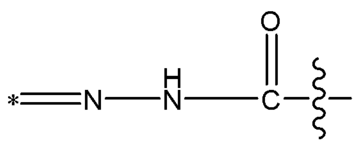 ;

wherein, the left * represents the connection point with $Z_1$, and the right

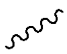

represents the connection point with $Z_3$;

$Z_3$ is selected from bond, $-C_1-C_{10}$ alkylene-C(=O)-, $-C_3-C_{10}$ alkynylene-C(=O)-, $-C_3-C_{10}$ alkenylene-C(=O)-, $-C_1-C_{10}$ heteroalkylene-C(=O)-, $-C_3-C_8$ cycloalkylene-C(=O)-, $-O-C_1-C_8$ alkylene-C(=O)-, -arylene-C(=O)-, $-C_1-C_{10}$ alkylene-arylene-C(=O)-, -arylene-$C_1-C_{10}$ -alkylene-C(=O)-, $-C_1-C_{10}$ -alkylene-$C_3-C_8$ -cycloalkylene-C(=O)-, $-C_3-C_8$ -cycloalkylene-$C_1-C_{10}$ -alkylene-C(=O)-, $-C_3-C_8$ -heterocyclylene-C(=O)-, $-C_1-C_{10}$ -alkylene-$C_3-C_8$ -heterocyclylene-C(=O)-, $-C_3-C_8$ -heterocyclylene-$C_1-C_{10}$ -alkylene-C(=O)-, $-C_1-C_{10}$ -alkylene-NH-, $-C_1-C_{10}$ -heteroalkylene-NH-, -$C_3-C_8$ -cycloalkylene-NH-, $-O-C_1-C_8$ -alkylene-NH-, -arylene-NH-, $-C_1-C_{10}$ -alkylene-arylene-NH-, -arylene-$C_1-C_{10}$-alkylene-NH-, $-C_1-C_{10}$ -alkylene-$C_3-C_8$ -cycloalkylene-NH-, $-C_3-C_8$ -cycloalkylene-$C_1-C_{10}$ -alkylene-NH-, $-C_3-C_8$ -heterocyclylene-NH-, $-C_1-C_{10}$ -alkylene-$C_3-C_8$ -heterocyclylene-NH- and $-C_3-C_8$ -heterocyclylene-$C_1-C_{10}$ -alkylene-NH-, wherein each group linked to -C(=O)- or -NH-in $Z_3$ is optionally substituted with the basic unit BU, and $Z_3$ is linked to the linker unit A via -C(=O)- or -NH-.

In some embodiments, $Z_1$ is the S atom; in other embodiments, $Z_1$ is the N atom; and in other embodiments, $Z_1$ is the C atom. In the ADC of the present invention, the preferred $Z_1$ is the sulfur atom of Ab.

In some embodiments, $Z_2$ is 5-10 membered heterocyclic groups, preferably 5-6 membered heterocyclic groups such as, but not limited to

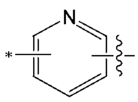, 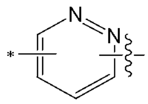, 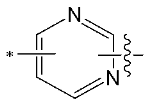, 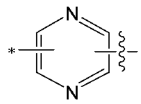,

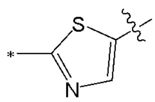, 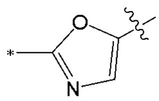, 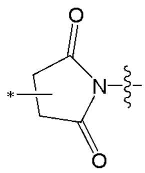,

wherein the left * represents the connection point with $Z_1$ and the right

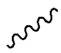

represents the connection point with $Z_3$.

In some embodiments, $Z_2$ is selected from

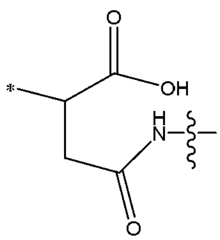 or 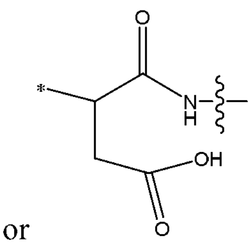

as the acid-amide moiety formed after hydrolysis of the succinimide moiety.

In a preferred embodiment, $Z_2$ is maleimidylidene group,

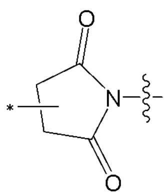.

In another preferred embodiment, $Z_2$ is

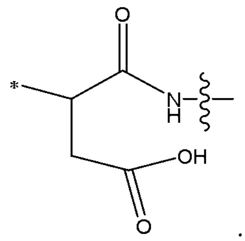

BU in $Z_3$ is selected from H, deuterium, halogen, $NO_2$, CN, $-OR^c$, $-OR^c$, $-N(R^c)_2$, $-COR^c$, $-CO_2R^c$, $-C-(O)C(O)R^c$, $-C(O)CH_2C(O)R^c$, $-S(O)R^c$, $-SO_2R^c$, $C(O)N(R^c)_2$, $-SO_2N(R^c)_2$, $-OC(O)R^c$, - $N(R^c)SO_2R^c$ and $-C_{1-6}$ alkyl optionally substituted with the preceding groups, wherein $R^c$ is H or $1-C_{1-6}$ alkyl optionally substituted with halogen, or wherein two $R^c$ groups attached to the same N atom form 4-7 heterocyclic groups together with the nitrogen to which they are attached. Preferably, BU is the aminoalkyl moiety, for example, $-(CH_2)_{1-6}NH_2$, $-(CH_2)_{1-6}NHR^c$ or $-(CH_2)_{1-6}N(R^c)_2$, wherein two $R^c$ groups attached to the same N atom and the nitrogen to which they are attached form azetidine, pyrrolidinyl, or piperidinyl groups.

In some embodiments, $Z_3$ is $-C_1-C_{10}$ -alkylene-C(=O)-, preferably $-C_1-C_5$ -alkylene-C(=O)-, where the -alkylene moiety is optionally substituted with the above BU moiety.

In some embodiments, $Z_3$ is $-C_1-C_{10}$ -alkylene-NH, preferably $-C_1-C_5$ -alkylene-NH-, wherein the -alkylene moiety is optionally substituted with the above BU moiety.

In an exemplary embodiment, $-Z_2-Z_3-$ can be the following groups:

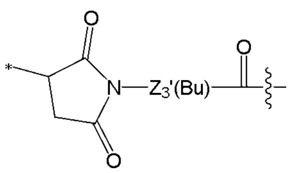
,
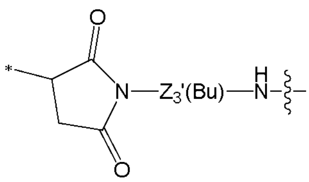
,
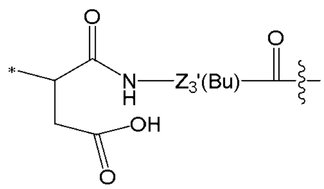

or

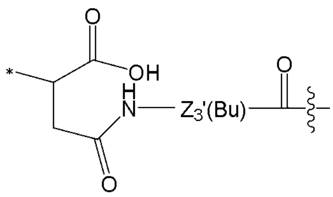
,

wherein $Z_3$' is $-C_1-C_5$ -alkylene-, wherein the -alkylene moiety is optionally substituted with the above BU moiety.

In the above $-Z_2-Z_3-$ structural formula, the left * represents the connection point with $Z_1$ and the right 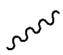 represents the antibody connector (when present) or property regulatory unit (when present, and antibody connector is absent) or cleavable linker (when neither the antibody connector nor the property regulatory unit are present).

In some embodiments, $-Z_1-Z_2-Z_3-$ can be the following groups:

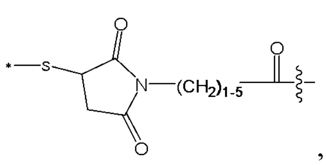
,
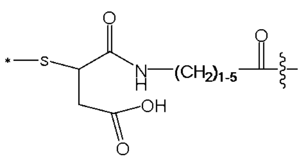
, and
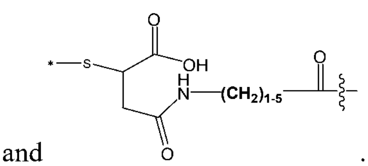

In the exemplary embodiments, $Z(-Z_1-Z_2-Z_3-)$ in Formula (III) of the present invention has the following structure:

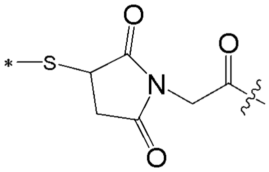, 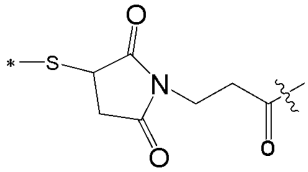, 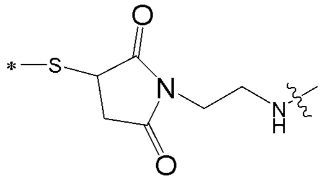,

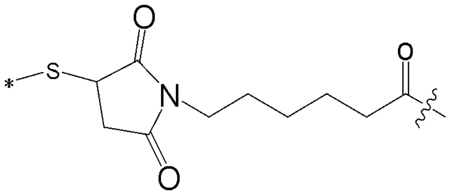, 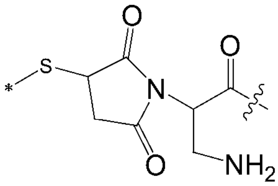, 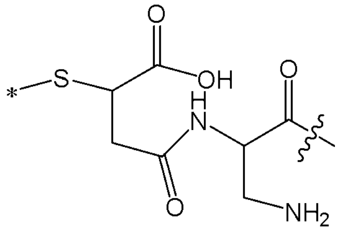,

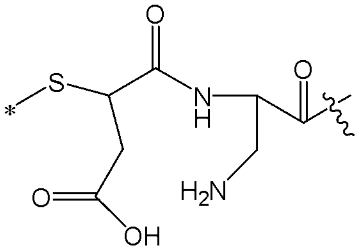.

In some preferred embodiments, Z in Formula (III) of the present invention has the following structure:

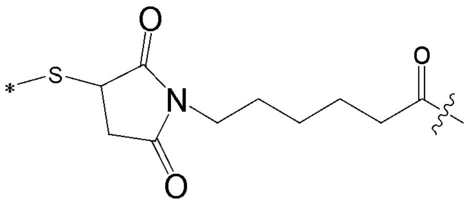, 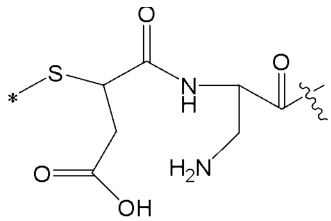.

## A - Linker unit

The linker unit connects the antibody connector Z to the property regulatory unit P, or when P is absent, connects the antibody connector to the cleavable linker Y, and is used to add an additional distance between the antibody connector and the cleavable linker to potentially aid activation of Y.

In some embodiments, linker unit A is selected from a direct bond, $C_{1-4}$ alkylene, -NH-, -NH-$C_{1-4}$ -alkylene -heteroaryl -, e.g., 5-membered or 6-membered azaheteroaryl, e.g., triazolyl, or the following groups:

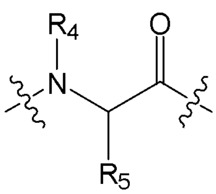, 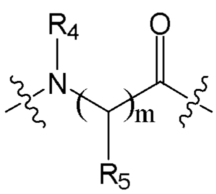, 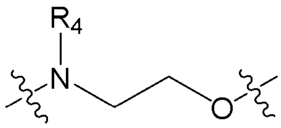, 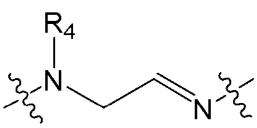,

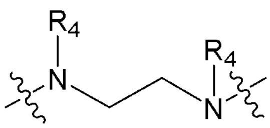, 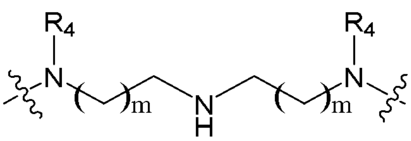, 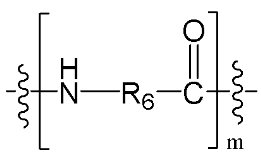, 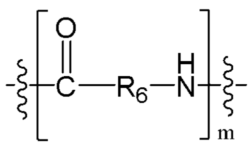,

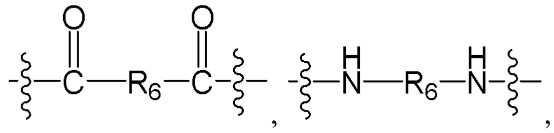

wherein $R_4$ is selected from H and $C_{1-6}$ alkyl, and preferably H or $C_{1-3}$ alkyl groups;

$R_5$ is selected from H, p-hydroxybenzyl, and optionally substituted -$C_{1-6}$ alkyl, wherein the substituents are selected from -OH, -SH, -$OC_{1-6}$ alkyl, -$SC_{1-6}$ alkyl, -$CONH_2$, -COOH, -$NH_2$, - NHC(=NH)$NH_2$, -NHCO-$C_{1-6}$ alkyl, -NHCHO, -$NHCONH_2$, pyridyl;
$R_6$ is selected from -$C_{1-6}$ -alkylene-, -arylene-, -$C_{1-10}$ -heteroalkylene-, -$C_{3-8}$ heterocyclyl-, -$C_{1-10}$ -alkylene-arylene-, -arylene-$C_{1-10}$ -alkylene-, -$C_{1-10}$ -alkylene-$C_{3-8}$ carbocyclic group-, -$C_{3-8}$ carbocyclic group-$C_{1-10}$ -alkylene-, -$C_{1-10}$ -alkylene-$C_{3-8}$ heterocyclyl-, -$C_{3-8}$ heterocyclyl-C $_{1-10}$ - alkylene-;

m is an integer from 1 to 10, preferably 1-4.
In a specific embodiment, linker unit A is

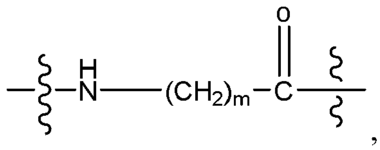

wherein m is an integer from 1 to 6, preferably 2 to 6, more preferably 2 to 4.
In a specific embodiment, linker unit A is -$C_{1-4}$ -alkylene-; in another specific embodiment, linker unit A is -NH-. In another specific embodiment, linker unit A is a directly linked bond.

**P-property regulatory unit**

The addition of property regulatory unit P may be beneficial in improving the properties of ADCs, such as stability in blood circulation, improving hydrophilicity, resulting in decreased clearance and increased exposure. However, increasing the amount of P also leads to an increase in ADC molecular weight as well as an increase in hydrodynamic radius, which leads to a decrease in diffusion, and a reduced diffusion rate may reduce the ability of ADC to penetrate into tumors. Because of these two competing pharmacokinetic effects, moderate P is required to reduce ADC clearance and thereby increase plasma exposure, but not so large as to reduce its diffusivity as to interfere with the ability of the ADC to achieve the desired target cell population.
The property regulating unit P can exist in series or branched form in the linker unit. P can be linked directly to the cleavable linker Y, or can be linked to the cleavable linker Y by an additional linker group, e.g., carbonyl, alkyl carbonyl, amide, ester, urea, disulfide bridge, carbamate, hydrazone, imide, oxime, triazolyl, maleimide, alkenyl, alkynyl, or -alkylene groups, for example, P can be linked to the cleavable linker Y by -C(=O)-, -$C_{1-4}$ -alkylene, -$C_{1-4}$ -alkylene-C(=O)-, -NH-C(=O)-($CH_2OCH_2$)-C(=O)-, -$C_{1-4}$ -alkylene-NH-C(=O)-($CH_2OCH_2$)-C(=O)-.
In some embodiments, P is absent.
In some embodiments, P is selected from polyamines, such as but not limited to polyethylenimine, polylysine, spermine, dimeric polyamines, arginine, amidine, protamine, cationic lipids, cationic porphyrins, quaternary ammonium salts of polyamines, alpha helical peptides.
In some embodiments, P is selected from peptides, preferably hydrophilic peptide.
In some embodiments, P is the cyclodextrin unit.
In some embodiments, P is polyamide.
In some embodiments, P is polysaccharide, dendrimer, or bifunctional hydrocarbon chain.
In some embodiments, P is PEG.
In an embodiment of the present invention, polydisperse PEG (a nonuniform mixture of size and molecular weight), monodisperse PEG (single chain length and molecular weight), and discrete PEG may be used as part of the property regulating unit in the ADC of the present invention, preferred PEG is discrete PEG, which are compounds synthesized in a stepwise manner rather than by a polymerization process, as individual molecules with defined chain lengths.
The ADC of the present invention may consist of one or more PEG chains consisting of at least two ethylene oxide ($CH_2CH_2O$) subunits. PEG chains can be linked together, for example, in linear, branched, or star configurations. Typically, at least one PEG chain is functionalized such that it can be covalently linked to other components in the linker unit,

including, for example, via amine, thiol, NHS ester, maleimide, alkyne, azide, carbonyl, or other functional groups. For example, functional derivatization of one end of the PEG chain can be covalently linked to an appropriate site in the linker unit, and the other end (or multiple ends) can be free and unbound, i.e., not linked to other components in the ADC, and can take the form of methoxy, carboxylic acid, alcohol, or other suitable functional groups; or the PEG chain can covalently link in series between two components of the two linker units, e.g., between linker unit A and the cleavable linker. PEG chains are linked to components within the linker unit as cleavable bonds that can be essentially insensitive to cleavage when circulating in plasma but sensitive to cleavage in the intracellular or intratumoral environment. Exemplary linkages include but are not limited to amide bonds, ether bonds, ester bonds, hydrazone bonds, oxime bonds, disulfide bonds, peptide bonds, or triazole bonds, etc.

The method of attaching a PEG unit to a linker unit of an ADC is well-known to technicians in the field. For example, PEG can covalently bind to amino acid residues via reactive groups. Reactive groups are those to which an activated PEG molecule can bind, such as free amino or carboxyl groups, and thiol groups on cysteine residues, for example, can also be used as reactive groups to attach PEG. In some embodiments, PEG can also be linked to an amino group using methoxylated PEG ("mPEG") with different reactive moieties, and unrestricted examples of such reactive moieties include succinimidyl succinate (SS), succinimidyl carbonate (SC), mPEG-imino acid ester, p-nitrophenyl carbonate (NPC), succinimidyl propionate (SPA), and cyanuric acid chloride. Accordingly, nonlimiting examples of this mPEG include mPEG-succinimidyl succinate (mPEG-SS), mPEG-succinimidyl carbonate (mPEG-SC), mPEG-imino acid ester, mPEG-p-nitrophenyl carbonate (mPEG-NPC), mPEG-succinimidyl propionate (mPEG-SPA), mPEG-N-hydroxy-succinimide (mPEG-NHS), mPEG-cyanuric acid chloride, $mPEG_2$-lyaminol-NPC, and mPEG2-Lys-NHS.

In some embodiments, PEG units contain one or more linear PEG chains, each having 2-12 ethylene oxide ($CH_2CH_2O$) subunits, such as at least 2, at least 3, at least 4, at least 5, at least 6, at least 7, at least 8, at least 9, at least 10, at least 11, and at least 12 ethylene oxide ($CH_2CH_2O$) subunits. In a preferred embodiment, the PEG unit contains at least 2, at least 4, at least 6, at least 8, at least 10 or at least 12 ethylene oxide ($CH_2CH_2O$) subunits, e.g., the PEG unit contains 2 to 12, 2 to 10, 2 to 8, 2 to 6, 2 to 4, 4 to 12, 4 to 10, 4 to 8, 4 to 6, 6 to 12, 6 to 10, 6 to 8, 8 to 12, 8 to 10 subunits.

An exemplary embodiment of PEG linked to the cleavable linker Y and linker unit A includes, but is not limited to, the following:

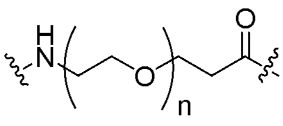,
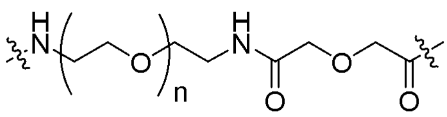,
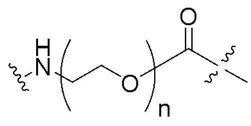,

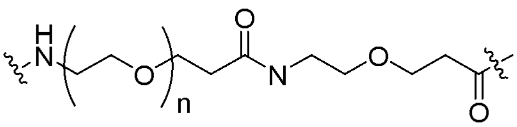,
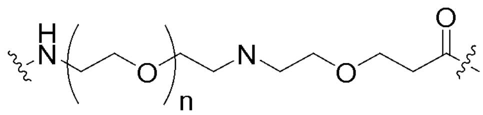;

An exemplary implementation for a linear PEG unit (i.e., a segment linked to a linker unit with the other end cap) is:

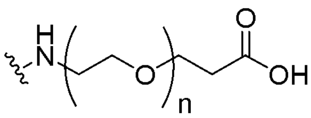,
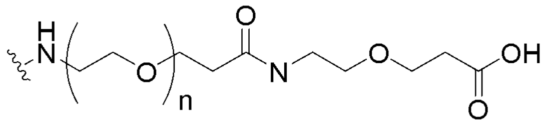,

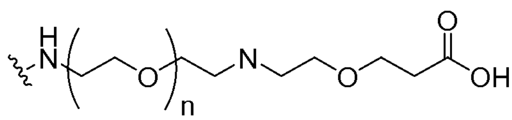,
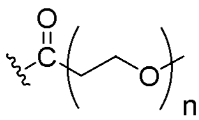;

wherein the wave line on the left represents the connection point with Z-A-, and the wave line on the right represents the connection point with Y, each n is independently selected from an integer of 2-12. In some embodiments, n is 2, 4, 8, or 12. In some embodiment, n is 2. In some embodiment, n is 4. In some embodiments, n is 8. In some embodiment, n is 12.

**Y-Cleavable linker**

On one aspect, the cleavable linker Y in the linker unit of the ADC of the present invention is a cleavable unit that is

a substrate that can be cleaved or broken by the enzyme. In order to limit or minimize the non-targeted toxicity of ADCs while ensuring the release of toxin molecules at target tumor sites, cleavable linkers are designed as substrates specifically cleaved by enzymes present near or within target cells, preferably at higher levels or activities near or within target cells than in other parts of the body, thereby ensuring CDNs are specifically recognized, cleaved, and released by enzymes in target cells/tissues to exert their biological function.

Therefore, the ADCs of the present invention are stable in biological fluids until they reach their targets, such as tumor cells/tissues. Because of the specific release of ADCs, the administration of the ADC of the present invention produces less toxicity than administration of CDNs alone, and the activity of CDNs is maximized.

In some embodiments, Y in the linker unit of the ADC of the present invention is a dipeptide, tripeptide or oligopeptide composed of amino acid sequences specifically recognized and cleaved by proteases, preferably specifically recognized and cleaved by non-mammalian proteases or endogenous mammalian proteases present near or within target cells, these enzymes are, for example, but not limited to β-position APP-cleaving enzyme 1 (BACE1), cathepsin D (CTSD), calpain-1 (CAPN1), aspartyl endonuclease (Lugemain), caspase 1 (Casp1), caspase 2 (Casp2), caspase 3 (Casp3), caspase 5 (CASPS), caspase 6 (Casp6), caspase 7 (Casp7), caspase 8 (Casp8), caspase 9 (Casp9), cathepsin B (CTSB), cathepsin K (CTSK), cathepsin L (CTSL), cathepsin S (CTSS), esterases (e.g., cholinesterase, alkaline phosphatase, phosphodiesterase, sulfatase), amidase angiotensin I converting enzyme (ACE), angiotensin I converting enzyme 2 (ACE2), ADAM metallopeptidase domain 10 (ADAM10), dipeptidyl peptidase 3 (DPP3), insulin degrading enzyme (IDE), matrix metalloproteinase 1 (MMP1), matrix metalloproteinase 12 (MMP12), matrix metalloproteinase 13 (MMP13), matrix metalloproteinase 14 (membrane insertion) (MMP14), matrix metalloproteinase 2 (MMP2), matrix metalloproteinase 3 (MMP3), matrix metalloproteinase 7 (MMP7), matrix metalloproteinase 8 (MMP8), matrix metalloproteinase 9 (MMP9), membrane metalloendopeptidase (NEPRILYSIN) (MME), ADAM metallopeptidase domain 17 (TACE) (ADAM17), dipeptidyl peptidase 4 (DPP4), dipeptidyl peptidase 8 (DPP8), dipeptidyl peptidase 9 (DPP9), coagulation factor Xa (Factor Xa), coagulation factor VILA (Factor VII), fibroblast activating protein α (FAP), furin (paired basic amino acid cleaving enzyme), granulase A (granulase 1, cytotoxic T lymphocytes associated (GZMA) serine esterase 3), granzyme B (granzyme 2, cytotoxic T lymphocyte associated (GZMB), serine esterase 1), granzyme K (granzyme 3, trypsin II) (GZMK), kallikrein-1 (KLK1), kallikrein-2 (KLK2), plasma kallikrein (PSA, KLK3), kallikrein-11 (KLK11), kallikrein-13 (KLK13), kallikrein-15 (KLK15), matriptase (ST14), Spinesin (TMPRSSS), plasmin (PLG), prolyl oligopeptidase (PREP), thrombin (F2), tPA, plasminogen activator tissue type (PLAT), UPA, plasminogen activator urokinase (PLAU), HtrA serine peptidase 2 (hTRA2), caseinolytic mitochondrial matrix peptidase proteolytic subunit (CIPP/X), constitutive proteasome chymotrypsin-like form (PSMBS, β5), constitutive proteasome trypsin-like form (PSMB7, β7), constitutive proteasome caspase-like form (PSMB6, β6), immunoproteasome chymotrypsin-like form (PSMB8, LMP7), immunoproteasome trypsin-like form (PSMB10, MECL1), immunoproteasome caspase-like form (PSMB9, LMP2).

In some embodiments, Y in the linker unit of the ADC of the present invention is specifically recognized and cleaved by cathepsin B, e.g., Val-Cit, Val-Ala, Gly-Phe-Leu-Gly, Gly-Gly-Phe-Gly.

In some embodiments, Y in the linker unit of the ADC of the present invention is specifically recognized and cleaved by aspartyl endonucleases, e.g., Ala-Ala-Asn, Ala-Ala.

In some embodiments, Y in the linker unit of the ADC of the present invention is specifically recognized and cleaved by matrix metalloproteinases 2/9, e.g., Pro-Leu-Gly-Leu-Ala-Gly (PLGLAG), Gly-Pro-Leu-Gly-Ile-Ala-Gly-Gln (GPLGIAGQ), Gly-Pro-Val-Gly-Leu-Ile-Gly-Lys (GPVGLIGK).

In some embodiments, Y as part of the linker unit of the ADC of the present invention is a cleavable peptide containing two or more (e.g., 2-12) continuous or discontinuous amino acids, such as dipeptide, tripeptide, tetrapeptide, pentapeptide, hexapeptide, heptapeptide, octapeptide, nonapeptide, decapeptide, undecapeptide, or dodecapeptide units, or consisting thereof. Each amino acid containing the peptide unit can be selected independently of each other from the natural or unnatural amino acid and/or as D- or L-isomers, provided that Y contains a cleavable bond that, when cleaved, will trigger the release of CDN. In some embodiments, Y consists only of natural amino acids. In other embodiments, Y consists of only unnatural amino acids. In some embodiments, Y consists of natural amino acids attached to unnatural amino acids. In some embodiments, Y consists of natural amino acids attached to the D-isomer of natural amino acids.

In some embodiments, amino acids contained in the Y of the ADC of the present invention are, for example, selected from alanine (Ala), arginine (Arg), aspartate (Asp), asparagine (Asn), histidine (His), glycine (Gly), glutamate (Glu), glutamine (Gln), phenylalanine (Phe), lysine (Lys), substituted lysine, leucine (Leu), serine (Ser), tyrosine (Tyr), threonine (Thr), isoleucine (Ile), proline (Pro), tryptophan (Typ), valine (Val), cysteine (Cys), methionine (Met), selenocysteine, ornithine (Orn), beta-alanine (β-Ala), citrulline (Cit), and derivatives thereof. In some embodiments, each amino acid is independently selected from alanine, arginine, aspartate, asparagine, histidine, glycine, glutamate, glutamine, phenylalanine, lysine, leucine, serine, tyrosine, threonine, isoleucine, proline, tryptophan, valine, cysteine, methionine, and their derivatives. In some embodiments, each amino acid is independently selected from alanine, arginine, aspartate, asparagine, histidine, glycine, glutamate, glutamine, phenylalanine, lysine, leucine, serine, tyrosine, threonine, isoleucine, proline, tryptophan and valine, N-methylglycine, β-alanine, and derivatives thereof.

In some embodiments, each amino acid is independently selected from the following L-(native) amino acids: alanine, arginine, aspartate, asparagine, histidine, glycine, glutamateglutamic acid, glutamine, phenylalanine, lysine, leucine, serine, tyrosine, threonine, isoleucine, tryptophan, and valine. In another embodiment, each amino acid is independently selected from the D-isomers of these natural amino acids: alanine, arginine, aspartate, asparagine, histidine, glycine, glutamate, glutamine, phenylalanine, lysine, leucine, serine, tyrosine, threonine, isoleucine, tryptophan, and valine.

In some embodiments, Y has the following structure:

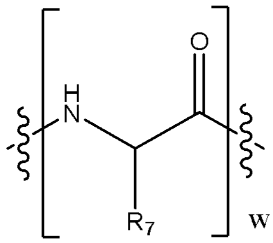 (Y1)

wherein the N-terminus is linked to the Z part of the linker unit, and the C-terminus is linked to the M part of the linker unit or directly linked to drug D.

w is an integer from 2 to 12;

$R_7$ is independently selected from hydrogen, methyl, isopropyl, isobutyl, sec-butyl, benzyl, p-hydroxybenzyl, $-CH_2OH$, $-CH(OH)CH_3$, $-CH_2CH_2SCH_3$, $-CH_2CONH_2$, $-CH_2COOH$, - $CH_2CH_2CONH2$, $-CH_2CH_2COOH$, $-(CH_2)_3NHC(=NH)NH_2$, $-(CH_2)_3NH_2$, $-(CH_2)_3NHCOCH_3$, - $(CH_2)_3NHCHO$, $-(CH_2)_4NHC(=NH)NH_2$, $-(CH_2)_4NH_2$, $-(CH_2)_4NHCOCH_3$, $-(CH_2)_4NHCHO$, - $(CH_2)_3NHCONH_2$, $-(CH_2)_4NHCONH_2$, $-CH_2CH_2CH(OH)CH_2NH_2$, 2-pyridinylmethyl-, 3-pyridinylmethyl-, 4-pyridinylmethyl-, phenyl, cyclohexyl,

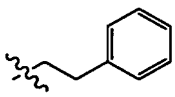, 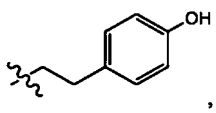,

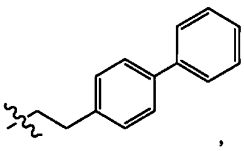, 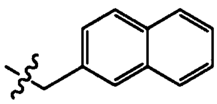, 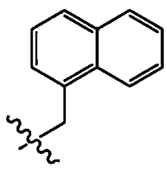, 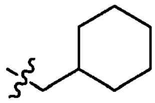, 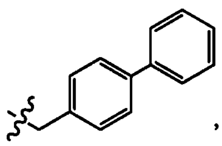,

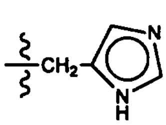, 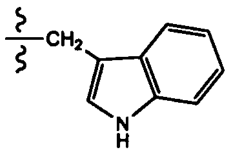;

or $R_7$ is the spiro

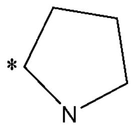

connected via *.

In some embodiments, each $R_7$ is independently hydrogen, methyl, isopropyl, isobutyl, sec-butyl, benzyl, $-CH_2CONH_2$ or $-(CH_2)_3NHCONH_2$. Accordingly, the amino acids are selected from alanine, glycine, asparagine, isoleucine, leucine, valine, phenylalanine, and citrulline.

In some embodiments, w is an integer from 2 to 8, such as 2 to 6, 2 to 4, 2 to 3, such as 2, 3, 4, 6, 8.

Alternatively, in some embodiments, Y or Y containing cleavable peptides has the following structure: $-(AA)_w-$(Y2)

wherein AA is an amino acid, selected from alanine (Ala), arginine (Arg), aspartate (Asp), asparagine (Asn), histidine (His), glycine (Gly), glutamate (Glu), glutamine (Gln), phenylalanine (Phe), lysine (Lys), substituted lysine, leucine (Leu), serine (Ser), tyrosine (Tyr), threonine (Thr), isoleucine (Ile), proline (Pro), tryptophan (Typ), valine (Val), cysteine (Cys), methionine (Met), selenocysteine,



ornithine (Asn), beta-alanine (β-Ala), citrulline (Cit), and derivatives thereof; preferably alanine, glycine, asparagine, isoleucine, leucine, valine, phenylalanine, citrulline, glutamate; more preferably alanine, glycine, asparagine, valine, phenylalanine, citrulline, glutamate;

w is an integer from 2 to 12; preferably 2 to 8 and preferably 2 to 4;

wherein each AA is linked by a peptide bond,

wherein the N -terminus of the peptide is linked to the property regulatory unit (when present), the linker unit (when present), or the Z part of the linker unit, and the C -terminus of the peptide is linked to a self-immolative linker of the linker unit or directly to the drug D.

In some embodiments, Y contains dipeptides, for example, dipeptides selected from the following: Ala-Ala, Ala-(D)Asp, Ala-Cit, Ala-Lys, Ala-Val, Asn-Cit, Asp-Cit, Asn-Lys, Asn-(D)Lys, Asp-Val, Cit-Ala, Cit-Asn, Cit-Asp, Cit-Cit, Cit-Lys, Cit-Ser, Cit-Val, Glu-Val, Gly-Glu, Phenyl Gly-(D)Lys, His-Val, Ile-Cit, Ile-Pro, Ile-Val, Leu-Cit, Lys-Cit, Me3Lys-Pro, Met-Lys, Met-(D)Lys, Phe-Arg, Phe-Cit, Phe-Lys, Pro-(D)Lys, Ser-Cit, Trp-Cit, Val-Ala, Val-(D)Asp, NorVal-(D)Asp, Val-Cit, Val-Glu, Val-Lys and salts thereof. In a specific embodiment, the dipeptide is Val-Cit. In another specific embodiment, the dipeptide is Val-Ala. In another specific embodiment, the dipeptide is Ala-Ala.

In some embodiments, Y contains tripeptides, such as Gly-Gly-Gly, Gly-Gly-Arg, Phe-Lys-Gly, Leu-Lys-Gly, Leu-Leu-Gly, Glu-Val-Cit, Val-Lys-Gly, Val-Lys-Ala, Val-Gly-Gly, Val-Cit-Gly, Val-Gln-Gly, Val-Glu-Gly, Val-Lys-Gly, Val-Lys-Leu, Ala-Ala-Ala, Ala-Ala-Asn. In a specific embodiment, the tripeptide is Ala-Ala-Asn.

In some embodiments, Y contains tetrapeptides, such asGly-Gly-Gly-Gly, Gly-Phe-Leu-Gly, Gly-Val-Lys-Gly, Ala-Leu-Ala-Leu, Gly-Phe-Leu-Gly, Ala-Leu-Ala-Leu, Gly-Gly-Phe-Gly, Val-Lys-Gly-Gly. In a specific embodiment, the tetrapeptide is Gly-Phe-Leu-Gly. In another specific embodiment, the tetrapeptide is Gly-Gly-Phe-Gly.

In some embodiments, Y contains hexapeptides or octapeptides such as Pro-Leu-Gly-Leu-Ala-Gly, Gly-Pro-Leu-Gly-Ile-Ala-Gly-Gln, Gly-Pro-Val-Gly-Leu-Ile-Gly-Lys.

In some embodiments, Y is a peptide containing a substituted lysine, such as dipeptide, tripeptide, tetrapeptide, hexapeptide, or octapeptide. In one embodiment, the substituted lysine is:

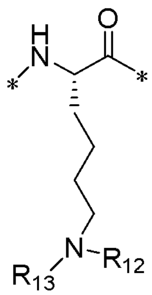

wherein, $R_{12}$ and $R_{13}$ are each independently selected from the following: H, $C_{1-6}$ alkyl, $-CO-NH_2$, - CONH($C_{1-6}$ alkyl) and -CONH($C_{1-6}$ alkyl)$_2$, wherein the alkyl group is optionally substituted with the following groups: halogen, $C_{1-6}$ alkoxy, $C_{1-6}$ haloalkyl, and $C_{3-6}$ cycloalkyl.

In the preferred embodiments, Y is selected from the following peptides: Val-Cit, Val-Ala, Ala-Ala-Asn, Gly-Gly-Phe-Gly as follows:

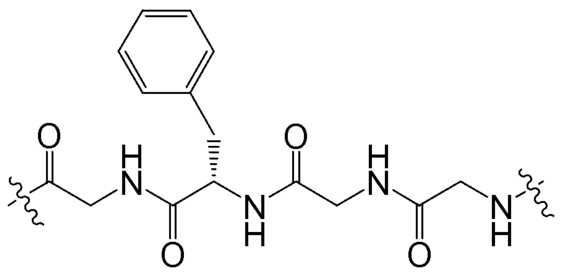, 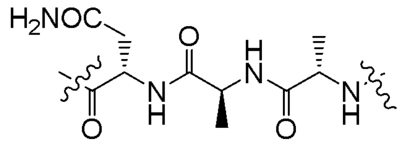,

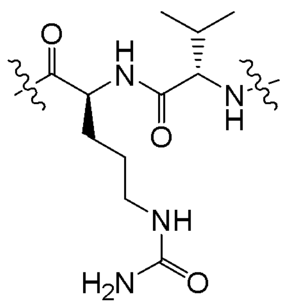, 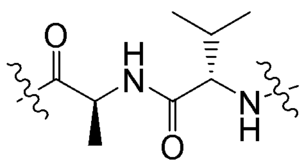,

wherein the left end carbonyl group is linked to the self-immolative linker M or drug D, the right end N is linked to the property regulatory unit P (when present), the linker unit A (when present), or the Z part of the linker unit.

As mentioned above, by designing antibody-drug conjugates biased toward tumor distribution, linker units with peptide Y-containing units would facilitate CDN release in tumor cells and the environment. Therefore, in some embodiments, after ADC is internalized into tumor cells, the amide bond in the peptide of the Y unit will be recognized and degraded by enzymes in tumor cells, releasing drug part D. In other embodiments, the amide bond in the peptide in the Y unit would be recognized and degraded by enzymes in the tumor environment, releasing drug part D.

In one embodiment, the peptide of the Y unit is the peptide cleaved by cathepsin B, e.g., but not limited to: Val-Cit, Val-Ala, Gly-Phe-Leu-Gly, Gly-Gly-Phe-Gly.

In one embodiment, the peptide of the Y unit is a peptide cleaved by high concentrations of Legumain in the tumor, such as Ala-Ala-Asn.

In one embodiment, the peptide of the Y unit is the peptide cleaved by high concentrations of MMP-2/9 in the tumor, e.g., Pro-Leu-Gly-Leu-Ala-Gly (PLGLAG), Gly-Pro-Leu-Gly-Ile-Ala-Gly-Gln (GPLGIAGQ), Gly-Pro-Val-Gly-Leu-Ile-Gly-Lys (GPVGLIGK).

On another aspect, Y in the linker unit of the ADC of the present invention contains reducible groups.

In some embodiments, Y in the linker unit of the ADC of the present invention contains disulfide groups. When the ADC is internalized into tumor cells, this group can induce an hypoxic state due to irregular blood flow in tumor cells, leading to an increased concentration of glutathione and its reduction by glutathione, thereby selectively releasing the drug CDN in the tumor cells. Exemplary ADCs containing disulfide bonds include, but are not limited to, the following formulas:

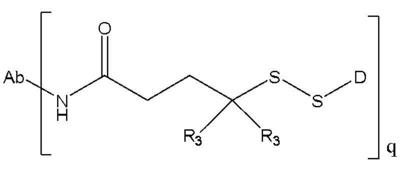, 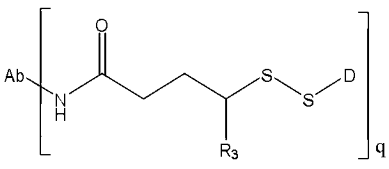 or 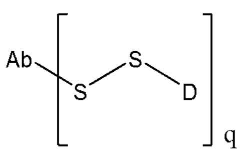

wherein Ab and D represent the antibody or antigen-binding fragment thereof and CDN, respectively, that target the antigen as defined in the present invention; S of S-D is from CDN, NH of Ab-NH is from Ab, and S of Ab-S is from Ab; q represents the number of times that CDNs are linked to Ab via linker units, and $R_3$ is independently H or $C_{1-6}$ alkyl at each occurrence, for example but not limited to methyl, ethyl.

In other embodiments, Y in the linker unit of the ADC of the present invention contains a nitro group which can be reduced under hypoxic conditions or by nitroreductase.

On another aspect, Y in the linker unit of the ADC of the present invention contains specific sugars.

In some embodiments, Y in the linker unit of the ADC of the present invention contains, for example, galactose. In this case, since β-galactosidase is mainly active intracellularly, it would facilitate the release of CDNs in target cells. Specifically,

In other embodiments, Y in the linker unit of the ADC of the present invention contains, for example, glucuronic acid. Because β-glucuronidase is highly concentrated in the microenvironment of a variety of solid tumors, including lung, breast, and gastrointestinal cancers, the presence of glucuronic acid in the Y of the linker unit of the ADC will enable specific release of the drug CDN into the tumor microenvironment. Specifically, the specified substance may be a glucuronic acid corresponding to the following formula:

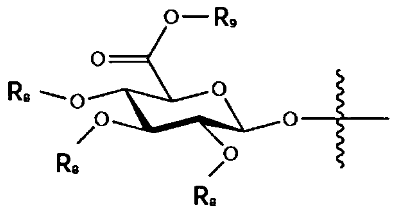

wherein each $R_8$ is independently H or protecting groups such as acetyl(-$COCH_3$), trifluoroacetyl(-$COCF_3$), trimethylacetyl(-CO-TBU), benzoyl(-COPH), silyl ether(-Si(O-alkyl)$_3$), benzyl(-$CH_2$Ph), p-methoxybenzyl(-$CH_2$-Ph-OMe), methoxymethyl(-$CH_2$-OME), triphenylmethyl(-$CPh_3$), tetrahydropyran or alkyl; and $R_9$ is H or alkyl, such as methyl.

**M - Self-immolative linker**

The self-immolative linker M in the linker unit L of the ADC of the present disclosure could be a covalent bond or a functional group that facilitates the linkage between the cleavable linker Y to drug D or could provide additional structural components to further facilitate the release of drug D from the rest of the conjugate.

In some embodiments, the self-immolative linker M in the linker unit of the ADC of the present disclosure is a covalent bond, i.e., the cleavable linker Y in the linker unit and the drug D are directly linked, e.g., via an amide bond.

In other embodiments, the self-immolative linker M between the drug D and the cleavable linker Y of the ADC of the present disclosure is a functional group that facilitates the linkage of the cleavable linker Y to the drug D, or additional structural components can be provided to further facilitate the release of the drug D from the rest of the ADC. In some embodiments, M linking Y and drug D units has the following formula:

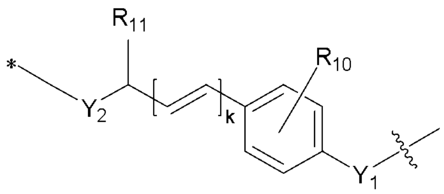
or
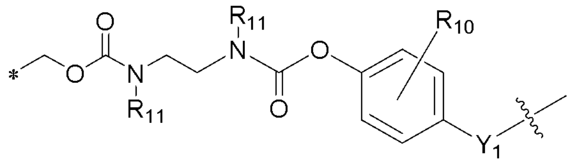

wherein * represents the connection point with the CDN and 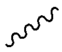 represents the connection point with the cleavable linker Y;

$Y_1$ is selected from -O- or -NH-;

$Y_2$ is selected from bond, -C(O)-O- or -C(O)-$NR_{11}$-;

$R_{10}$ is selected from H, -$NO_2$, -$NH_2$ or -$CF_3$;

$R_{11}$ is selected from H or $C_{1-6}$ alkyl, preferably H or methyl;

k is 0 or 1.

In a specific embodiment, M linking Y and drug D units has the following formula:

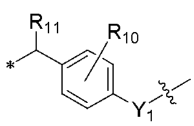,

specific examples include

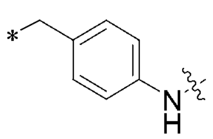, 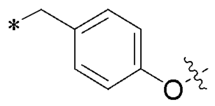, 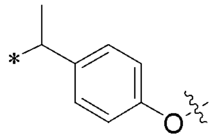,

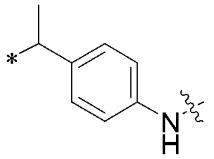, 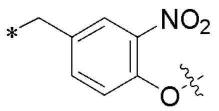, 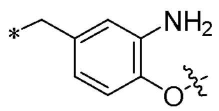, 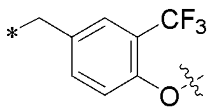, 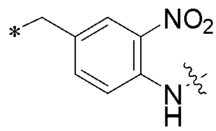,

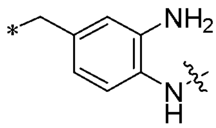, 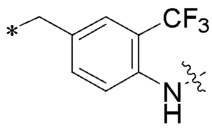.

In a specific embodiment, M linking Y and drug D units has the following formula:

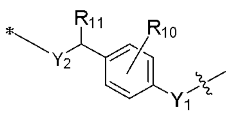,

specific examples include

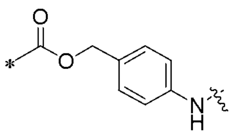, 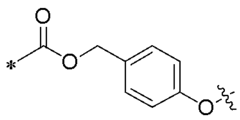,

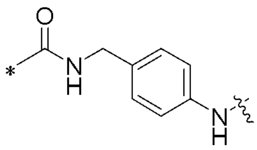, 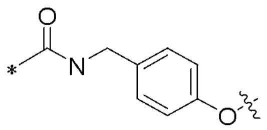.

In a specific embodiment, M linking Y and drug D units has the following formula:

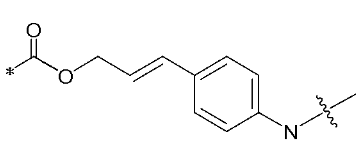 or 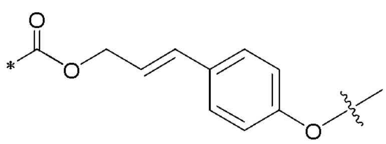.

In a specific embodiment, M linking Y and drug D units has the following formula:

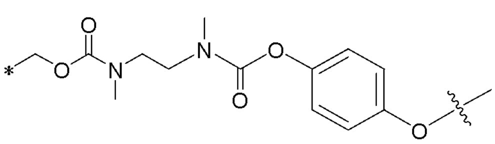 or 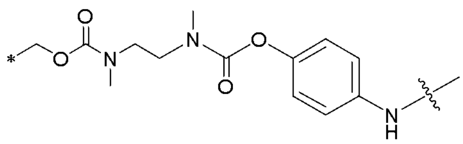.

In the preferred embodiment of the present invention, M linking Y and drug D units is selected from

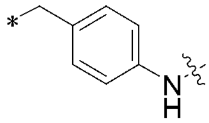 or 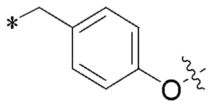,

preferably

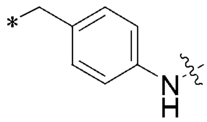.

In other embodiments, M is a covalent bond and the Y in Formula (X) of the present invention and drug D are directly linked , such as via an amide bond.

**Exemplary linker unit L**

In the preferred embodiment, in the linker unit L of Formula (III),

M is selected from

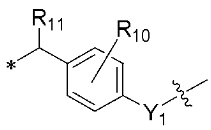 or 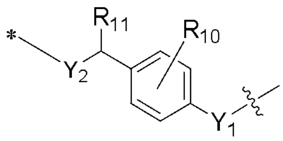,

wherein * represents connection point with CDN, 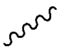 represents connection point with a cleavable linker Y, wherein $Y_1$ is selected from -O- or -NH-, $Y_2$ is selected from bond, -C(O)-O- or -C(O)-$NR_{11}$-, $R_{10}$ is selected from H, -$NO_2$, -$NH_2$ or -$CF_3$, $R_{11}$ is selected from H or $C_{1-6}$ alkyl;

Y is selected from enzymatically cleavable peptides of 2-8 amino acid ;

P is absent or is one or more linear, branched or star shaped PEG chains consisting of at least two ethylene oxide ($CH_2CH_2O$) subunits;

A is selected from a direct bond, -$C_{1-4}$ -alkylene-, -NH- or -NH-$C_{1-4}$ -alkylene-heteroaryl-;

Z is selected from

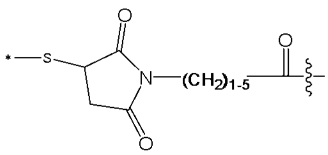, 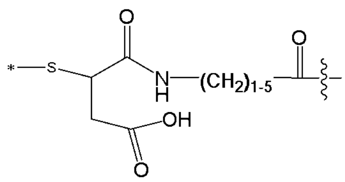,

and

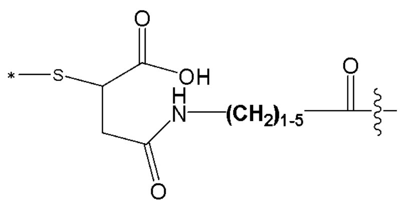.

In some specific embodiments, Y has

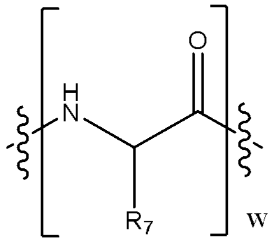 (Y1),

, wherein the N -terminus is linked to the Z part of the linker unit, and C -terminus is linked to the M part of the linker unit or directly to the drug D; w is an integer from 2 to 8, preferably an integer of 2-4; each $R_7$ is independently hydrogen, methyl, isopropyl, isobutyl, sec-butyl, benzyl, $-CH_2CONH_2$ or - $(CH_2)_3NHCONH_2$, accordingly, each amino acid described is selected from alanine, glycine, asparagine, isoleucine, leucine, valine, phenylalanine, citrulline; or

Y has the following structure: $-(AA)_w-$(Y2), wherein AA is selected from alanine, glycine, asparagine, isoleucine, leucine, valine, phenylalanine, citrulline, glutamate; more preferably selected from alanine, glycine, asparagine, valine, phenylalanine, citrulline, glutamate; w is an integer from 2 to 8, preferably an integer from 2 to 4; wherein each AA is linked by a peptide bond, wherein the N -terminus of the peptide is linked to the property regulatory unit P (when present), A (when present), or Z part, and the C -terminus of the peptide is linked to the self-immolative linker M or directly to the drug D.

In the preferred embodiment, Y is selected from the following peptides: Val-Cit, Val-Ala, Ala-Ala-Asn, Gly-Gly-Phe-Gly as follows:

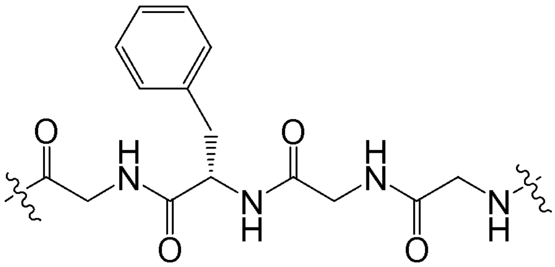

,

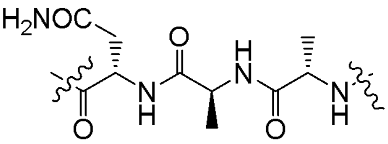

,

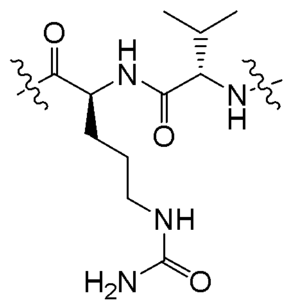

,

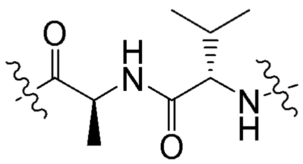

,

wherein the left end carbonyl group is linked to the self-immolative linker M or drug D, the right end N is linked to the property regulatory unit P (when present), linker unit A (when present) or Z part of the linker unit.

In a further preferred embodiment, L in Formula (X) of the present invention, is the following linker unit:

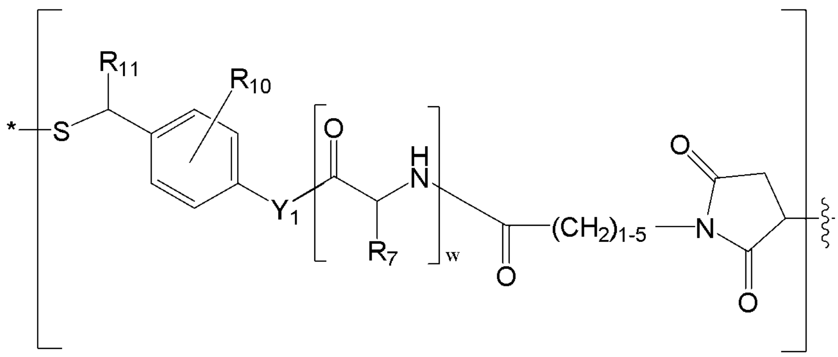

,

wherein the S atom is derived from -SH in CDN, the wavy line represents the connection point with the antibody , $R_7$, $R_{10}$, $R_{11}$, $Y_1$, w is as defined generally or specifically above. Specific examples of linker units include:

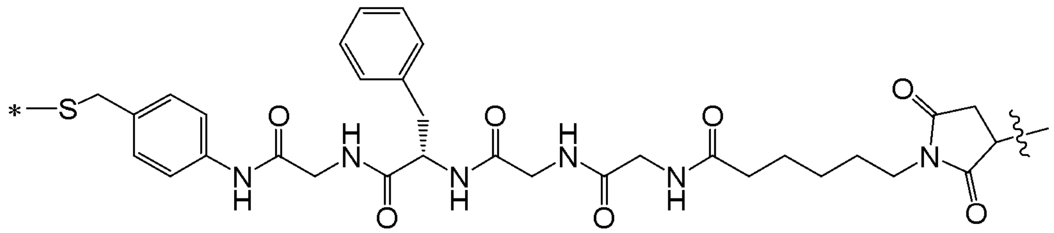

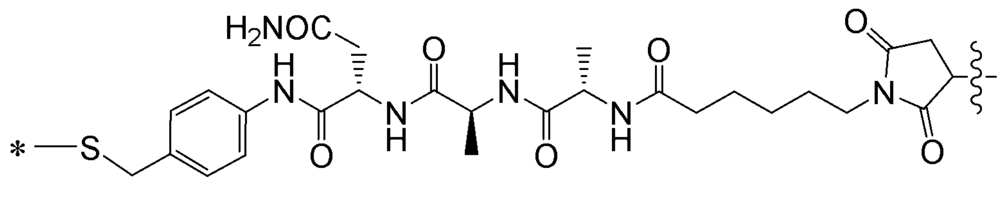

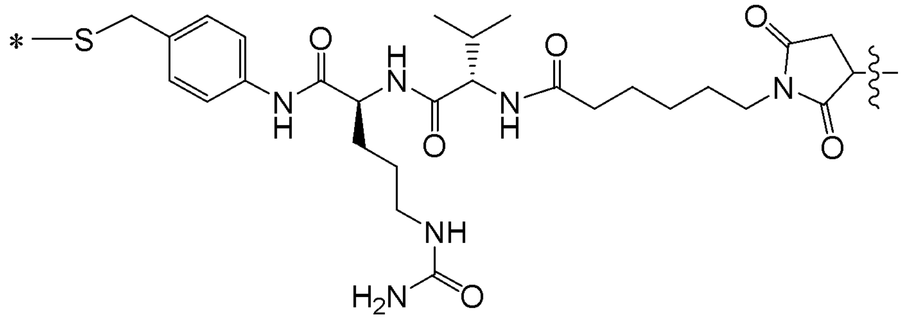

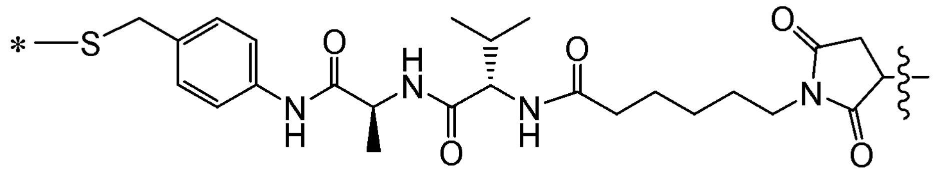

**CDN-linker unit of the present invention**

Based on the above, [L-D] in Formula (X) of the present invention has the following formula:

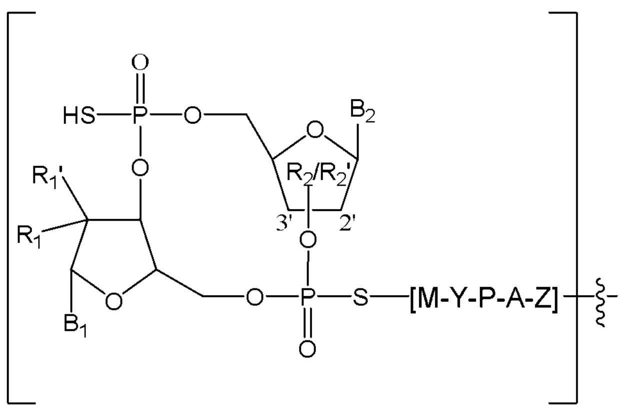

(IV),

e.g.

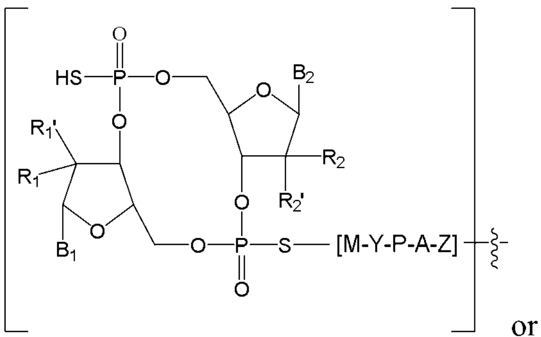

or

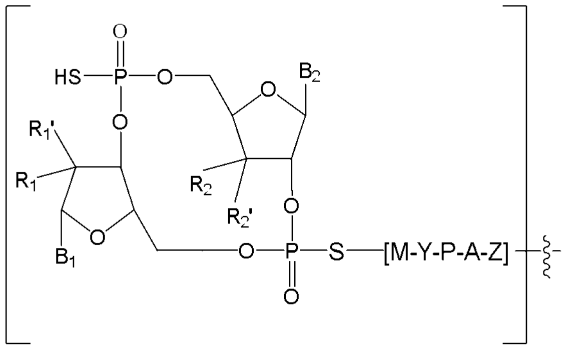

(IV-a) (IV-b)

wherein the wavy line represents the connection point with the Ab; $B_1$, $B_2$, $R_1$, $R_1$', $R_2$, $R_2$', M, Y, P, A, Z have the meanings defined above for drug part D, respectively, which include the resulting technical protocols formed by the general, preferred, or specific definition of each group, and the resulting technical protocols formed by any combination of the general, preferred, or specific definition.

In the preferred embodiment, [L-D] in Formula (X) of the present invention has the following formula:

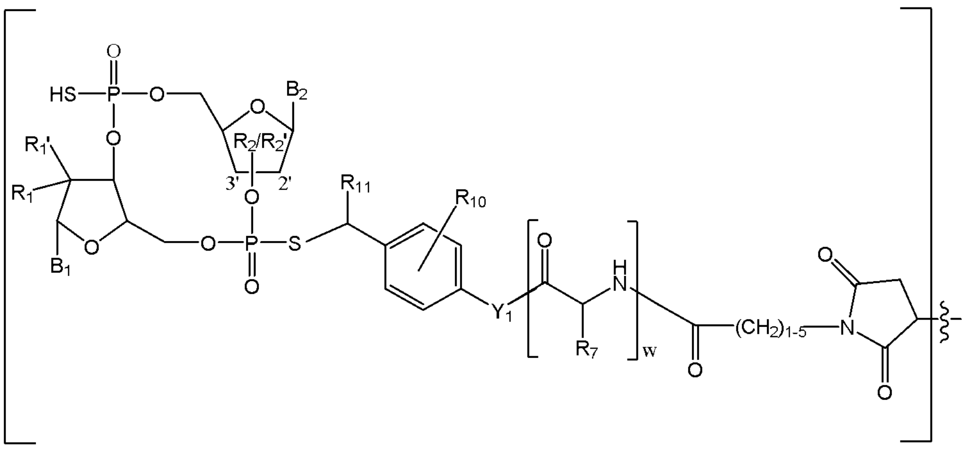

(IV-1),

e.g.

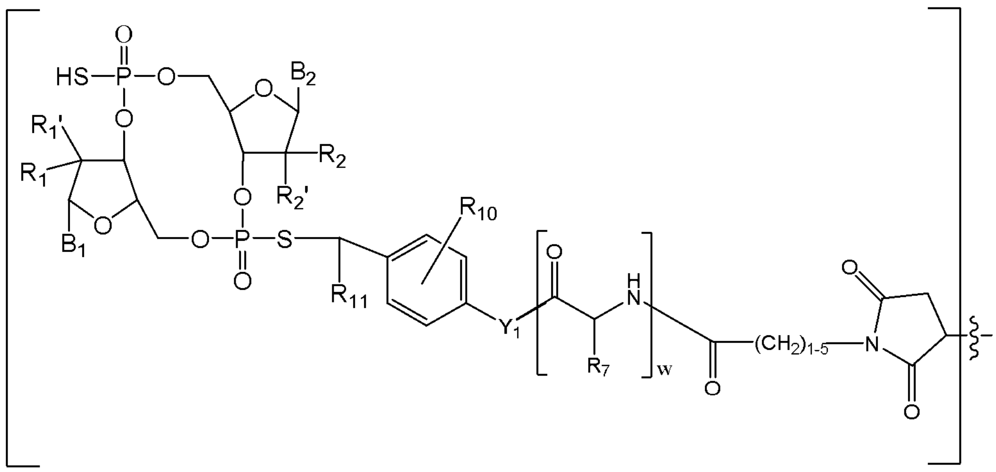

(IV-a-1)

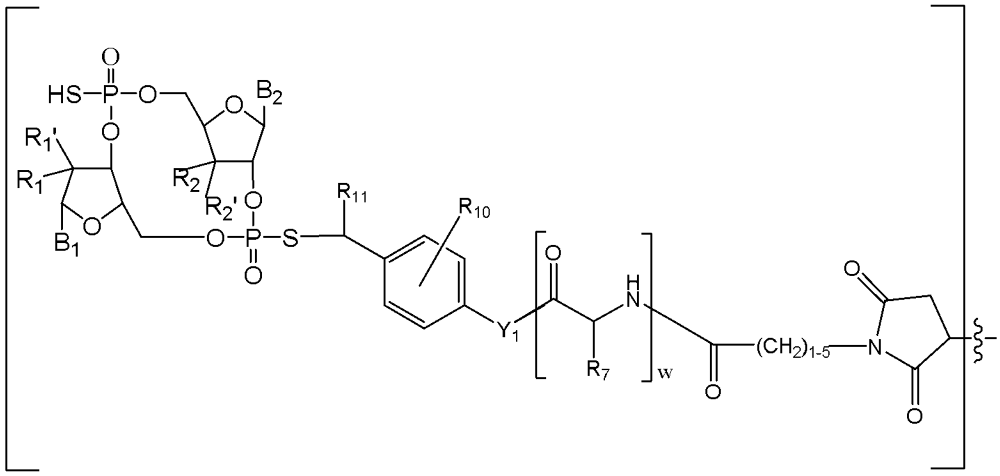

(IV-b-1)

wherein the wavy line represents the connection point with Ab; $B_1$, $B_2$, $R_1$, $R_1$', $R_2$, $R_2$', $R_7$, $R_{10}$, $R_{11}$, $Y_1$, and w have the meaning defined above for drug part D, respectively, which include the resulting technical protocols formed by the general, preferred, or specific definitions defined of each group, and the resulting technical protocols formed by any combination of the general, preferred, or specific definitions.

In the more preferred embodiment, in each of the Formula (IV), (IV-a), (IV-b), (IV-1), (IV-a-1), or (IV-b-1),

$B_1$ is adenine

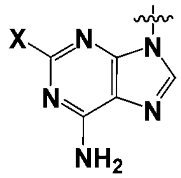

optionally substituted with X, wherein X is selected from H, Cl, F or -NH$C_{1-6}$ alkyl; or guanine

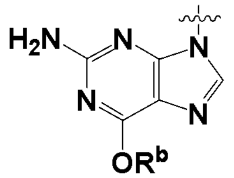

optionally substituted with $R^b$, wherein $R^b$ is selected from -$C_{1-6}$ alkyl;
$B_2$ is guanine

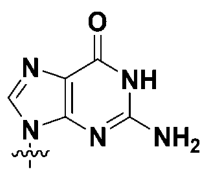

;

$R_1$ and $R_1$' are both H, or one is H and the other is F, or one is H and the other is OH;
$R_2$ and $R_2$' are both H, or one is H and the other is F, or one is H and the other is OH;
$Y_1$ is selected from -O- or -NH-;
$R_{10}$ is selected from H, -$NO_2$, -$NH_2$ or -$CF_3$;
$R_{11}$ is selected from H or $C_{1-6}$ alkyl;
$R_7$ is independently hydrogen, methyl, isopropyl, isobutyl, sec-butyl, benzyl, -$CH_2CONH_2$ or - $(CH_2)_3NHCONH_2$;
w is an integer from 2 to 8.

In a more preferred embodiment, in each Formula (IV), (IV-a), (IV-b), (IV-1), (IV-a-1) or (IV-b-1), $B_1$ is for example

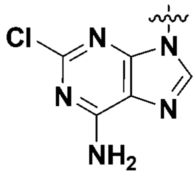, 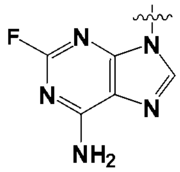, 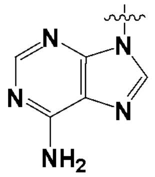, 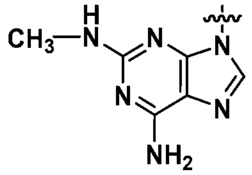

and

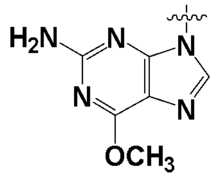,

preferably

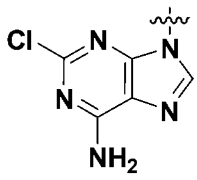, 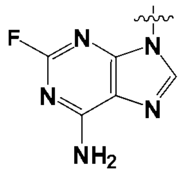, 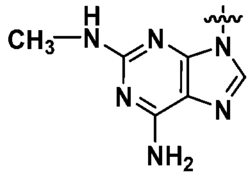;

$B_2$ is guanine

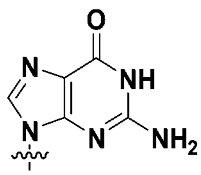.

In the specific embodiment, $R_1$ and $R_1$' are both H, or one H and the other is F, or one H and the other is OH. In a further specific embodiment, one of $R_2$ and $R_2$' is H and the other is OH. In a further specific embodiment, $Y_1$ is -NH. In a further specific embodiment, $R_{10}$ and $R_{11}$ are both H. In a further specific embodiment, $R_7$ is independently hydrogen, methyl, isopropyl, benzyl, - $CH_2CONH_2$ or $-(CH_2)_3NHCONH_2$, and w is an integer from 2 to 4.

In a further preferred embodiment, [L-D] in Formula (X) of the present invention has the following formula:

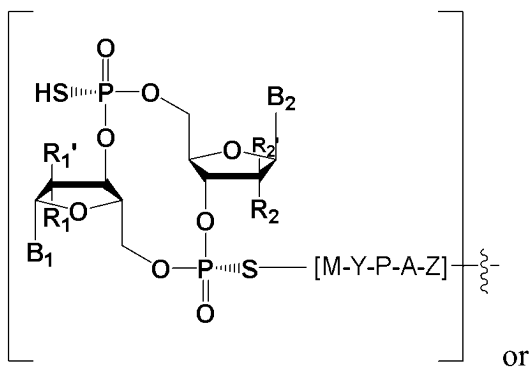

(IV-a')

or

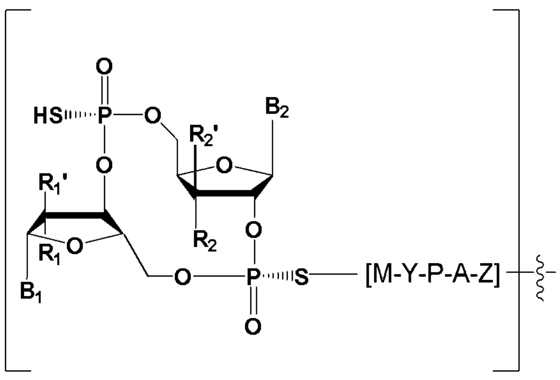

(IV-b')

More specifically:

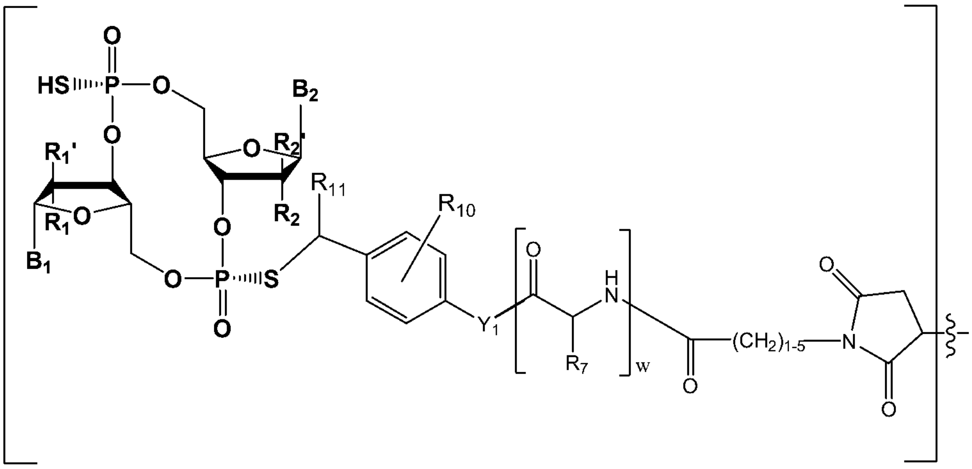

(IV-a'-1)

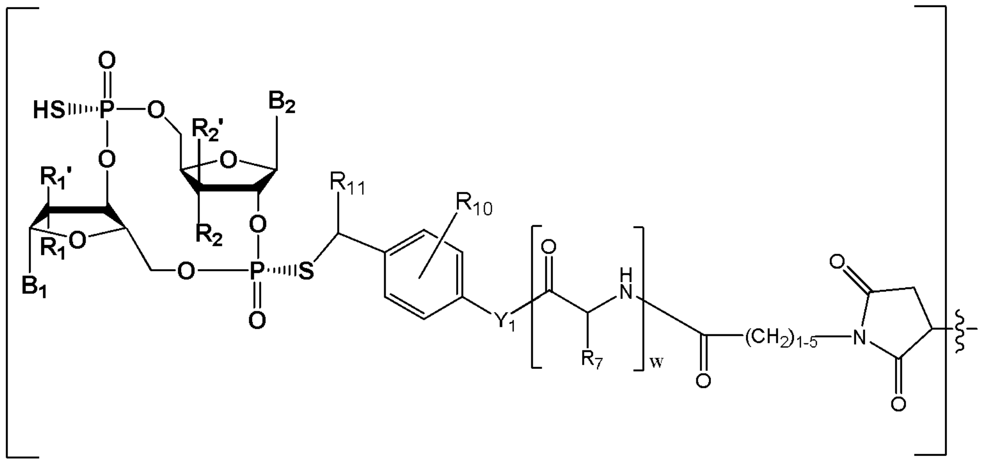

(IV-b'-1)

wherein the wavy line represents the connection point with the Ab; $B_1$, $B_2$, $R_1$, $R_1'$, $R_2$, $R_2'$, $R_7$, $R_{10}$, $R_{11}$, $Y_1$, and w have the meaning defined above for drug part D, respectively, which include the resulting technical protocols formed by the general, preferred or specific definitions of each group, and the resulting technical protocols formed by any combination of the general, preferred or specific definitions.

In a further preferred embodiment, in Formula (IV-a'), (IV-b'), (IV-a'-1) or (IV-b'-1),

$B_1$ is adenine

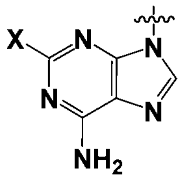

optionally substituted with X, wherein X is selected from H, Cl, F or $-NHC_{1-6}$ alkyl; or guanine

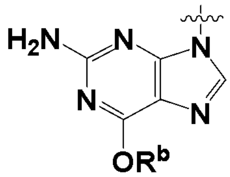

optionally substituted with $R^b$, wherein $R^b$ is selected from -$C_{1-6}$ alkyl;
$B_2$ is guanine

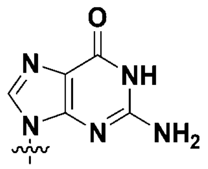
;

$R_1$ and $R_1$' are both H, or wherein $R_1$' is H and $R_1$ is F, or wherein $R_1$' is H and $R_1$ is OH; $R_2$ and $R_2$' are both H, or wherein $R_2$' is H and $R_2$ is F, or wherein $R_2$' is H and $R_2$ is OH
$Y_1$ is selected from -O- or -NH-;
$R_{10}$ is selected from H, -$NO_2$, -$NH_2$ or -$CF_3$;
$R_{11}$ is selected from H or $C_{1-6}$ alkyl;
$R_7$ is independently hydrogen, methyl, isopropyl, isobutyl, sec-butyl, benzyl, -$CH_2CONH_2$ or - $(CH_2)_3NHCONH_2$;
w is an integer from 2 to 8.

In a further preferred embodiment, in Formula (IV-a'), (IV-b'), (IV-a'-1) or (IV-b'-1), $B_1$ is for example

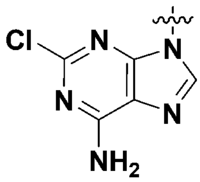
,
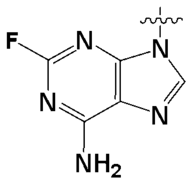
,
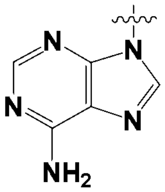
,
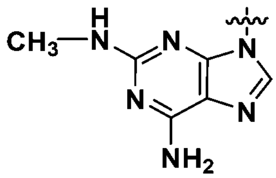
and
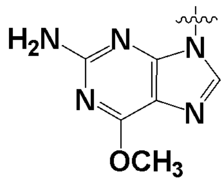

preferably

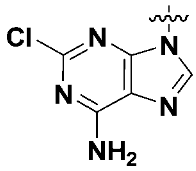
,
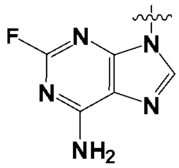
,
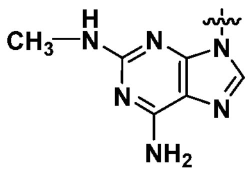
;

$B_2$ is guanine

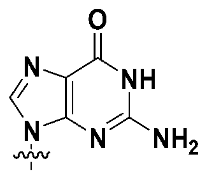
.

In this specific embodiment, both $R_1$ and $R_1$' are H, or wherein $R_1$' is H and $R_1$ is F, or wherein $R_1$' is H and $R_1$ is OH. In a further specific embodiment, $R_2$ is OH and $R_2$' is H. In a further specific embodiment, $Y_1$ is -NH. In a specific embodiment, $R_{10}$ and $R_{11}$ are both H. In a further specific embodiment, $R_7$ is independently hydrogen, methyl, isopropyl, benzyl, -$CH_2CONH_2$ or - $(CH_2)_3NHCONH_2$ and w is an integer from 2 to 4.
The specific examples of [L-D] in Formula (X) of the present invention include:

| | |
|---|---|
| **PL-1** | 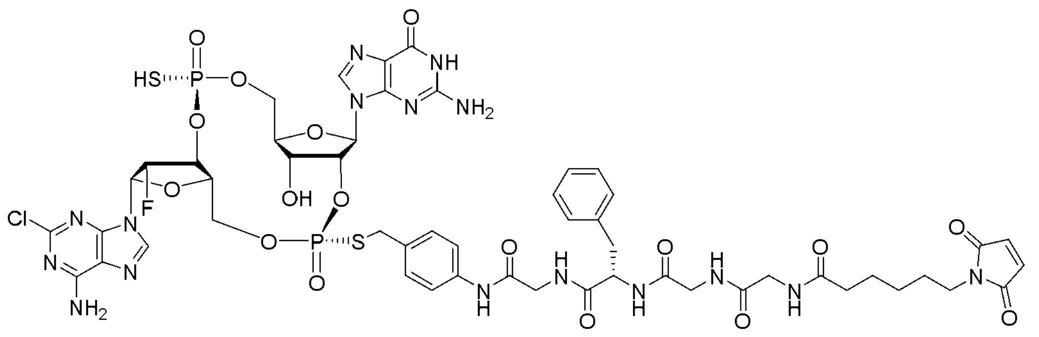 |
| **PL-2** | 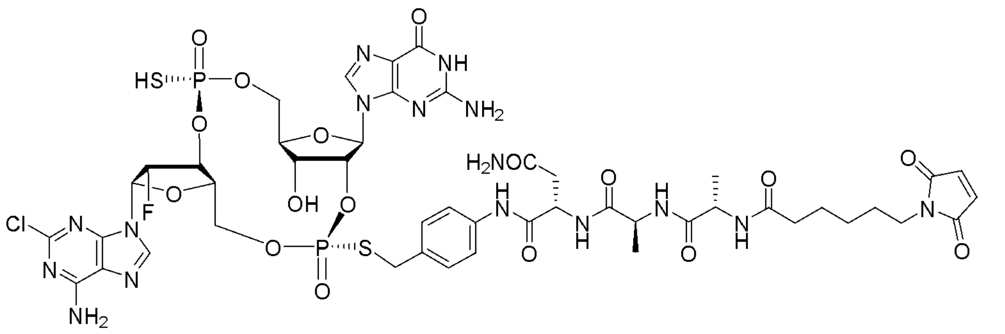 |
| **PL-3** | 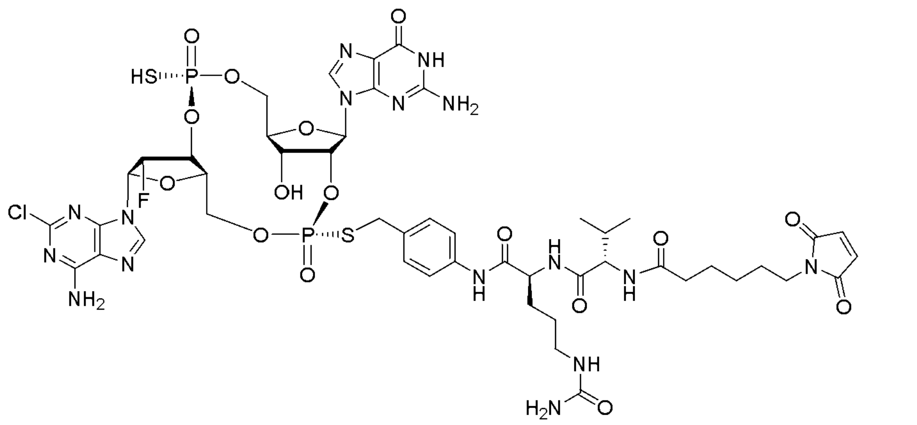 |
| **PL-4** | 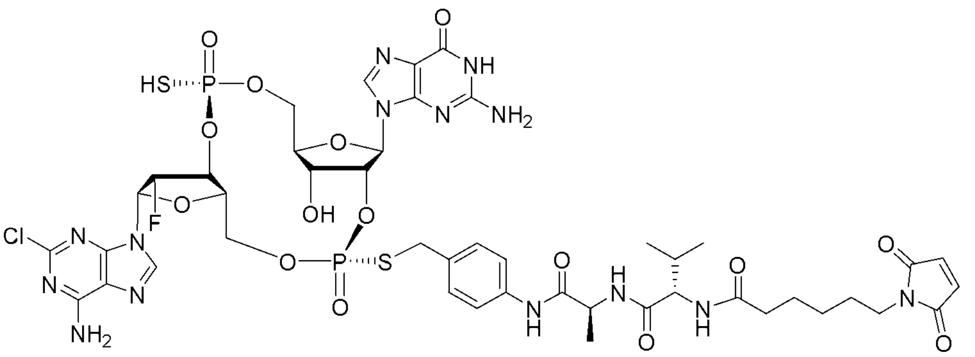 |

(continued)

| | |
|---|---|
| **PL-5** | 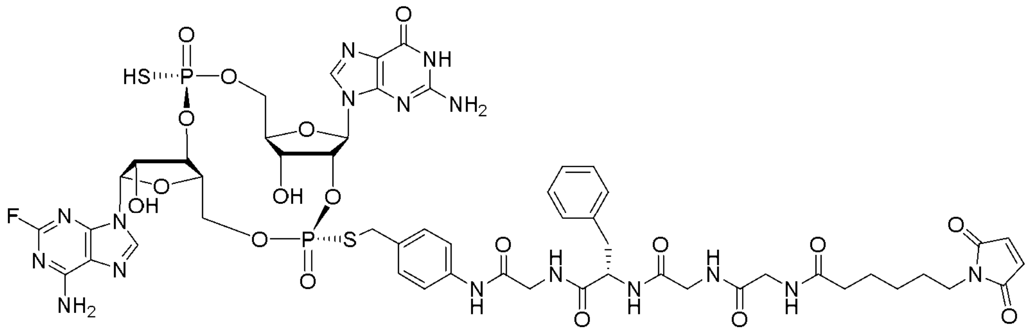 |
| **PL-6** | 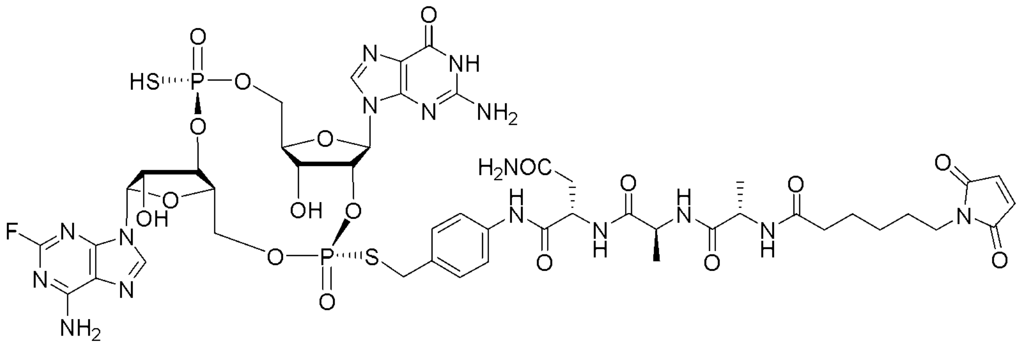 |
| **PL-7** | 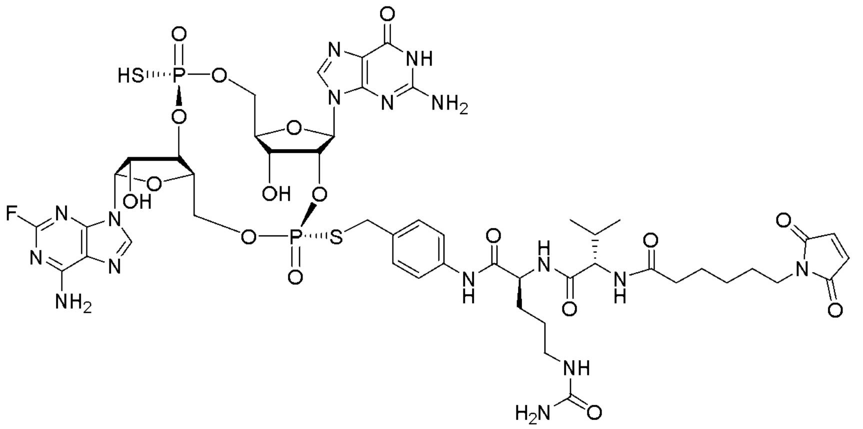 |
| **PL-8** | 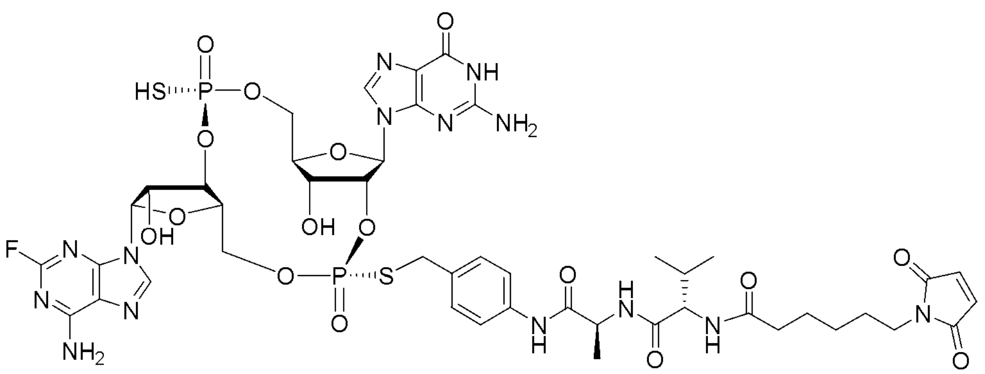 |

(continued)

| | |
|---|---|
| **PL-9** | 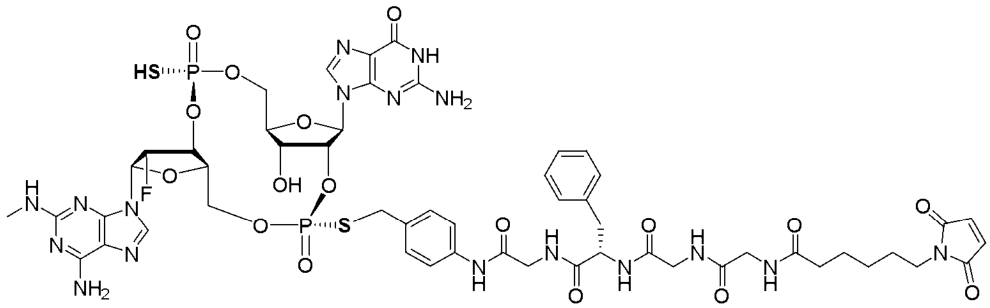 |
| **PL-10** | 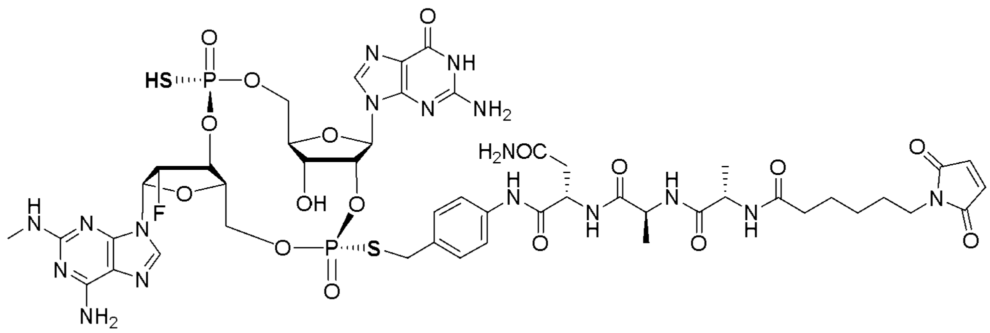 |
| **PL-11** | 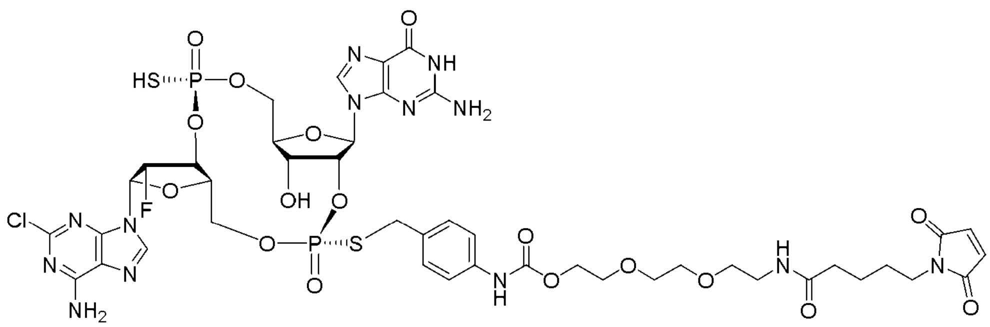 |
| **PL-12** | 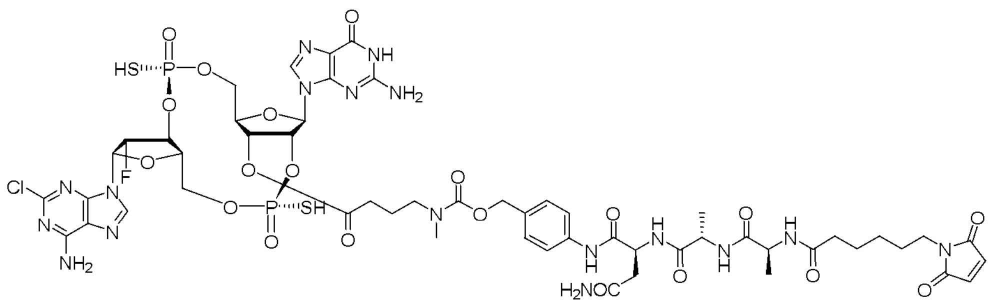 |
| **PL-13** | 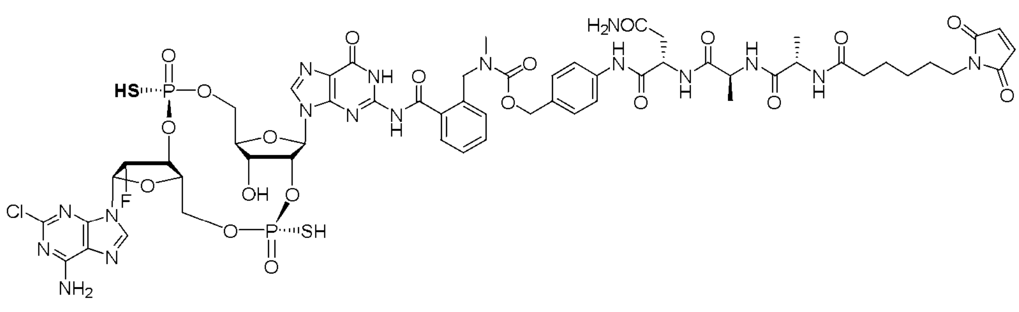 |

stereoisomers, tautomers, stable isotopic variants, pharmaceutically acceptable salts or solvates thereof.

**ExemplaryADC of the present invention**

Based on the above, ADC of Formula (X) of the present invention is specifically:

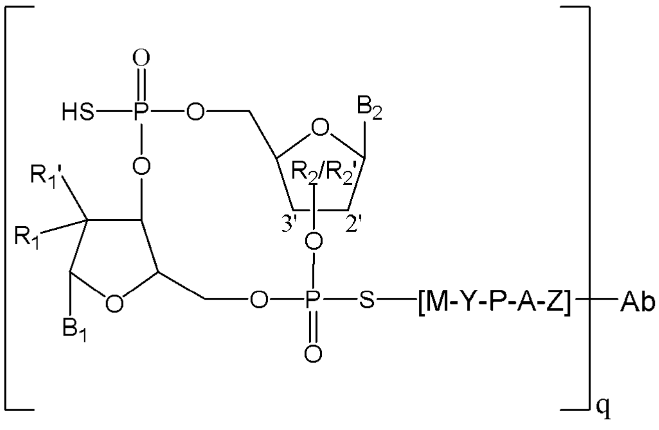

(X1),

e.g.

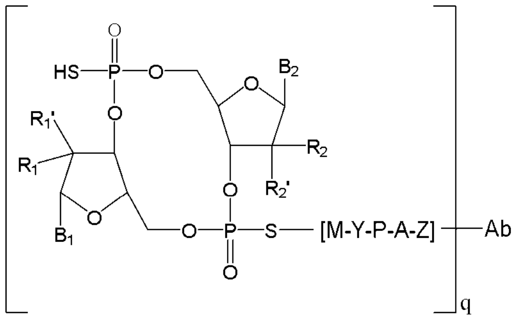

or

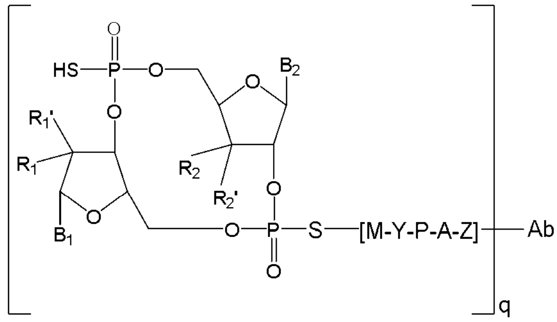

(X1-a) (X1-b)

wherein, $B_1$, $B_2$, $R_1$, $R_1$', $R_2$, $R_2$', M, Y, P, A, Z, q and Ab have the meanings defined above for Drug D, respectively, which include the resulting technical protocols formed by the general, preferred or specific definitions of each group, and the resulting technical protocols formed by any combination of the general, preferred or specific definitions.

In a preferred embodiment, the ADC in Formula (X) of the present invention has the following formula:

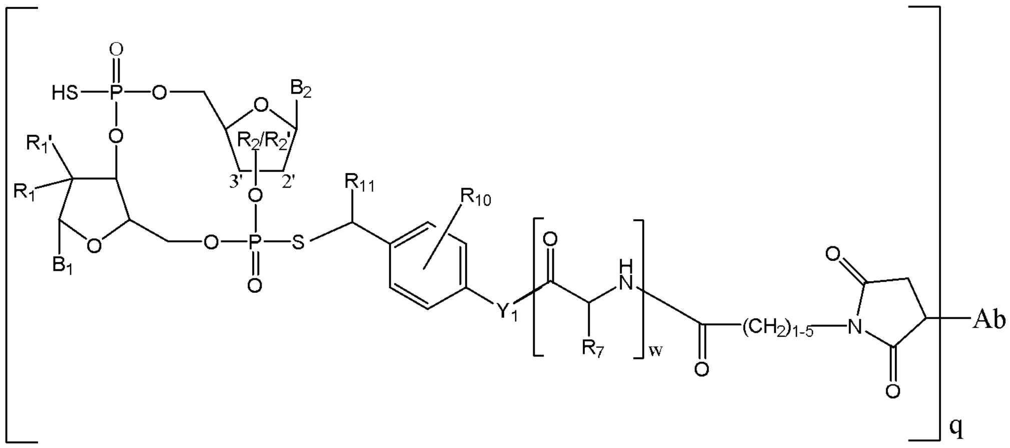

(X1-1),

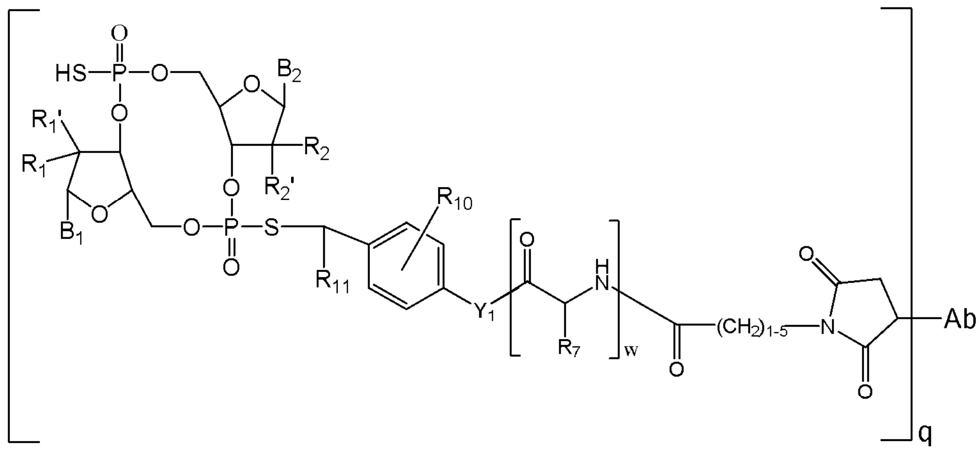

(X1-a-1)

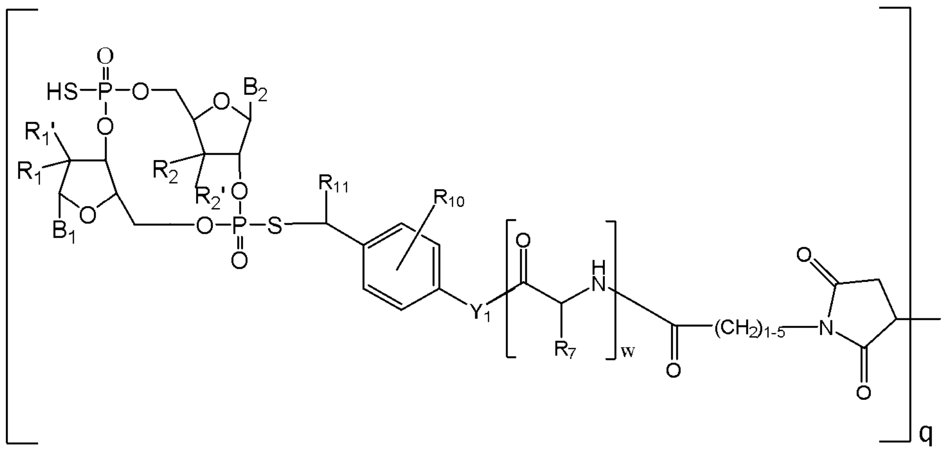

(X1-b-1)

In a further preferred embodiment, the ADC in Formula (X) of the present invention has the following formula:

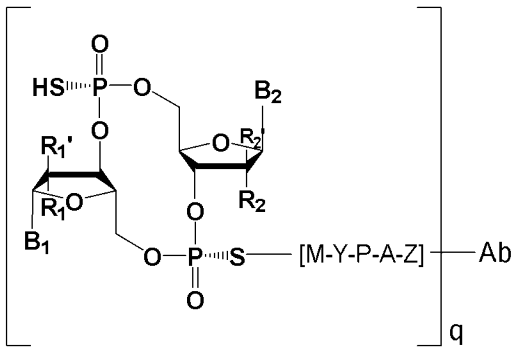

or

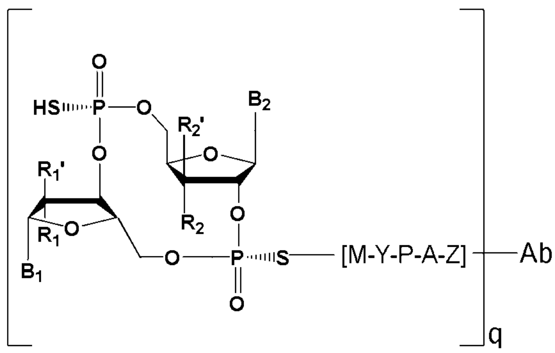

(X1-a') (X1-b')

More specifically:

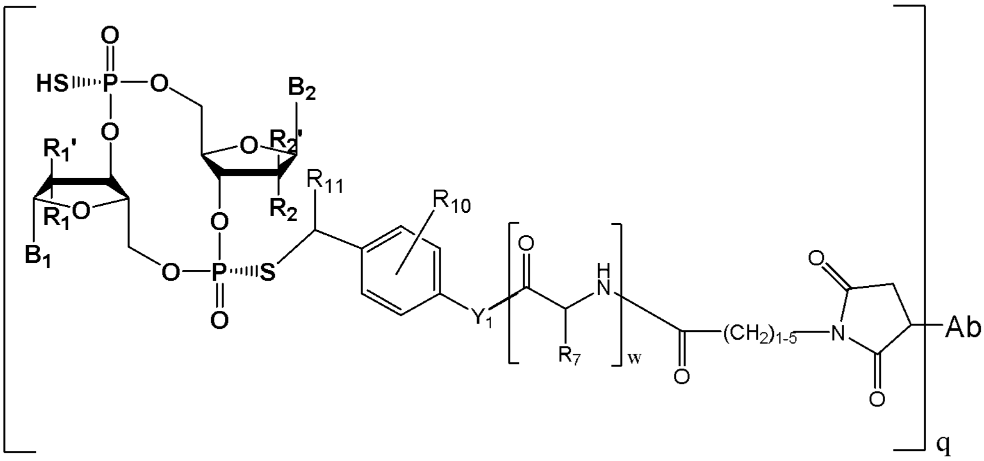

(X1-a'-1)

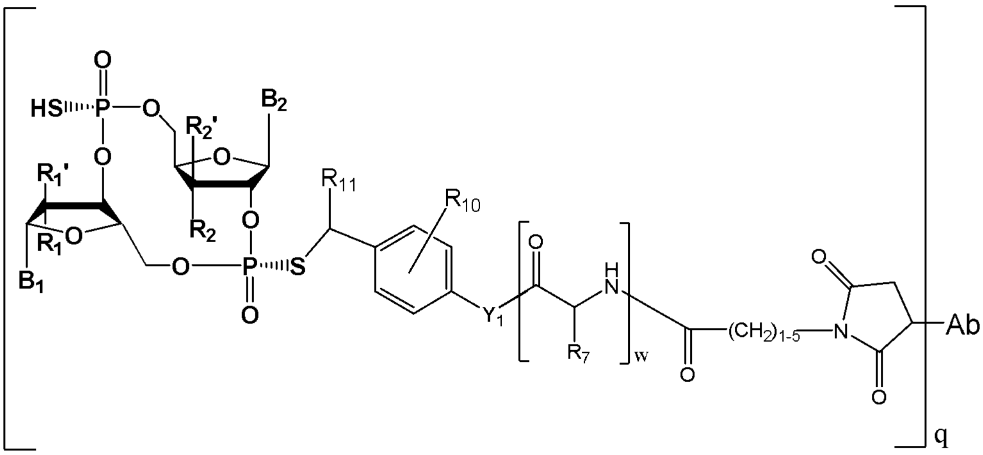

(X1-b'-1)

It should be mentioned that the above listed definitions of each group in the ADC of Formula (X1), (X1-a), (X1-b), (X1-1), (X1-a-1), (X1-b-1), (X1-a'), (X1-b'), (X1-a'-1), (X1-b'-1) of the present invention correspond to the definitions given above for each [L-D] general formula (Formula (IV), (IV-a), (IV-b), (IV-1), (IV-a-1), (IV-b-1), (IV-a'), (IV-b'), (IV-a'-1), and (IV-b'-1)) under **"exemplary CDN-linker units,** respectively, including the resulting technical protocols formed by the general, preferred, or specific definitions, and the resulting technical protocols formed by any combination of the general, preferred, or specific definitions.

In a preferred set of embodiment, q in the ADC of Formula (X1), (X1-a), (X1-b), (X1-1), (X1-a-1), (X1-b-1), (X1-a'), (X1-b'), (X1-a'-1), (X1-b'-1) of the present invention represents an average DAR value in a range of 1 to 10 or any two values between 1 and 10, e.g. about 1, 2, 3, 4, 5, 6, 7, 8, 9, 10, 1 to 2, 1 to 3, 1 to 4, 1 to 5, 1 to 6, 1 to 7, 1 to 8, 1 to 9, 1 to 10, 2 to 10, 2 to 8, 2 to 6, 2 to 4, 4 to 6, 4 to 8, 4 to 10, 6 to 8, or 6 to 10.

In a preferred set of embodiments, q in the ADC of Formula (X1), (X1-a), (X1-b), (X1-1), (X1-a-1), (X1-b-1), (X1-a'), (X1-b'), (X1-a'-1), (X1-b'-1) of the present invention represents an average DAR value in a range of 1 to 8 or any two values between 1 and 8, e.g. about 1, 2, 3, 4, 5, 6, 7, 8, 1 to 2, 1 to 3, 1 to 4, 1 to 5, 1 to 6, 1 to 7, 1 to 8, 2 to 8, 2 to 6, 2 to 4, 4 to 6, 4 to 8, or 6 to 8.

In some preferred embodiments, q represents an average DAR of about 3. In some preferred embodiments, q represents an average DAR of about 6. In some preferred embodiments, q represents an average DAR of about 8.

In a preferred set of embodiments, Ab in ADC of Formula (X1), (X1-a), (X1-b), (X1-1), (X1-a-1), (X1-b-1), (X1-a'), (X1-b'), (X1-a'-1), (X1-b'-1) of the present invention are antibodies or antigen-binding fragments thereof that bind tumor-specific antigens or tumor-associated antigens.

In a preferred set of embodiments, Ab-bound tumor-specific antigens or tumor-associated antigens in the ADC of Formula (X1), (X1-a), (X1-b), (X1-1), (X1-a-1), (X1-b-1), (X1-a'), (X1-b'), (X1-a'-1), (X1-b'-1) of the present invention are selected from: HER2, Her3, HER1 (ErbB1), HER4 (ErbB4), TROP2, Nectin4, tissue factor, PD-L1, PD-1, MET, CLDN18.2, KIT, CTLA-4, RPR1, epinephrine A2 receptor (EphA2), folate receptor (FRa), mesothelin, endothelin receptor, GCPII, IL-13Ra, BCMA, GD2, CLL-1, CA-IX, MUC1, 5T4, AOC3, ALK, AXL, C242, CA-125, CCL11, CCR5, CD138, CD2, CD3, CD4, CDS, CD15, CA15-3, CD18, CD19, CD20, CD21, CD22, CD30, CD33, CD37, CD38, CD52, CD56, CD66e, CD70, CD72, CD74, CD79a, CD79b, CD123, CD174, CD276, CCD79b, CA19-9, AGS-16, IGF1R, IGF2R, VEGFR1, VEGFR2,

VEGFR3, PDGFR-α, PDGFR-β, EGFR, EGFRvIII, SLTRK6, BMPR1B, E16, TOP1, STEAP1, 0772P, MUC16, Napi3b, Sema 5b, PSCA hIg, ETBR, RNF124, prostate cancer-related gene 1, TrpM4, teratoma derived growth factor 1, C3DR, FcRH2, NCA, MDP, IL20R-α, Brevican, EphB2R, ASLG659, prostate stem cell antigen precursor, GEDA, BAFF-R, CXCR5, HLA-DOB, P2X5, LY64, FcRH1, IRTA2, TENB2, Integrin α5β6, Integrin α4β7, FGF2, FGFR1, FGFR2, FGFR3, FGFR4, PSMA, Somatostatin receptor, RANK, SLAMF7, ITGB6, CEACAM5, MUC1, CA9, EGFRvlll, IL2RA, AXL receptor tyrosine kinase, TGF-βR, TNFRSF8, carcinoma/testis associated antigen, CLEC14A, GRP78, stem cell-specific antigen, ASG-5, PRR4, GUCY2C, SLC39A6, TPBG, middle tumor-associated antigen CA242, FOLR1, GPNMB, HAVCR1, prostate tumor target Mindin, VTCN1, PTK7 protein tyrosine kinase 7, macrophage stimulation 1 receptor, TACSTD2, CA6, DLL3, DLL4, EpCAM, FAP, DKK-1, Endoglin, VCAM1, GPC3, DR5, ASCT2, B7H4.

In a more preferred set of embodiments, in the present invention, the antigens bound by the Ab in ADCs with Formula (X1), (X1-a), (X1-b), (X1-1), (X1-a-1), (X1-b-1), (X1-a'), (X1-b'), (X1-a'-1), (X1-b'-1) are selected from HER2, KIT, FOLR1, CD276, PD-L1, NECTIN4, Mesothelin, MUC1, GCPII, BCMA, cMet, RPR1, CD22, CD19, TOP1, Claudin 18.2, EGFR, Trop-2; and more preferably from HER2, Claudin 18.2, and Trop-2.

In a more preferred set of embodiments, Ab in ADCof Formula (X1), (X1-a), (X1-b), (X1-1), (X1-a-1), (X1-b-1), (X1-a'), (X1-b'), (X1-a'-1), (X1-b'-1) of the present invention are antibodies that specifically bind HER2 or antigen-binding fragments thereof.

In this set of more preferred embodiments, Ab in the ADC of Formula (X1), (X1-a), (X1-b), (X1-1), (X1-a-1), (X1-b-1), (X1-a'), (X1-b'), (X1-a'-1), (X1-b'-1) of the present invention contains 3 heavy chain complementarity determining regions (HCDRs) and 3 light chain complementarity determining regions (LCDRs), wherein: as defined by Kabat,

HCDR1 contains or consists of the amino acid sequence of SEQ ID NO: 6,
HCDR2 contains or consists of the amino acid sequence of SEQ ID NO: 7,
HCDR3 contains or consists of the amino acid sequence of SEQ ID NO: 8,
LCDR1 contains or consists of the amino acid sequence of SEQ ID NO: 3,
LCDR2 contains or consists of the amino acid sequence of SEQ ID NO: 4, and
LCDR3 contains or consists of the amino acid sequence of SEQ ID NO: 5.

In this set of more preferred embodiments, Ab in the ADC of Formula (X1), (X1-a), (X1-b), (X1-1), (X1-a-1), (X1-b-1), (X1-a'), (X1-b'), (X1-a'-1), (X1-b'-1) of the present invention contains a heavy chain variable region and a light chain variable region, wherein the heavy chain variable region contains the amino acid sequence shown in SEQ ID NO: 10, and the light chain variable region contains the amino acid sequence shown in SEQ ID NO: 9.

In this set of more preferred embodiments, the Ab in the ADC of Formula (X1), (X1-a), (X1-b), (X1-1), (X1-a-1), (X1-b-1), (X1-a'), (X1-b'), (X1-a'-1), (X1-b'-1) of the present invention contains:

(a) a heavy chain containing the amino acid sequence of SEQ ID NO: 2, and
(b) a light chain containing the amino acid sequence of SEQ ID NO: 1.

In this set of more preferred embodiments, the Ab in the ADC of Formula (X1), (X1-a), (X1-b), (X1-1), (X1-a-1), (X1-b-1), (X1-a'), (X1-b'), (X1-a'-1), (X1-b' -1) of the present invention is Trastuzumab.

In another set of more preferred embodiments, the Ab in the ADC of Formula (X1), (X1-a), (X1-b), (X1-1), (X1-a-1), (X1-b-1), (X1-a'), (X1-b'), (X1-a'-1), (X1-b'-1) of the present invention is an antibody or antigen-binding fragment thereof that specifically binds Trop-2.

In this set of more preferred embodiments, Ab in the ADC of Formula (X1), (X1-a), (X1-b), (X1-1), (X1-a-1), (X1-b-1), (X1-a'), (X1-b'), (X1-a'-1), (X1-b'-1) of the present invention contains 3 heavy chain complementarity determining regions (HCDRs) and 3 light chain complementarity determining regions (LCDRs), wherein: as defined by Kabat,

HCDR1 contains or consists of the amino acid sequence of SEQ ID NO: 18,
HCDR2 contains or consists of the amino acid sequence of SEQ ID NO: 19,
HCDR3 contains or consists of the amino acid sequence of SEQ ID NO: 20,
LCDR1 contains or consists of the amino acid sequence of SEQ ID NO: 15,
LCDR2 contains or consists of the amino acid sequence of SEQ ID NO: 16, and
LCDR3 contains or consists of the amino acid sequence of SEQ ID NO: 17.

In this set of more preferred embodiments, Ab in the ADC of Formula (X1), (X1-a), (X1-b), (X1-1), (X1-a-1), (X1-b-1), (X1-a'), (X1-b'), (X1-a'-1), (X1-b'-1) of the present invention contains a heavy chain variable region and a light chain variable region, wherein the heavy chain variable region contains the amino acid sequence shown in SEQ ID NO: 22, and the light chain variable region contains the amino acid sequence shown in SEQ ID NO: 21.

In this set of more preferred embodiments, the Ab in the ADC of Formula (X1), (X1-a), (X1-b), (X1-1), (X1-a-1),

(X1-b-1), (X1-a'), (X1-b'), (X1-a'-1), (X1-b'-1) of the present invention contains:

(a) a leavy chain containing the amino acid sequence of SEQ ID NO: 14, and
(b) a light chain containing the amino acid sequence of SEQ ID NO: 13.

In this group of more preferred embodiments, the Ab in the ADC of Formula (X1), (X1-a), (X1-b), (X1-1), (X1-a-1), (X1-b-1), (X1-a'), (X1-b'), (X1-a'-1), (X1-b'-1) of the present invention is Sacituzumab.
In another set of more preferred embodiments, the Ab in the ADC of Formula (X1), (X1-a), (X1-b), (X1-1), (X1-a-1), (X1-b-1), (X1-a'), (X1-b'), (X1-a'-1), (X1-b'-1) of the present invention is an antibody or antigen-binding fragment thereof that specifically binds Claudin 18.2.
In this group of more preferred embodiments, the Ab in the ADC of Formula (X1), (X1-a), (X1-b), (X1-1), (X1-a-1), (X1-b-1), (X1-a'), (X1-b'), (X1-a'-1), (X1-b'-1) of the present invention contains 3 heavy chain complementarity determining regions (HCDR) and 3 light chain complementarity determining regions (LCDR), wherein: as defined by Kabat,

HCDR1 contains or consists of the amino acid sequence of SEQ ID NO: 29,
HCDR2 contains or consists of the amino acid sequence of SEQ ID NO: 30,
HCDR3 contains or consists of the amino acid sequence of SEQ ID NO: 31,
LCDR1 contains or consists of the amino acid sequence of SEQ ID NO: 26,
LCDR2 contains or consists of the amino acid sequence of SEQ ID NO: 27, and
LCDR3 contains or consists of the amino acid sequence of SEQ ID NO: 28.

In this group of more preferred embodiments, the Ab in the ADC of Formula (X1), (X1-a), (X1-b), (X1-1), (X1-a-1), (X1-b-1), (X1-a'), (X1-b'), (X1-a'-1), (X1-b'-1) of the present invention contains a heavy chain variable region and a light chain variable region, wherein the heavy chain variable region contains the amino acid sequence shown in SEQ ID NO: 33 and the light chain variable region contains the amino acid sequence shown in SEQ ID NO: 32.
In this group of more preferred embodiments, the Ab in the ADC of Formula (X1), (X1-a), (X1-b), (X1-1), (X1-a-1), (X1-b-1), (X1-a'), (X1-b'), (X1-a'-1), (X1-b'-1) of the present invention contains:

(a) a heavy chain containing the amino acid sequence of SEQ ID NO: 25, and

(b) a light chain containing the amino acid sequence of SEQ ID NO: 24.

In this group of more preferred embodiments, the Ab in the ADC of Formula (X1), (X1-a), (X1-b), (X1-1), (X1-a-1), (X1-b-1), (X1-a'), (X1-b'), (X1-a'-1), (X1-b'-1) of the present invention is Zolbetuximab.
In another set of more preferred embodiments, the Ab in the ADC of Formula (X1), (X1-a), (X1-b), (X1-1), (X1-a-1), (X1-b-1), (X1-a'), (X1-b'), (X1-a'-1), (X1-b'-1) of the present invention is an antibody or antigen-binding fragment thereof that specifically binds EGFR.
In this group of more preferred embodiment, the Ab in the ADC of Formula (X1), (X1-a), (X1-b), (X1-1), (X1-a-1), (X1-b-1), (X1-a'), (X1-b'), (X1-a'-1), (X1-b'-1) of the present invention contains 3 heavy chain complementarity determining regions (HCDR) and 3 light chain complementarity determining regions (LCDR), wherein: as defined by Kabat,

HCDR1 contains or consists of the amino acid sequence of SEQ ID NO: 40,
HCDR2 contains or consists of the amino acid sequence of SEQ ID NO: 41,
HCDR3 contains or consists of the amino acid sequence of SEQ ID NO: 42,
LCDR1 contains or consists of the amino acid sequence of SEQ ID NO: 37,
LCDR2 contains or consists of the amino acid sequence of SEQ ID NO: 38, and
LCDR3 contains or consists of the amino acid sequence of SEQ ID NO: 39.

In this group of more preferred embodiments, the Ab in the ADC of Formula (X1), (X1-a), (X1-b), (X1-1), (X1-a-1), (X1-b-1), (X1-a'), (X1-b'), (X1-a'-1), (X1-b'-1) of the present invention contains a heavy chain variable region and a light chain variable region, wherein the heavy chain variable region contains the amino acid sequence shown in SEQ ID NO: 44 and the light chain variable region contains the amino acid sequence shown in SEQ ID NO: 43.
In this group of more preferred embodiments, the Ab in the ADC of Formula (X1), (X1-a), (X1-b), (X1-1), (X1-a-1), (X1-b-1), (X1-a'), (X1-b'), (X1-a'-1), (X1-b'-1) of the present invention contains:

(a) a heavy chain containing the amino acid sequence of SEQ ID NO: 36, and

(b) a light chain containing the amino acid sequence of SEQ ID NO: 35.

(c) in this group of more preferred embodiments, the Ab in the ADC of the present disclosure is Cetuximab.

It should be mentioned that the ADC of the present disclosure covers ADC obtained from any combination of any embodiment generally or specifically defined in this disclosure for a drug D unit, any embodiment generally or specifically defined in this disclosure for a linker unit L, and any embodiment generally or specifically defined in this disclosure for an antibody Ab.

In specific preferred embodiments, specific examples of ADCs of Formula (I) of the present invention include:

| | | |
|---|---|---|
| 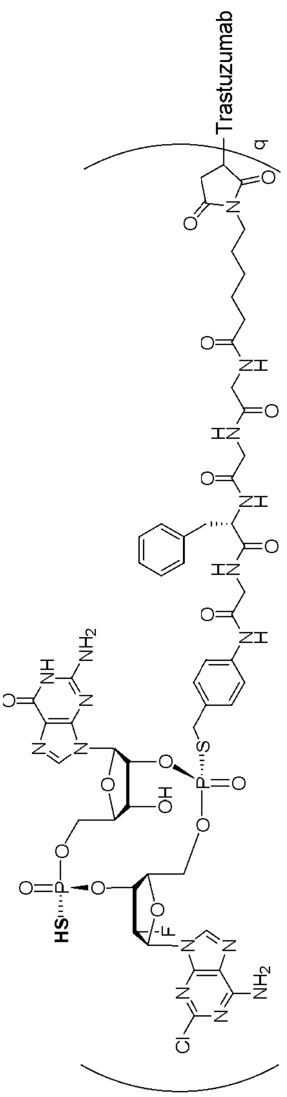 | 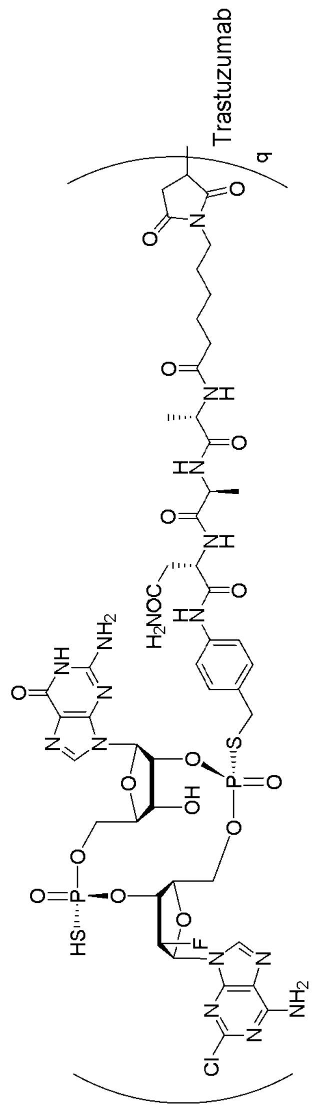 | 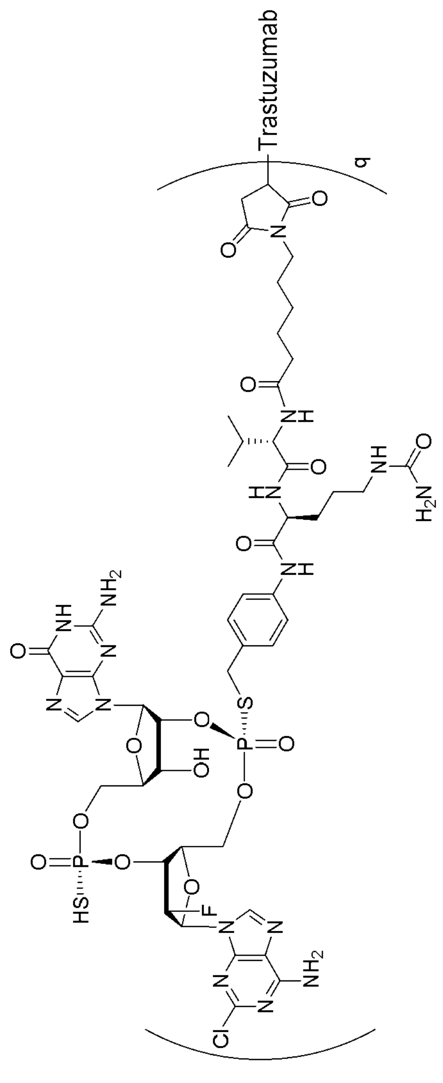 |
| ADC-1 | ADC-2 | ADC-3 |

(continued)

| | |
|---|---|
| ADC-4 | 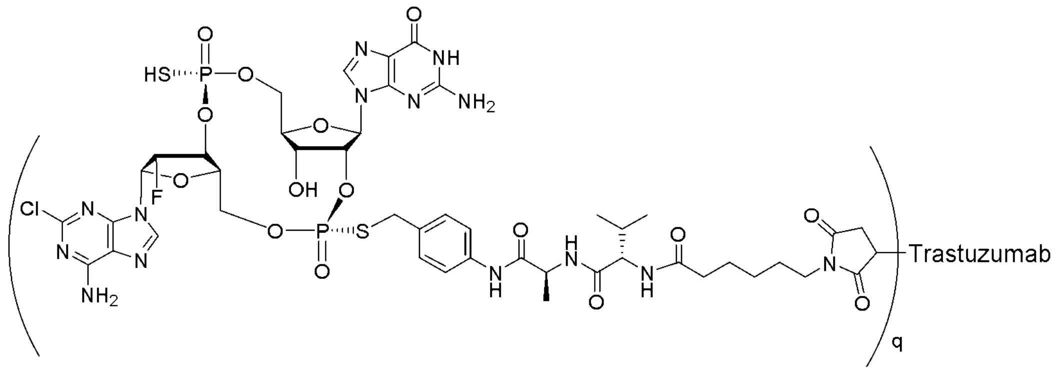 |
| ADC-5 | 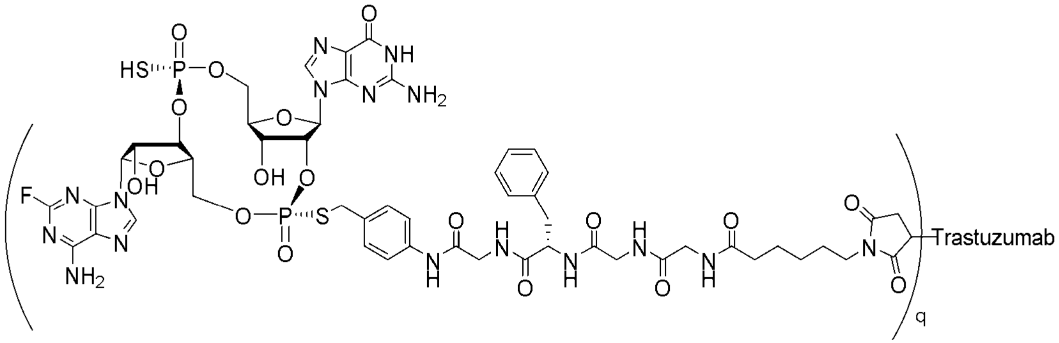 |
| ADC-6 | 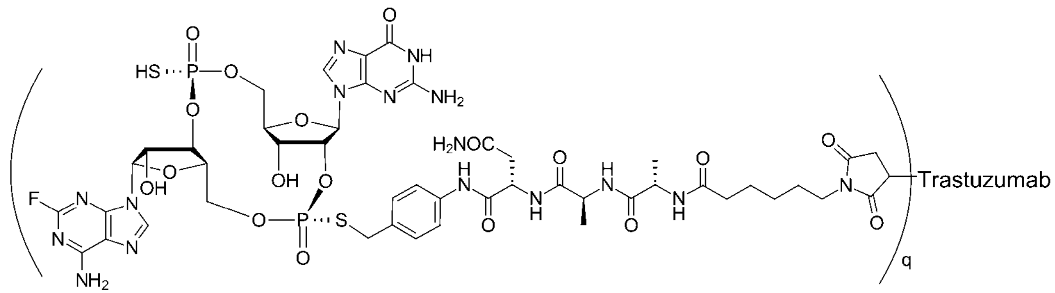 |

(continued)

| | |
|---|---|
| ADC-7 | 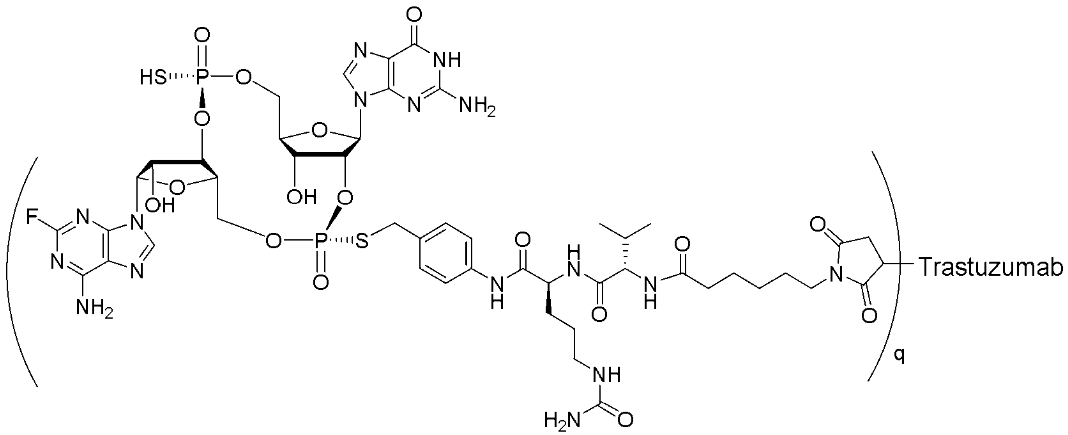 |
| ADC-8 | 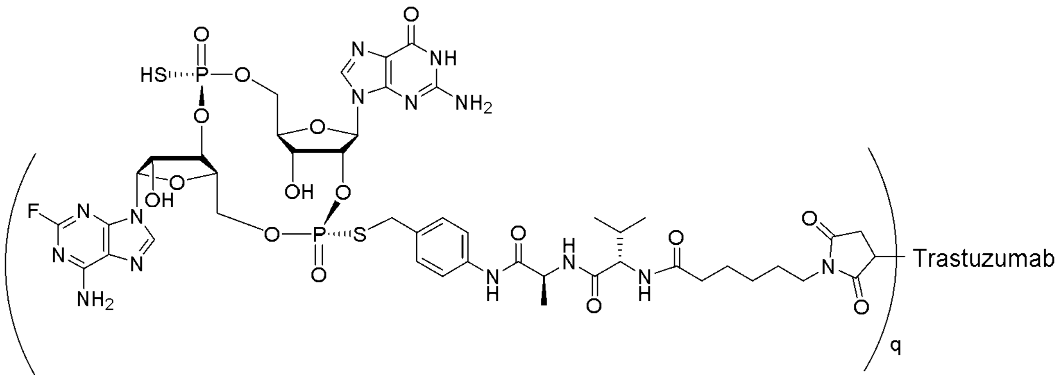 |
| ADC-9 | 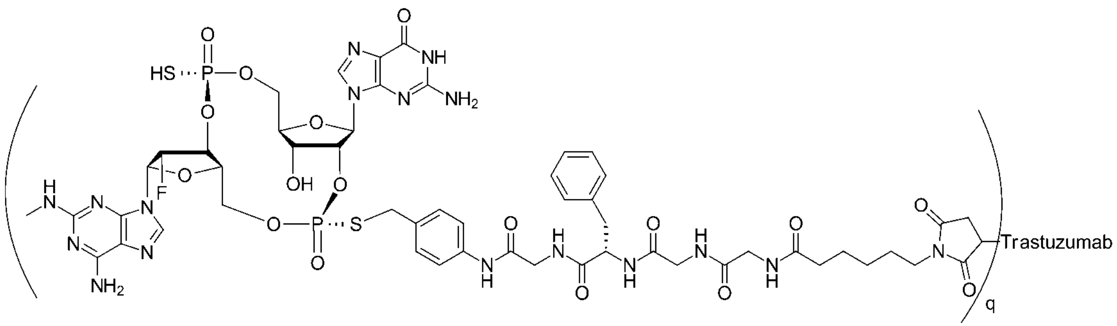 |

(continued)

| | |
|---|---|
| ADC-10 | 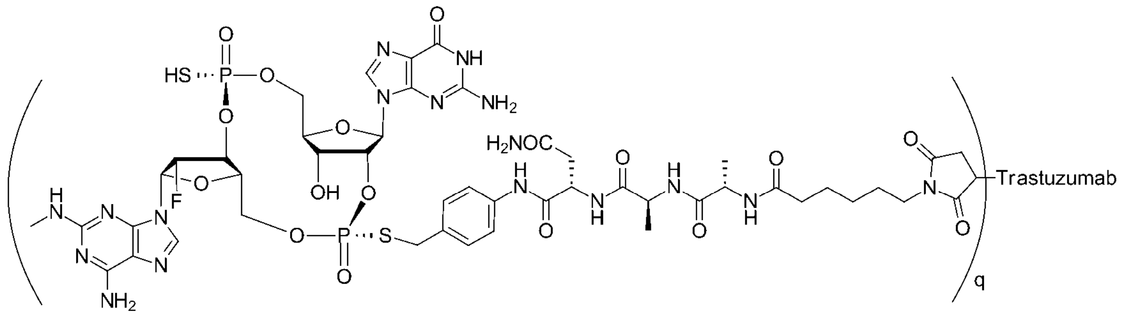 |
| ADC-11 | 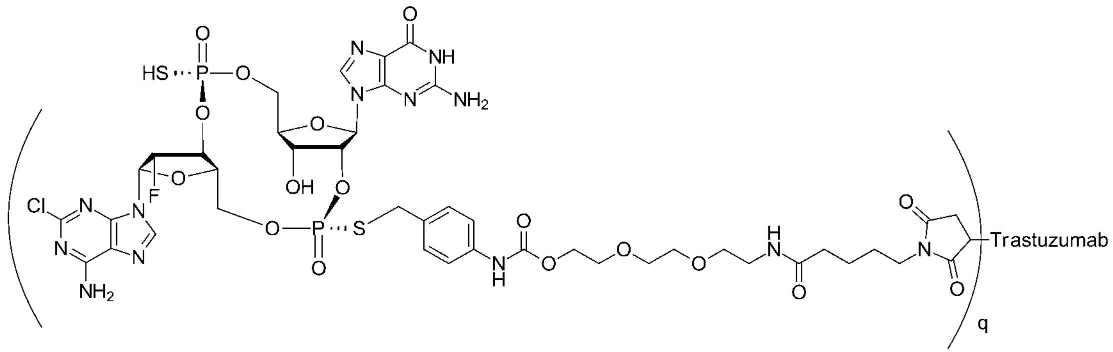 |
| ADC-12 | 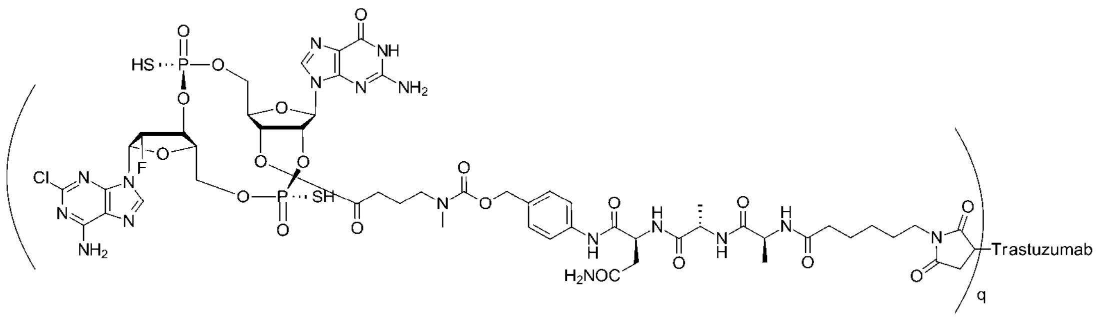 |

(continued)

| | |
|---|---|
| ADC-13 | 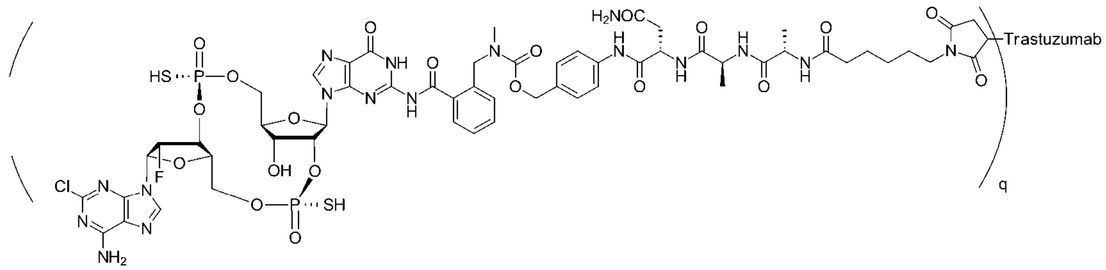 |
| ADC-15 | 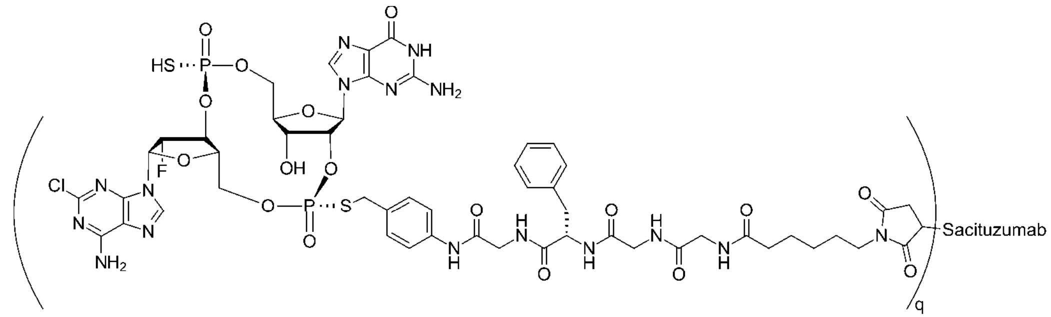 |
| ADC-16 | 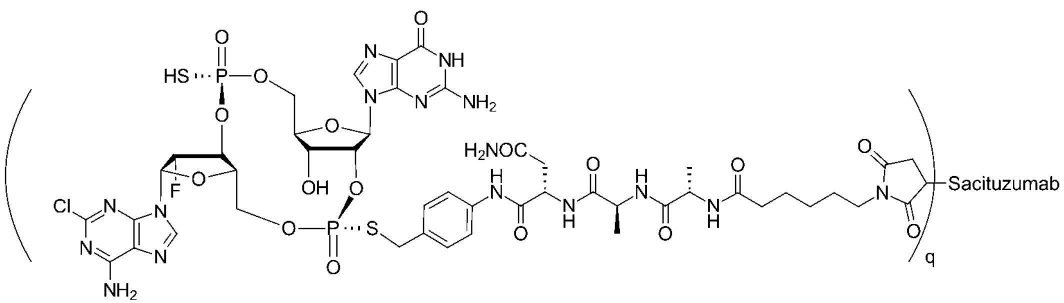 |

(continued)

| | |
|---|---|
| ADC-17 | 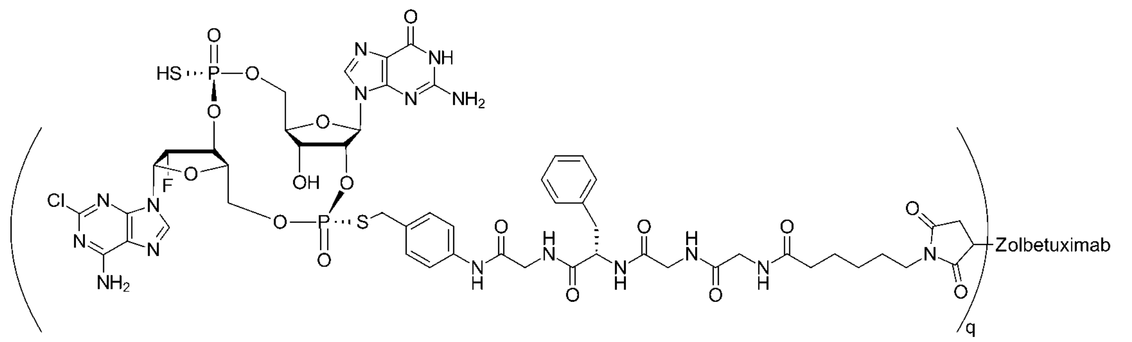 |
| ADC-18 | 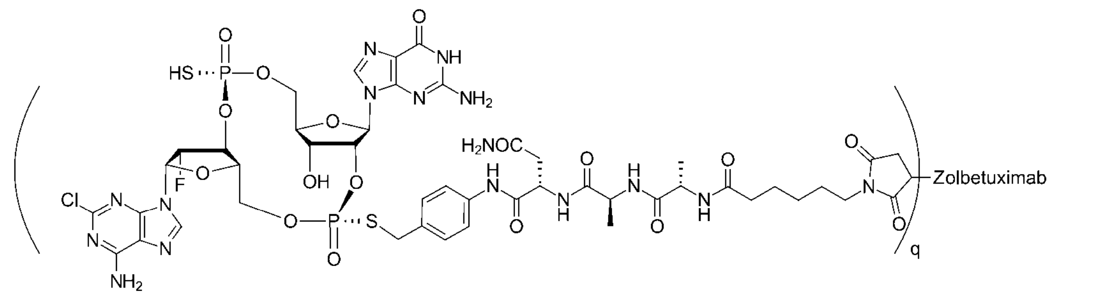 |
| ADC-19 | 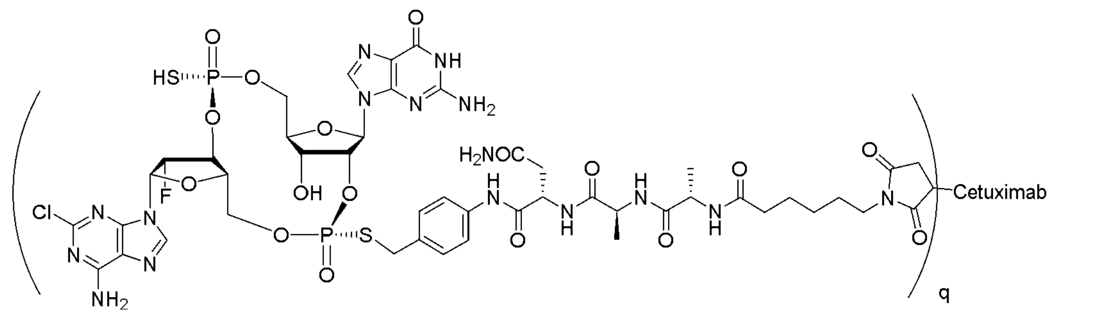 |

stereoisomers, tautomers, stable isotopic variants, pharmaceutically acceptable salts or solvates thereof,

wherein q represents an average DAR value of about 1-20, preferably an average DAR value of about 1 to 10, 1 to 8, e.g., about 2, 3, 4, 5, 6, 7, 8, 9, 10, about 1 to 2, 1 to 3, 1 to 4, 1 to 5, 1 to 6, 1 to 7, 1 to 8, 1 to 9, 1 to 10, 2 to 10, 2 to 8, 2 to 6, 2 to 4, 4 to 6, 4 to 8, 4 to 10, 6 to 8, or 6 to 10.

In some preferred embodiments, q represents an average DAR of about 3. In some preferred embodiments, q represents an average DAR of about 6. In some preferred embodiments, q represents an average DAR of about 8.

**II: Beneficial effects of the ADC of the present invention**

ADCs according to the present invention have at least one or more of the following advantages:

➢ Targeting tumor cells, activating STING innate immune pathway in tumor cells, killing tumor cells, releasing predesigned cytotoxic sub-structural units after CDN decomposition, further killing tumor cells, endowing efficient tumor killing effect in animals, providing tumor marker fragments for the acquired immune system, endowing the body with immune memory, and consolidating long-term immune effect;

➢ Compared with blood circulation distribution of CDN, the distribution of ADC to tumor environment is significantly promoted, which effectively limits the premature release of CDN load in plasma and reduces off-target toxicity;

➢ No obvious aggregation phenomenon, can achieve high drug loading, DAR can be as high as 8, through the branch chain, it is possible to obtain higher DAR value;

➢ ADC-CDN conjugate can effectively protect CDN from hydrolysis by phosphatases in blood, thus greatly improving the blood circulation stability of drug CDN;

➢ With the antibody acting as a guide, ADC-CDN is endocytosed by cancer cells that highly express the corresponding antigen, and can provide rapid and effective CDN release in tumor cells, and CDNs decompose and then release cytotoxic molecules after exerting STING agonist function;

➢ CDN obtains phosphorylated fragment molecules after intracellular breakdown, which crosses the key activation step of traditional cytotoxic molecules and reduces drug resistance;

➢ Phosphorylated fragment molecules obtained from CDNs after intracellular decomposition are not easily pumped out of cancer cells and reduce drug resistance;

➢ Significant tumor growth inhibition activity compared to antibodies alone and bifunctional CDN STING agonists alone was demonstrated in animal models;

➢ It shows a wide safety window in all (numerous) in vivo efficacy models;

➢ Acceptable PK profile;

➢ More traditional, mature and friendly administration methods (such as intravenous, subcutaneous, intramuscular, external application, etc.) can be used instead of intratumoral administration to prolong the efficacy and reduce toxic and side effects.

Specifically, the target inhibition of ADC compounds described in this application can be that the proliferation ability of tumor cells with high expression of specific targets is reduced by more than 1%, more than 5%, more than 20%, more than 50% or even 90%, and more than 95% when the proposed compound is added to the culture medium of tumor cells with high expression of specific targets compared with the addition of negative control or control drugs. For example, the said target inhibition could be an IC50 value (nM) of less than 1000, less than 500, or less than 100 for tumor cells with high expression of a particular target, such as 1 to 500, 1 to 100; for example, the said tumor cells could include, but not be limited to, solid tumor cells, such as breast adenocarcinoma cells, gastric cancer cells, lymphoma cells, ovarian adenocarcinoma cells, etc.; and the said specific targets include but are not limited to HER2, TROP2, EGFR, and **Claudin 18.2.**

Specifically, the in vivo antitumor effect of ADC compounds described in this application can be applied to animals

compared to the addition of control or control drugs, i.e. animals' tumors showed a volume reduction in 1, 3, 5, 7, 14, 21 or 30 days by more than 1%, more than 5%, more than 20%, more than 50%, even more than 90% or 95%. For example, compared to the administration of negative control and control drugs, the tumors of the animals showed a volume reduction of more than 1.1-fold, more than 1.5-fold, more than 2-fold, more than 5-fold, more than 10-fold, even more than 100-fold or more than 500 in 1, 3, 5, 7, 14, 21 or 30 days. The animals described include, but are not limited to, mammals. The administration described includes, but is not limited to, oral, intravenous, instillation, intraperitoneal, or topical administration.

Specifically, the distribution of ADC compounds described in this application in tumor tissues of animals following administration can be more than 1%, more than 5%, more than 20%, more than 50%, or even more than 90%, 95% increase in distribution compared to other tissues and organs, or compared to CDNs. For example, there can be an increase in distribution of more than 1.1-fold, more than 1.5-fold, more than 2-fold, more than 5-fold, more than 10-fold, or even more than 100-fold or more than 500-fold. The animals include, but are not limited to, mammals, and the tissues or organs include, but are not limited to, heart, liver, spleen, lung, kidney, brain, etc. The administration described include, but is not limited to, oral, intravenous, instillation, intraperitoneal, or topical administration.

Specifically, the ADC compounds described in this application have good in vivo safety, with free toxin release rates of no more than 50%, no more than 20%, no more than 10%, no more than 1%, and no more than 0.1% in vivo after administration to animals. For example, the in vivo safety described is that compounds can be administered at concentrations above 0.5 mg/kg, 5 mg/kg, 10 mg/kg, 50 mg/kg, 100 mg/kg, or even 500 mg/kg in animals without producing toxic manifestations. The animals described include but are not limited to mammals. The administration described include, but is not limited to, oral, intravenous, instillation, intraperitoneal, or topical administration.

Specifically, ADC compounds described in this application are plasma stable, Cytotoxic drugs released after addition to plasma did not release more than 50%, no more than 20%, no more than 10%, and even no more than 1%, no more than 0.1% in 1, 3, 5, 7, 14, 21, or 30 days.

## III. Drug Composition

On another aspect, the present invention provides a pharmaceutical composition comprising ADC or CDN compounds of the present invention and one or more pharmaceutically acceptable excipients, which may comprise conventional components in pharmaceutical preparations, such as diluents, carriers, pH regulators, preservatives, solubilizers, stabilizers, humectants, emulsifiers, sweeteners, colorants, flavorants, salts used to alter osmolality, buffering agents, masking agents, antioxidants and other active agents. Suitable carriers and excipients are well-known to technicians in the field and are described in detail in e.g., Gennaro A.R. et al, Remington: The Science and Practice of Pharmacy (2000) Lippincott, Williams & Wilkins, Philadelphia.

The present invention also provides methods for preparing the compositions described using ADC or CDN compounds of the present invention, typical pharmaceutical compositions or dosage forms prepared by mixing the compounds of the present invention with carriers or excipients, which may be formulated in a manner well-known to technicians in the field and consistent with good medical practice, as shown in e.g., Gennaro A.R. et al., Remington: The Science and Practice of Pharmacy (2000) Lippincott, Williams & Wilkins, Philadelphia, or various countries' pharmacopoeias.

The drug composition may be in any suitable form, such as tablets, powders, capsules, sterile injectable preparations, solutions, dispersions, suspensions, syrups, sprays, suppositories, gels, emulsions, patches, etc., and may be administered to the patient by multiple routes, such as intravenous, intratumoral, subcutaneous, intramuscular, oral, intranasal, intrathecal, transdermal, or topical. The route of administration in any given situation may depend on the specific antibody and/or ADC, the subject, the nature and severity of the disease, and the subject's physical condition. In some embodiments, drug compositions will be administered intravenously, intratumorally, subcutaneously, or intramuscularly as liquid formulations.

In a preferred embodiment, ADC and/or CDNs and pharmaceutical compositions including them described herein are systemically administered, such as subcutaneously, intraperitoneally, intramuscularly, intravenously, particularly intravenously, especially sterile injectable formulations, such as sterile injectable solutions or suspensions in nontoxic and parenterally acceptable diluents or solvents, or prepared as lyophilized powders; in acceptable media or solvents, solutions such as water, 1,3-butanediol, Ringer's solution, or isotonic sodium chloride solution may be used; in addition, sterile nonvolatile oils may be routinely used as solvents or suspension media, for which any mild nonvolatile oils, including synthetic monoglycerides or diglycerides, fatty acids, may be used. In other embodiments, ADCs and/or CDNs and drug compositions including them described herein are administered topically at tumor sites, such as intratumoral or in the tumor microenvironment.

The compound of the present invention can be administered in a wide range and, of course, can be adjusted by the clinician on an individual basis in each specific case. For preventive or therapeutic purposes, the appropriate dosage of the ADC of the present invention (when used alone or in combination with one or more other therapeutic agents) will

depend on the type of disease to be treated, the type of antibody, the severity and course of the disease, whether the antibody is administered for preventive purposes or for therapeutic purposes, previous treatments, the patient's clinical history and response to the antibody, and at the discretion of the treating physician.

The pharmaceutical composition or preparation of the present invention may also contain more than one active ingredient, which is required for a particular indication to be treated, preferably having complementary activities that do not adversely affect each other. For example, it is desirable to also provide other therapeutic agents, including chemotherapeutic agents, angiogenesis inhibitors, cytokines, cytotoxic agents, other antibodies, small molecule drugs, or immunomodulators (e.g., immune checkpoint inhibitors or agonists). The active ingredients are combined appropriately in amounts that are effective for the purpose of use.

**IV: Drug combination and kit**

In some embodiments, the present invention also provides a pharmaceutical combination or pharmaceutical combination product comprising ADC and/or CDN of the present invention or pharmaceutically acceptable salts or solvates thereof and one or more other therapeutic agents for the prevention or treatment of diseases associated with or mediated by STING, more specifically for the treatment or prevention of inflammation, allergic or autoimmune diseases, infectious diseases or hyperproliferative diseases, especially tumors or viral infections; and thus the present invention also provides methods for the treatment or prevention of diseases associated with or mediated by STING, more specifically inflammation, allergic or autoimmune diseases, infectious diseases or hyperproliferative diseases, especially tumors or viral infections, in subjects, including administering a pharmaceutical combination of the present invention to humans or animals.

An additional purpose of the present invention is to provide a kit comprising a pharmaceutical combination of the present invention preferably in a pharmaceutical dosage unit form. Dosage units can thus be provided according to the dosing schedule or the interval between drug administrations.

In one embodiment, the kit of the present invention in the same package comprises:

- the first container containing a pharmaceutical composition comprising the ADC of the present invention or a pharmaceutically acceptable salt or solvate thereof;
- the second container containing a drug composition containing other therapeutic agents.

Depending on factors such as the disease to be treated and the condition of the individual, the technicians in the field can determine the mode and order of administration of each component of the combined finished product. A combination product of the present invention may be used in a therapeutic method of the present invention. In some embodiments, the present invention provides a combination product among which other therapeutic agents are, for example, therapeutic agents such as antibodies that effectively stimulate an immune response thereby further enhancing, stimulating, or up-regulating the immune response of a subject. In some embodiments, the combination product is indicated for the prevention or treatment of STING related or mediated diseases, especially tumors.

**V. Use and Methods**

In view of the ability of the CDN compound loaded with the ADC of the present invention to activate STING, induce expression of type I interferons and pro-inflammatory cytokines such as IL-6, TNF-$\alpha$ and IFN-$\gamma$, and exhibit cytotoxic activity, on another aspect, the present invention provides therapeutic uses and methods of the ADC and/or CDN compound of the present invention.

In some embodiments, the present invention provides ADCs (i.e., ADCs of Formula A and its various subformulas) or CDNs (i.e., CDNs of Formula Y and its various sub-formulas, e.g., CDN-1 or CDN-2) disclosed herein for therapeutic purposes.

The ADC and/or CDNs provided in the present invention may be used alone or in combination with each other and/or in combination with other therapeutic agents. As shown in the following example, ADC and CDN can promote immune response when administered to subjects. For example, the ADC and/or CDN provided in the present invention, alone or in combination, are capable of inducing interferon B (IFNB) in human subjects, thanks in part to their ability to activate STING. CDNs in ADCs bind to antibodies or antigen-binding fragments thereof that bind tumor-associated antigens or immune cell antigens and target them for delivery to tumor-associated immune cells or the tumor microenvironment to trigger the activation of STING and produce an immune response, kill tumor cells and produce anti-inflammatory and antiviral effects, while prolonging or enhancing the immune response; subsequently, CDNs released from ADCs further decompose and release the cytotoxic molecule part in the structure and further inhibit the survival of tumor cells.

Therefore, the ADC of the present invention can promote immune responses as well as tumor cell killing, which is

more potent than antibodies contained in unconjugated CDNs or ADCs. In other words, the present invention achieves synergy by combining the prepared CDN with a specific immunotherapeutic antibody, which in turn is further reinforced by the cytotoxic part released by further decomposition of the CDN, thereby achieving a triple synergistic effect.

Therefore, in one aspect, the present invention accordingly provides methods for inducing, stimulating or assisting immune responses in an individual, including administering the ADC of the present invention or a pharmaceutically acceptable salt or solvate thereof or a pharmaceutical composition comprising the same to an individual. In one embodiment, the ADC or a pharmaceutically acceptable salt or solvate thereof or a pharmaceutical composition comprising the same is administered to an individual as immunotherapy to induce in vivo production of one or more cytokines useful for human or animal therapy, including type I interferons and pro-inflammatory cytokines such as IL-6, TNF-$\alpha$ and IFN-$\gamma$, to modulate the immune system of that person or animal for certain therapeutic benefits.

On another aspect, the present invention correspondingly provides methods for treating or preventing diseases associated with or mediated by an immune response, specifically diseases associated with or mediated by STING, including inflammatory, allergic or autoimmune diseases, infectious diseases, or cancers, including administering therapeutically effective amounts of the ADC of the present invention or a pharmaceutically acceptable salt or solvate thereof or a pharmaceutical composition comprising the same to subjects in need.

On another aspect, the present invention provides the use of the ADC or a pharmaceutically acceptable salt or solvate thereof or a pharmaceutical composition comprising the same for the prevention or treatment of diseases associated with or mediated by STING, and more specifically for the treatment or prevention of inflammatory, allergic or autoimmune diseases, infectious diseases or hyperproliferative diseases, especially tumors or viral infections.

On another aspect, the present invention provides the ADC of the present invention or a pharmaceutically acceptable salt or solvate thereof or a pharmaceutical composition comprising the same for use as a therapeutic agent for the treatment or prevention of diseases associated with or mediated by STING, more specifically as a therapeutic agent for the treatment or prevention of inflammation, allergic or autoimmune diseases, infectious diseases or hyperproliferative diseases, especially anti-tumor or antiviral therapies, or as vaccine adjuvants; specifically, as cytotoxic agents for the treatment or prevention of hyperproliferative diseases, especially tumors, or as cytotoxic agents for the treatment or prevention of viral infections.

In a preferred embodiment, the ADC or a pharmaceutically acceptable salt or solvate thereof or a pharmaceutical composition comprising the same is used as a cytotoxic agent for the treatment or prevention of hyperproliferative diseases, especially tumors. In another preferred embodiment, the ADC or a pharmaceutically acceptable salt or solvate thereof or a pharmaceutical composition comprising the same is used to treat recurrent tumors or to prevent tumor recurrence.

On another aspect, the present invention provides ADCs or pharmaceutically acceptable salts or solvates thereof or pharmaceutical compositions comprising the same as multifunctional active agents, which combine immunotherapeutic activity with cytotoxic therapeutic activity, including the ability to activate the immune system by activating the STING signaling pathway for antitumor and antiviral replication, to cause tumor cell death or prevent viral replication by releasing cytotoxic agents, to continuously activate STING by releasing tumor DNA to kill tumor cells, and to generate antibody-antigen responses by releasing tumor neoantigens to provide an "immunological memory" or long-lasting immunity to tumors. In this aspect, the present invention also provides the use of the ADC of the present invention or a pharmaceutically acceptable salt or solvate thereof or a pharmaceutical composition comprising the same for achieving the various functions mentioned above.

On another aspect, the present invention provides methods for treating or preventing diseases associated with or mediated by STING, more specifically inflammation, allergic or autoimmune diseases, infectious diseases or hyperproliferative diseases, especially tumors or viral infections, in subjects, including administering the ADC of the present invention or a pharmaceutically acceptable salt or solvate thereof or a pharmaceutical composition comprising the same to humans or animals; specifically, the present invention provides methods for treating or preventing hyperproliferative diseases, especially tumors, in subjects, or for treating or preventing viral infections in subjects, which includes administering the ADC of the present invention or a pharmaceutically acceptable salt or solvate thereof or a pharmaceutical composition comprising the same to humans or animals.

On another aspect, the present invention provides the use of the ADC or a pharmaceutically acceptable salt or solvate thereof or a pharmaceutical composition comprising the same in the preparation of a medicament for the prevention or treatment of diseases associated with or mediated by STING, more specifically inflammatory, allergic or autoimmune diseases, infectious diseases or hyperproliferative diseases, especially tumors or viral infections, or in the preparation of vaccine adjuvants; specifically, the present invention provides the use of the ADC of the present invention or a pharmaceutically acceptable salt or solvate thereof or a pharmaceutical composition comprising the same in the preparation of a medicament for the treatment or prevention of hyperproliferative diseases, especially tumors, and the use of the ADC or a pharmaceutically acceptable salt or solvate thereof or a pharmaceutical composition comprising the same in the preparation of a medicament for the treatment or prevention of viral infections.

Hyperproliferative disease, defined for theuses and methods described above, refers to a physiological condition

in a subject characterized by uncontrolled or dysregulated cell growth or death, especially tumor or cancer, including solid and hematogenous tumors, including, but not limited to, brain cancer, skin cancer, bladder cancer, ovarian cancer, breast cancer, gastric cancer, pancreatic cancer, prostate cancer, colon cancer, blood cancer, lung cancer, and bone cancer, for example neuroblastoma, intestinal cancer such as rectal cancer, colon cancer, familial adenomatous polyposis carcinoma, and hereditary nonlymphocytic colorectal cancer, esophageal cancer, lip cancer, laryngeal cancer, nasopharyngeal carcinoma, oral cancer, salivary gland cancer, peritoneal cancer, soft tissue sarcoma, urothelial carcinoma, hidradenocarcinoma, gastric cancer, adenocarcinoma, medullary thyroid cancer, papillary thyroid cancer, renal carcinoma, renal parenchymal carcinoma, ovarian cancer, cervical cancer, uterine corpus cancer, endometrial cancer, pancreatic cancer, prostate cancer, testicular cancer, breast cancer (including HER2-negative breast cancer), urinary cancer, melanoma, brain tumors such as glioblastoma, astrocytoma, meningioma, medulloepithelioma, and peripheral neuroectodermal tumor, Hodgkin lymphoma, non-Hodgkin lymphoma, Burkitt's lymphoma, acute lymphoblastic leukemia (ALL), chronic lymphocytic leukemia (CLL), chronic myelogenous leukemia (CLL), and lymphocytic carcinoma, acute myelogenous leukemia (AML), myelogenous leukemia (chronic myelogenous leukemia (CML), adult T-cell lymphoma, diffuse lymphoma (DLBCL), hepatoma, multiple myeloma, seminoma, osteosarcoma, chondrosarcoma, anal canal cancer, adrenocortical carcinoma, chordoma, fallopian tube cancer, gastrointestinal stromal tumor, myeloproliferative disorder, mesothelioma, biliary tract cancer, Ewing's sarcoma, and other rare tumor types, as well as recurrent forms of these tumors.

In a preferred embodiment, the hyperproliferative disease for the above uses and methods are small cell lung cancer, non-small cell lung cancer, colorectal cancer, liver cancer, breast cancer, ovarian cancer, gastric cancer, prostate cancer, melanoma, renal cell carcinoma, head and neck cancer, pancreatic cancer, Hodgkin lymphoma, leukemia, or bladder cancer.

For the above uses and methods, virus infection refers to the process that the virus invades the body through a variety of ways and proliferates in susceptible host cells, and the viruses involved include but are not limited to double-stranded DNA virus and single-stranded DNA virus, single-positive-sense RNA virus, single-negative-sense RNA virus and double-stranded RNA virus and retrovirus, with examples of hepatitis B virus, TTV virus, adenovirus, papillomavirus, herpes zoster virus, smallpox virus and vaccinia virus, influenza virus, classical swine fever virus, hepatitis A virus, hepatitis C virus, hepatitis D virus, hepatitis E virus, hepatitis G virus, rabies virus, ebolavirus, enterovirus and human immunodeficiency virus. The therapeutic uses and methods provided in the present invention may be used for the above virus infections and the resulting disease.

Regarding the use of the ADC or a pharmaceutically acceptable salt or solvate thereof or a pharmaceutical composition comprising the same as a vaccine adjuvant and in the preparation of a vaccine adjuvant, this description means that the ADC or a pharmaceutically acceptable salt or solvate thereof or a pharmaceutical composition comprising the same may be used as an adjuvant in therapeutic or preventive strategies employing vaccines, i.e., the ADC or a pharmaceutically acceptable salt or solvate thereof or a pharmaceutical composition comprising the same is used in conjunction with one or more vaccines selected to stimulate immune responses to one or more predetermined antigens, the vaccine contains inactivated or attenuated bacteria or viruses, such as containing one or more inactivated tumor cells expressing and secreting GM-CSF, CCL-20, CCL3, IL-12p70, FLT-3 ligands, cytokines.

## VI: Preparation of ADC of the present invention

### 1. General synthesis method

The compounds of the present invention, stereoisomers, tautomers, stable isotopic variants, pharmaceutically acceptable salts or solvates thereof may be prepared by a variety of methods well-known in the field of organic synthesis, including those given below, those given in examples, or those similarly understood by technicians in the field.

The following are examples of general synthetic schemes for the synthesis of compounds of the present invention. For each reaction step, the appropriate reaction conditions are known or can be routinely determined by the technical staff in the field. Specifically, the process steps used to synthesize the compounds of the present invention may be carried out under reaction conditions known to itself (including those specifically mentioned), in the absence or generally in the presence of solvents or diluents (including, for example, solvents or diluents that are inert to the reagents used and dissolve the reagents used), in the absence or presence of catalysts, condensing agents, or neutralizers (e.g., ion exchangers, such as cation exchangers, such as H + forms), at reduced, normal, or elevated temperatures (e.g., about -100°C to about 190°C, including, e.g., about -78°C to about 150°C, e.g., about 0°C to about 125°C, room temperature, -20°C to 40°C, or reflux temperature), under atmospheric pressure or in a closed container, under pressure when appropriate and/or under inert atmosphere, such as oxygen or nitrogen atmosphere.

The raw materials and reagents used in the preparation of these compounds are generally commercially available or may be prepared by methods below, similar to those given below, or known in the field. If desired, the raw materials and intermediates in the synthetic reaction process can be isolated and purified using conventional techniques,

which include but are not limited to filtration, distillation, crystallization, chromatography, etc. The materials can be characterized by conventional methods including physical constants and spectral data.

Unless otherwise indicated in the description of the method, the solvents suitable for any particular reaction include: those solvents specifically mentioned, or, for example, water; esters, such as lower fatty acid lower alkyl esters, such as ethyl acetate; ethers, such as aliphatic ethers, such as ether, or cyclic ethers, such as tetrahydrofuran or dioxane; liquid aromatic hydrocarbons, such as benzene or toluene; alcohols, such as methanol, ethanol, or 1- or 2-propanol, such as acetonitrile; halogenated hydrocarbons, such as dichloromethane or chloroform; amides, such as *N,N*-dimethylformamide or *N,N*-dimethylacetamide; bases, such as heterocyclic azoles, such as pyridine; carboxylic anhydrides, such as lower aliphatic carboxylic anhydrides, such as acetate; cyclic, linear, or branched hydrocarbons, such as cyclohexane, hexane, or isopentane; or mixtures of these solvents, such as aqueous solutions. Such solvent mixtures may also be used for post-processing, such as by chromatography or partition.

Technicians in this field can recognize the presence of stereocenters in compounds of Formula I. At all stages of the reaction, mixtures of formed isomers can be separated into individual isomers, such as diastereomers or enantiomers, or into mixtures of any desired isomers, such as racemates or mixtures of diastereomers, see e.g., E. L. Eliel, S. H. Wilen, and L. N. Mander's "Stereochemistry of Organic Compounds" (Wiley-Interscience, 1994).

**2. Bifunctional cyclic dinucleotide CDN fragment synthesis**

Process 1 below illustrates a general synthetic route that can be used to prepare the bi-functional **CDN** compound herein and each specific embodiment. The variables of the general formulae in the following processes have the same meaning as in the compound defined herein or in each of its specific embodiments unless otherwise indicated.

**Process 1**

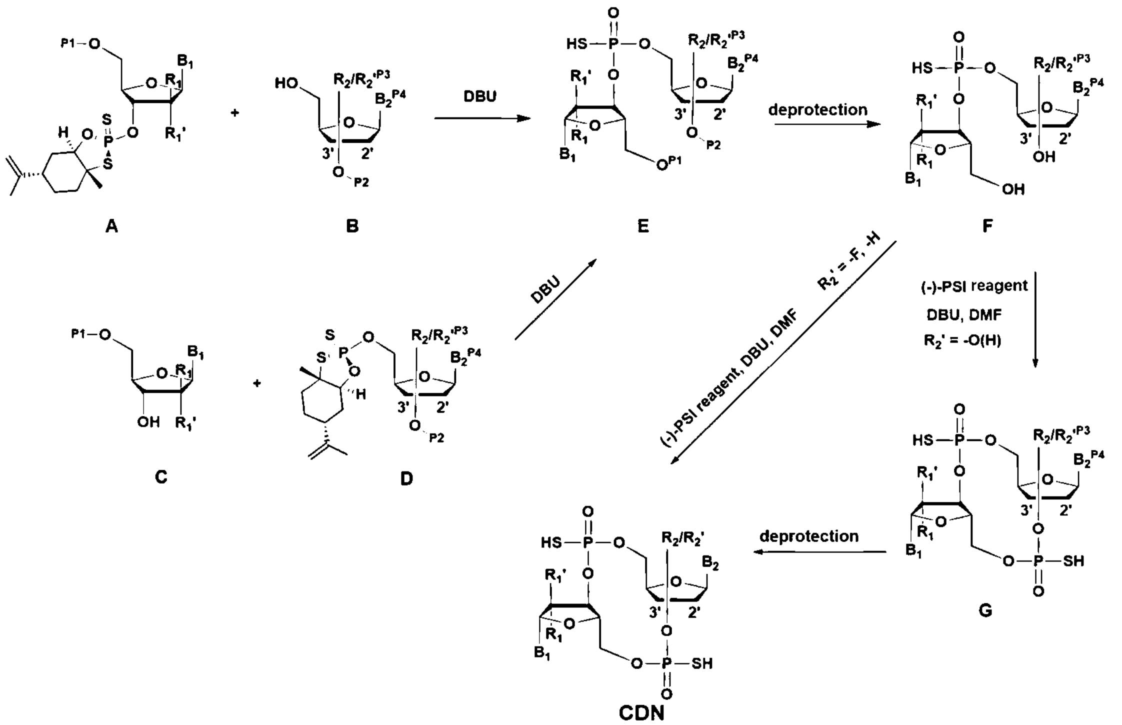


Among them, $P_1$ and $P_2$ are suitable hydroxyl protecting groups, and $P_3$ and $P_4$ are suitable hydroxyl or amino protecting groups, including but not limited to, TBS(tert-butyldimethylsilyl), DMTr (bis(4-methoxyphenyl)benzyl), Bz (benzoyl), $^{i}$-BuCO (isobutyryl). The deprotection used in the compound synthesis process of the present invention is carried out under acidic conditions (including but not limited to acetic acid/water, trifluoroacetic acid/water, etc.), alkaline conditions (including but not limited to ammonia water, ammonia/methanol solution, etc.), or fluorine-containing negative ion anion compounds (including but not limited to tetrabutylammonium fluoride, triethylamine trihydrofluorate, etc.).

Process 2 illustrates the route of synthesis of compounds of Formula A and Formula C in Process 1, as well as the synthesis of intermediates further used therein.

**Process 2**

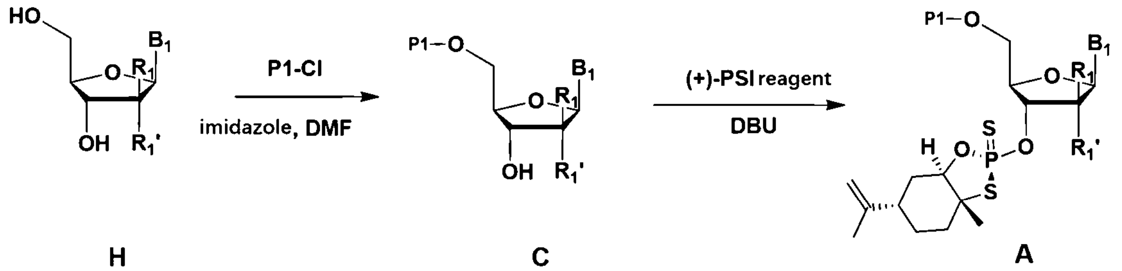


H C A

Processes 3-6 illustrates the route of synthesis of the compound of Formula B in Process 1 and the synthesis of intermediates further used therein.

**Process 3**

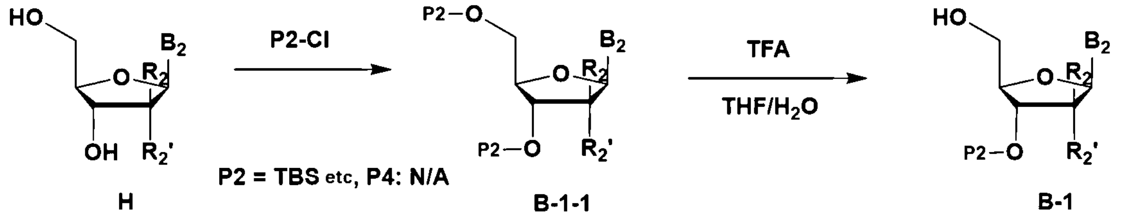


H B-1-1 B-1

Process 4

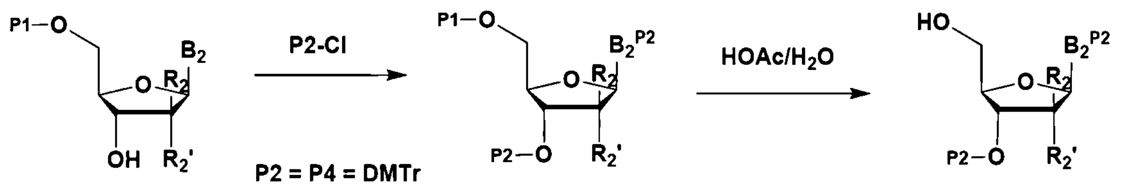


C B-2-1 B-2

**Process 5**

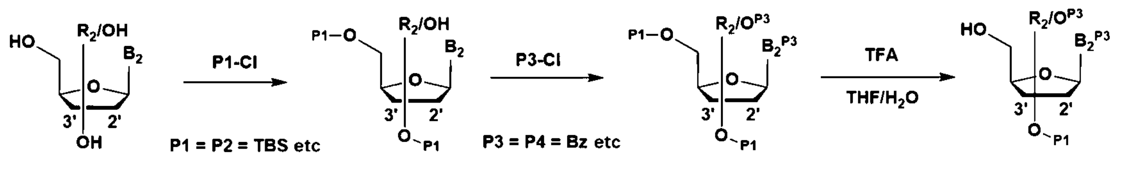


B-3-1 B-3-2 B-3-3 B-3

**Process 6**

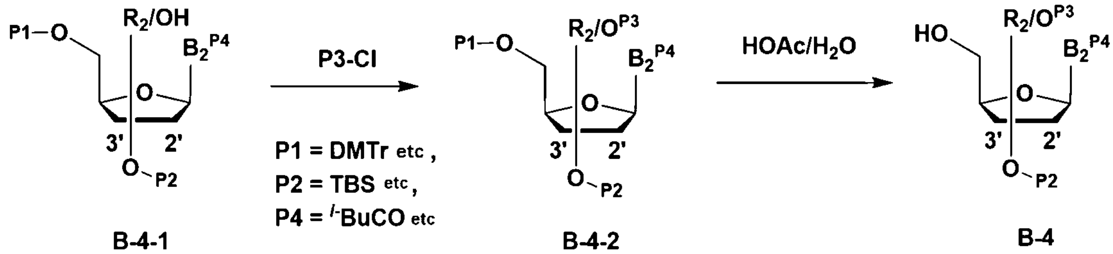


B-4-1 B-4-2 B-4

Process 7 illustrates the route of synthesis of the compound of Formula Din Process 1.

**Process 7**

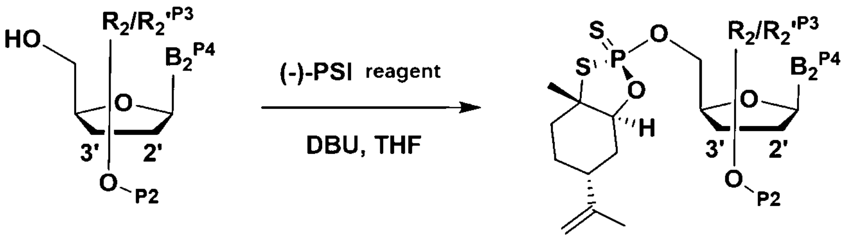


B D

Specifically, the present invention provides a preparation method for the said compound of the present invention, including:

reacting the compound of Formula A

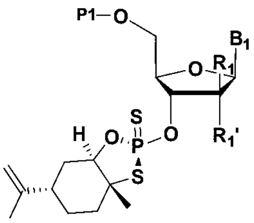

A

wherein $B_1$, $R_1$, $R_1$' have the meaning as defined above for the compound of Formula (II) of the present invention or each of its specific examples; P1 is a suitable hydroxyl protecting group, such as, but not limited to TBS (tert-butyldimethylsilyl), DMTr (bis (4-methoxyphenyl)benzyl), Bz (benzoyl), $^{i}$-BuCO (isobutyryl);

with the compound of Formula B in the presence of base, e.g., DBU,

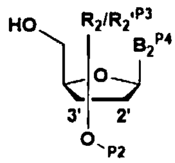

B

wherein $B_2$, $R_2$, $R_2$' have the meaning as defined above for the compound of Formula (II) of the present invention or each of its specific examples; P2 is the suitable hydroxyl protecting group, P3 and P4 are each suitable hydroxyl or amino protecting groups, such as, but not limited to TBS (tert-butyldimethylsilyl), DMTr (bis(4-methoxyphenyl) benzyl), Bz (benzoyl), $^{i}$-BuCO (isobutyryl);

or reacting the compound of Formula C

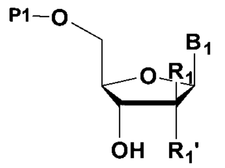

C

wherein $B_1$, $R_1$, $R_1$' and P1 are defined above for the compound of Formula A;

with the compound of Formula D in the presence of base, e.g., DBU,

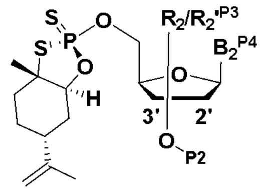

D

wherein $B_2$, $R_2$, $R_2$', P2, P3 and P4 are defined above for the compound of Formula B;

to obtain the compound of Formula E,

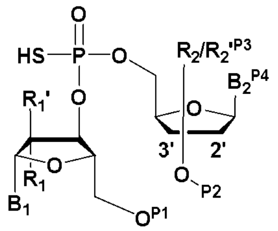

E

the compound of Formula E is selectively deprotected under conditions such as trifluoroacetic acid/water, tetrabutylammonium fluoride, or triethylamine trihydrofluoride, to give the compound of Formula F

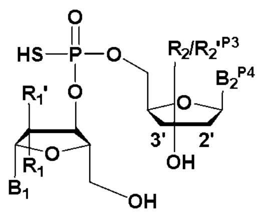

F

wherein each group has the meaning defined above;

a) When $R_2$'=-O(H) and protecting group P3 is benzoyl, the compound of formula F is closed ring with (-)-PSI reagent in the presence of a base, such as DBU, to obtain cyclic dinucleotide compound of formula G,

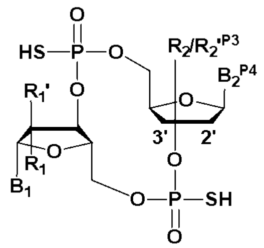

G

which is debenzoylated in ammonia water or aminomethanol solution, to obtain cyclic dinucleotide **CDN** compound,

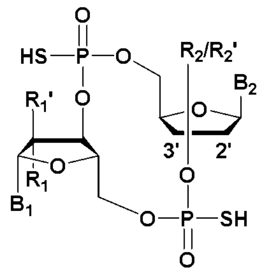

CDN

wherein $R_2$'=-O(H), each of the remaining groups has the meaning defined above for the CDN compound or each of its specific examples;

or b) When $R_2$'=-F or -H, the compound of formula F is closed ring with (-)-PSI reagent in the presence of a base, such as DBU, to obtain cyclic dinucleotide CDN compound,

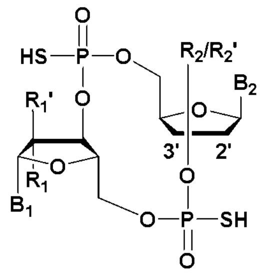

CDN

wherein $R_2$'=-F or -H, each of the remaining moieties has the meaning defined above for the CDN compound or for each of its specific examples.

The compound of formula A and the compound of formula C can be prepared as follows:

selectively protecting the primary alcohol of the compound of Formula H in the presence of base, e.g., imidazole,

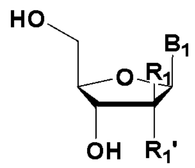

H

to obtain the compound of Formula C,

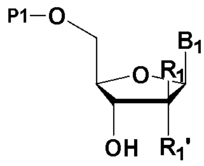

C

the above compound of Formula C is reacted with (+)-PSI reagent in the presence of base, such as DBU, to obtain the compound of Formula A,

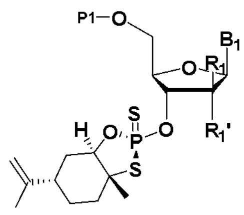

A

wherein $B_1$, $R_1$, $R_1$', and P1 are defined above for the compound of Formula A.

The compound of Formula H is protected at both hydroxyl groups in the presence of base, e.g. imidazole,

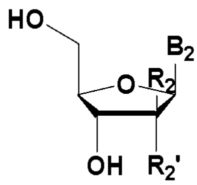

H

to obtain the compound of Formula B-1-1

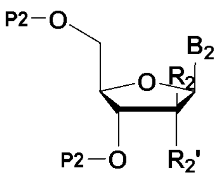

B-1-1

the compound of Formula B-1-1 is selectively deprotected of primary alcohol under conditions such as trifluoroacetic acid/water to obtain the compound of Formula B-1

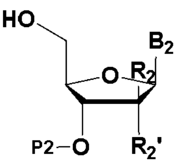

B-1

wherein $B_2$, $R_2$, $R_2$', P2 are defined above for the compound of Formula B.

The compound of Formula B may also be prepared in the form of Formula B-2 as follows: further protecting the secondary alcohol of the compound of Formula C in the presence of base, e.g. DBU/pyridine,

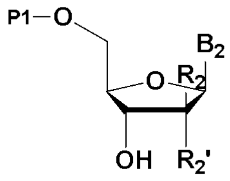

C

to obtain the compound of Formula B-2-1,

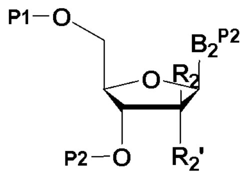

B-2-1

the compound of Formula B-2-1 is selectively deprotected of primary alcohol under conditions such as acetic acid/water to yield the compound of Formula B-2,

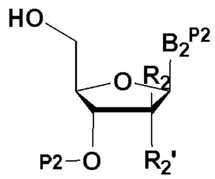

B-2

wherein $B_2$, $R_2$, $R_2$', P1, P2 are defined above for the compound of Formula A or Formula B.

The compounds of Formula B compounds may also be prepared in the form of Formula B-3 as follows:

selectively protecting the compound of Formula B-3-1 at the primary alcohol and one secondary alcohol in the presence of a base, e.g. imidazole,

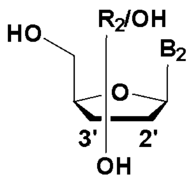

B-3-1

to obtain the compound of formula B-3-2,

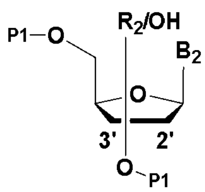

B-3-2

reacting the compound of Formula B-3-2 with the unprotected secondary alcohol and the amino group on the base

using a protecting group such as benzoyl chloride in the presence of a base, e.g. *N*-methylimidazole to obtain the compound of formula B-3-3.

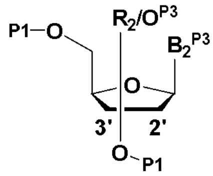

B-3-3

the compound of Formula B-3-3 is selectively deprotected of primary alcohol under conditions such as trifluoroacetic acid/water, to give the of compound Formula B-3,

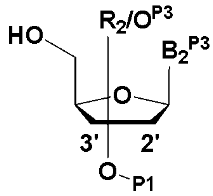

B-3

wherein $B_2$, $R_2$, $R_2$', P1, P2 and P3 are defined above for the compound of Formula A or Formula B.

The compound of Formula B may also be prepared in the form of Formula B-4 as follows:

reacting the compound of Formula B-4-1 with the unprotected secondary alcohol using a protecting group, e.g. benzoyl chloride in the presence of a base, e.g. N-methylimidazole,

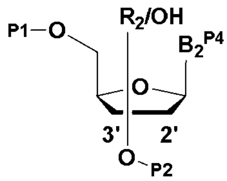

B-4-1

to obtain the compound of Formula B-4-2,

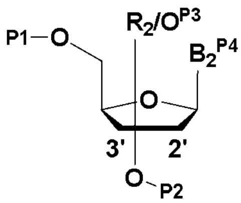

B-4-2

the compound of Formula B-4-2 is selectively deprotected of primary alcohol under conditions such as acetic acid/water to yield the compound of Formula B-4

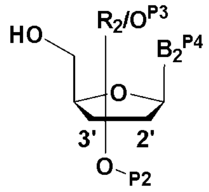

B-4

wherein $B_2$, $R_2$, $R_2$', P1, P2 and P4 are defined above for the compound of Formula A or Formula B.

The compound of Formula D can be prepared as follows:

reacting the compound of Formula B with (-)-PSI reagent in the presence of base, e.g. DBU,

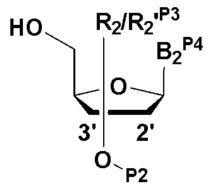

B

to obtain the compound of Formula D

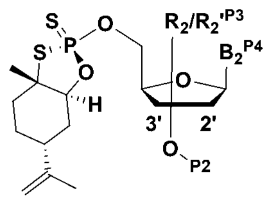

D

wherein $B_2$, $R_2$, $R_2$', $P_2$, $P_3$, and $P_4$ are defined above for the compound of Formula B.

**3. Synthesis of linker unit L fragments as well as bifunctional CDN-linker units**

As mentioned above, the bifunctional CDNs of the ADC of the present invention are linked to antibodies via linker units.

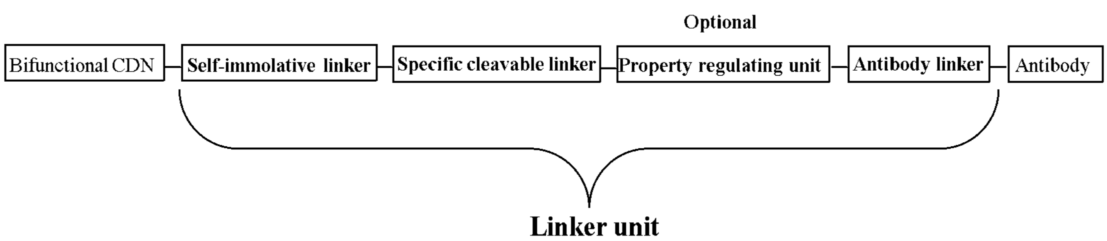


Process 8 below illustrates a general synthetic route that can be used to prepare this bi-functional **CDN** compound and each specific example. The variables of the general formulae in the following processes have the same meaning as in the compound defined herein or in each of its specific examples unless otherwise indicated.

**Process 8**

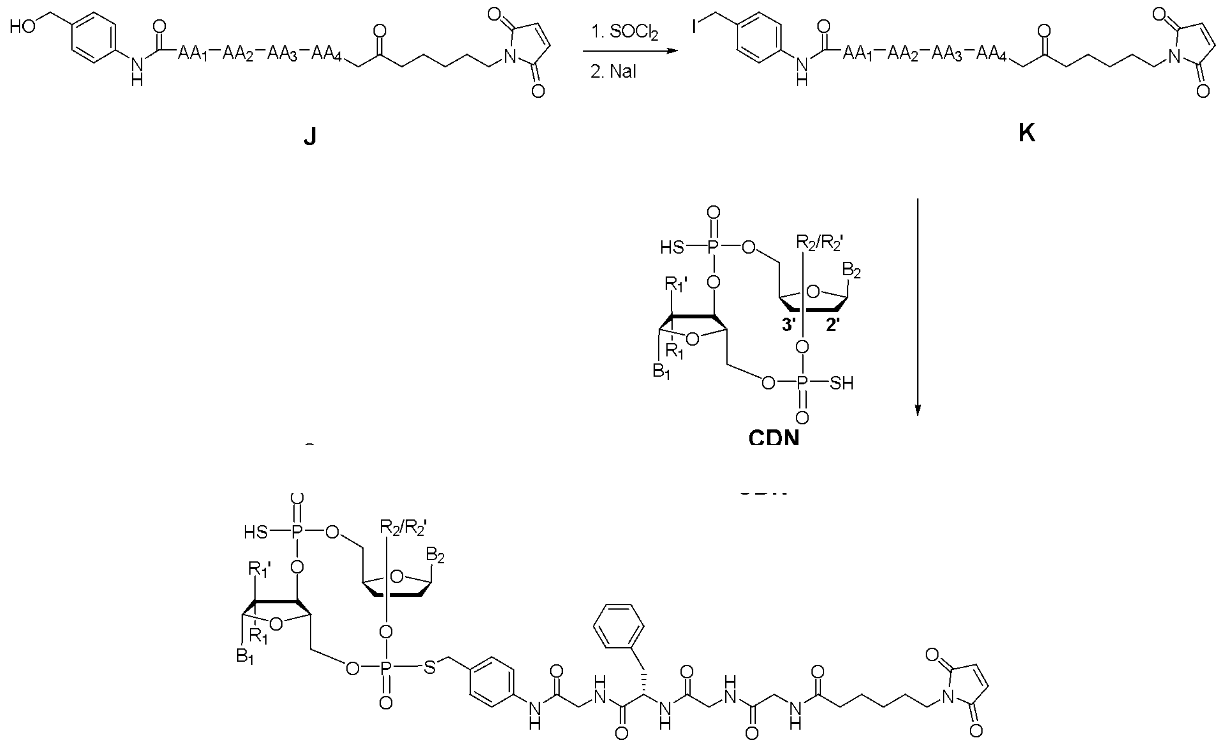


J

K

CDN

PL

The Intermediate **J** is commercially available, wherein $AA_1$, $AA_2$, $AA_3$, and $AA_4$ represent amino acids, respectively, and conversion of benzyl alcohol from intermediate J into benzyl halogen, preferably benzyl iodine **K,** is achieved with halogenated reagents (e.g., dichlorodisulfoxide, etc.), as defined by the Formula (Y1) in the "cleavable linker" section of the present invention.

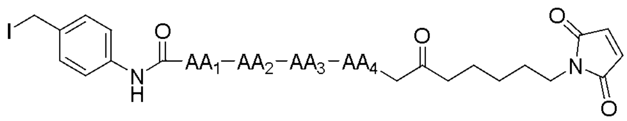

**K**

In a suitable solvent, such as DMF, the iodine in the molecule of intermediate K, predominantly over succinimide, reacts directly with one of the -SH molecules in the CDN molecule to generate intermediate **PL,** while the remaining succinamide serves to link the antibody molecule.

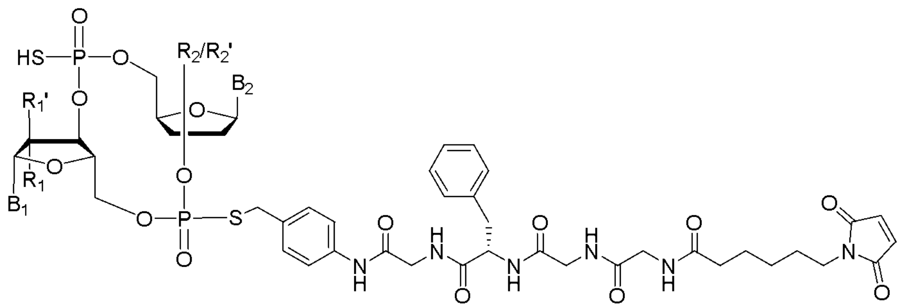

**PL**

## 4. Antibody selection and synthesis

The antibodies used in ADC of the present invention are derived from commercial sources and include, but are

not limited to, antibodies to the following targets: KIT, FOLR1, CD276, PD-L1, NECTIN4, Mesothelin, MUC1, GCPII, BCMA, cMet, RPR1, CD22, CD19, TOP1, Claudin 18.2, EGFR, TROP2, HER2, etc.

**5. Synthesis of bifunctional CDN-linker-antibody conjugate ADC**

Generation of antibody-drug conjugates can be accomplished by any technique known to technicians in the field. In some embodiments, conjugation of the drug-linker to the antibody is accomplished by reacting with amino acid residues of the antibody. In some embodiments, drug D is coupled to a cysteine residue of an antibody using linker L with a leaving group to prepare the conjugate of Formula A. In some embodiments, the interchain disulfide bonds of the antibody can be disrupted and free thiols exposed for conjugation to the linker-drug by controlling the conditions under which the antibody is treated with a reducing agent such as tris(2-hydroxyethyl)phosphine (TCEP). For IgG1 antibodies, up to four linked disulfide bonds can be reduced to generate up to eight reactive thiol moieties for conjugation. Conjugates prepared by this method can contain zero, one, two, three, four, five, six, seven, or eight drugs in each antibody molecule.

When the prepared conjugate is a combination of conjugates with different drug conjugation sites and/or numbers, the drug loading of the conjugate is expressed by the average DAR, and at this point the average DAR of the antibody-drug conjugate combination prepared can be characterized by conventional means such as mass spectrometry, ELISA assay, and HPLC, and the quantitative distribution of the antibody-drug conjugate expressed as q can also be determined. Wherein q refers to the separation, purification and characterization of homogeneous antibody-drug conjugate with a certain value and antibody-drug conjugate with other drug loading capacity, which can be achieved by means of reversed-phase HPLC or electrophoresis.

Process 9 below illustrates a general synthetic route that can be used to prepare this bi-functional **CDN** compound and each specific example. The variables of the general formulae in the following processes have the same meaning as in the compound defined herein or in each of its specific examples unless otherwise indicated.

**Process 9**

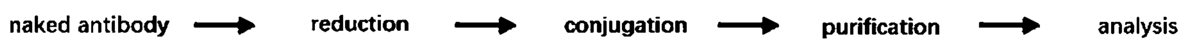


The antibody molecule is first reduced, preferably reducing the disulfide bonds of the antibody molecule. The heavy and light chains of the antibody molecule are interconnected by four pairs of disulfide bonds. These disulfide bonds can be reduced with reducing agents (e.g., tris(2-carboxyethyl)phosphine TCEP, mercaptoethanol, dithiothreitol, cysteine, reduced glutathione, etc.) to a maximum of 0-8 thiols, each of which may react with succinamide to generate 0-8 bifunctional CDN-linker-derived antibody conjugate ADCs. The average drug loading capacity DAR (drug/antibody ratio) of this compound can be experimentally measured.

Experimental materials and reagents used in the above synthesis methods and processes, unless otherwise specified, may be obtained from commercial sources, prepared using methods based on existing technology or similar to those disclosed in this application. The synthesis conditions used in the above synthesis methods and processes may be routinely determined by technical personnel in the field unless otherwise specified.

The present invention also relates to a preparation method wherein a compound which may be obtained in the form of an intermediate at any step of each preparation method and process described herein is used as a starting material and the remaining method steps are performed, or wherein the starting material is formed in situ under reaction conditions or in the form of a derivative such as a protected form or salt form, or a compound which may be obtained according to the method of the present invention is generated and further processed in situ under the method conditions described.

**Specific Enbodiments**

The present invention is further described in the following in combination with examples. It should be mentioned that the following examples cannot be taken as a limitation to the scope of protection of the present invention.

Unless there is an apparent error in the structural formula, the structural formula is more accurate when the chemical name of any compound of the present invention is inconsistent with the structural formula given.

Experimental methods for which specific conditions are not indicated in the following examples are generally in accordance with the usual conditions for such reactions, or as recommended by the manufacturer. The experimental materials and reagents used in the following examples may be obtained from commercial sources, prepared according to existing technology or similar methods disclosed in this application, unless otherwise specified.

Unless otherwise stated, percentages and fractions are weight percentages and fractions; ratios of liquids are volume ratios; all temperatures are given in degrees Celsius unless otherwise stated.

In the following examples, $^{1}$H NMR spectrum $^{31}$P NMR spectrum were typically recorded on Bruker 400 MHz

NMR and 500 MHz NMR nuclear magnetic resonance apparatus was used for recording, with chemical shifts expressed as δ (ppm); mass spectra were recorded on Agilent 1290 liquid chromatography+6120B mass spectrometry LCMS liquid chromatography apparatus; silica column purification was performed on Biotage SelektSEL-2SV or ISO-1SV; preparative liquid chromatography purification was performed on Gilson 281 (column: waters Xbridge 19 mm x 250 mm, 5 μm or WELCH C18, 21.2 mm x 250 mm, 10 μm). Mobile phase: A: water (10 mM $NH_4HCO_3$ or 0.05% formic acid), B: acetonitrile (or containing 0.05% formic acid). Flow rate: 20-30 mL/min. Detection wavelength: 214 nm/254 nm) or otherwise indicated.

The following abbreviations are used in the following synthesis examples and each abbreviation not listed has the meaning normally understood by technicians in the field.

Abbreviations List

| | |
|---|---|
| $CDCl_3$ | Deuterated chloroform |
| DMSO-$d_6$ | Deuterated Dimethyl Sulfoxide |
| MHz | Megahertz |
| MS-ESI | Electrospray mass spectrometry |
| DBU | 1,8-Diazabicycloundeca-7-ene |
| DCC | N,N'- Dicyclohexylcarbodiimide |
| DIEA | N,N- Diisopropylethylamine |
| DMAP | 4-Dimethylaminopyridine |
| DMF | *N,N*-Dimethylformamide |
| CDI | *N,N'*-Carbonyldiimidazole |
| TMSCl | Trimethylchlorosilane |
| TFA | Trifluoroacetic acid |
| THF | Tetrahydrofuran |
| $H_2O$ | Water |
| HOAc | Acetic acid |
| HOBt | 1-Hydroxybenzotriazole |
| BzCl | Benzoyl chloride |
| IFN | Interferon |
| FBS | Fetal bovine serum |
| PBS | Phosphate buffer solution |
| ELISA | Enzyme Linked Immunosorbent Assay |
| (+)-PSI reagent | (*2R,3aR,6S,7aR*)-3a-methyl-2-((perfluorophenyl)thio)-6-(propyl-1-en-2-yl)hexahydrobenzo[d] [1,3,2] oxothiophos heterocyclopentan-2-sulfide) (CAS: 2245335-71-9) 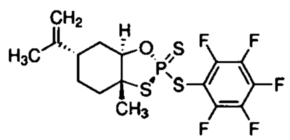 |
| (-)-PSI reagent | (*2S,3aS,6R,7aS*)-3a-methyl-2-((perfluorophenyl)thio)-6-(propyl-1-en-2-yl)hexahydrobenzo[d][1,3,2] oxothiophos heterocyclopentan-2-sulfide (CAS No. 2245335-70-8) |

(continued)

| | |
|---|---|
| | 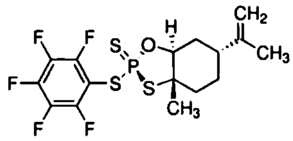 |

**Synthesis examples**

**Preparation of bifunctional CDNs**

**Preparation of Intermediate 2:**

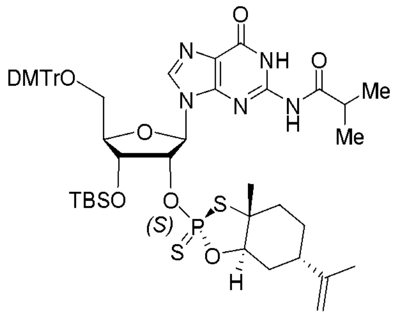

5'-O-(4,4'-Dimethoxytrityl)-3'-O-tert-butyldimethylsilyl-N2-isobutyrylguanosine (10 g, 13.0 mmol) was dissolved in acetonitrile (100 mL), followed by addition of (+)-PSI reagent (7.0 g, 15.6 mmol) and 4Å molecular sieve (10 g, 325 mesh), respectively, and after the system was displaced twice with nitrogen, the reaction solution was cooled with an ice salt bath. When the internal temperature of the reaction solution was decreased to 0~5°C, DBU(3.0 g, 19.5 mmol) was slowly added dropwise into the above reaction solution, after the completion of addition the internal temperature was maintained at 0~5°C and stirred for 1 hour. After the completion of TLC monitored reaction, the reaction solution was filtered with Celite, then the filter cake was washed with dichloromethane (200 mL), and the resulting filtrate was washed twice with 10% potassium dihydrogen phosphate aqueous solution (100 mL each time). The resulting organic phase was dried over anhydrous sodium sulfate and filtered with silica gel (50 g of silica gel, 100-200 mesh), and the filter cake was washed with a mixture of dichloromethane and ethyl acetate (200 mL, 1:1 volume ratio of the two solvents). The resulting filtrate was concentrated under reduced pressure at a temperature not exceeding 40°C to give 13.5 g as a foamy solid. N-heptane (50 mL) and isopropyl ether (50 mL) were added to the crude product, stirred for 1 hour at 10-15°C, then filtered, and the filter cake was dried under vacuum at a temperature not exceeding 40°C to give **Intermediate 2** as a yellow solid (12.6 g, 95.4% yield).

$^1$H NMR (400 MHz, Chloroform-d, δ): 11.88 (s, 1H), 7.90 (s, 1H), 7.79 (s, 1H), 7.53 (d, *J* = 7.3 Hz, 2H), 7.40 (d, *J* = 8.7 Hz, 4H), 7.27-7.14 (m, 3H), 6.79 (t, *J* = 9.1 Hz, 4H), 6.36-6.23 (m, 1H), 5.95 (d, *J* = 7.6 Hz, 1H), 5.01 (s, 1H), 4.87 (s, 1H), 4.49-4.35 (m, 2H), 4.13 (d, *J* = 7.1 Hz, 2H), 3.76 (d, *J* = 3.6 Hz, 6H), 3.47 (d, *J* = 10.9 Hz, 1H), 3.07-2.95 (m, 1H), 2.57 (s, 1H), 2.26 (d, *J* = 14.6 Hz, 1H), 1.92 (d, *J* = 9.5 Hz, 2H), 1.75 (d, *J* = 20.9 Hz, 9H), 0.98 (d, *J* = 6.9 Hz, 3H), 0.86 (s, 9H), 0.74 (d, *J* = 6.9 Hz, 3H), 0.11 (s, 3H), 0.00 (s, 3H). MS-ESI [M+H]$^+$: 1016.4.

**Preparation of Intermediate 4:**

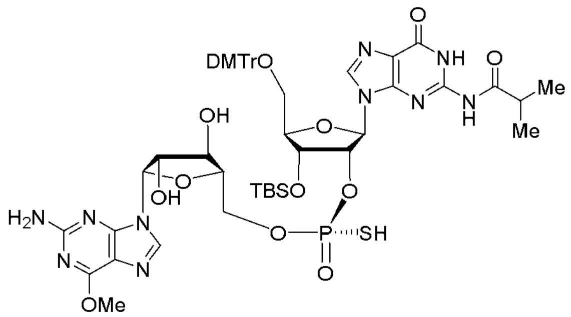

**Intermediate 2** (3.4 g, 3.34 mmol) was dissolved in DMF (30 mL), followed by addition of 2-amino-3-methoxy-adenosine (nelarabine, 1.0 g, 3.36 mmol) and 4Å molecular sieve (3.4 g, 325 mesh), and the above reaction mixture was displaced twice with nitrogen and stirred at room temperature. DBU (1.79 g, 11.78 mmol) was added dropwise after 5 minutes and followed by continued stirring for 1 hour. After completion of the TLC monitored reaction, the reaction solution was filtered by Celite pad, and then the filter cake was washed with dichloromethane (100 mL). The resulting filtrate was washed twice with water (50 mL water each time) and once with saturated brine (50 mL) and then dried over anhydrous sodium sulfate and concentrated under reduced pressure to obtain the crude product. Further purified by silica gel column chromatography (40 g silica gel, mobile phase A: dichloromethane, mobile phase B: 0-10% methanol, flow rate: 30 mL/min), the resulting product solution was concentrated under reduced pressure to obtain **intermediate 4** as a white solid (2.5 g, yield 65.1%).

$^1$H NMR (400 MHz, DMSO-$d_6$, δ): 8.12 (s, 1H), 7.95-7.91 (d, $J$ = 15.6 Hz, 2H), 7.37-7.35 (d, $J$ = 7.6 Hz, 2H), 7.28-7.16 (m, 7H), 6.84-6.82 (d, $J$ = 8.0 Hz, 4H), 6.43 (s, 2H), 6.05-6.02 (m, 2H), 5.70-5.65 (m, 2H), 5.42-5.36 (m, 1H), 4.62 (s, 1H), 3.99-3.93 (m, 5H), 3.70-3.69 (d, $J$= 5.2 Hz, 6H), 3.61 (s, 2H), 3.47-3.41 (m, 2H), 3.24-3.19 (m, 2H), 2.79-2.75 (m, 1H), 1.12-1.10 (d, $J$ = 6.8 Hz, 6H), 0.83 (s, 9H), 0.17-0.13 (d, $J$ = 14.8 Hz, 6H).

**Preparation of Intermediate 5:**

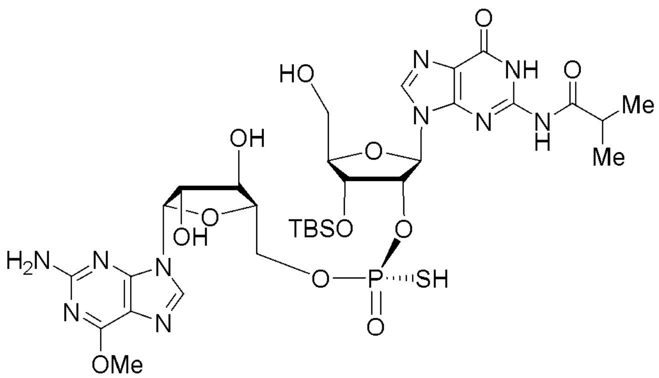

**Intermediate 4** (1.0 g, 0.87 mmol) was dissolved in dichloromethane (10 mL), to which Amberlyst 15 resin (1.2 g) and triethylsilane (101 mg, 0.87 mmol) were added, and stirred overnight at room temperature. Following completion of the TLC monitored reaction, methanol (1 mL) was added to the reaction solution and stirred for another 0.5 h. The reaction solution was filtered with Celite and the filter cake was subsequently washed with dichloromethane (50 mL). The resulting filtrate was concentrated under reduced pressure to yield the crude product, followed by addition of methyl tert-butyl ether (10 mL) to the crude product and filtered after stirring at room temperature for 1 h. The resulting filter cake was dried under vacuum at 45°C to give **intermediate 5** as a pale yellow solid (480 mg, 66.2% yield).

**Preparation of Intermediate 6:**

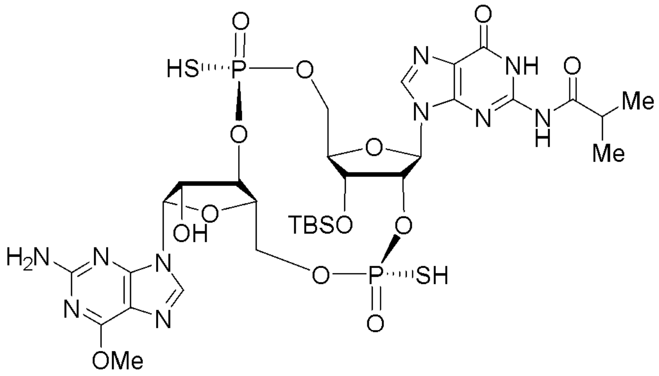

**Intermediate 5** (200 mg, 0.24 mmol) was dissolved in DMF (3 mL) to which (-) -PSI reagent (107 mg, 0.24 mmol) and 4Å molecular sieve (0.5 g, 325 mesh) were added. The system was displaced twice with nitrogen, then cooled to 0~5°C with an ice salt bath, stirred, and DBU (127.9 mg, 0.84 mmol) was slowly added dropwise, and then stirred at room temperature for 2 hours. After the reaction was complete as detected by LC-MS, the reaction solution was filtered with Celite and the filter cake was washed with methanol (5 mL). The resulting filtrate was subjected to reversed-phase column chromatography (packed column model Biotage Sfar C18 D-Duo 100Å 30 μm column, filler volume 60 g, mobile phase A: 10 mM ammonium bicarbonate aqueous solution, phase B: acetonitrile, gradient elution from 0 to 40% B, flow rate: 30 mL/min), and the product solution obtained from the column chromatography was further freeze-dried to obtain **intermediate 6** as a white color solid (50 mg, yield 22.6%). MS-ESI $[M + H]^+$: 921.2.

**Preparation of Intermediate 7:**

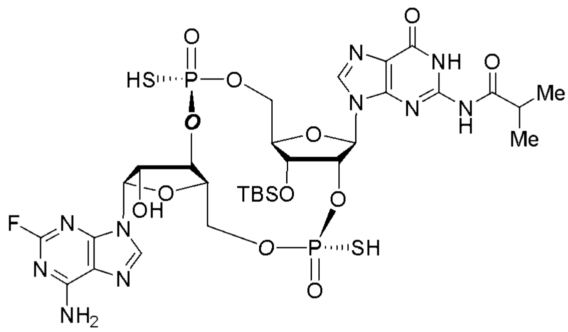

**Intermediate 7** was prepared by replacing 2-amino-3-methoxy-adenosine with 2-fluoro-adenosine using the route of **intermediate 6.** MS-ESI $[M + H]^+$: 831.3.

**Preparation of CDN-1: (2',3')-cyclo-(*Rp,Rp*)-[2'-O-dithiophosphodiester-guanosine]-[3'-O-dithiophosphate diester -2-amino-3-methoxy-adenosine]diammonium salt**

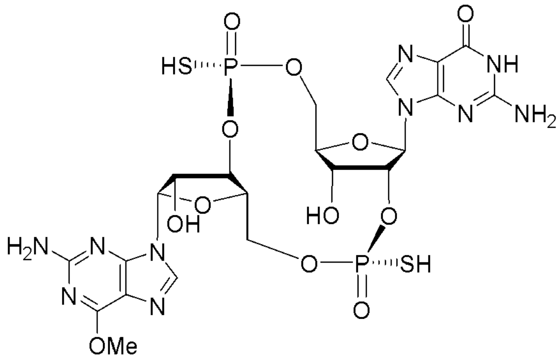

**Intermediate 6** (50 mg, 0.054 mmol) and deionized water (1 mL) were mixed and stirred, then cooled to 0 to 5°C with an ice bath, and then aqueous NaOH (2 mol/L, 1 mL) was slowly added dropwise. After completion of dripping, the ice bath was removed and stirred at room temperature for 16 hours. After the reaction was complete as detected by LC-MS, the reaction solution was purified by preparative liquid chromatography (Gilson281 preparative HPLC, Welch 10 μm 19 mm × 250 mm preparative column, mobile phase A: 10 mM ammonium bicarbonate aqueous solution, phase B: acetonitrile, flow rate: 20 mL/min, dual wavelength UV absorption monitoring at 214 nm and 254 nm, gradient elution: 0-2.2 min, 3% B; 2.2-15.6 min, 3-15% B; 15.6-15.8 min, 15-95% B; 15.8-17.8 min, 95% B), and the resulting product solution was a white solid **CDN-1** (14.7 mg) after lyophilization.

$^1$H NMR (400 MHz, DMSO-$d_6$, δ): (ppm) 8.12 (s, 1H), 7.82 (s, 1H), 6.22-6.21 (d, *J* = 3.6 Hz, 1H), 5.88-5.86 (d, *J* = 8.5 Hz, 1H), 5.15 - 5.12 (m, 1H), 4.99-4.97 (d, *J* = 10.4 Hz, 1H), 4.50-4.49 (d, *J* = 4.0 Hz, 1H), 4.23-4.12 (m, 4H), 4.00-3.93 (m, 5H), 3.73-3.70 (d, *J* = 12.0 Hz, 1H). $^{31}$P NMR (400 MHz, DMSO-$d_6$, δ): 53.75, 47.44. MS-ESI [M+H]$^+$: 737.2.

**Preparation of CDN-2: (2',3')-cyclo-(*Rp,Rp*)-[2'-O-thiophosphodiester-guanosine]-[3'-O-thiophosphodiester-2-fluoro-adenosine]diammonium salt**

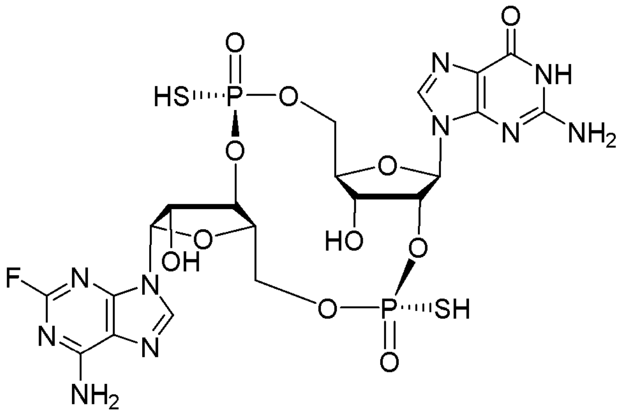

**CDN-2** was prepared by replacing **Intermediate 6** with **Intermediate 7** using the route of**CDN-1. Intermediate 7** (30 mg, 0.038 mmol), methanol (1 mL) and water (1 mL) were mixed well, triethylamine (57.3 mg, 0.566 mmol) was added while stirring, and the resulting mixture was then stirred at room temperature for 48 hours. After completion of the reaction monitored by LCMS, the pH of the reaction solution was adjusted to 3 with 1*N* hydrochloric acid and the resulting mixture was concentrated under reduced pressure to give the crude product. The crude product was re-dissolved in DMSO (4 mL) and purified using preparative liquid chromatography (Gilson 281 preparative HPLC, Waters XBridge C18 10μm 19 mm × 250 mm preparative column, mobile phase A: 10 mM ammonium bicarbonate aqueous solution, phase B: acetonitrile, flow rate: 20 mL/min, dual wavelength UV absorption monitoring at 214 nm and 254 nm, gradient elution: 0-2.3 min, 5% B; 2.30-15.50 min, 5-20% B; 15.50-15.70 min, 20-95% B; 15.70-17.70 min, 95% B, 17.90-22.50 min, 5% B. The retention time of the compound was 9.0 min, and the resulting product solution was further freeze-dried to give compound **CDN-2** as a white solid (19.4 mg).

$^1$H NMR (400 MHz, DMSO-$d_6$, δ): 10.60 (s, 1H), 8.07 (d, *J* = 7.6 Hz, 2H), 7.88 (d, *J* = 61.4 Hz, 2H), 7.58-6.75 (m, 8H), 6.50 (d, *J* = 30.7 Hz, 3H), 6.17 (d, *J* = 4.3 Hz, 1H), 5.89 (d, *J* = 8.4 Hz, 1H), 5.17 - 5.05 (m, 1H), 4.98 (d, *J* = 16.0 Hz, 2H), 4.50 (s, 1H), 4.33 (s, 1H), 4.25-4.08 (m, 3H), 4.06-3.91 (m, 2H), 3.74 (d, *J* = 12.4 Hz, 1H). $^{19}$F NMR (400 MHz, DMSO-$d_6$, δ):

-52.25. $^{31}$P NMR (400 MHz, DMSO-d6, δ): 54.84, 47.31. MS-ESI $[M+H]^+$: 725.2.

**Preparation of CDN-3 to CDN-8:**

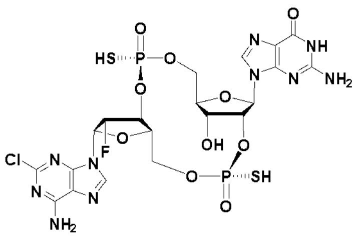

CDN-3

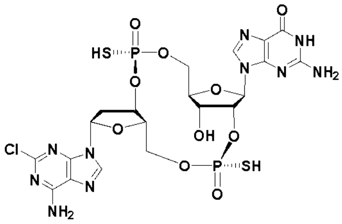

CDN-4

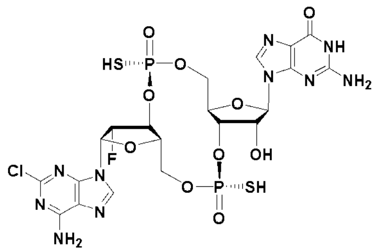

CDN-5

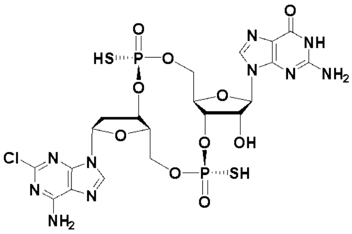

CDN-6

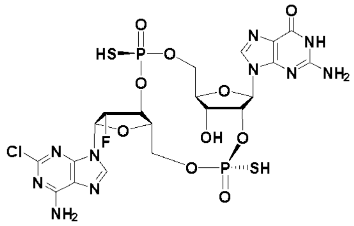

CDN-7

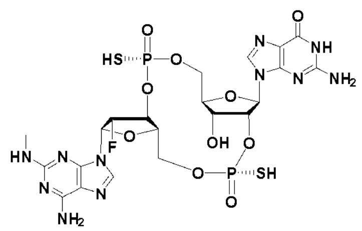

CDN-8

**Synthesis and characterization of CDN-3 to CDN-8 (diammonium salt form)** were provided in Examples 10, 12, 16, 17, 25, 26 of WO2022083584A1, respectively.

**CDN-3** ((2',3')-cyclo-(*Rp*,*Rp*)-[2'-O-thiophosphodiester-guanosine]-[3'-O-thiophosphodiester-2-chloro-2'-deoxy-2'-fluoro-beta-adenosine]diammonium salt) was prepared using the preparation route of Example 10 of WO2022083584A1**,** intermediate 75 ((2',3')-cyclo-[2'-O-thiophosphodiester-3'-O-benzoyl-guanosine]-[3'-O-thiophosphodiester-2-chloro-2'-deoxy-2'-fluoro-beta-adenosine]-isomer 2, MS-ESI $[M-H]^-$: 844.9 was used and after reaction with 28% ammonia water, the compound was purified using preparative liquid chromatography (Gilson 281 Preparation HPLC, 19 x 250 mm Welch 10 μm preparation column, mobile phase A: 10 mM ammonium bicarbonate aqueous solution, B: acetonitrile, flow rate: 25 ml/min, dual wavelength UV absorption monitoring at 214 and 254 nm with gradient elution: 0-3 min, 0-3% B; 3-14 min, 3-38% B; 14-14.3 min, 38-95% B; 14.3-20 min, 95% B). The retention time of the compound was 13 min.

$^1$H NMR (400 MHz, DMSO-$d_6$) δ 10.63 (brs, 1H), 8.22 (s, 1H), 8.18 (s, 1H), 7.93 (brs, 2H), 7.12 (t, *J* = 51.2 Hz, 8H), 6.69 (brs, 2H), 6.28 (dd, *J* = 24.0, 2.4 Hz, 1H), 5.88 (d, *J* = 8.4 Hz, 1H), 5.42-4.80 (m, 4H), 4.45 (d, *J* = 4.0 Hz, 1H), 4.39-4.10 (m, 3H), 4.08-3.94 (m, 1H), 3.93-3.70 (m, 2H). $^{31}$P NMR (162 MHz, DMSO-$d_6$) δ 54.07, 50.13. MS-ESI $[M-H]^-$: 741.0.

**CDN-4** ((2',3')-cyclo-(*Rp*,*Rp*)-[2'-O-thiophosphodiester-guanosine]-[3'-O-thiophosphodiester-2-chloro-2'-deoxy-adenosine]diammonium salt) was prepared using the preparation route of Example 12 of WO2022083584A1**,** intermediate 77 ((2',3')-cyclo-[2'-O-thiophosphodiester-N2-isobutyryl-3'-O-benzoyl-guanosine]-[3'-O-thiophosphodiester-2-chloro-2'-deoxy-adenosine]-isomer 2, MS-ESI $[M-H]^-$: 897.2) was used and after reaction with 7 M ammonia in methanol purified by preparative liquid chromatography (10 mM ammonium bicarbonate as additive). Analyze with LCMS: Agilent 1100+G1946D LCMS, 4.6 x 150 mm Waters XBridge C18 3.5 μm analytical column, mobile phase A: 10 mM ammonium bicarbonate aqueous solution, B: acetonitrile, flow rate: 1 ml/min, dual wavelength UV absorption monitoring at 214 and 254 nm, gradient elution: 0-8 min, 5-95% B; 8-15 min, 95% B. The retention time of the compound was 3.3 min.

$^1$H NMR (400 MHz, DMSO-$d_6$) δ 10.61 (brs, 1H), 8.39 (s, 1H), 8.20 (s, 1H), 7.82 (brs, 2H), 7.11 (t, *J* = 51.2 Hz, 8H), 6.70 (brs, 2H), 6.30-6.18 (m, 1H), 5.87 (d, *J*= 8.4 Hz, 1H), 5.25-5.13 (m, 2H), 4.96 (s, 1H), 4.50 (d, *J* = 4.4 Hz, 1H), 4.31-3.71 (m, 6H), 3.03-2.82 (m, 1H), 2.65-2.50 (m, 1H). $^{31}$P NMR (162 MHz, DMSO-$d_6$) δ 54.26, 49.51. MS-ESI $[M-H]^-$: 722.8.

**CDN-5** ((3',3')-cyclo-(*Rp*,*Rp*)-[3'-O-thiophosphodiester-guanosine]-[3'-O-thiophosphodiester-2-chloro-2'-deoxy-2'-fluoro-beta-adenosine]diammonium salt) was prepared using the preparation route of Example 16 of WO2022083584A1**,** intermediate 81 ((3',3')-cyclo-[3'-O-thiophosphodiester-N2-isobutyryl-2'-benzoyl-guanosine]-[3'-O-thiophosphodiester-2-chloro-2'-deoxy-2'-fluoro-beta-adenosine]-isomer 2, MS-ESI $[M-H]^-$: 915.0 was used and after reaction with 7 M ammonia in methanol. The compound was purified using preparative liquid chromatography chromatography (Gilson 281 preparative HPLC, 19 x 250 mm Welch 10 μm preparative column, mobile phase A: 10 mM ammonium bicarbonate in water, B: acetonitrile, flow rate: 25 ml/min, dual wavelength UV absorption monitoring at 214 and 254 nm,

gradient elution: 0-3 min, 0-3% B; 3-14 min, 3-33% B; 14-14.3 min, 33-95% B; 14.3-20 min, 95% B). The retention time of the compound was 13 min.

$^{1}$H NMR (400 MHz, DMSO-$d_6$) δ 10.63 (brs, 1H), 8.22 (s, 1H), 8.09 (s, 1H), 7.94 (brs, 2H), 7.38-6.94 (m, 8H), 6.65 (brs, 2H), 6.28 (dd, *J* = 24.0, 2.4 Hz, 1H), 5.87 (d, *J* = 8.8 Hz, 1H), 5.42-5.11 (m, 3H), 5.00 (s, 1H), 4.56-4.42 (m, 1H), 4.39-4.21 (m, 2H), 4.13 (s, 1H), 4.05-3.94 (m, 1H), 3.89-3.71 (m, 2H). $^{31}$P NMR (162 MHz, DMSO-$d_6$) δ 53.97, 49.08. MS-ESI [M-H]$^-$: 740.9.

**CDN-6** ((3',3')-cyclo-(*Rp*,*Rp*)-[3'-O-thiophosphodiester-guanosine]-[3'-O-thiophosphodiester-2-chloro-2'-deoxy-adenosine]diammonium salt) was prepared using the preparation route of Example 17 of WO2022083584A1**,** intermediate 82 ((3',3')-ring-[3'-O-thiophosphodiester-N2-isobutyryl-2'-benzoyl-guanosine]-[3'-O-thiophosphodiester-2-chloro-2'-deoxy-adenosine], MS-ESI [M-H]$^-$: 896.8) was useed and after reaction with 7 M ammonia in methanol. The compound was purified using preparative liquid chromatography (Gilson 281 Preparation HPLC, 19 x 250 mm Welch 10 μm preparation column, mobile phase A: 10 mM ammonium bicarbonate aqueous solution, B: acetonitrile, flow rate: 25 ml/min, dual wavelength UV absorption monitoring at 214 and 254 nm with gradient elution: 0-3 min, 0-3% B; 3-14 min, 3-33% B; 14-14.3 min, 33-95% B; 14.3-20 min, 95% B. The retention time of the compound was 11 min.

$^{1}$H NMR (400 MHz, DMSO-$d_6$) δ 10.59 (brs, 1H), 8.41 (s, 1H), 8.11 (s, 1H), 7.85 (brs, 2H), 7.15 (brs, 8H), 6.66 (brs, 2H), 6.25 (dd, *J* = 12.8, 6.0 Hz, 1H), 5.85 (d, *J* = 8.8 Hz, 1H), 5.35-5.11 (m, 2H), 4.91 (s, 1H), 4.59-4.50 (m, 1H), 4.31-3.71 (m, 6H), 3.03-2.87 (m, 1H). $^{31}$P NMR (162 MHz, DMSO-$d_6$) δ 53.46, 47.84. MS-ESI [M-H]$^-$: 740.9.

**CDN-7** ((2',3')-cyclo-(*Rp*,*Sp*)-[2'-O-thiophosphodiester-guanosine]-[3'-O-thiophosphodiester-2-chloro-2'-deoxy-2'-fluoro-beta-adenosine]diammonium salt) was prepared using the preparation route of Example 25 of WO2022083584A1, intermediate 90 ((2',3')-cyclo-(*Rp*,*Sp*)-[2'-O-thiophosphodiester-N2-isobutyryl-3'-O-tert-butyldimethylsilyl-guanosine]-[3'-O-thiophosphodiester-2-chloro-2'-deoxy-2'-fluoro-beta-adenosine], MS-ESI [M + H]$^+$: 926.8) (100 mg, crude) was dissolved in 7 M ammonia in methanol (3 mL) and stirred at room temperature (28 °C) for 4 hours before rotary drying The resulting residue was dissolved in methanol (1 mL), ammonium fluoride (60.5 mg, 1.63 mmol) was added, heated (60°C) and stirred for 20 hours, after completion of reaction monitored by LCMS and HPLC, the reaction solution was purified using preparative liquid chromatography (Gilson 281 Preparation HPLC, 19 x 250 mm Waters XBridge C18 10 μm preparative column, mobile phase A: 10 mM ammonium bicarbonate aqueous solution, B: acetonitrile, flow rate: 25 ml/min, dual wavelength UV absorption monitoring at 214 and 254 nm, gradient elution: 0-2 min, 5% B; 2-18.9 min, 5-15% B; 18.9-19.4 min, 15-95% B; 19.4-22.4 min, 95% B. The compound was purified with retention time of 10 min and lyophilized to give a white solid (21 mg).

$^{1}$H NMR (400 MHz, DMSO-$d_6$) δ 10.71 (s, 1H), 8.44 (s, 1H), 8.23 (s, 1H), 7.92 (s, 2H), 7.11 (t, *J*= 52Hz, 6H), 6.56 (s, 2H), 6.29 (d, *J* = 28Hz, 1H), 5.95 (d, *J* = 8.4 Hz, 1H), 5.35 (d, *J* = 50.4 Hz, 1H), 5.20 (t, *J* = 11.8 Hz, 1H), 5.11-5.05 (m, 1H), 4.48 (d, *J* = 3.6 Hz, 1H), 4.39-4.28 (m, 2H), 4.14 (s, 1H), 4.02-3.96 (m, 1H), 3.88-3.83 (m, 1H), 3.81-3.76 (m, 1H). $^{31}$P NMR (162 MHz, DMSO-$d_6$) δ 53.30, 50.07. $^{19}$F NMR (376 MHz, DMSO-$d_6$) δ -196.87. MS-ESI [M+H]$^+$:742.8.

**CDN-8** *((2',3')-cyclo-(Rp,Rp)* - [2'-O-thiophosphodiester-guanosine]-[3'-O-thiophosphodiester-2-methylamino-2'-deoxy-2'-fluoro-beta-adenosine] diammonium salt) was prepared using the preparation route of Example 26 of WO2022083584A1, use intermediate 98 ((2',3')-cyclo-(*Rp*,*Rp*) - [2'-O-thiophosphodiester-N2-isobutyryl-3'-O-tert-butyldimethylsilyl-guanosine]-[3'-O-thiophosphodiester-2-chloro-2'-deoxy-2'-fluoro-beta-adenosine], MS + ESI-- [M + H]: 1030.8), produced byproduct from deprotection reaction with methanamine in ethanol, the reaction solution was purified by preparative liquid chromatography (Gilson 281 preparative HPLC, 19 x 250 mm Waters XBridge C18 10 μm preparative column, mobile phase A: 10 mM ammonium bicarbonate aqueous solution, B: acetonitrile, flow rate: 25 ml/min, dual wavelength UV absorption monitoring at 214 and 254 nm with gradient elution: 0-2 min, 5% B; 2-15.6 min, 5-15% B; 15.6-15.8 min, 15-95% B; 15.8-18 min, 95% B. The compound was got with retention time of 14.1 min and lyophilized to give a white solid.

$^{1}$H NMR (400 MHz, DMSO-$d_6$) δ 10.69 (brs, 1H), 8.27 (s, 1H), 8.02 (s, 1H), 7.34-6.97 (m, 3H), 6.31 (brs, 1H), 6.28 (d, *J* = 2.4 Hz, 1H), 5.90 (d, *J* = 0.8 Hz, 1H), 5.44-5.15 (m, 3H), 4.44 (d, *J* = 0.4 Hz, 1H), 4.35-4.27 (m, 1H), 4.24-4.12 (m, 2H), 4.03-3.94 (m, 1H), 3.93-3.79 (m, 2H), 2.85 (s, 3H). $^{31}$P NMR (162 MHz, DMSO-$d_6$) δ 54.02, 51.06. MS-ESI [M+H]$^+$: 738.0.

**Preparation of CDN-9 to CDN-12 (diammonium salt form):**

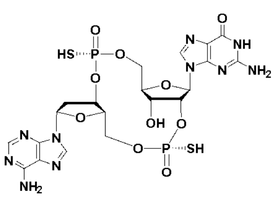

CDN-9

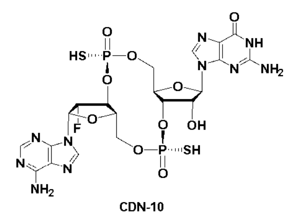

CDN-10

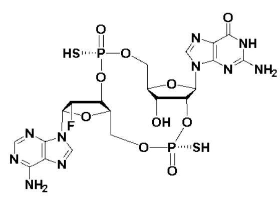

CDN-11

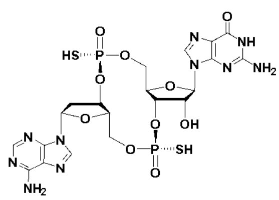

CDN-12

**CDN-9** was prepared using the preparation route of Example 10 of WO2022083584A1**, 2'-chloro-2'-deoxy-2'-fluoro-beta-adenosine** was displaced by **2'-deoxy-adenosine** to obtain the crude product. The crude product was then purified by preparative liquid chromatography (Gilson 281 preparative HPLC, 19 mm × 250 mm Welch 10 μm preparative column, mobile phase A: 10 mM ammonium bicarbonate aqueous solution, B: acetonitrile, flow rate: 20 ml/min, dual wavelength UV absorption monitoring at 214 and 254 nm, gradient elution: 0-5.2 min, 0-5% B; 5.2-10.2 min, 5-20% B; 10.2-10.4 min, 25-95% B; 10.4-12.4 min, 95% B. The retention time of the compound was 1.8 min.

$^{1}H$ NMR (400 MHz, DMSO-$d_6$) δ 8.37 (s, 1H), 8.16 (s, 1H), 8.07 (s, 1H), 7.28 (s, 2H), 6.64 (brs, 1H), 6.34 (dd, *J* = 9.2, 5.4 Hz, 1H), 5.86 (d, *J* = 8.4 Hz, 1H), 5.28 (m, 2H), 4.31 (d, *J* = 4.3 Hz, 1H), 4.19 - 3.94 (m, 4H), 3.76 (m, 2H), 3.11 - 2.96 (m, 1H), 2.69-2.61 (m, 2H). $^{31}P$ NMR (162 MHz, DMSO), δ 56.33, 53.51. MS-ESI [M-H]$^-$: 689.2.

**CDN-10** was prepared by using the route of Example 16 of WO2022083584A1 and replacing **2-chloro-2'-deoxy-2'-fluoro-beta-adenosine** with **2'-deoxy-2'-fluoro-beta-adenosine** to obtain the crude product. The crude product was then purified by preparative liquid chromatography (Gilson 281 preparative HPLC, 19 mm × 250 mm Welch 10 μm preparative column, mobile phase A: 10 mM ammonium bicarbonate aqueous solution, B: acetonitrile, flow rate: 20 mL/min, dual wavelength UV absorption monitoring at 214 and 254 nm, gradient elution: 0-2.4 min, 0-10% B; 2.4-19.0 min, 10-30% B; 19.0-19.6 min, 30-95% B; 19.6-20.0 min, 95% B. The retention time of the compound was 2.9 min.

$^{1}H$ NMR (400 MHz, DMSO-$d_6$, δ): 10.64 (s, 1H), 8.25-8.17 (m, 3H), 7.64 (brs, 2H), 6.68 (brs, 2H), 6.40 (dd, *J* = 20.0, 2.6 Hz, 1H), 5.89 (d, *J* = 8.0 Hz, 1H), 5.38-5.18 (m, 3H), 4.48 (d, *J* = 4.0 Hz, 1H), 4.34 (d, *J* = 12.0 Hz, 1H), 4.27 (t, *J* =8.0 Hz, 1H), 4.15 (s, 1H), 4.02 (t, *J* =6.0 Hz, 1H), 3.89-43.8 (m, 2H). $^{31}P$ NMR (162 MHz, DMSO-$d_6$, δ): 54.01. MS-ESI [M+H]$^+$: 707.0.

**CDN-11** was prepared by using the route of preparation of Example 10 of WO2022083584A1**,** replacing **2-chloro-2'-deoxy-2'-fluoro-beta-adenosine** with **2'-deoxy-2'-fluoro-beta-adenosine** to obtain the crude product. The crude product was then purified by preparative HPLC (Gilson 281 preparative HPLC, 19 mm × 250 mm Welch 10 μm preparative column, mobile phase A: 10 mM ammonium bicarbonate aqueous solution, B: acetonitrile, flow rate: 20 ml/min, dual wavelength UV absorption monitoring at 214 and 254 nm, gradient elution: 0-2.0 min, 0-5% B; 2.0-10.0 min, 5-25% B; 10.0-12.5 min, 25-95% B; 12.5-20 min, 95% B. The retention time of the compound was 6.8 min.

$^{1}H$ NMR (400 MHz, DMSO-$d_6$, δ): 10.71 (s, 1H), 8.39-8.24 (m, 3H), 7.90 (brs, 1H), 7.11(t, *J* = 52.0 Hz,3H), 6.75 (brs, 1H), 6.42 (dd, *J* = 23.4, 2.6 Hz, 1H), 5.91 (d, *J* = 8.4 Hz, 1H), 5.43-5.27 (m, 2H), 5.22 (t, *J* = 10.6 Hz, 1H), 4.46 (d, *J* = 4.2 Hz, 1H), 4.35 (dd, *J* = 10.6, 5.0 Hz, 1H), 4.29-4.21 (m, 1H), 4.15 (s, 1H), 4.02 (t, *J* = 11.2 Hz, 1H), 3.95-3.81 (m, 3H). $^{31}P$ NMR (162 MHz, DMSO-$d_6$, δ): 54.20, 51.26. MS-ESI [M-H]$^-$: 707.0.

**CDN-12** was prepared by using the route of preparation of Example 16 of WO2022083584A1**,** replacing **2'-chloro-2'-deoxy-2'-fluoro-beta-adenosine** with **2'-deoxy-adenosine** to obtain the crude product. The crude product 1 was then purified by preparative liquid chromatography (Gilson 281 preparative HPLC, 19 mm × 250 mm Welch 10 μm preparative column, mobile phase A: 10 mM ammonium bicarbonate aqueous solution, B: acetonitrile, flow rate: 20 ml/min, dual wavelength UV absorption monitoring at 214 and 254 nm, gradient elution: 0-2.4 min, 0-5% B; 2.4-19.0 min, 5-10% B; 19.0-19.5 min, 10-95% B; 19.5-20.0 min, 95% B. The retention time of the compound was 3.0 min.

$^{1}H$ NMR (400 MHz, DMSO-$d_6$, δ): 10.58 (s, 1H), 8.37 (s, 1H), 8.16 (s, 1H), 8.06 (s, 1H),7.28 (s, 2H), 7.12 (brs, 2H), 6.60 (brs, 2H), 6.35-6.32(m, 1H), 5.86 (d, *J* = 8.0 Hz, 1H), 5.39 - 5.28 (m, 2H), 4.31 (s, 1H), 4.18-4.03 (m, 3H), 3.75 (d, *J* = 8.0 Hz, 1H), 3.06-3.01 (m, 2H). $^{31}P$ NMR (162 MHz, DMSO-$d_6$, δ): 56.31, 53.50. MS-ESI [M-H]$^-$: 689.2.

It should be mentioned that the CDNs used in the following examples were diammonium salt forms.

**Preparation of CDN-Linker Intermediate PL**

**Preparation of Intermediate 9:**

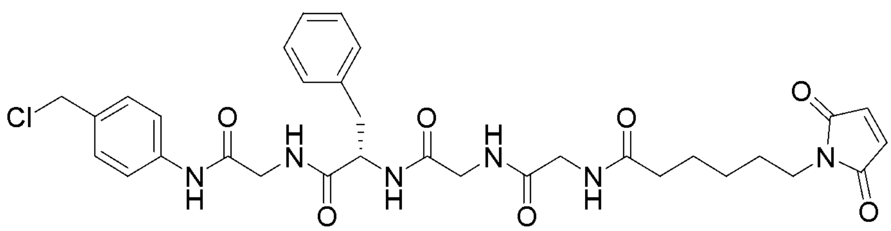

Sulfoxide chloride solution in dichloromethane solution (1.0 mol/L, 6.93 mL, 6.93 mmol) was added to a mixture of (*S*)-6-(2,5-dioxo-2,5-dihydro-1*H*-pyrrol-1-yl)-*N*-(2-((2-(1-((2-((4-(hydroxymethyl)phenyl)amino)-2-oxoethyl)amino)-1-oxo-3-phenylpropyl-2-yl)amino)-2-oxoethyl)amino)-2-oxoethyl)hexamide (2.2 g, 3.47 mmol, CAS No. 2632342-05-1, purchased from Shanghai Bide Pharma) and THF (40 mL) mixture under stirring at 0°C, 5 minutes dropwise addition. The

mixture was stirred continuously at 0°C to room temperature for 2 h, then the solvent was removed under reduced pressure, methyl tert-butyl ether (20 mL) was then added and stirred for 10 min. The filter cake was filtered and collected and dried under vacuum to give **intermediate 9** as a yellow solid (2.2 g, 97.1% yield). MS-ESI [M + H]+: 653.1.

**Preparation of Intermediate 10:**

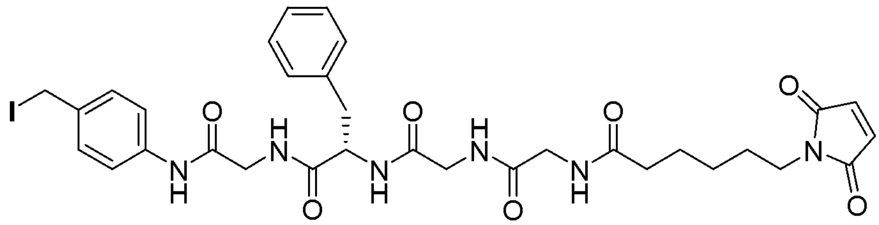

Sodium iodide (5.05 g, 33.68 mmol) was added in portions to a mixture of **intermediate 9** (2.2 g, 3.37 mmol) and acetone (50 mL) under stirring at 0°C and stirred continuously for 16 hours at 0°C to room temperature in the dark. The solvent was then concentrated, and ice-cold water (20 mL) was added and stirred at room temperature for 10 minutes. The filter cake was filtered and collected and dried under vacuum to give **intermediate 10** as a yellow solid (1.5 g, yield 59.7%). MS-ESI [M + H]+: 745.1.

**Preparation of Intermediate 11:**

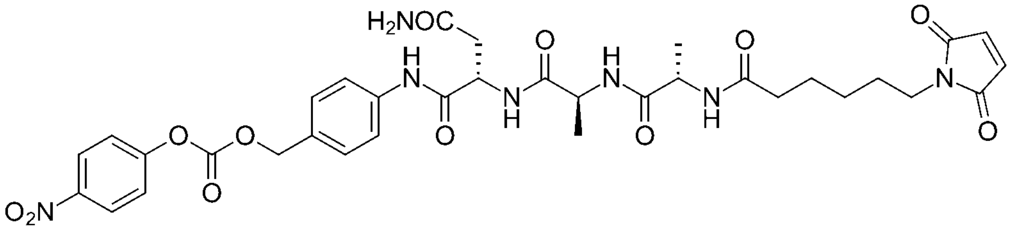

(S)-2-((S)-2-((S)-2-(6-(2,5-dioxo-2,5-dihydro-1H-pyrrole-1-yl)hexanoamido)propionamido)propionamido)-N1-(4-(hydroxymethyl)phenyl)succinamide (3.0 g, 5.2 mmol, CAS No.1638970-44-1) and bis(p-nitrophenyl)carbonate (4.78 g, 15.7 mmol) were dissolved in anhydrous DMF (20 mL), cooled to 0°C, DIEA (3.36 g, 26 mmol) was added, and the mixture was stirred for 2 hours at 0°C. Upon completion, the solution was concentrated under reduced pressure to remove the solvent and purified by column chromatography (methanol/dichloromethane = 20%-10%) to give **intermediate 11** as a yellow solid (550 mg). MS-ESI [M + H]+: 738.3.

**Preparation of PL-1:**

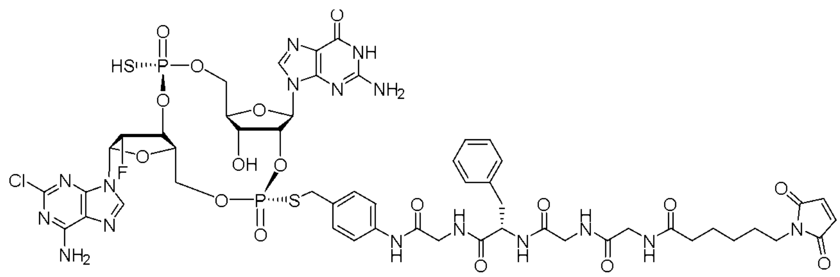

At 0°C under stirring, p-toluenesulfonic acid monohydrate (384 mg, 2.019 mmol) was added portionwise to a solution of compound **CDN-3** (500 mg, 0.673 mmol) in anhydrous DMF (10 mL), followed by **intermediate 10** (500 mg, 0.671 mmol). The reaction mixture was stirred at 0°C to room temperature in the dark for 2 hours. Prep-HPLC purification

was then prepared (Gilson281 preparative HPLC, Waters XBridge C18 10 μm 19 mm × 250 mm preparative column, mobile phase A: 0.1% TFA aqueous solution, B: acetonitrile, flow rate: 25 mL/min, dual wavelength UV absorption monitoring at 214 and 254 nm, gradient elution: 0-2.1 min, 0 - 20% B; 2.1-16.5 min, 20-40% B; 16.5-22.7 min, 40-95% B; 22.7-25.5 min, 95-20% B, the retention time of the compound was 13.2 min), to obtain **PL-1** as a pale yellow solid (80 mg). $^1$H NMR (400 MHz, DMSO-$d_6$ + $D_2O$, δ): 8.25 (s, 1H), 8.17 (s, 1H), 7.58 (d, *J* = 7.9 Hz, 2H), 7.38 (d, *J* = 7.9 Hz, 2H), 7.23 (m, 5H), 6.95 (s, 2H), 6.41 (dd, *J* = 15.8, 4.5 Hz, 1H), 5.95 (d, *J* = 8.0 Hz, 1H), 5.64 (d, *J* = 49.4 Hz, 2H), 5.11 (s, 1H), 4.67 (d, *J* = 3.8 Hz, 1H), 4.49 (m, 2H), 4.38-4.13 (m, 4H), 4.05 (m, 2H), 3.93-3.75 (m, 5H), 3.36 (t, *J* = 7.0 Hz, 2H), 2.86 (d, *J* = 11.7 Hz, 2H), 2.12 (m, 2H), 1.47 (m, 4H), 1.18 (m, 2H). MS-ESI [M+H]$^+$: 1359.1.

**Preparation of PL-2:**

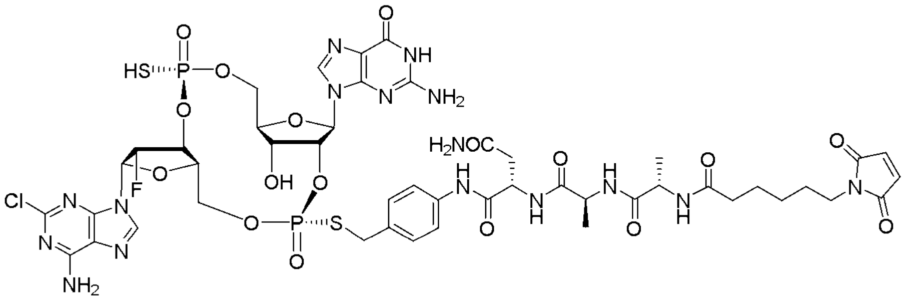

**PL-2** was obtained by using the synthesis route of **PL-1** from (*S*)-2-((*S*)-2-((*S*)-2-(6-(2,5-dioxo-2,5-dihydro-1H-pyrrol-1-yl)hexanamido)propionamido)-N-(4-(hydroxymethyl)phenyl)succinamide (CAS No. 1638970-44-1).

$^1$H NMR (400 MHz, DMSO-$d_6$ + $D_2O$, δ): 8.55 (s, 1H), 8.21 (s, 1H), 7.63 (d, *J* = 7.4 Hz, 2H), 7.37 (d, *J* = 7.9 Hz, 2H), 6.95 (s, 2H), 6.52-6.37 (m, 1H), 6.00 (d, *J* = 8.2 Hz, 1H), 5.67 (d, *J* = 50.5 Hz, 2H), 5.23 (m, 1H), 4.62-4.04 (m, 12H), 3.36 (d, *J*= 7.0 Hz, 2H), 2.57 (m, 2H), 2.08 (d, *J* = 7.4 Hz, 2H), 1.52-1.39 (m, 4H), 1.33-1.02 (m, 6H). MS-ESI [M+H]$^+$: 1297.4; [M/2+H]$^+$: 649.0.

**Preparation of PL-3:**

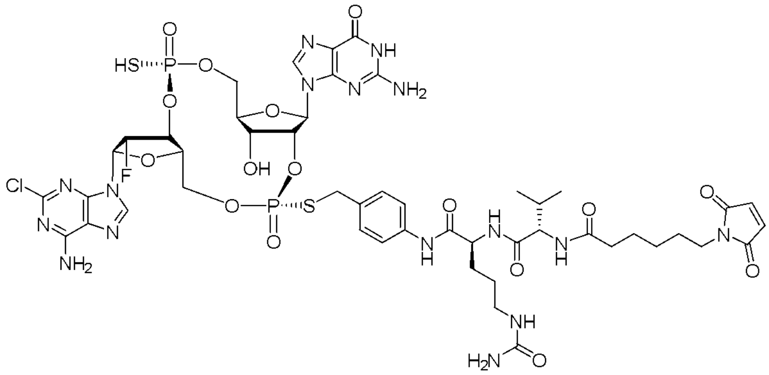

**PL-3** was obtained by using the synthesis route of **PL-1** from 6-(2,5-dioxo-2,5-dihydro-*1H*-pyrrol-1-yl)-*N*-(*S*)-1-((*S*)-1-(4-(hydroxymethyl)phenyl)amino)-1-oxo-5-ureidopentyl-2-yl)amino-3-methyl-1-oxobutyl-2-yl)hexanamide (CAS No. 159857-80-4).

$^1$H NMR (400 MHz, DMSO-$d_6$ + $D_2O$, δ): 8.35 (s, 1H), 8.22 (d, *J* = 2.3 Hz, 1H), 7.58 (d, *J* = 8.3 Hz, 2H), 7.36 (d, *J* = 8.3 Hz, 2H), 6.96 (s, 2H), 6.41 (dd, *J* = 15.8, 4.5 Hz, 1H), 5.96 (d, *J* = 8.6 Hz, 1H), 5.75-5.54 (m, 2H), 5.17 (m, 1H), 4.58 (d, *J* = 3.7 Hz, 1H), 4.46 (d, *J* = 4.0 Hz, 1H), 4.39-3.98 (m, 11H), 3.38 (t, *J* = 7.0 Hz, 2H), 2.99-2.88 (m, 2H), 2.13 (dt, *J* = 14.1, 7.1 Hz, 2H), 1.96 (q, *J* = 6.6 Hz, 1H), 1.48 (m, 8H), 1.18 (m, 2H), 0.86-0.80 (m, 6H). MS-ESI [M+H]$^+$: 1297.4.

**Preparation of PL-4:**

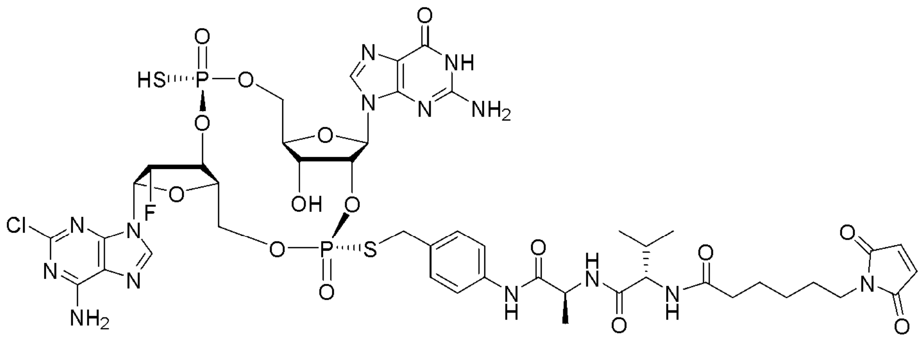

**PL-4** was obtained by using the synthesis route of **PL-1,** from 6-(2,5-dioxo-2,5-dihydro-*1H*-pyrrol-1-yl)-*N*-(*S*)-1-((*S*)-1-(4-(hydroxymethyl)phenyl)amino)-1-oxopropyl-2-yl)amino-3-methyl-1-oxobutyl-2-yl)hexanamide (CAS No. 1870916-87-2).

$^1$H NMR (400 MHz, DMSO-$d_6$ + $D_2O$, δ): 8.26 (s, 1H), 7.96 (s, 1H), 7.56 (d, *J* = 8.3 Hz, 2H), 7.36 (d, *J* = 8.3 Hz, 2H), 6.97 (s, 2H), 6.38 (dd, *J* = 16.2, 4.1 Hz, 1H), 5.90 (d, *J* = 8.7 Hz, 1H), 5.68 (s, 2H), 5.55 (d, *J* = 4.0 Hz, 1H), 5.09-4.98 (m, 1H), 4.70 (d, *J* = 3.8 Hz, 1H), 4.50 (dd, *J* = 9.4, 4.8 Hz, 1H), 4.39-4.29 (m, 1H), 4.28-4.08 (m, 6H), 4.00 (m, 1H), 3.36 (t, *J* = 6.9 Hz, 2H), 2.14 (m, 2H), 2.03-1.91 (m, 1H), 1.48 (m, 3H), 1.27-1.15 (m, 6H), 0.83 (dd, *J* = 14.8, 6.8 Hz, 6H). MS-ESI [M+H]$^+$: 1211.3.

**Preparation of PL-5:**

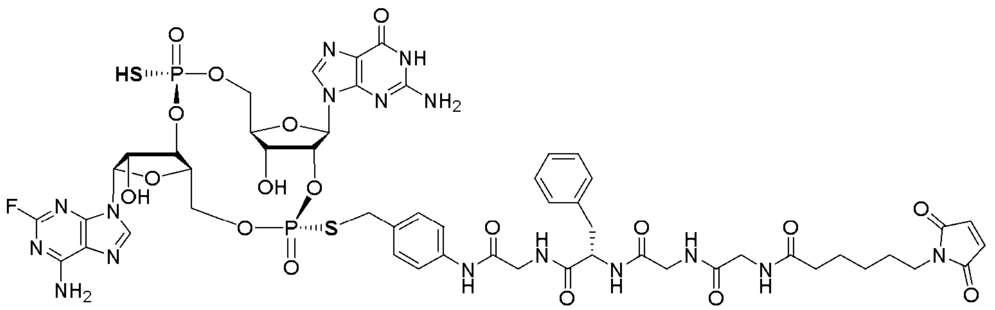

**PL-5** was obtained by using the synthetis route of **PL-1,** with **CDN-2** replacing **CDN-3.** MS-ESI [M + H]$^+$: 1341.2.

**Preparation of PL-6:**

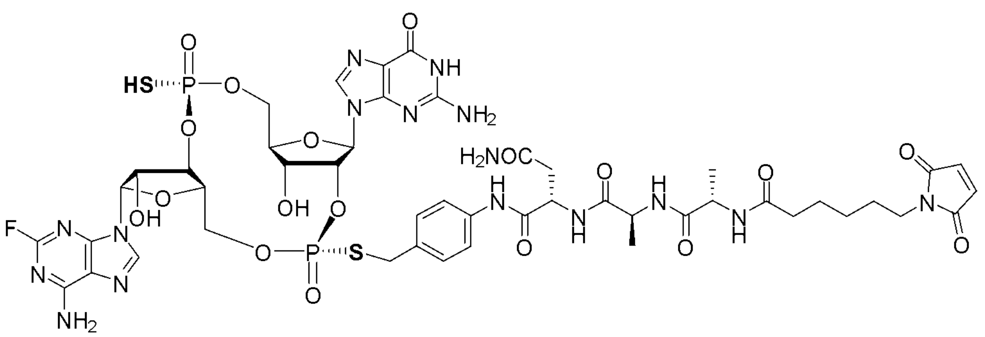

**PL-6** was obtained by using the synthesis route of **PL-2,** with **CDN-2** replacing **CDN-3.** MS-ESI [M + H]+: 1279.3.

**Preparation of PL-9:**

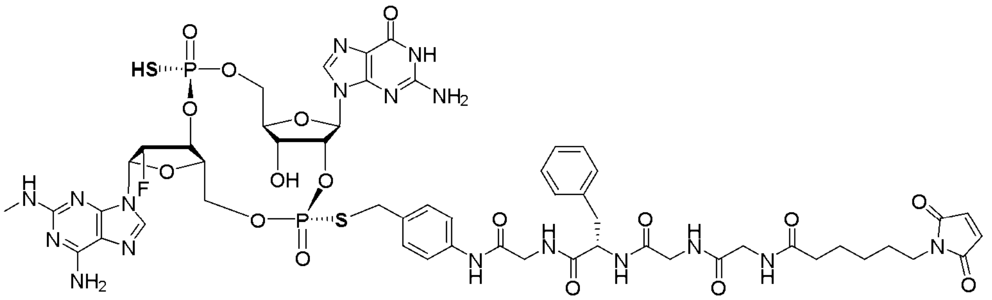

**PL-9** was obtained by using the synthesis route from **PL-1,** with **CDN-8** replacing **CDN-3.**

$^1$H NMR (400 MHz, DMSO-$d_6$ + $D_2O$) δ 9.89 (s, 1H), 8.08 (s, 1H), 8.02 - 7.94 (m, 1H), 7.57 (d, *J* = 8.4 Hz, 2H), 7.35 (d, *J* = 8.8 Hz, 2H), 7.29 - 7.17 (m, 5H), 6.97 (s, 2H), 6.39 - 6.31 (m, 1H), 6.06 - 5.87 (m, 2H), 5.80 - 5.68 (m, 1H), 5.68 - 5.55 (m, 1H), 5.16 - 5.06 (m, 1H), 4.69 - 4.62 (m, 1H), 4.52 - 4.43 (m, 2H), 4.25 - 4.16 (m, 4H), 3.87 - 3.84 (m, 1H), 3.82 - 3.71 (m, 2H), 3.67 (s, 2H), 3.62 (s, 1H), 3.36 (t, *J* = 7.2 Hz, 2H), 3.10 - 3.04 (m, 1H), 2.91 - 2.79 (m, 4H), 2.73 (s, 1H), 2.11 (t, *J* = 7.2 Hz, 2H), 1.53 - 1.41 (m, 4H), 1.22 - 1.15 (m, 2H). MS-ESI [M+H]$^+$: 1354.3.

**Preparation of PL-10:**

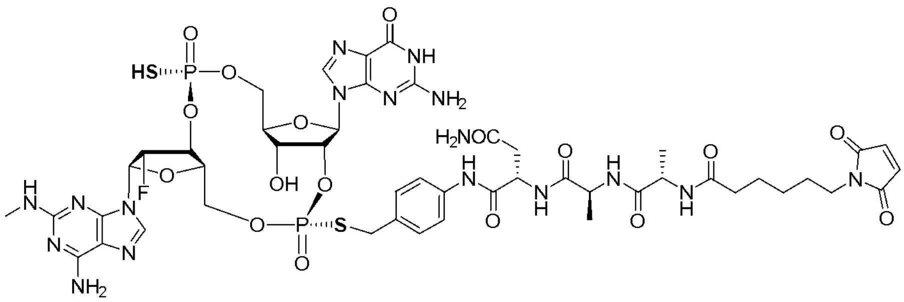

**PL-10** was obtained by using the synthesis route of **PL-2,** with **CDN-8** replacing **CDN-3.**

$^1$H NMR (400 MHz, DMSO-$d_6$ + $D_2O$) δ 9.68 (s, 1H), 8.07 (s, 1H), 8.00 (s, 1H), 7.61 (d, *J* = 8.0 Hz, 2H), 7.34 (d, *J* = 8.4 Hz, 2H), 6.98 (s, 2H), 6.40 - 6.34 (m, 1H), 6.03 - 5.91 (m, 2H), 5.83 - 5.72 (m, 1H), 5.70 - 5.59 (m, 1H), 5.17 - 5.09 (m, 1H), 4.67 - 4.62 (m, 1H), 4.61 - 4.56 (m, 1H), 4.49 - 4.44 (m, 1H), 4.29 - 4.14 (m, 7H), 3.36 (t, *J* = 7.2 Hz, 2H), 2.85 (s, 3H), 2.58 - 2.53 (m, 2H), 2.09 (t, *J* = 7.2 Hz, 2H), 1.51 - 1.41 (m, 4H), 1.25 - 1.15 (m, 8H). MS-ESI [M+H]$^+$: 1292.3 (M+H).

**Preparation of PL-11:**

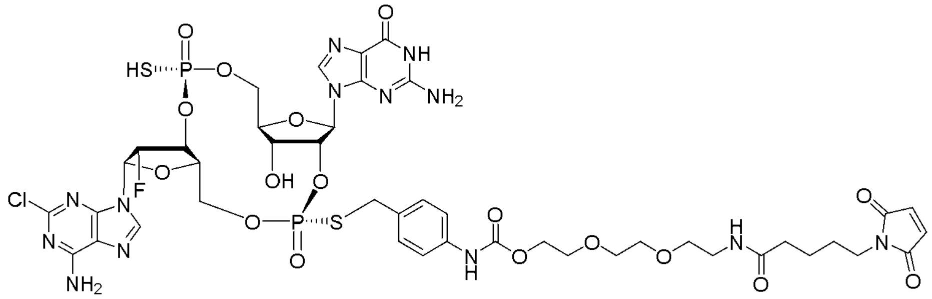

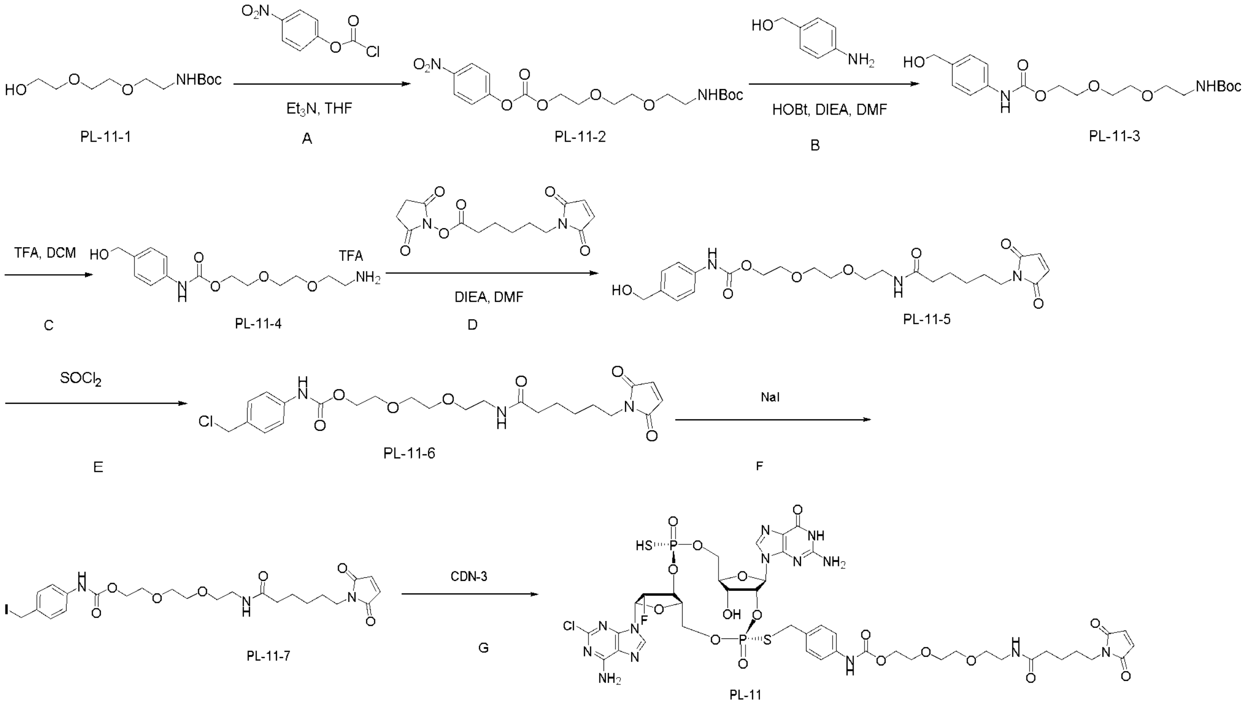


**Step A:** p-nitrophenyl chloroformate (2.67 g, 13.24 mmol) was added slowly to a solution of (2-(2-(2-hydroxyethoxy)ethoxy)ethyl)tert-butyl carbamate (3.0 g, 12.03 mmol) in anhydrous tetrahydrofuran (50 mL) at 0°C, triethylamine (3.65 g, 36.10 mmol) was then added dropwise, and the resulting mixture was stirred for 16 hours at 0°C to room temperature. After completion of the reaction, the reaction solution was concentrated under reduced pressure to remove the solvent and purified by column chromatography (ethyl acetate/petroleum ether = 0-20%) to give **compound PL-11-2** as a yellow solid (2.2 g, yield 90.2%). MS-ESI [M + H]$^+$: 415.1.

**Step B:** To a solution of **compound PL-11-2** (4.50 g, 10.86 mmol) in tetrahydrofuran (50 mL), (4-aminophenyl) methanol (1.34 g, 10.86 mmol) was added, 1-hydroxybenzotriazole (440 mg, 3.26 mmol), N,N-diisopropylethylamine (2.11 g, 16.29 mmol) was then added at room temperature. The mixture was stirred at room temperature for 36 hours. Upon completion of the reaction, the mixture was concentrated to remove the solvent and purified by flash column chromatography (ethyl acetate/petroleum ether = 20% to 50%) to give **compound PL-11-3** as a yellow solid (3.6 g, yield 83.2%). MS-ESI [M + H]$^+$: 399.1.

**Step C:** To a solution of **compound PL-11-3** (3.6 g, 9.03 mmol) in dichloromethane (100 mL), trifluoroacetic acid (20 mL) was added dropwise for 5 minutes at 0°C, stirred for 3 hours at room temperature. The mixture was concentrated to remove the solvent and stirred for 10 minutes with methyl tert-butyl ether (50 mL), filtered, and the filter cake was collected and dried under vacuum to give **compound PL-11-4** as a yellow solid (3.6 g, yield 96.6%). MS-ESI [M + H]$^+$: 299.1.

**Step D:** To a solution of **compound PL-11-4** (3.6 g, 8.73 mmol) in DMF (20 mL), 6-(maleimidyl)succinimidyl hexanoate (3.23 g, 10.48 mmol) and N,N-diisopropylethylamine (2.26 g, 17.46 mmol) were added, the mixture was stirred for 3 hours at 25°C. Tert-Butyl methyl ether (50 mL) was added at 0°C and stirred for 10 minutes, the mixture was filtered, and the filter cake was collected and dried under vacuum to give **compound PL-11-5** as a yellow solid (0.9 g, yield 20.9%). MS-ESI [M + H]$^+$: 492.2.

**Step E: compound PL-11-5** (300 mg, 0.61 mmol) was dissolved in anhydrous tetrahydrofuran (5 mL), after nitrogen displacement, stirred in an external ice bath, and slowly dripped dichlorosulfoxide in dichloromethane solution (1 M, 0.91 mL, 0.91 mmol) into the above reaction system, after completion of dripping, the reaction solution was stirred at

room temperature for about 4 hours, and the reaction was completed, and the solution was concentrated under reduced pressure to remove solvent to obtain **compound PL-11-6** (300 mg, yield 96.3%) as a yellow oily crude, which was directly used for the next reaction. MS-ESI $[M + H]^+$: 510.1.

**Step F:** Sodium iodide (147 mg, 0.98 mmol) was added in portions to a stirred mixture of **compound PL-11-6** (50 mg, 0.098 mmol) in 10 mL of anhydrous acetone at 0° C. The resulting mixture was stirred for 16 hours at 0°C to room temperature in the dark. Upon completion, the resulting mixture was concentrated under reduced pressure to remove the solvent, ice-cold water (20 mL) was added, the mixture was stirred at room temperature for 10 minutes, filtered, and the filter cake was collected and dried under vacuum to give **compound PL-11-7** (50 mg, yield 84.7%) as a yellow solid. MS-ESI $[M + H]^+$: 602.1.

**Step G:** Intermediate **CDN-3** (50 mg, 0.067 mmol) was dissolved in anhydrous DMF (10 mL) and cooled to 0°C. Under dark and light-protected conditions, p-toluenesulfonic acid monohydrate (38 mg, 0.201 mmol) and compound **PL-11-7** (47 mg, 0.081 mmol) were added in portions. The mixture was stirred in the dark at 0°C to room temperature for 2 hours. Upon completion, HPLC purification was performed by reverse phase preparation, (Gilson281 preparative HPLC, Waters XBridge C18 10 μm 19 mm × 250 mm preparative column, mobile phase A: 0.1% TFA in water, B: acetonitrile, flow rate: 25 mL/min, dual wavelength UV absorption monitoring at 214 and 254 nm, gradient elution: 0-2.1 min, 0-20% B; 2.1-18.5 min, 20-40% B; 18.5-22.7 min, 40-95% B; 22.7-25.5 min, 95-20% B, the retention time of the compound was 12.5 min), to obtain **PL-11** as a pale yellow solid (15 mg, yield 18.5%).

$^1H$ NMR (400 MHz, DMSO-$d_6$ + $D_2O$, δ) 8.22 (s, 1H), 8.14 (d, *J* = 2.1 Hz, 1H), 7.32 (q, *J* = 8.7 Hz, 4H), 6.82 (s, 2H), 6.16 (dd, *J* = 16.5, 4.1 Hz, 1H), 5.89 (d, *J* = 8.6 Hz, 1H), 5.54 (t, *J* = 12.2 Hz, 1H), 5.41 (s, 1H), 5.28 (s, 1H), 5.07 (s, 1H), 4.59 (d, *J* = 3.7 Hz, 1H), 4.52 - 4.44 (m, 1H), 4.24 (d, *J* = 3.8 Hz, 1H), 4.00 - 3.89 (m, 2H), 3.57 (dd, *J* = 5.6, 3.5 Hz, 2H), 3.49 (ddt, *J* = 8.9, 6.4, 3.3 Hz, 5H), 3.37 (t, *J* = 5.6 Hz, 2H), 3.31 (t, *J* = 7.0 Hz, 2H), 3.15 (t, *J* = 5.7 Hz, 2H), 2.01 (t, *J* = 7.3 Hz, 2H), 1.41 (m, 4H). MS-ESI $[M+H]^+$: 1202.2.

**Preparation of PL-12:**

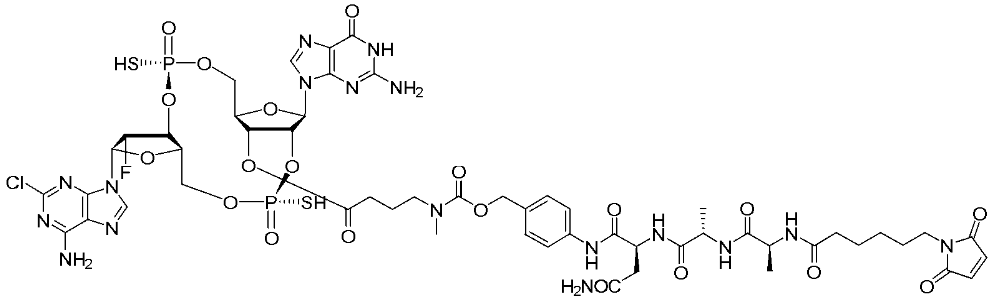

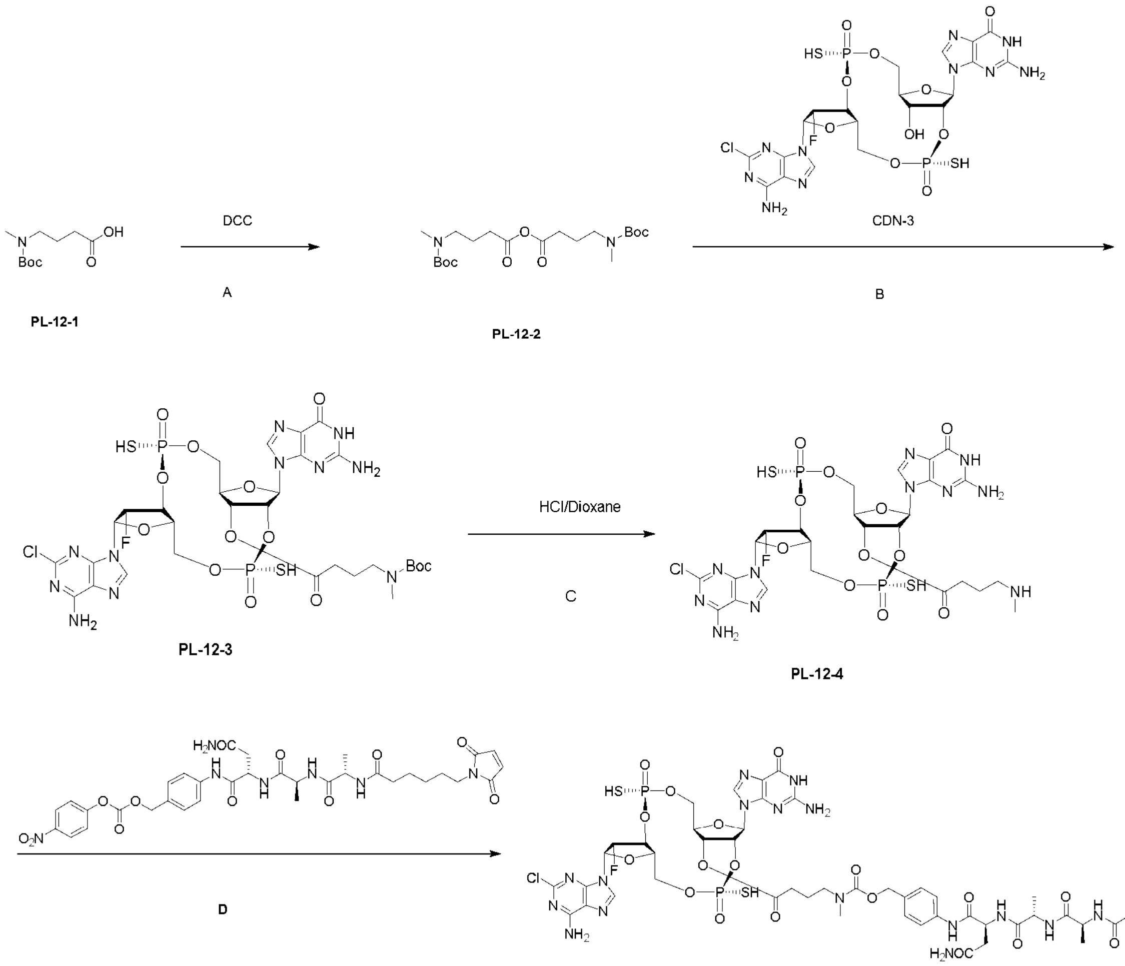

PL-12-1
PL-12-2
PL-12-3
PL-12-4
PL-12

**Step A:** 4-(tert-butylcarbonyl (methyl) amino) butyric acid (4.0 g, 18.4 mmol), DCC (3.8 g, 18.4 mmol) was sequentially added to anhydrous dichloromethane (40 mL) at 0°C, the resulting mixture was stirred for 2 h at room temperature. After completion of the reaction, the reaction solution was cooled to 0°C, filtered after 0.5 h, and the filtrate was collected and concentrated to give **compound PL-12-2** as a clear oil (3.8 g, yield 99.0%).

$^1$H NMR (400 MHz, Chloroform-*d*) δ 3.29 (t, *J* = 6.9 Hz, 4H), 2.85 (d, *J* = 1.6 Hz, 6H), 2.47 (t, *J* = 7.3 Hz, 4H), 1.87 (p, *J* = 7.2 Hz, 4H), 1.45 (s, 18H). MS-ESI [M + H]$^+$: 417.2.

**Step B:** Compound **CDN-3** (200.0 mg, 0.269 mmol), DMAP (32.8 mg, 0.269 mmol), triethylamine (272 mg, 2.69 mmol) were sequentially added to DMF (10 mL) at 0°C, the resulting mixture was stirred for 5 minutes at room temperature, then **PL-12-2** (1.12 g, 2.69 mmol) was added and the resulting mixture was stirred overnight at room temperature. After completion of the reaction, the reaction solution was filtered, collected, and purified by reverse-phase preparative HPLC to give compound **PL-12-3** as a white solid (150 mg, yield 59.1%). MS-ESI [M + H]$^+$: 942.1.

**Step C:** At 0°C, a solution of HCl in 1,4-dioxane (4 M, 5 mL) was added dropwise to a stirred mixture of compound **PL-12-3** (140 mg, 0.148 mmol) in anhydrous 1,4-dioxane (5 mL). The resulting mixture was stirred at 0°C to 20°C for 2 hours. After completion, the mixture was concentrated under reduced pressure to remove the solvent. The residue was then treated with methyl tert-butyl ether (MTBE, 10 mL) at room temperature, stirred for 0.5 h, and filtered. The filter cake

was collected and dried under vacuum to afford the white solid compound **PL-12-4.** (130 mg, yield 99.5%). $^{1}H$ NMR (400 MHz, DMSO-$d_6$ + $D_2O$) δ 8.66 (s, 1H), 8.21 (d, *J* = 2.6 Hz, 1H), 6.32 (dd, *J* = 22.5, 2.7 Hz, 1H), 5.97 (d, *J* = 8.2 Hz, 1H), 5.71 - 5.52 (m, 2H), 5.51 - 5.34 (m, 1H), 5.27 (t, *J* = 10.9 Hz, 1H), 4.38 (q, *J* = 6.4, 4.8 Hz, 2H), 4.15 (dd, *J* = 27.1, 16.4 Hz, 2H), 3.92 (m, 4H), 3.03 (t, *J* = 7.8 Hz, 2H), 2.60 (s, 3H), 1.95 (q, *J* = 8.4 Hz, 2H). MS-ESI $[M+H]^+$: 842.1.

**Step D: Intermediate PL-12-4** (130 mg, 0.154 mmol) and **Intermediate 11** (148 mg, 0.185 mmol) were dissolved in anhydrous DMF (5 mL) and cooled to 0°C. Triethylamine (155.6 mg, 1.54 mmol) was added dropwise, and the mixture was stirred at 0°C for 2 hours. After completion, the crude product was purified by reversed-phase preparative liquid chromatography (Gilson 281 preparative HPLC system, Waters XBridge C18 10 μm, 21.2 mm × 250 mm column; mobile phase A: 10 mM NH4HCO3, mobile phase B: acetonitrile; flow rate: 20 mL/min; UV detection at 214 and 254 nm; gradient elution: 0-2.5 min, 15% B; 2.5-15.5 min, 15-35% B; 15.5-15.7 min, 35-95% B; 15.7-17.7 min, 95% B; 17.7-17.9 min, 95-15% B; 17.9-22.5 min, 15% B). The target compound eluted at 10.7 min, yielding **PL-12** as a white solid (16 mg, 7.1% yield).

$^{1}H$ NMR (400 MHz, DMSO-$d_6$ + $D_2O$) δ 8.20 - 8.15 (m, 2H), 7.65 - 7.60 (m, 2H), 7.32 (d, *J* = 8.4 Hz, 2H), 6.92 (s, 2H), 6.29 (dd, *J* = 23.6, 2.4 Hz, 2H), 5.92 (d, *J* = 8.5 Hz, 1H), 5.52 - 5.38 (m, 3H), 5.33 - 5.23 (m, 2H), 5.02 (s, 3H), 4.61 (t, *J* = 6.5 Hz, 1H), 4.34 (t, *J* = 7.6 Hz, 1H), 4.26 - 4.19 (m, 2H), 3.40 - 3.26 (m, 5H), 2.87 (s, 3H), 2.61 (dd, *J* = 6.6, 3.6 Hz, 2H), 2.41 (d, *J* = 6.9 Hz, 2H), 2.11 (t, *J* = 7.4 Hz, 2H), 1.87 - 1.81 (m, 2H), 1.49 (dt, *J* = 15.4, 7.7 Hz, 4H), 1.23 (q, *J* = 9.7, 8.6 Hz, 8H). MS-ESI $[M+H]^+$: 1440.2.

**Preparation of PL-13:**

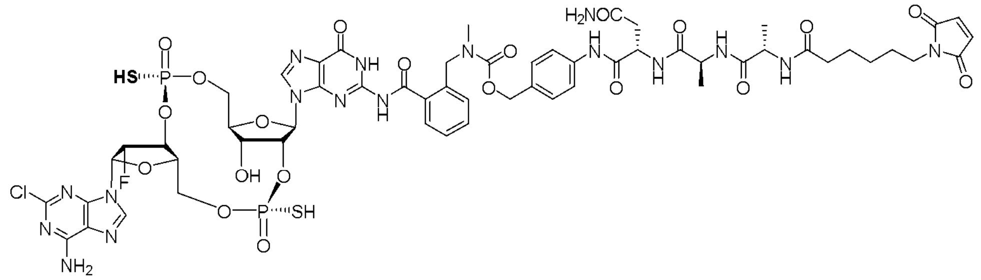

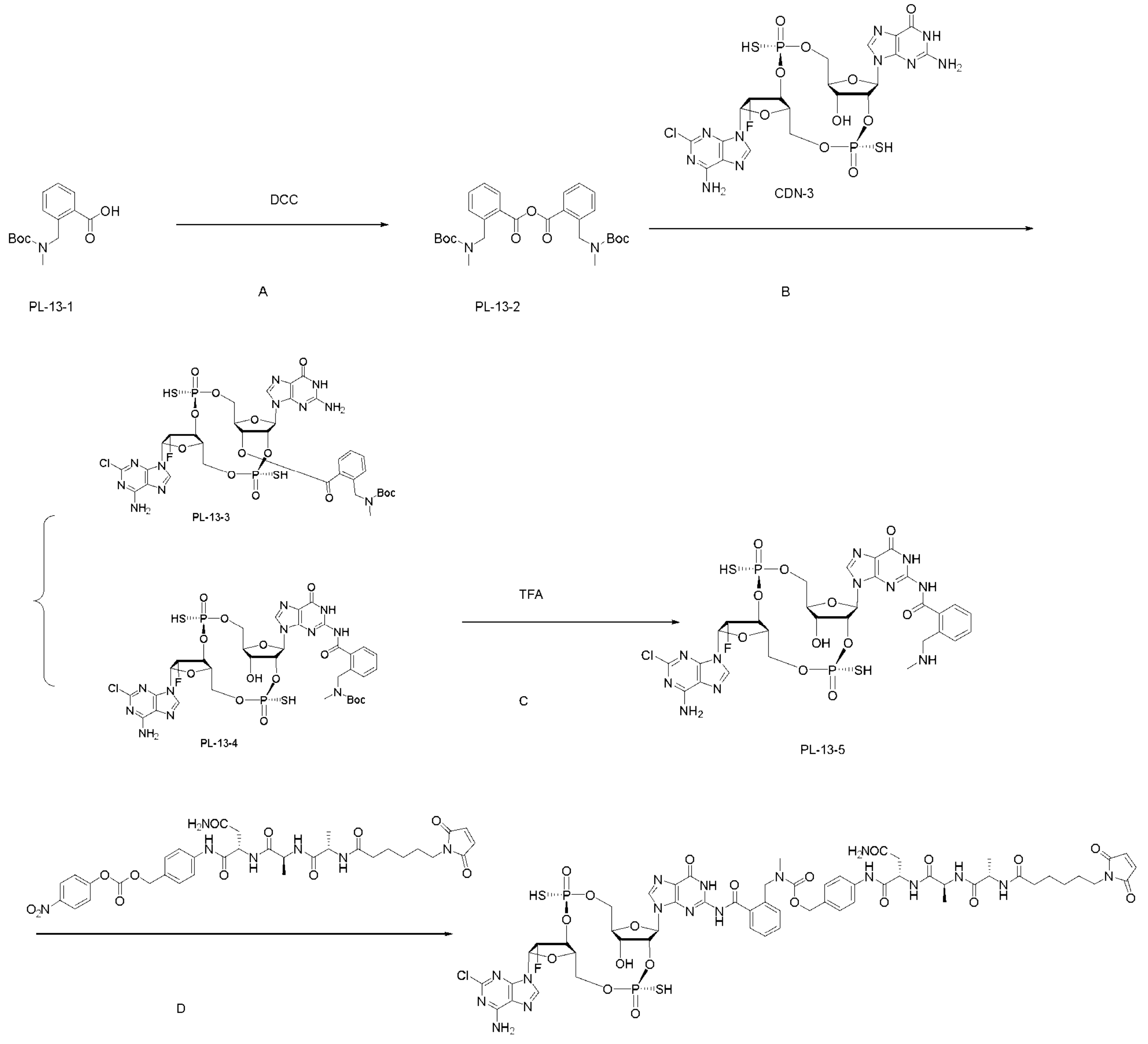


PL-13-1 PL-13-2 CDN-3 PL-13-3 PL-13-4 PL-13-5 PL-13

**Step A:** 2-[(tert-butoxycarbonyl-methyl-amino)methyl]benzoic acid (referring to "Journal of the American Chemical Society, 2015, vol. 137, # 15, p. 4924 - 4927" published method to prepare) (5.0 g, 18.8 mmol) and DCC (3.88 g, 18.8 mmol) were successively added to anhydrous dichloromethane (40 mL) at 0°C, and the resulting mixture was stirred for 2 h at room temperature. After completion of the reaction, the reaction solution was cooled to 0°C, filtered after 0.5 h, and the filtrate was collected and concentrated to give **compound PL-13-2** as a clear oil (1.8 g, yield 18.6%).

$^1$H NMR (400 MHz, Chloroform-*d*) δ 8.10 (s, 2H), 7.65 (t, *J* = 7.7 Hz, 2H), 7.38 (t, *J* = 7.6 Hz, 4H), 4.93 (d, *J* = 12.3 Hz, 4H), 2.95 (s, 6H), 1.50 (s, 9H), 1.39 (s, 9H). MS-ESI [M+ Na]$^+$: 535.2.

**Step B: CDN-3** (300.0 mg, 0.4 mmol), DMAP (97.7 mg, 0.8 mmol), triethylamine (202 mg, 2.69 mmol) were successively added to DMF (10 mL) under nitrogen, followed by **compound PL-13-2** (1.65 g, 3.2 mmol) in DMF (10 mL)

and the resulting mixture was stirred overnight at 100°C. After completion of the reaction, the reaction solution was filtered, the filtrate was collected and purified by reversed-phase preparation to give **compound PL-13-4** as a white solid (50 mg, yield 12.5%) and **compound PL-13-3** as a white solid (150 mg, yield 37.5%), respectively.

**PL-13-3:** MS-ESI $[M + H]^+$: 990.0, HPLC Retention Time: 1.975 min;

**PL-13-4:** MS-ESI $[M + H]^+$: 990.0, HPLC Retention Time: 2.111 min;

HPLC Method: Agilent Poroshell 120 SB-C18, 3.0 x 50 mm, 2.7 μm analytical column, mobile phase A: 0.05% TFA in water, B: acetonitrile, flow rate: 1.0 mL/min, dual wavelength UV absorption monitoring at 214 and 254 nm, gradient elution: 0-0.2 min, 0-5% B; 0.2-2 min, 5-95% B; 2-4 min, 95% B; 4-4.2 min, 95-5% B; 4.2-5 min, 5% B.

**Step C:** TFA (1 mL) was added to a stirred mixture of **compound PL-13-4** (50 mg, 0.05 mmol) in anhydrous dichloromethane (8 mL) at 0°C, and the resulting mixture was stirred for 1.5 hours at 0°C to room temperature. Upon completion, the resulting mixture was concentrated under reduced pressure to remove the solvent, the mixture was stirred with methyl tert-butyl ether (10 mL) at room temperature, filtered, and the filter cake was collected and dried under vacuum to give **compound PL-13-5** as a white solid (50 mg, yield 100%). MS-ESI $[M + H]^+$: 890.0.

**Step D: Compound PL-13-5** (50 mg, 0.056 mmol), **intermediate 11** (61.99 mg, 0.084 mmol) were dissolved in anhydrous DMF (5 mL), cooled to 0°C, triethylamine (16.9 mg, 0.168 mmol) was added, the mixture was stirred for 2 hours at 0°C. Upon completion, purification was performed by reverse-phase preparation (Gilson281 preparative HPLC, Waters XBridge C18 10 μm 21.2 mm × 250 mm preparative column, mobile phase A: 10 mM $NH_4HCO_3$, B: acetonitrile, flow rate: 20 mL/min, dual wavelength UV absorption monitoring at 214 and 254 nm, gradient elution: 0-2.5 min, 20% B; 2.5-15.5 min, 20-35% B; 15.5-15.7 min, 35-95% B; 15.7-17.7 min, 95% B; 17.7-17.9 min, 95-20% B; 17.9-22.5 min, 20% B; the retention time of the compound was 14.7 min, to obtain compound **PL-13** as a white solid (7 mg, yield 8.3%). MS-ESI $[M + H]^+$: 1488.2.

**Preparation of PL-14:**

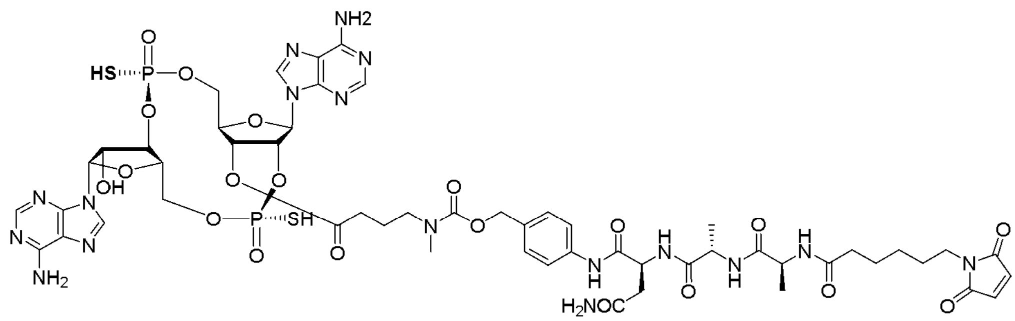

**PL-14** was obtained by using the synthesis route of **PL-12,** with ADU-S100 (CAS No. 1638750-95-4, purchased from MCE, Cat. No. #HY12885A) replacing **CDN-3.** MS-ESI $[M + H]^+$: 1388.3.

<u>**Preparation of bifunctional CDN-antibody conjugated compounds**</u>

**Preparation of ADC-1:**

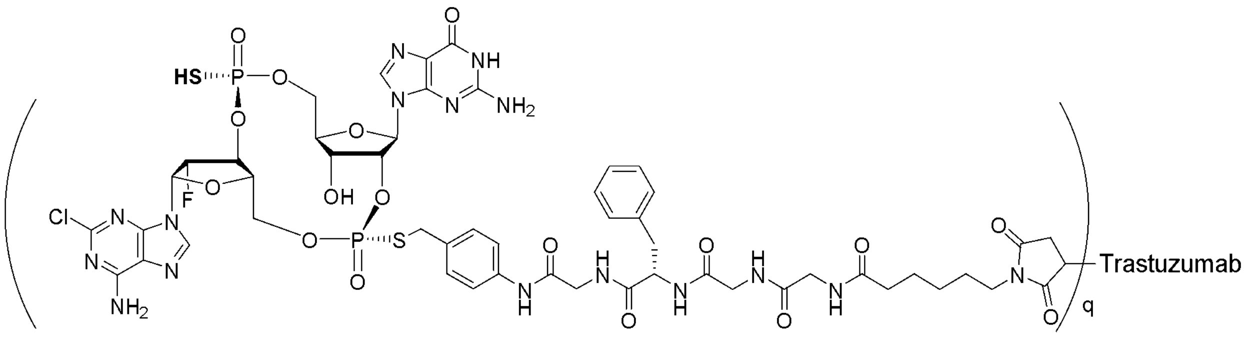

1) Antibody Reduction

Tri-(2-carboxyethyl)phosphine aqueous solution (Sigma) (10 mM, 0.134 mL, 1.344 μmol, 8.0 eq) was added to the anti-HER2 antibody Trastuzumab (CAS No. 180288-69-1, purchased from Shanghai Aladdin Reagent) in buffer (pH = 6.4, 30 mM His-HAc + 5 mM EDTA; 2.5 mL, 10 mg/mL, 0.168 μmol, 1.0 eq) at 25°C. The mixture was then placed in a 25°C water bath shaker and allowed to react for 2 hours.

2) Coupling small molecule

**PL-1** (2.7 mg, 2.02 μmol, 12 eq) was dissolved in 0.10 mL DMSO and added dropwise to the above solution. The reaction was allowed to proceed for 1.0 hour. The reaction mixture was purified using an ultrafiltration centrifugal device (Merck, Cat. No. #UFC903096) with a 30 kDa molecular weight cutoff (MWCO). The solution was exchanged to 15 mM His-Tris + 8% sucrose buffer(18.72 mg/mL, 1.1 mL) and stored at -78°C.

3) Determination of the Concentration of Antibody Conjugate **ADC-1**

The concentration of the ADC was determined by ultraviolet spectrophotometry (a Thermo NanoDrop 2000 spectrophotometer). At a specific wavelength, the total absorbance value of the ADC solution is equal to the sum of the absorbance value of the toxin molecule and the monoclonal antibody at that wavelength.

After the cuvette containing sodium succinate buffer were placed in the reference absorption cell and the sample assay absorption cell, respectively, the solvent blank was subtracted, the cuvette containing the test article solution was placed in the sample assay absorption cell and the absorbance at 280 nm and 258 nm was measured.

Result calculation:

$$(1)\ A_{280\ nm} = (C_{drug} \times E_{drug\text{-}280} + C_{mab} \times E_{mab\text{-}280}) \times I$$

$$(2)\ A_{258\ nm} = (C_{drug} \times E_{drug\text{-}258} + C_{mab} \times E_{mab\text{-}258}) \times I$$

$$(3)\ R = (E_{drug\text{-}258})/(E_{drug\text{-}280})$$

wherein,

$A_{280\,nm}$: average absorbance value at 280 nm for a single sample of the test article solution at an optical length of 1 cm;

$A_{258\,nm}$: average absorbance value at 258 nm for a single sample of the test article solution at an optical length of 1 cm;

$E_{mab\text{-}280}$: mass extinction coefficient of the protein at 280 nm, 1.48 L/(g × cm);

$E_{mab\text{-}258}$: mass extinction coefficient of the protein at 258 nm, 0.653 L/(g × cm);

R: ratio of toxin extinction coefficient at 258 nm and 280 nm;

$C_{mab}$: Concentration of protein, mg/mL;

$C_{drug}$: Toxin concentration, mg/mL;

I: Optical length, cm

The concentration of the antibody conjugate can be calculated from the extinction coefficient and absorbance of monoclonal antibodies and drugs at two detection wavelengths by combining the (1), (2), and (3) three equations.

$$C_{mab} = (A_{280\ nm} \times R - A_{258\ nm})/(E_{mab\text{-}280} \times R - E_{mab\text{-}258})$$

The concentration of antibody conjugate **ADC-1** was determined as 18.72 mg/mL by the method described above.

4) Determination of DAR Value (Drug-Antibody Ratio) of Antibody Conjugate **ADC-1**

DAR values for antibody conjugate **ADC-1** were determined and calculated using TOF MS (500-8000) ESI+ in conjunction with Maxent1 software and the results are presented in the table below.

| DAR value | Corresponding mass spectra number | Mass spectra intensity percentage (%) |
|---|---|---|
| DAR0 | 145157.0 | 7.86 |
| DAR4 | 150602.0 | 7.94 |
| DAR5 | 151960.0 | 3.09 |
| DAR6 | 153321.0 | 10.40 |
| DAR7 | 154682.0 | 11.90 |
| DAR8 | 156040.0 | 58.81 |

The drug-antibody ratio DAR value for antibody conjugate **ADC-1,** as calculated by percent mass spectra intensity, was 7.86% × 0 + 7.94% × 4 + 3.09% × 5 + 10.40% × 6 + 11.90% × 7 + 58.8 1% × 8 = 6.6.

5) Determination of Protein Monomer Content of Antibody Conjugate **ADC-1**

The antibody-drug conjugate (**ADC-1**) was analyzed by SEC chromatography (size-exclusion chromatography) (SHIMADZU SHIMSEN Ankylo SEC-300, 3 μm, 7.8 mm × 300 mm) using a mobile phase of 300 mM NaCl in 50 mM PBS (pH 6.8)/MeCN = 90/10 (v/v) at a flow rate of 0.5 mL/min. The column temperature was maintained at 20°C, and the injection volume was 20 μL. The chromatographic run time was 30 min. The analysis revealed that the protein monomer content of **ADC-1** was 84.39%.

**Preparation of ADC-2:**

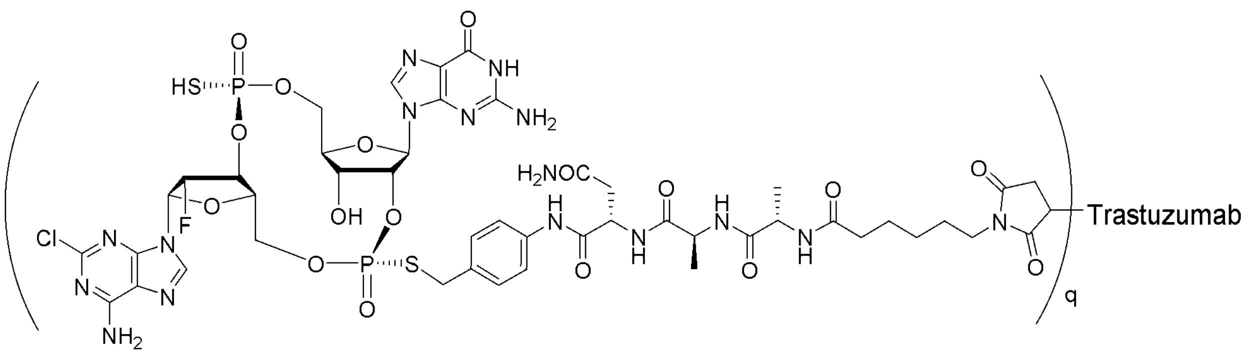

The antibody conjugate **ADC-2** (22.38 mg/mL, 1.2 mL in 15 mM His-Tris + 8% sucrose buffer) was obtained by coupling the intermediate **PL-2** with the anti-HER2 antibody Trastuzumab using the synthesis and analysis method of **ADC-1** and the DAR was 5.7, and the protein monomer content was 96.78%.

**Preparation of ADC-3:**

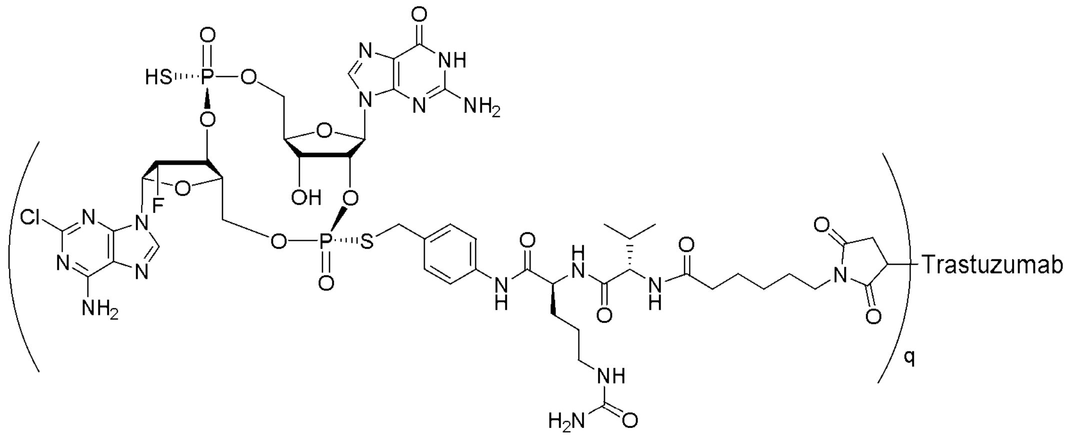

The antibody conjugate **ADC-3** (14.80 mg/mL, 1.5 mL, stored in 15 mM His-Tris + 8% sucrose buffer) was obtained by coupling the intermediate **PL-3** and anti-HER2 antibody Trastuzumab using the synthesis and analysis method of **ADC-1** and the DAR was 7.9, and the protein monomer content was 96.80%.

**Preparation of ADC-4:**

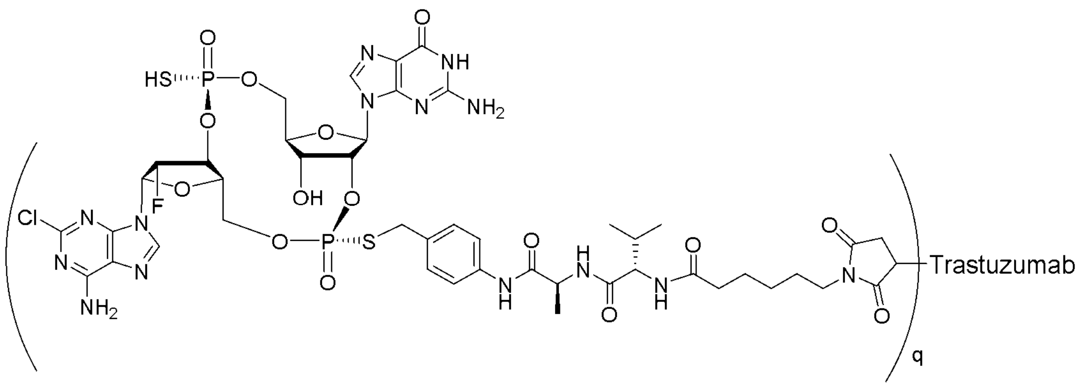

The antibody conjugate **ADC-4** (3.79 mg/mL, 1.5 mL, stored in 15 mM His-Tris + 8% buffer) was obtained by coupling the intermediate **PL-4** with anti-HER2 antibody Trastuzumab using the synthesis and analysis method of **ADC-1,** and the DAR was 8.0, and the protein monomer content was 99.20%.

**Preparation of ADC-5:**

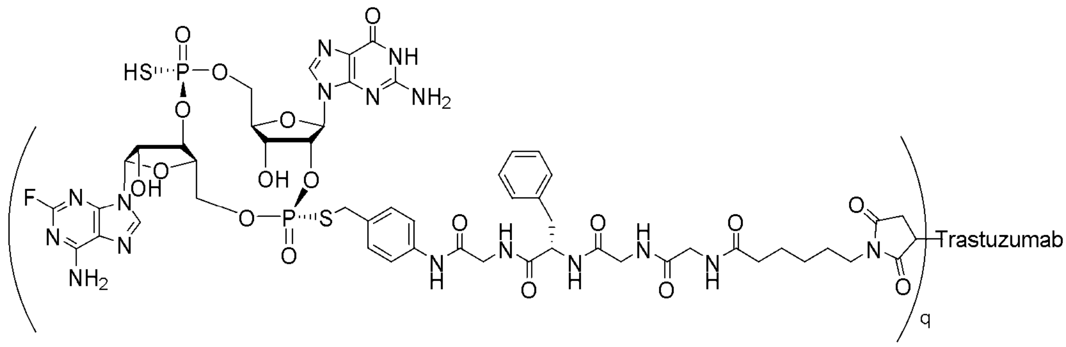

The antibody conjugate **ADC-5** (1.00 mg/mL, 1.0 mL, stored in 15 mM His-Tris + 8% sucrose buffer) was obtained by coupling the intermediate **PL-5** and anti-HER2 antibody Trastuzumab using the synthesis and analysis method of **ADC-1,** and the DAR was 6.9, and the protein monomer content was 97.60%.

**Preparation of ADC-6:**

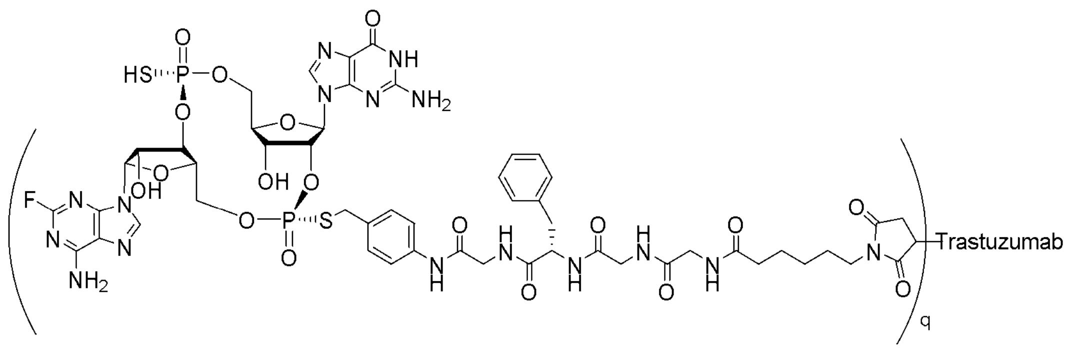

The antibody conjugate **ADC-6** (1.38 mg/mL, 0.9 mL, stored in 15 mM His-Tris + 8% sucrose buffer) was obtained by coupling the intermediate **PL-6** with the anti-HER2 antibody Trastuzumab using the synthesis and analysis method of **ADC-1,** and the DAR was 5.0, and the protein monomer content was 98.70%.

**Preparation of ADC-9:**

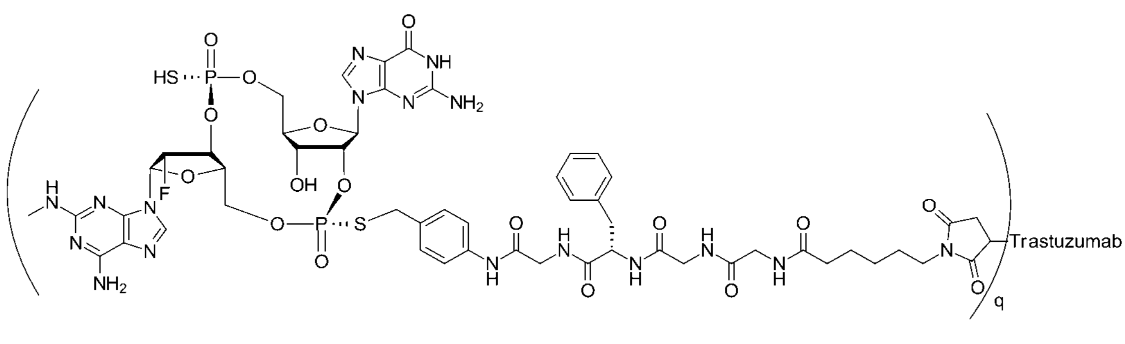

The antibody conjugate **ADC-9** (7.50 mg/mL, 0.55 mL, stored in 15 mM His-Tris + 8% sucrose buffer) was obtained by coupling the intermediate **PL-9** with the anti-HER2 antibody Trastuzumab using the synthesis and analysis method of **ADC-1,** and the DAR was 7.5, and the protein monomer content was 88.00%.

**Preparation of ADC-10:**

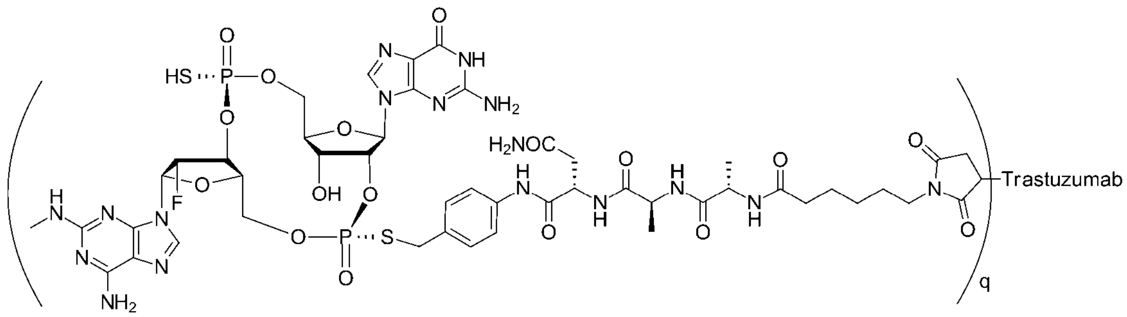

The antibody conjugate **ADC-10** (4.80 mg/mL, 0.70 mL, stored in 15 mM His-Tris + 8% sucrose buffer) was obtained by coupling the intermediate **PL-10** with anti-HER2 antibody Trastuzumab using the synthesis and analysis method of **ADC-1,** and the DAR was 7.2, and the protein monomer content was 98.70%.

**Preparation of ADC-11:**

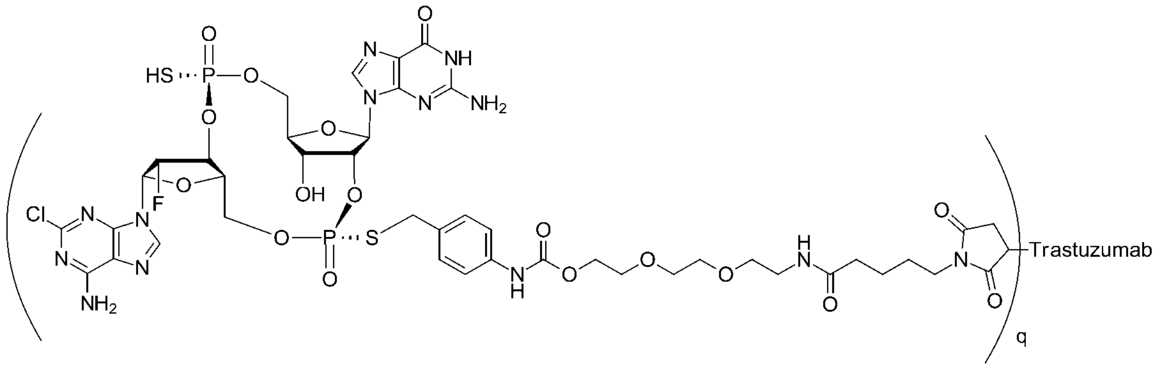

The antibody conjugate **ADC-11** (5.70 mg/mL, 0.60 mL, stored in 15 mM His-Tris + 8% sucrose buffer) was obtained by coupling the intermediate **PL-11** with the anti-HER2 antibody Trastuzumab using the synthesis and analysis method of **ADC-1,** and the DAR was 8.0, and the protein monomer content was 96.85%.

**Preparation of ADC-12:**

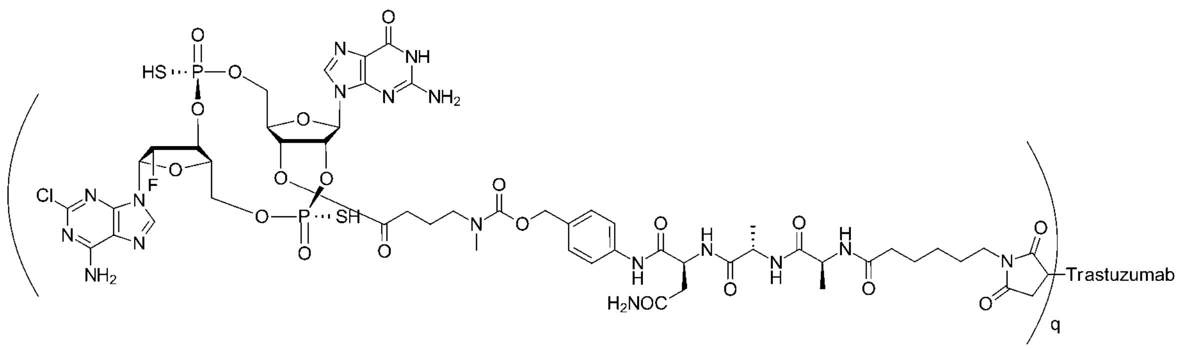

The antibody conjugate **ADC-12** (2.00 mg/mL, 2.00 mL, stored in 15 mM His-Tris + 8% sucrose buffer) was obtained by coupling the intermediate **PL-12** with anti-HER2 antibody Trastuzumab using the synthesis and analysis method of **ADC-1,** and the DAR was 8.0, and the protein monomer content was 99.90%.

**Preparation of ADC-13:**

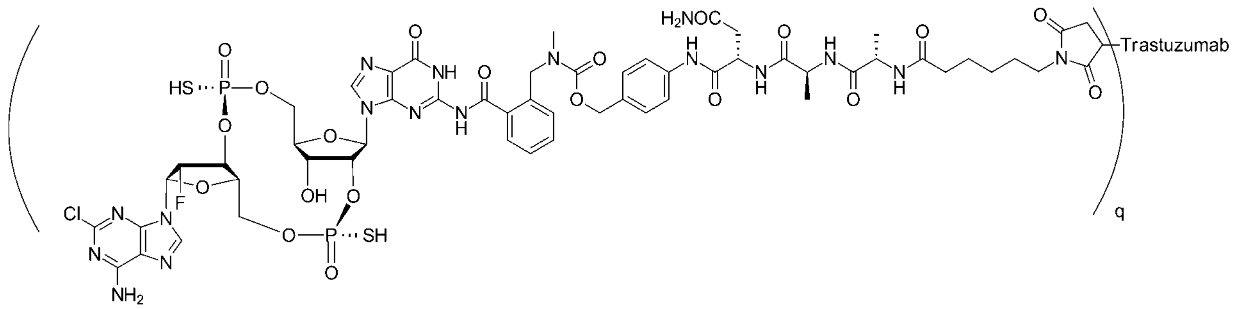

The antibody conjugate **ADC-13** (1.58 mg/mL, 1.70 mL, stored in 15 mM His-Tris + 8% sucrose buffer) was obtained by coupling the intermediate **PL-13** with the anti-HER2 antibody Trastuzumab using the synthesis and analysis method of **ADC-1,** and the DAR was 8.0, and the protein monomer content was 99.50%.

**Preparation of ADC-14 (Positive Control Compound):**

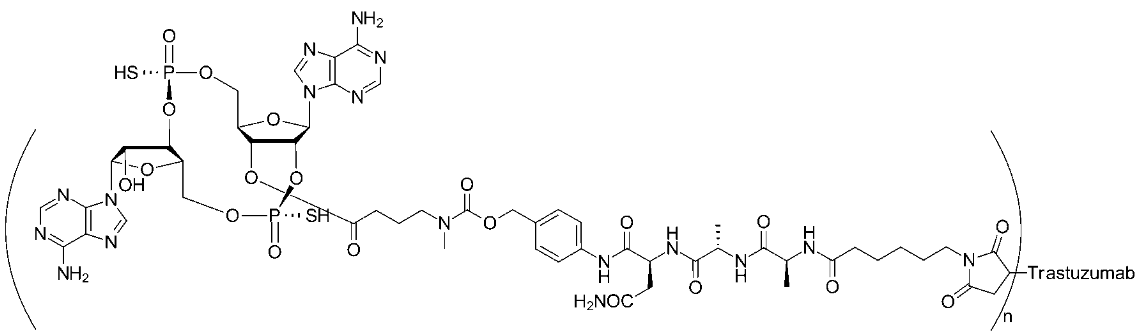

The antibody conjugate **ADC-14** (4.60 mg/mL, 1.50 mL, stored in 15 mM His-Tris + 8% sucrose buffer) was obtained by coupling the intermediate **PL-14** and anti-HER2 antibody Trastuzumab using the synthesis and analysis method of **ADC-1,** and the DAR was 7.7, and the protein monomer content was 97.70%.

**Preparation of ADC-15:**

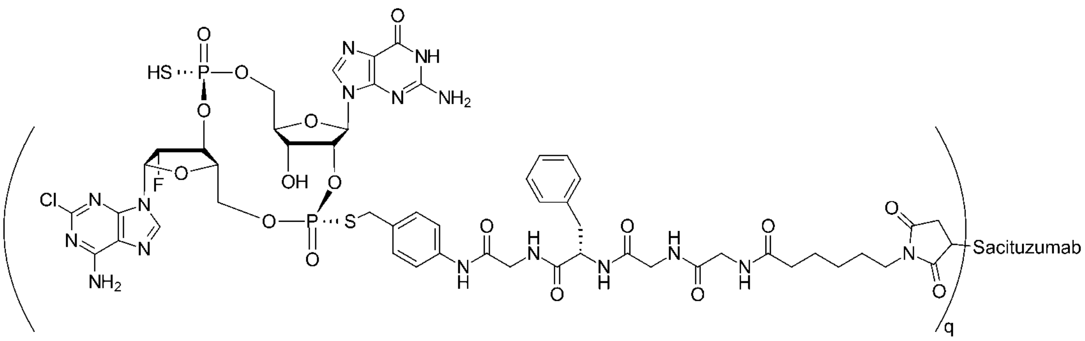

The antibody conjugate **ADC-15** (5.12 mg/mL, 0.90 mL, stored in 15 mM His-Tris + 8% sucrose buffer) was obtained by coupling the intermediate **PL-1** and anti-TROP2 antibody Sacituzumab (CAS No. 1796566-95-4, purchased from Zhejiang BioRay Pharmaceutical Co., Ltd.) using the synthesis and analysis method of ADC-1, and the DAR was 7.1, and the protein monomer content was 98.40%.

**Preparation of ADC-16:**

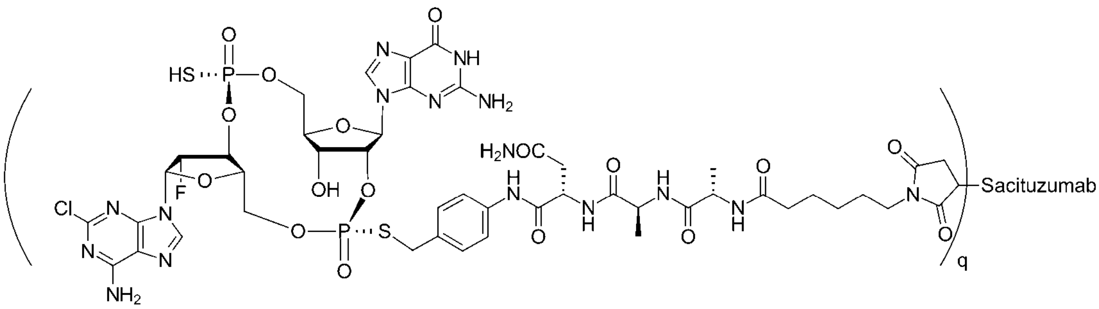

The antibody conjugate **ADC-16** (6.92 mg/mL, 0.90 mL, stored in 15 mM His-Tris + 8% sucrose buffer) was obtained by coupling the intermediate **PL-2** and anti-TROP2 antibody Sacituzumab (CAS No. 1796566-95-4, purchased from Zhejiang BioRay Pharmaceutical Co., Ltd.) using the synthesis and analysis method of ADC-1, DAR was 6.5, and protein monomer content was 99.20%.

The amino acid sequence of Sacituzumab (purchased from Zhejiang BioRay Pharmaceutical Co., Ltd.) is as follows:

Heavy chain (SEQ ID NO: 14)

QVQLQQSGSE LKKPGASVKV SCKASGYTFT NYGMNWVKQA PGQGLKWMG WINTYTGEPTY TDDFKGRFAF SLDTSVSTAY LQISSLKADD TAVYFCARGG FGSSYWYFDV WGQGSLVTVS SASTKGPSVF PLAPSSKSTS GGTAALGCLVKDYFPEPVTV SWNSGALTSG VHTFPAVLQS SGLYSLSSVV TVPSSSLGTQTYICNVNHKP SNTKVDKRVE PKSCDKTHTC PPCPAPELLG GPSVFLFPPK PKDTLMISRT PEVTCVVVDV SHEDPEVKFN WYVDGVEVHN AKTKPREEQY NSTYRVVSVL TVLHQDWLNG KEYKCKVSNK ALPAPIEKTI SKAKGQPREP QVYTLPPSRE EMTKNQVSLT CLVKGFYPSD IAVEWESNGQ PENNYKTTPP VLDSDGSFFL YSKLTVDKSR WQQGNVFSCS VMHEALHNHY TQKSLSLSPG K

Light chain (SEQ ID NO: 13)

DIQLTQSPSS LSASVGDRVS ITCKASQDVS IAVAWYQQKP GKAPKLLIYSASYRYTGVPD

RFSGSGSGTD FTLTISSLQP EDFAVYYCQQ HYITPLTFGA GTKVEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC

**Preparation of ADC-17:**

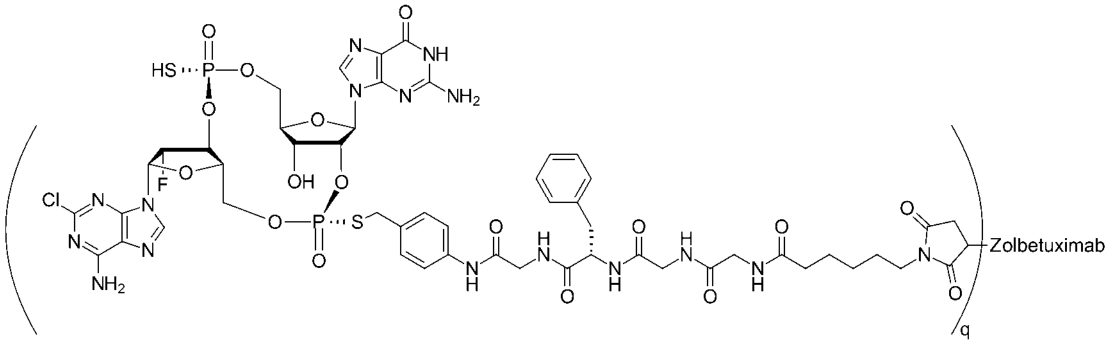

The antibody conjugate **ADC-17** (4.13 mg/mL, 1.10 mL, stored in 15 mM His-Tris + 8% sucrose buffer) was obtained by coupling intermediate **PL-1** and anti-CLDN18.2 antibody Zolbetuximab (CAS No. 1496553-00-4, purchased from Shanghai Sanyou Bio) using the synthesis and analysis method of **ADC-1** and the was 7.9, and the protein monomer content was 98.90%.

**Preparation of ADC-18:**

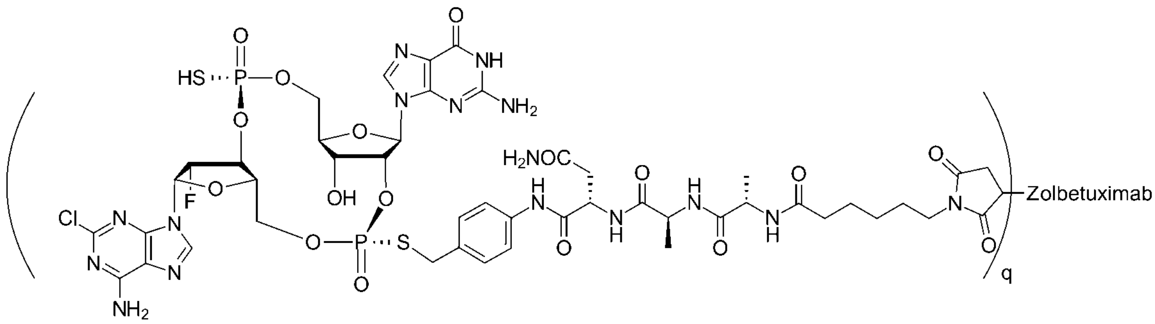

The antibody conjugate **ADC-18** (3.33 mg/mL, 1.00 mL, stored in 15 mM His-Tris + 8% sucrose buffer) was obtained by coupling the intermediate **PL-2** and anti-CLDN18.2 antibody Zolbetuximab (CAS No. 1496553-00-4, purchased from Shanghai Sanyou Biological) using the synthesis and analysis method of **ADC-1,** and the DAR was 8.0, and the protein monomer content was 97.70%.

The amino acid sequence of Zolbetuximab (purchased from Shanghai Sanyou Bio) is as follows:

Heavy chain (SEQ ID NO: 25)

QVQLQQPGAELVRPGASVKLSCKASGYTFTSYWINWVKQRPGQGLEWIGNIYPSDSY TNYNQKFKDKATLTVDKSSSTAYMQLSSPTSEDSAVYYCTRSWRGNSFDYWGQGTTLTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKRVEPKSCDKTHTCPPCPAPELLGGPSVF LFPPKPKDTLMISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLPPSREEMTKNQ

VSLTCLVKGFYPSDIAVEWESNGQPENNYKTTPPVLDSDGSFFLYSKLTVDKSRWQQGNVF SCSVMHEALHNHYTQKSLSLSPGK

Light chain (SEQ ID NO: 24)

DIVMTQSPSSLTVTAGEKVTMSCKSSQSLLNSGNQKNYLTWYQQKPGQPPKLLIYWAS TRESGVPDRFTGSGSGTDFTLTISSVQAEDLAVYYCQNDYSYPFTFGSGTKLEIKRTVAAPSV FIFPPSDEQLKSGTASVVCLLNNFYPREAKVQWKVDNALQSGNSQESVTEQDSKDSTYSLS STLTLSKADYEKHKVYACEVTHQGLSSPVTKSFNRGEC

**Preparation of ADC-19:**

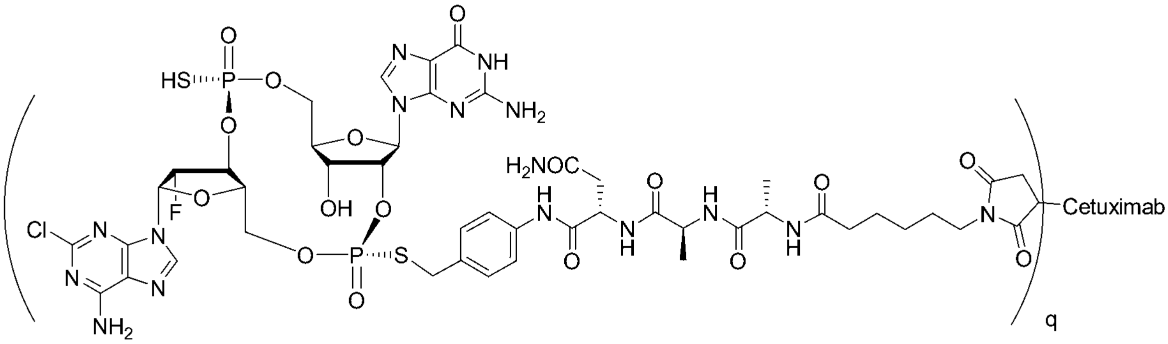

The antibody conjugate **ADC-19** (2.40 mg/mL, 1.50 mL, stored in 15 mM His-Tris + 8% sucrose buffer) was obtained by coupling the intermediate **PL-2** and anti-EGFR antibody Cetuximab (CAS No. 205923-56-4, purchased from Taizhou Mai Bo Tai Ke Pharmaceutical) using the synthesis and analysis method of **ADC-1,** and the DAR was 8.0, and the protein monomer content was 99.30%.

The amino acid sequence of Cetuximab (purchased from Taizhou Mabpharm Ltd) is as follows:

Heavy chain (SEQ ID NO: 36)

QVQLKQSGPG LVQPSQSLSI TCTVSGFSLT NYGVHWVRQS PGKGLEWLGV IWSGGNTDYN TPFTSRLSIN KDNSKSQVFF KMNSLQSNDT AIYYCARALT YYDYEFAYWG QGTLVTVSAA STKGPSVFPL APSSKSTSGG TAALGCLVKD YFPEPVTVSW NSGALTSGVH TFPAVLQSSG LYSLSSVVTV PSSSLGTQTY ICNVNHKPSN TKVDKRVEPK SCDKTHTCPP CPAPELLGGP SVFLFPPKPK DTLMISRTPE VTCVVVDVSH EDPEVKFNWY VDGVEVHNAK TKPREEQYNS TYRVVSVLTV LHQDWLNGKE YKCKVSNKAL PAPIEKTISK AKGQPREPQV YTLPPSREEM TKNQVSLTCL VKGFYPSDIA VEWESNGQPE NNYKTTPPVL DSDGSFFLYS KLTVDKSRWQ QGNVFSCSVM HEALHNHYTQ KSLSLSPGK

Light chain (SEQ ID NO: 35)

DILLTQSPVI LSVSPGERVS FSCRASQSIG TNIHWYQQRT NGSPRLLIKY ASESISGIPS RFSGSGSGTD FTLSINSVES EDIADYYCQQ NNNWPTTFGA GTKLELKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG

LSSPVTKSFN RGEC

## Examples of Drug Activity

### Example 1: Activation effect of CDN fragment of the present invention on IFN-β secretion by THP-1 cells

THP-1 cells are a human monocytic leukemia cell line whose STING phenotype is HAQ type, namely R71H-G230A-R293Q. In this experiment, Human IFN-beta DuoSet ELISA Kit from R&D Company (R&D Company, Cat. No. #DY814-05) and DuoSet ELISA Ancillary Reagent Kit 2 (R&D Company, Cat. No. #DY008) were used to evaluate the activation effect of representative compounds of the present invention on IFN-β secretion by THP-1 cells.

When THP-1 cells (ATCC #TIB-202) were revived, the cryovial was rapid shaken in a 37°C water bath to thaw it with 1 min. The thawed cell suspension was mixed well with RPMI1640 medium (Hyclone Company, Cat. No. #SH30027.01) containing 10% FBS (Lifetechnology Company, Cat. No. #10099-141), centrifuged at 1000 rpm for 5 min, and the supernatant was discarded. The cell pellet was resuspended in 5 mL of complete medium (RPMI1640 medium containing 10% FBS), placed in a cell culture flask with a bottom area of 25 $cm^2$, and cultured in a cell incubator at 37°C, 5% $CO_2$, and 95% humidity. Cell passage was performed when the confluence rate of cells reached approximately 80%. For cell passage, all cells in the flask were transferred to a 15 mL centrifuge tube, centrifuged at 1000 rpm for 5 min, and the supernatant was discarded. The cell pellet was resuspended in 5 mL of new complete medium, 1 mL was placed in a cell culture flask with a bottom area of 25 $cm^2$, and 4 mL of fresh complete medium was added to continue the culture. Plating was performed when the confluence rate of cells reached approximately 80% again. Referring to the cell passage method at plating, 1/5 of the cell suspension was maintained and the remaining 4/5 of the cell suspension was placed in a 15 mL centrifuge tube. The cells were centrifuged, and the old medium was discarded. The cells were washed once with RPMI1640 medium (without serum), and centrifuged to remove the supernatant. The cells were resuspended with RPMI 1640 medium (without serum). Cell viability was measured by trypan blue exclusion to ensure plating when cell viability was above 95%. RPMI1640 medium (without serum) was used to prepare a cell suspension at a density of $1.1 \times 10^6$ viable cells/mL, and 180 μL of the cell suspension was added to wells of a 96-well cell culture plate (NUNC, Cat. No. #167008) to achieve a cell density of $2 \times 10^5$ viable cells/well in the culture plate.

A DMSO stock solution of 10 mM compound was first serially diluted at a 3.16-fold dilution ratio with DMSO (Sigma, Cat. No.# D2650) to a fifth concentration and a DMSO control without compound was set at a sixth concentration. DMSO solutions containing different concentrations of compounds were then diluted at a 10-fold dilution ratio with PBS, to yield a DMSO content of 10% in each concentration of compound solution. Finally, 20 μL of the above solution was added to the corresponding cell culture plate to achieve a starting concentration of 100 μM compound, a 3.16-fold dilution ratio of adjacent concentrations, and a DMSO content of 1% in the cell culture plate. The plates were maintained in a cell incubator for an additional 24 hours.

The ELISA detection procedure was performed according to the instructions of the R&D

ELISA plate coating: the capture antibody (mouse anti-human IFN-β capture antibody PART No. 844508) was diluted with PBS (R & D System Cat. No. #DY006) to prepare the working concentration, 100 μL of capture antibody working solution was added to a 96-well ELISA plate, the plate was sealed and incubated at room temperature overnight. The capture antibody working solution was discarded, the cell plate was washed 3 times with washing buffer (0.05% Tween-20 in PBS, pH 7.2-7.4, R & D System Cat. No. #WA126), 400 μL of washing buffer was used per well, the washing buffer was fully removed per wash, and the plate was inverted and tapped on clean paper after the last wash to completely remove the washing buffer. 300 μL of blocking buffer (1% BSA in PBS, pH 7.2-7.4, R & D System Cat. No. #DY995) was added to each well and incubated at room temperature for 1-2 hours. The wash steps above were repeated to prepare each plate for sample addition.

Sample detection: 100 μL of sample or standard (recombinant human IFN-β standard, PART No. #844510) was added into each well, the plate was sealed and incubated at room temperature for 2 hours, and the washing steps of the above plate coating method were repeated. Then, 100 μL of detection antibody (biotinylated mouse anti-human IFN-β detection antibody, PART No. #844509) was added to each well, the plate was sealed and incubated at room temperature for 2 hours, and the washing steps of the above plate coating method were repeated. Subsequently, 100 μL of Streptavidin-HRP (PART No. #893975) working solution was added to each well, the plate was sealed and incubated at room temperature for 20 min. This procedure was protected from light, and the washing steps of the above plate coating method were repeated. Then, 100 μL of a 1:1 mixture of substrate solution (Color Reagent A ($H_2O_2$)) and Color Reagent B (tetramethylbenzidine) (R & D System Cat. No. #DY999) was added to each well, and the plate was sealed and incubated at room temperature for 20 min, this process was protected from light. Finally, 50 μL of stop solution (2 N $H_2SO_4$, R & D System Cat. No. #DY994) was added to each well and the plates were tapped to ensure adequate mixing.

OD450 was measured in each well using a multiplate reader (Molecular Devices, Spectramax M3). If the wavelength calibration function was available, it was set to 540 nm or 570 nm. If wavelength calibration was not available, OD540 or OD570 was subtracted from OD450. This process was completed within 30 min after the addition of the stop solution.

2'3'-cGAMP (Invivogen, Cat. No. #tlrl-nacga23) was used as the positive control compound and ADU-S100

(MCE, Cat. No. #HY12885A) was used as the control compound:

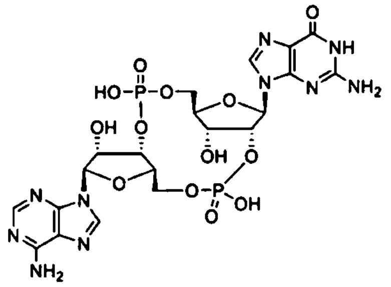

2',3'-cGAMP

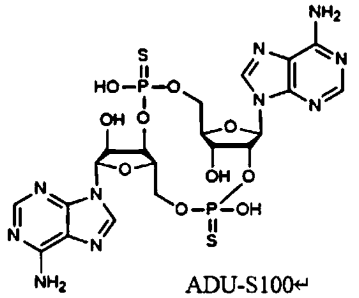

ADU-S100

Data was analyzed using GraphPad Prism 7.0 software and a standard curve for ELISA IFN-β content was obtained using a log-log plot. The OD values obtained from each sample well were substituted into the standard curve equation to determine the corresponding IFN-β concentrations. Dose-response curves of IFN-β concentration versus compound concentration were obtained by regression fitting the data with a non-linear S-curve and EC50 values were calculated.

Table 1 - Activation of IFN-β secretion by representative CDN fragments in THP-1 cells (EC50, μM)

| CDN | THP-1 activity | CDN | THP-1 activity | CDN | THP-1 activity |
|---|---|---|---|---|---|
| 2',3'-cGAMP | B | CDN-4 | A | CDN-9 | B |
| ADU-S100 | A | CDN-5 | A | CDN-10 | A |
| CDN-1 | B | CDN-6 | A | CDN-11 | A |
| CDN-2 | A | CDN-7 | B | CDN-12 | A |
| CDN-3 | A | CDN-8 | A | | |

wherein: A: EC50 1~10 μM; B: EC50 10.1~100 μM

The experimental results showed that the CDN fragments in the present invention activated the secretion of IFN-β in THP-1 cells. As can be seen from Table 1, **CDN-2, 3, 4, 5, 6, 8, 10, 11** and **12** compounds in particular have significant STING agonist activity: they have EC50 stronger than 2',3'-cGAMP, all comparable to ADU-S100.

**Example 2: Inhibitory activity of the CDN fragment of the present invention against CT26 cell proliferation**

CT26 cells are a mouse colorectal cancer cell line. In this experiment, CellTiter-Glo Luminescence Cell Viability Assay kit from Promega was used to assess the inhibitory activity of the compounds on CT26 cell proliferation.

When CT26 cells (ATCC #CRL-2638) were revived, the cryovial was rapid shaken in a 37°C water bath to thaw with 1 min. The thawed cell suspension was mixed well with DMEM medium (GE Company, Cat. No. #SH30243.01) containing 10% FBS (GIBCO, Cat. No. #10099-141), centrifuged at 1000 rpm for 5 min, and the supernatant was discarded. The cell pellet was resuspended in 5 mL of complete medium (DMEM medium containing 10% FBS), placed in a cell culture flask with a bottom area of 25 $cm^2$, and cultured in a cell incubator at 37°C, 5% $CO_2$, and 95% humidity. Passage was performed when the confluence rate of cells reached 70%~80%. Referring to the cell passage method at plating, all cells in the flask were transferred to a 15 mL centrifuge tube, centrifuged at 1000 rpm for 5 minutes, and the supernatant was discarded. The cell pellet was resuspended in 5 mL of new complete medium, 1 mL was placed in a cell culture flask with a bottom area of 25 $cm^2$, and 4 mL of fresh complete medium was added to continue the culture. Plating was performed when the cell confluence rate of cells reached 70% to 80% again. Referring to the cell passage method at plating, 1/5 of the cell suspension was maintained and the remaining 4/5 of the cell suspension was placed in a 15 mL centrifuge tube. The cells were centrifuged, the old medium was discarded, the cells was washed once with DMEM medium (without serum), and centrifuged to remove the supernatant. Cells were resuspended with DMEM medium (without serum). Cell viability was measured by trypan blue exclusion to ensure plating when cell viability was above 95%. A cell suspension at a density of 1.1 x $10^4$ viable cells/mL was prepared using DMEM medium (without serum) and 90 μL of the cell suspension was added to 96-well clear flat-bottom black-walled cell culture plate wells (Corning, Cat. No. #3603) to yield a cell density of 1000 viable cells/well in the plate. A control group without cells, without compounds, and with complete medium only (i.e., culture medium control) was set up, and a control group without compounds and with cells (i.e.,

cell control) was set up. The plates were incubated overnight in a cell incubator.

A DMSO stock solution of 10 mM compound was first serially diluted at a 3.16-fold dilution ratio with DMSO (Sigma, Cat. No. #D2650) to a ninth concentration and a tenth concentration of DMSO control without compound was set. DMSO solutions containing different concentrations of the compound were then diluted a 10-fold dilution ratio with PBS (Solarbio company, Cat. No. #P1020) to achieve a DMSO content of 10% in each concentration of the compound solution. Finally, 10 μL of the above solution was added to the corresponding cell culture plate to achieve a starting concentration of 100 μM compound, a 3.16-fold dilution ratio of adjacent concentrations, and a DMSO content of 1% in the cell culture plate. The plates were maintained in a cell incubator for an additional 120 hours.

After 120 hours, the CellTiter-Glo reagent (Promega, Cat. No. #G7572) was thawed and the plate was equilibrated at room temperature for 30 min. Then, 100 μL CellTiter-Glo was added to each well of the plate and shaken on an orbital shaker for 5 min to fully lyse the cells. The plate was placed at room temperature for 20 min to stabilize the cold light signal, and the cold light value of each well was scanned with a multifunctional microplate reader (Molecular Devices, Spectramax M3) at full wavelength.

The compound of the present invention and ADU-S100 (MCE, Cat. No. #HY12885A) were used as controls: Clofarabine (Wuhu Huaren Technology, Cat. No. #HR-00701002), Cladribine (CSNpharm, Cat. No. #CSN10004), Gemcitabine Hydrochloride (Shaoyuan, Cat. No. #SY014538), Gemcitabine Prodrug LY2334737 (home-made).

Cell survival was calculated for each concentration of compound using the following formula:

$$\text{Cell survival rate (\%)} = (\text{Lum}_{\text{test article}} - \text{Lum}_{\text{culture medium control}})/(\text{Lum}_{\text{cell control}} - \text{Lum}_{\text{culture medium control}}) \times 100\%.$$

Data was analyzed using GraphPad Prism 7.0 software, and non-linear S-curve regression was used to fit the data to generate dose-response curves from which IC50 values were calculated.

The results showed that each CDN compound of the present invention showed an inhibitory effect on CT26 cell proliferation, with IC50 values between 0.1 and 100 μM preferably in the range of <10 μM and more preferably in the range of <1 μM. IC50 values for representative compounds are shown in Table 2.

Table 2 - Inhibitory effect of representative CDN fragments on proliferation of CT26 cells (IC50, μM)

| Sample | CT-26 activity | Sample | CT-26 activity | Sample | CT-26 activity |
|---|---|---|---|---|---|
| ADU-S100 | NA | Cladribine | A | CDN-5 | B |
| Gemcitabine | A | CDN-3 | A | CDN-6 | B |
| Clofarabine | A | CDN-4 | B | CDN-8 | B |

wherein: A: IC50<1 μM; B: IC50 1~10 μM; NA: IC50>10 μM

Experimental results showed that representative CDN fragments were able to inhibit the growth of CT26 tumor cells in vitro; among them, **CDN-1, 2, 3, 4, 5, 6** and **8** showed particularly significant inhibitory ability, which was superior to tumor cell inhibitory ability of ADU-S 100. Combined with the results of compound activity from Example 1, this result suggests that the CDN fragment has multifunctional antitumor attributes, i.e., antitumor immunity of STING agonists and cytotoxic effects of antimetabolite anticancer agents.

According to the similar experimental methods mentioned above, the inhibition activity of CDN-3 fragment on cell proliferation in several other tumor cell lines (purchased from Kyinno Biotechnology (Beijing) Co., Ltd.) was also investigated, and the results are shown in Table 3.

| **Serial Number** | **Cell Strain** | **Tumor type** | **$IC_{50}$ (μM)** | |
|---|---|---|---|---|
| | | | **CDN-3** | **ADU-S100** |
| 1 | **A20** | Mouse B lymphoma cells | 0.032 | 5.299 |
| 2 | **A375** | Human melanoma cells | 0.206 | >10 |
| 3 | **B16F10** | Mouse melanoma cells | 8.698 | >10 |
| 4 | **Daudi** | Human Burkitt's lymphoma cells | 0.423 | >10 |
| 5 | **FaDu** | Human pharyngeal squamous carcinoma cell | 0.499 | >10 |
| 6 | **H22** | Mouse hepatoma cells | 0.982 | >10 |

(continued)

| Serial Number | Cell Strain | Tumor type | $IC_{50}$ (μM) | |
|---|---|---|---|---|
| | | | CDN-3 | ADU-S100 |
| 7 | **MC38** | Mouse colon cancer cells | 1.024 | >10 |
| 8 | **SK-MEL5** | Human melanoma cells | 0.505 | >10 |
| 9 | **4T1** | Mouse breast cancer cells | 2.271 | >10 |

**Example 3: Antitumor activity of the CDN fragment of the present invention in a CT26 syngeneic mouse bilateral transplanted tumor model**

BALB/c mice aged 6-8 weeks (purchased from Shanghai Lingchang Biotechnology Co., Ltd.) were subcutaneously inoculated with $5\times10^5$ CT26 cells (provided by Taicang Zexin Biotechnology Co., Ltd., ATCC#CRL-2638) on the left and right posterior backs at a volume of 0.1 mL/side. When tumors grew to an average volume of 100 $mm^3$, treatments were randomized and started based on tumor size and mouse body weight. The first day of dosing was Day 0. Two doses were administered, 50 μg compound/mouse (i.e., 2.5 mg/kg) was injected intratumorally into the right flank on days 0 and 4, and the same volume of PBS (Hyclone Company, Cat. No. #SH30258.01) was used as a control. The left tumor was left untreated. Control mice were sacrificed after 13 days of dosing and treated mice were sacrificed 21 days later. During the experiment, the tumor volume and body weight were measured three times a week on the left and right sides, and the tumor volume was calculated as $V = D \times d \times d/2$ (wherein D represents the long diameter of the tumor and d represents the short diameter of the tumor).

The data at 13 days post treatment showed that the tested example compounds of the present invention were capable of significantly inhibiting bilateral tumor growth. The effect was more significant in the right tumor group, with most tumors disappeared (FIG. 1-A). Among them, the tumor growth inhibition rate of ADU-S100 treatment group was 99.1%, CDN-6 was 99.5%, and the tumors of other groups were 100% inhibited without touching the tumor. Meanwhile, untreated left tumors showed decreased growth (Fig. 1-B). On day 13 after treatment, the growth inhibition rates of each group of left tumors were ADU-S100, 64.2%; CDN-3, 91.2%; CDN-4, 75.3%; CDN-5, 71.7%; CDN-6, 67.4%. In addition, a decrease in body weight was observed in individual mice in this experiment, which was within 15% after treatment, and body weight was recovered after stopping the dosing (FIG. 1-C). The above results show that the CDN fragment of the present invention exhibited comparable or superior tumor inhibition ability to ADU-S100 in the CT26 syngeneic bilateral tumor model.

**Example 4: Inhibition activity of ADC molecule of the present invention on proliferation of human breast adenocarcinoma SK-BR-3 cells**

SK-BR-3 cells (Cell Bank of Chinese Academy of Sciences, Cat. No. #TCHu225) were cultured at 37°C, 5%CO2 and 95% humidity in McCoy's 5a + 10% FBS (ExCell Bio, Cat. No. #FND500). Cells in the logarithmic growth phase were harvested and counted, and cell viability was measured using Countstar automated cell counter based on the classical trypan blue staining principle to ensure that the cell viability was above 90%. The cell concentration was adjusted, 135 μL of cell suspension was added into 96 well clear flat bottom black wall plate (Corning, Cat. No. #3904), 3000/well, respectively, and the cells were incubated in 96 well plate at 37°C and 5% $CO_2$.

IC50 determination of drug preparation: A 10 fold drug solution was prepared with complete medium, and 15 μL drug solution was added to each well of 96 well plate inoculated with cells to make the working concentration up to 600 nM, 3× dilution, 9 concentration levels, and duplicate wells were set for each drug concentration. The cells in 96 well plates that had been dosed were incubated at 37°C, 5% $CO_2$ for 5 days before performing CTG assay. The plate was equilibrated to room temperature for 30 min and CTG reagent (CellTiter-Glo® Luminescent Cell Viability Assay (Promega, Cat. No. #G7573) was melt, 50 μL of CTG solution was added to each well, and shaked on an orbital shaker for 2 min to lyse the cells. The plate was left at room temperature for 10 min to stabilize the cold light signal and the cold light value was read (EnVision® Multifunctional Plate Reader, PerkinElmer#2105).

$$\text{Cell survival rate (\%)} = (\text{Lum}_{\text{test drug}} - \text{Lum}_{\text{culture medium control}})/(\text{Lum}_{\text{cell control}} - \text{Lum}_{\text{culture medium control}}) \times 100\%.$$

GraphPad Prism software was used to analyze the data, and Dose-response-inhibition equation was used to fit the data to obtain the dose-response curve from which IC50 value was calculated. IC50 values for representative ADC molecules are shown in Table 4.

Table 4 Inhibition of SK-BR-3 Cell Proliferation by Representative ADC Molecules (IC50, nM)

| Serial Number | Sample | $IC_{50}$ (nM) |
|---|---|---|
| 1 | ADC-2 | 66.9 |
| 2 | ADC-12 | 255.8 |
| 3 | ADC-14 | >600 |
| 4 | Trastuzumab | >600 |

The results showed that ADCs with **CDN-3** as Payload, i.e., **ADC-2**, **ADC-12**, showed a strong inhibitory effect on SK-BR-3 cell proliferation, while the control of ADC with **ADU-S100** as Payload, i.e., **ADC-14** weakly inhibited the proliferation of SK-BR-3 cells.

**Example 5: Inhibition activity of ADC molecule of the present invention on proliferation of human gastric cancer NCI-N87 cells**

NCI-N87 cells (Cell Bank of Chinese Academy of Sciences, Cat. No. #TCHu130) were cultured at 37°C, 5% $CO_2$, 95% humidity in RPMI 1640 + 10% FBS (ExCell Bio, Cat. No. #FND500), the cells in logarithmic growth phase were harvested and counted, and cell viability was measured using Countstar automated cell counter based on classical trypan blue staining principle to ensure that the cell viability was above 90%. The cell concentration was adjusted, 135 μL of cell suspension was added into 96 well clear flat bottom black wall plate (Corning, Cat. No. #3904), 2000/well, and the cells was incubated in 96 well plate at 37°C and 5% $CO_2$.

IC50 determination of drug preparation: A 10 fold drug solution was prepared with complete medium, and 15 μL drug solution was added to each well of 96 well plate inoculated with cells to make the working concentration up to 600 nM, 3× dilution, 9 concentration levels, and duplicate wells were set for each drug concentration. The cells in the 96 well plates that had been dosed were incubated at 37°C, 5% $CO_2$ for 5 days before performing CTG assay. the plate was equilibrated to room temperature for 30 min and CTG Reagent (CellTiter-Glo® Luminescent Cell Viability Assay (Promega, Cat. No. #G7573) was melt, 50 μL of CTG Solution was added to each well, and shaked on an orbital shaker for 2 min to lyse the cells. The plate was left at room temperature for 10 min to stabilize the cold light signal and the cold light value was read (EnVision® Multifunctional Plate Reader, PerkinElmer#2105).

Cell survival rate (%) = (Lum test drug -Lum $_{\text{culture medium control}}$)/(Lum $_{\text{cell control}}$ - Lum $_{\text{culture medium control}}$) × 100%.

GraphPad Prism software was used to analyze the data, and Dose-response-inhibition equation was used to fit the data to obtain the dose-response curve from which IC50 value was calculated. IC50 values for representative ADC molecules are shown in Table 5.

Table 5 Inhibition of NCI-N87 Cell Proliferation by Representative ADC Molecules (IC50, nM)

| Serial Number | Sample | $IC_{50}$ (nM) |
|---|---|---|
| 1 | ADC-15 | 70 |
| 2 | ADC-16 | 143 |
| 3 | ADC-17 | 368 |
| 4 | ADC-18 | 245 |
| 5 | CDN-3 | 209 |
| 6 | Zolbetuximab | >600 |
| 7 | Sacituzumab | >600 |

The results showed that Payload CDN-3 and four ADC molecules, i.e., **ADC-15**, **ADC-16**, **ADC-17**, **ADC-18** in the present invention, showed strong inhibitory effects on NCI-N87 cell proliferation, while two naked antibodies, i.e., Zolbetuximab and Sacituzumab used as controls, showed little inhibitory effects on the proliferation of the cells.

**Example 6: Inhibition activity of ADC molecule of the present invention on proliferation of lymphoma Raji-hClaudin 18.2 cells**

Raji-hClaudin 18.2 cells (provided by Elpiscience) were cultured at 37°C, 5% $CO_2$ and 95% humidity in RPMI 1640 + 10% FBS (ExCell Bio, Cat. No. #FND500) + 10 ug/mL Blasticidin (Beyotime, Cat. No. #ST-018), the cells in logarithmic growth phase were harvested and counted, and cell viability was measured using Countstar automated cell counter based on classical trypan blue staining principle to ensure that the cell viability was above 90%. The cell concentration was adjusted, 135 μL of cell suspension was added into 96 well clear flat bottom black wall plate (Corning, Cat. No. #3904), 10000/well, and the cells were incubated in 96 well plate at 37°C and 5% $CO_2$.

IC50 determination of drug preparation: A 10 fold drug solution was prepared with complete medium, and 15 μL drug solution was added to each well of 96 well plate inoculated with cells to make the working concentration up to 600 nM, 3 × dilution, 9 concentration levels, and duplicate wells were set for each drug concentration. The cells in the 96 well plates that had been dosed were incubated at 37°C, 5% $CO_2$ for 5 days before performing CTG assay. The plate was equilibrated to room temperature for 30 min and CTG Reagent (CellTiter-Glo® Luminescent Cell Viability Assay (Promega, Cat. No. #G7573) was melt, 50 μL of CTG Solution was added to each well, and shaked on an orbital shaker for 2 min to lyse the cells. The plate was left at room temperature for 10 min to stabilize the cold light signal and the cold light value was read (EnVision ® Multifunctional Plate Reader, PerkinElmer#2105).

$$\text{Cell survival rate (\%)} = (\text{Lum}_{\text{test drug}} - \text{Lum}_{\text{culture medium control}})/(\text{Lum}_{\text{cell control}} - \text{Lum}_{\text{culture medium control}}) \times 100\%.$$

GraphPad Prism software was used to analyze the data, and Dose-response-inhibition equation was used to fit the data to obtain the dose-response curve from which IC50 value was calculated. IC50 values for representative ADC molecules are presented in Table 6.

Table 6 Inhibition of Raji-hClaudin 18.2 Cell Proliferation by Representative ADC Molecules (IC50, nM)

| **Serial Number** | **Sample** | **$IC_{50}$ (nM)** |
|---|---|---|
| 1 | ADC-17 | 71.3 |
| 2 | ADC-18 | 36.6 |
| 3 | CDN-3 | 131 |
| 4 | Zolbetuximab | >600 |

The results showed that Payload CDN-3 and two ADC molecules, i.e., **ADC-17, ADC-18** in the present invention showed strong inhibition effects of Raji-hClaudin 18.2 cell proliferation, while the control naked antibody, ie., Zolbetuximab showed little inhibition effects on the proliferation of Raji-hClaudin 18.2 cells.

**Example 7: Antitumor activity of ADC molecules (ADC-1** and **ADC-2) of the present invention on SK-OV-3 tumor-bearing mice**

This experiment evaluated the efficacy of antibody conjugates **ADC-1** and **ADC-2** administered intravenously against the subcutaneous xenograft human ovarian cancer SK-OV-3 model in BALB/c Nude mice.

0.1 mL ($1\times10^7$) of human ovarian cancer SK-OV-3 cells (purchased from ATCC, Cat#HTB-77) (PBS: matrigel volume ratio of 1:1) was subcutaneously inoculated into the right posterior back of each mouse (BALB/c Nude mice, 6-8 weeks old, female, Jiangsu GemPharmatech Co., Ltd.). When the mean tumor volume reached approximately 150 $mm^3$, the mice were randomly divided into 9 experimental groups, with 5 mice in each group. Groups 1 to 8 were respectively the vehicle control (PBS buffer), **ADC-2** 3 mg/kg, **ADC-2** 0.6 mg/kg, **ADC-1** 3 mg/kg, **ADC-1** 0.6 mg/kg, anti-HER2 antibody 3 mg/kg, anti-HER2 antibody 0.1 mg/kg, and **CDN-3** 0.1 mg/kg, which were administered intravenously once on the day of group assignment. Group 9 was received intratumoral injection of **CDN-3** 0.1 mg/kg on Days 0, 4, and 7 of the study for a total of 3 drug doses (see Table 4 for animal grouping). The vehicle was a PBS buffer. Tumor volume and body weight were measured twice weekly during the experimental period, and data was recorded. Tumor volume was calculated according to the formula in Example 3. The antitumor efficacy TGI (%) of the test compound was calculated according to the following formula:

$$\mathrm{TGI}(\%) = \frac{1 - \begin{pmatrix}\text{Mean tumor volume at end of administration in the treatment group} - \\ \text{Mean tumor volume at start of administration in the treatment group}\end{pmatrix}}{\begin{pmatrix}\text{Mean tumor volume at end of administration in the vehicle control group} - \\ \text{Mean tumor volume at the start of administration in the vehicle control group}\end{pmatrix}} * 100\%$$

The experimental results are presented in Table 7 and FIG. 2 and 3.

Antibody-conjugated drugs **ADC-1** and **ADC-2** presented target-related, excellent antitumor activity in the HER2-positive human ovarian cancer SK-OV-3 xenograft model. At 3 mg/kg dose, tumor growth inhibition (TGI) was 123% and 102%, and complete tumor regression was 100% and 80% when measured 21 days after a single dose of **ADC-1** and **ADC-2**, respectively. At 0.6 mg/kg, TGI was 84% and 91%, respectively, and complete tumor regression was achieved in 20% of **ADC-1**-treated mice. **ADC-1** and **ADC-2** showed dose-related efficacy, and both were significantly better than their small molecule conjugate **CDN-3** administered intravenously or intratumorally alone (the latter having TGI of 10% and 39%, respectively). Naked antibody (HER2 ab.) showed little antitumor activity at 3 mg/kg and 0.6 mg/kg, with TGI of 22% and 26%, respectively. Mice treated with a single dose of **ADC-1** or **ADC-2** exhibited a brief, transient body weight loss, with maximal body weight loss observed on Day 3 post dose, with a decrease of 19.5% in the **ADC-2** 3 mg/kg group, 8.5% in the **ADC-2** 0.6 mg/kg group, 14.5% in the **ADC-1** 3 mg/kg group, and 11.8% in the **ADC-1** 0.6 mg/kg group. Mice gradually regained body weight after day 3 and overall animals showed good tolerability.

Table 7 Efficacy analysis of human ovarian cancer cell line SK-OV-3 tumor model

| Group | Treatment | Tumor Volume ($mm^3$, Day 21) | Tumor growth inhibition (TGI) | Rate of complete tumor regression (TV = 0 $mm^3$) | *P* value (relative to Group 1) |
|---|---|---|---|---|---|
| Group 1 | Vehicle Control (PBS, i.v., day0) | 790 ± 78 | / | / | / |
| Group 2 | ADC-2 (3 mg/kg, i.v., day0) | 0 ± 0 | 123% | 100% (5/5) | 0.0000 |
| Group 3 | ADC-2 (0.6 mg/kg, i.v., day0) | 253 ± 80 | 84% | 0% (0/5) | 0.0013 |
| Group 4 | ADC-1 (3 mg/kg, i.v., day0) | 136 ± 136 | 102% | 80% (4/5) | 0.0031 |
| Group 5 | ADC-1 (0.6 mg/kg, i.v., day0) | 206 ± 64 | 91% | 20% (1/5) | 0.0004 |
| Group 6 | HER2 ab. (3 mg/kg, i.v., day0) | 649 ± 73 | 22% | 0% (0/5) | 0.2221 |
| Group 7 | HER2 ab. (0.6 mg/kg, i.v., day0) | 623 ± 76 | 26% | 0% (0/5) | 0.1624 |
| Group 8 | CDN-3 (0.1 mg/kg, i.v., day0) | 723 ± 140 | 10% | 0% (0/5) | 0.6853 |
| Group 9 | CDN-3 (0.1 mg/kg, i.t., day 0, 4, 7) | 540 ± 47 | 39% | 0% (0/5) | 0.0253 |

**Example 8: Anti-tumor activity of ADC molecules (ADC-2, ADC-3, ADC-4, ADC-6, ADC-10, ADC-11, ADC-12, ADC-13) of the present invention on SK-OV-3 tumor-bearing mice**

This experiment evaluated the efficacy of antibody conjugates **ADC-2**, **ADC-3**, **ADC-4**, **ADC-6**, **ADC-10**, **ADC-11**, **ADC-12**, **ADC-13** in the subcutaneous human ovarian cancer xenograft model SK-OV-3 following intravenous administration in the BALB/c Nude mice.

0.1 mL ($1\times10^7$) human ovarian carcinoma SK-OV-3 cells (purchased from ATCC, Cat. #HTB-77) (PBS: matrigel volume ratio 1:1) were subcutaneously inoculated into each mouse (BALB/c Nude mice, 6-8 weeks old, female, Jiangsu GemPharmatech Co., Ltd.) in the right posterior back. When the mean tumor volume reached approximately 150 $mm^3$, the mice were randomly divided into 9 experimental groups, with 4 mice in each group. Groups 1 to 9 were respectively the vehicle control (PBS buffer), ADC-2 0.6 mg/kg, ADC-10 0.6 mg/kg, ADC-3 0.6 mg/kg, ADC-4 0.6 mg/kg, ADC-11 0.6 mg/kg, ADC-12 0.6 mg/kg, ADC-13 0.6 mg/kg, ADC-6 0.6 mg/kg, which were administered intravenously one time on the day of group assignment (set as day 0). The vehicle was a PBS buffer. Tumor volumes and body weights were measured twice weekly during the experimental period, and data was recorded. Tumor volume was calculated according to the formula in Example 3. The anti-tumor efficacy TGI (%) of the test compound was calculated according to the formula in Example 5.

The experimental results are shown in Table 8 and FIG. 4 and 5. The assay results evaluated the impact of **CDN-3** different conjugation sites and Linker on efficacy of the antibody conjugates on HER2 -positive human ovarian cancer SK-OV-3 xenograft model, as well as different Payloads (**CDN-2**, **CDN-3** and **CDN-8**) on ADC efficacy. The results showed

that, from the investigation of the efficacy of linker, the TGI of compounds (**ADC-2**, **ADC-3**, **ADC-4**, **ADC-11**) linked to the same Payload (**CDN-3**) indicated that the linkers of **ADC-2**, **ADC-3** and **ADC-4** were superior to **ADC-11**, and the linker of **ADC-3** had the best drug efficacy. From the investigation of different conjugation sites, it emerged that **ADC-2** (conjugated S site), TGI = 87% and **ADC-13** (conjugated N site), TGI = 87% had significantly better efficacy than **ADC-12** (coupled O site) TGI = 13%. By investigating the efficacy from different Payload, it emerged that **ADC-10** (Payload: **CDN-8**) and **ADC-2** (Payload: **CDN-3**) had better efficacy than **ADC-6** (Payload: **CDN-2**). In terms of body weight, except for **ADC-2**, which showed a decrease in body weight after one dose (7.7%) and gradually recovered after the third day, the body weight of mice in the other groups gradually recovered after slight changes (less than 5%) in the first three days, and the overall animals showed good tolerability.

Table 8

| Group | Treatment | Tumor Volume ($mm^3$, Day 21) | Tumor growth inhibition (TGI) |
|---|---|---|---|
| Group 1 | Vehicle control group (PBS, i.v., day0) | 944 | / |
| Group 2 | ADC-2 (0.6 mg/kg, i.v., day0) | 259 | 87% |
| Group 3 | ADC-10 (0.6 mg/kg, i.v., day0) | 182 | 97% |
| Group 4 | ADC-3 (0.6 mg/kg, i.v., day0) | 120 | 104% |
| Group 5 | ADC-4 (0.6 mg/kg, i.v., day0) | 186 | 96% |
| Group 6 | ADC-11 (0.6 mg/kg, i.v., day0) | 698 | 31% |
| Group 7 | ADC-12 (0.6 mg/kg, i.v., day 0) | 845 | 13% |
| Group 8 | ADC-13 (0.6 mg/kg, i.v., day 0) | 260 | 87% |
| Group 9 | ADC-6 (0.6 mg/kg, i.v., day 0) | 425 | 66% |

The specific embodiments provided herein are only used for exemplifying the present invention and do not constitute a limitation to the scope defined by the claims. Based on the content disclosed in this application, technicians in the field can obviously understand the equivalent variants of the technical solutions of this application, and these variants are also covered within the scope of this application.

## Claims

**1.** An antibody-drug conjugate of Formula (X) or a pharmaceutically acceptable salt or solvate thereof:

$$Ab\text{-}[L\text{-}D]_q \qquad (X)$$

wherein,

Ab represents an antibody or antigen-binding fragment that binds to a target antigen;
L represents a linker unit linking D to Ab;
D represents a compound of Formula (II):

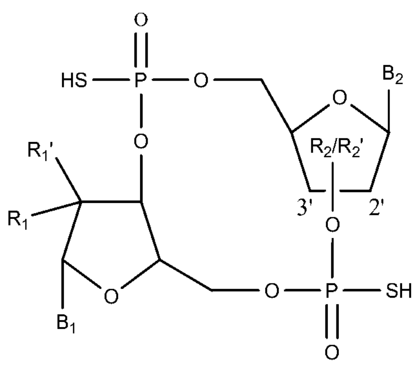

(II)

wherein
$B_1$ is adenine

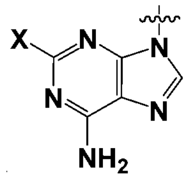

optionally substituted with X, wherein X is selected from an H, Cl, F or $-NHC_{1-6}$ alkyl; or guanine

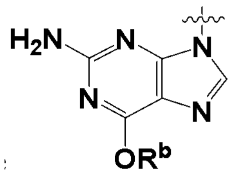

optionally substituted with $R^b$, wherein $R^b$ is selected from H or $-C_{1-6}$ alkyl,
$R_1$ and $R_1$' are each independently selected from H, F or -OH,
$B_2$ is guanine

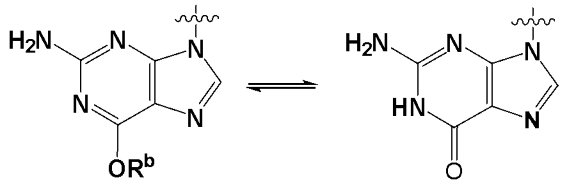

,

wherein $R^b$ is selected from H or $-C_{1-6}$ alkyl,

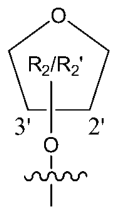

means that the phosphate bond can be attached to either the 2' or 3' position of the pentose, where the site not looped to the phosphate is substituted with $R_2$ and $R_2$', and
$R_2$ and $R_2$' are each independently selected from H, -OH or F,
or stereoisomers, tautomers, stable isotopic variants, pharmaceutically acceptable salts, prodrugs or solvates thereof;
q represents the number of D linked to Ab via the linker unit L;
wherein the compound of Formula (II) is covalently bonded to the linker unit L via either of its -SH.

**2.** The antibody-drug conjugate of claim 1, wherein $B_1$ in Formula (II) is adenine

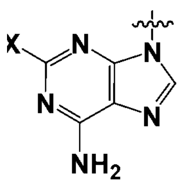

, wherein X is selected from H, Cl, F or $-NHC_{1-6}$ alkyl, or guanine

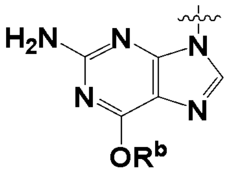,

wherein $R^b$ is-$C_{1-6}$ alkyl, e.g.

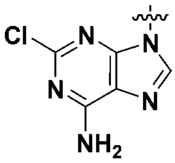, 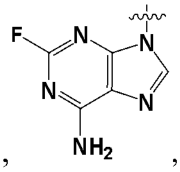, 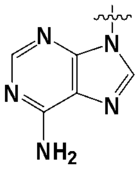, 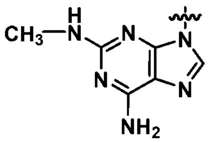 and 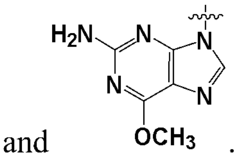.

3. The antibody-drug conjugate of claim 1 or 2, wherein $R_1$ and $R_1$' in Formula (II) are both H, or one is H and the other is F, or one is H and the other is OH.

4. The antibody-drug conjugate of any one of claims 1 to 3, wherein $B_2$ in Formula (II) is guanine

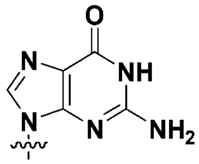.

5. The antibody-drug conjugate of any one of claims 1 to 4, wherein in Formula (II), one of $R_2$ and $R_2$' is H and the other is OH.

6. The antibody-drug conjugate of any one of claims 1 to 5, wherein Formula (II) has the following formula:

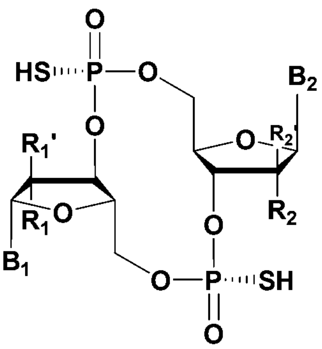 (II-a') 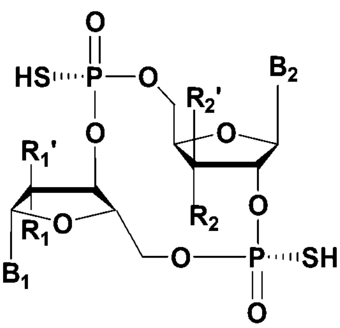 (II-b').

7. The antibody-drug conjugate of claim 6, wherein $R_1$ and $R_1$' are both H, or wherein $R_1$' is H and $R_1$ is F, or wherein $R_1$' is H and $R_1$ is OH.

8. The antibody-drug conjugate of claim 6 or 7, wherein $B_1$ is

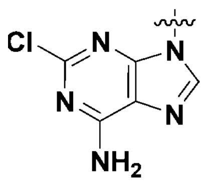

and $R_1$ is F and $R_1$' is H; or $B_1$ is

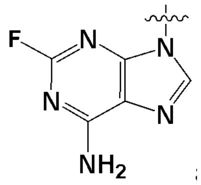

;

and $R_1$ is OH and $R_1$' is H; or $B_1$ is

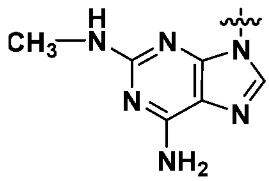

and $R_1$ is F and $R_1$' is H.

**9.** The antibody-drug conjugate of any one of claims 6 to 8, wherein $B_2$ is guanine

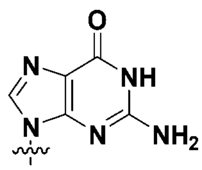

.

**10.** The antibody-drug conjugate of any one of claims 6 to 9, wherein $R_2$' is H and $R_2$ is -OH.

**11.** The antibody-drug conjugate of claim 1, wherein Formula II is selected from the following compounds, stereoisomers, tautomers, stable isotopic variants, pharmaceutically acceptable salts or solvates thereof,

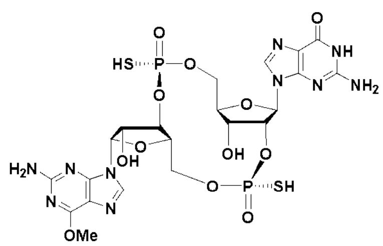

CDN-1

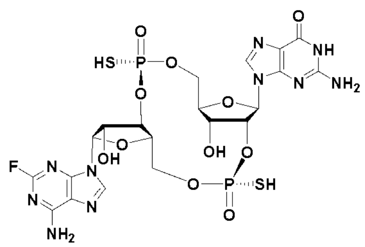

CDN-2

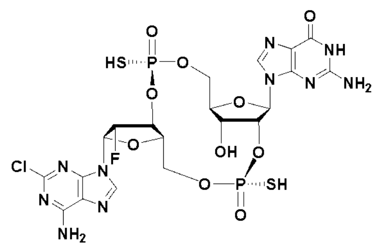

CDN-3

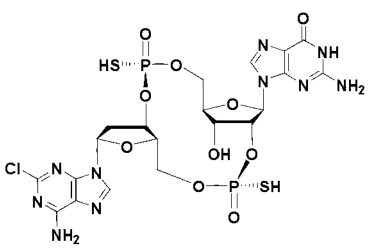

CDN-4

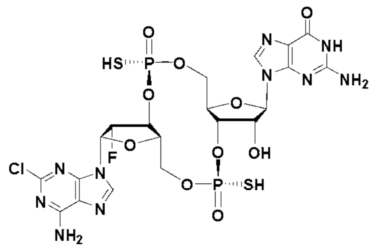

CDN-5

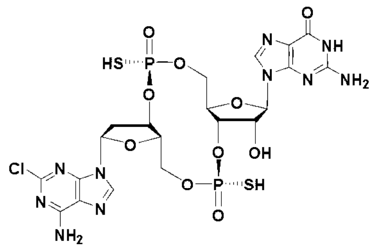

CDN-6

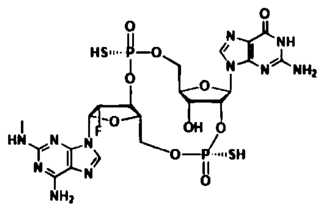

CDN-8

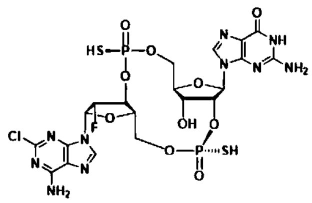

CDN-7

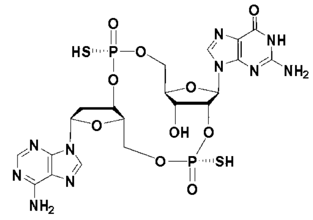

CDN-9

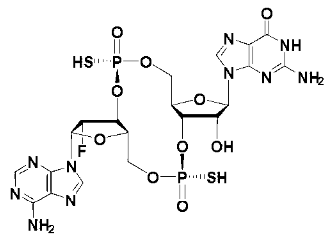

CDN-10

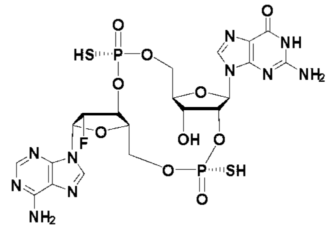

CDN-11

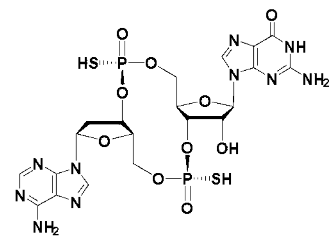

CDN-12

.

**12.** The antibody-drug conjugate of any one of claims 1 to 11, wherein the linker unit L has the structure of Formula (III) as follows:

-Z-A-P-Y-M- (III),

wherein

M is a self-immolative linker for connection to drug D;
Y is a cleavable linker, selected from peptides of 2-8 amino acids, preferably dipeptide, tripeptide, or tetrapeptide; disulfide; ester; amide; and sugar;
P is a property regulating unit, which is absent or selected from polyethylene glycol (PEG), hydrophilic peptide, cyclodextrin unit, polyamine, polyamide, polysaccharide, dendrimer, and bifunctional hydrocarbon chain;
A is a connector unit;
Z is an antibody linker linked to Ab.

**13.** The antibody-drug conjugate of claim 12, wherein in the linker unit L,

M is selected from

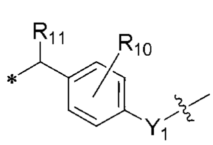 or 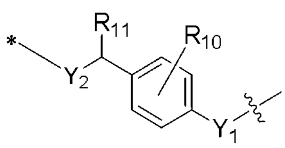 ,

wherein * represents the connection point with CDN and

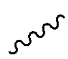

represents the connection point with the cleavable linker Y, wherein $Y_1$ is selected from -O- or -NH-, $Y_2$ is selected from bond, -C(O)-O- or -C(O)-$NR_{11}$-, $R_{10}$ is selected from H, $-NO_2$, $-NH_2$ or $-CF_3$, $R_{11}$ is selected from H or $C_{1-6}$ alkyl;
Y is selected from enzymatically cleavable peptide of 2-8 amino acids;
P is absent or is one or more linear, branched, or star shaped PEG chains consisting of at least two ethylene oxide ($CH_2CH_2O$) subunits;
A is selected from a directl bond, $-C_{1-4}$ alkylene-, -NH-, or -NH-$C_{1-4}$ alkylene-heteroaryl-;
Z is selected from

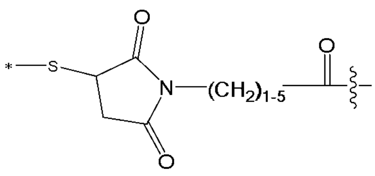

,

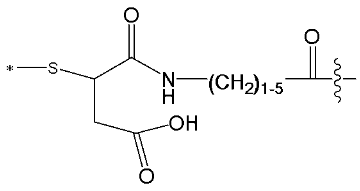

and

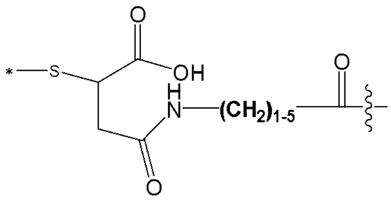

.

**14.** The antibody-drug conjugate of claim 12 or 13, wherein in the linker unit L, M is

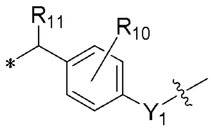

, e.g.

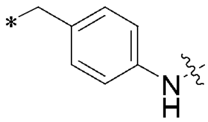

,

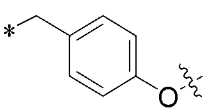

,

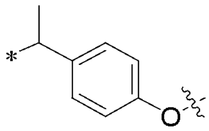

,

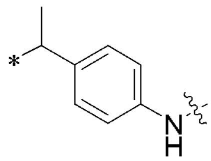

,

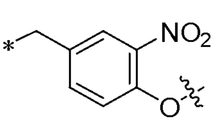

,

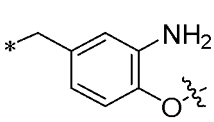

,

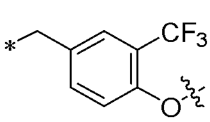

,

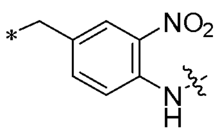

,

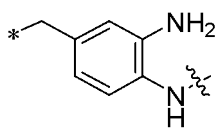

,

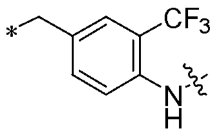

,

preferably

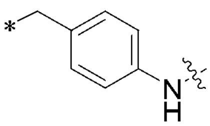

.

**15.** The antibody-drug conjugate of claims 12 to 14, wherein in the linker unit L, Y is specifically recognized and cleaved by cathepsin B, e.g. Val-Cit, Val- Ala, Gly-Gly-Phen-Gly, Gly-Phen-Leu-Gly; or specifically recognized and cleaved by asparagine endonuclease, e.g. Ala-Ala-Asn; or specifically recognized and cleaved by matrix metalloproteinases 2/9, e.g. Pro-Leu-Gly-Leu-Ala-Gly, Gly-Pro-Leu-Gly-Ile-Ala-Gly-Gln, Gly-Pro-Val-Gly-Leu-Ile-Gly-Lys.

**16.** The antibody-drug conjugate of claims 12 to 14, wherein in the linker unit L, Y has the following formula:

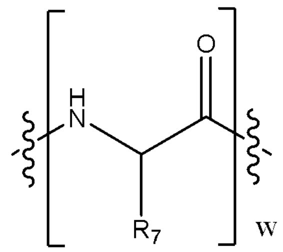

(Y1)

wherein the N-terminus is linked to the Z-portion of the linker unit and the C-terminus is linked to the M-portion of the linker unit or directly to drug D; w is an integer from 2 to 8, e.g., an integer from 2 to 4; each $R_7$ is independently hydrogen, methyl, isopropyl, isobutyl, sec-butyl, benzyl, - $CH_2CONH_2$, or $-(CH_2)_3NHCONH_2$;
preferably Y is selected from Val-Cit, Val-Ala, Ala-Ala-Asn, Gly-Gly-Phe-Gly.

**17.** The antibody-drug conjugate of claims 12 to 14, wherein the linker unit L is the following linker unit:

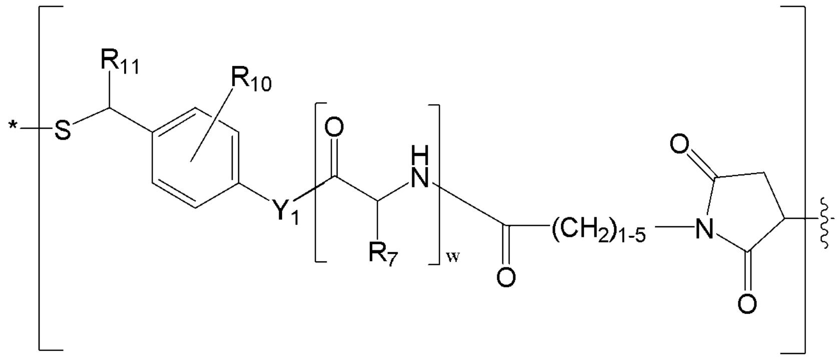

,

wherein the S atom is from - SH in the CDN, the wavy line represents the connection point with the antibody, and $R_7$, $R_{10}$, $R_{11}$, $Y_1$, w are defined accordingly in claims 13-16.

**18.** The antibody-drug conjugate of claims 1 to 17, wherein Ab is an antibody or antigen-binding fragment thereof that binds a tumor-specific antigen or a tumor-associated antigen, wherein the tumor specific antigen or tumor associated antigen is selected from HER2, Her3, HER1 (ErbB1), HER4 (ErbB4), Trop-2, Nectin4, Tissue Factor, PD-L1, PD-1, MET, Claudin 18.2, KIT, CTLA-4, RPR1, Adrenaline A2 Receptor (EphA2), Folate Receptor (FRa), Mesothelin, Endothelin Receptor, GCPII, IL-13Ra, BCMA, GD2, CLL-1, CA-IX, MUC1, 5T4, AOC3, ALK, AXL, C242, CA-125, CCL11, CCR5, CD138, CD2, CD3, CD4, CDS, CD15, CA15-3, CD18, CD19, CD20, CD21, CD22, CD30, CD33, CD37, CD38, CD52, CD56, CD66e, CD70, CD72, CD74, CD79a, CD79b, CD123, CD174, CD276, CCD79b, CA19-9, AGS-16, IGF1R, IGF2R, VEGFR1, VEGFR2, VEGFR3, PDGFR-$\alpha$, PDGFR-$\beta$, EGFR, EGFRvIII, SLTRK6, BMPR1B, E16, TOP1, STEAP1, 0772P, MUC16, Napi3b, Sema 5b, PSCA hlg, ETBR, RNF124, prostate cancer-related gene 1, TrpM4, teratoma-derived growth factor 1, C3DR, FcRH2, NCA, MDP, IL20R-$\alpha$, Brevican, EphB2R, ASLG659, prostate stem cell antigen precursor, GEDA, BAFF-R, CXCR5, HLA-DOB, P2X5, LY64, FcRH1, IRTA2, TENB2, integrin $\alpha5\beta6$, integrin $\alpha4\beta7$, FGF2, FGFR1, FGFR2, FGFR3, FGFR4, PSMA, Somatostatin receptor, RANK, SLAMF7, ITGB6, CEACAM5, MUC1, CA9, EGFRvIII, IL2RA, AXL receptor tyrosine kinase, TGF-$\beta$R, TNFRSF8, cancer/testis associated antigen, CLEC14A, GRP78, stem cell specific antigen, ASG-5, PRR4, GUCY2C, SLC39A6, TPBG, middle cancer associated antigen CA242, FOLR1, GPNMB, HAVCR1, prostate tumor target Mindin, VTCN1, PTK7 protein tyrosine kinase 7, macrophage stimulating 1 receptor, TACSTD2, CA6, DLL3, DLL4, EpCAM, FAP, DKK-1, Endoglin, VCAM1, GPC3, DR5, ASCT2, B7H4; preferably selected from HER2, KIT, FOLR1, CD276, PD-L1, NECTIN4, Mesothelin, MUC1, GCPII, BCMA, cMet, RPR1, CD22, CD19, TOP1, Claudin 18.2, EGFR, Trop-2; more preferably antibodies specifically binding HER2, Claudin 18.2, and/or Trop-2 or antigen-binding fragments thereof.

**19.** The antibody-drug conjugate of claims 1 to 18, wherein Ab contains 3 heavy chain complementarity determining regions (HCDRs) and 3 light chain complementarity determining regions (LCDRs), wherein: as defined by Kabat,

HCDR1 contains or consists of the amino acid sequence of SEQ ID NO: 6,
HCDR2 contains or consists of the amino acid sequence of SEQ ID NO: 7,

HCDR3 contains or consists of the amino acid sequence of SEQ ID NO: 8,
LCDR1 contains or consists of the amino acid sequence of SEQ ID NO: 3,
LCDR2 contains or consists of the amino acid sequence of SEQ ID NO: 4, and
LCDR3 contains or consists of the amino acid sequence of SEQ ID NO: 5;
or
HCDR1 contains or consists of the amino acid sequence of SEQ ID NO: 18,
HCDR2 contains or consists of the amino acid sequence of SEQ ID NO: 19,
HCDR3 contains or consists of the amino acid sequence of SEQ ID NO: 20,
LCDR1 contains or consists of the amino acid sequence of SEQ ID NO: 15,
LCDR2 contains or consists of the amino acid sequence of SEQ ID NO: 16, and
LCDR3 contains or consists of the amino acid sequence of SEQ ID NO: 17;
or
HCDR1 contains or consists of the amino acid sequence of SEQ ID NO: 29,
HCDR2 contains or consists of the amino acid sequence of SEQ ID NO: 30,
HCDR3 contains or consists of the amino acid sequence of SEQ ID NO: 31,
LCDR1 contains or consists of the amino acid sequence of SEQ ID NO: 26,
LCDR2 contains or consists of the amino acid sequence of SEQ ID NO: 27, and
LCDR3 contains or consists of the amino acid sequence of SEQ ID NO: 28;
or
HCDR1 contains or consists of the amino acid sequence of SEQ ID NO: 40,
HCDR2 contains or consists of the amino acid sequence of SEQ ID NO: 41,
HCDR3 contains or consists of the amino acid sequence of SEQ ID NO: 42,
LCDR1 contains or consists of the amino acid sequence of SEQ ID NO: 37,
LCDR2 contains or consists of the amino acid sequence of SEQ ID NO: 38, and
LCDR3 contains or consists of the amino acid sequence of SEQ ID NO: 39.

**20.** The antibody-drug conjugate of claims 1 to 18 , wherein Ab contains a heavy chain variable region and a light chain variable region, wherein the heavy chain variable region contains the amino acid sequence shown in SEQ ID NO: 10 and wherein the light chain variable region contains the amino acid sequence shown in SEQ ID NO: 9; or

wherein the heavy chain variable region contains the amino acid sequence shown in SEQ ID NO: 22 and the light chain variable region contains the amino acid sequence shown in SEQ ID NO: 21; or
wherein the heavy chain variable region contains the amino acid sequence shown in SEQ ID NO: 33 and the light chain variable region contains the amino acid sequence shown in SEQ ID NO: 32; or
wherein the heavy chain variable region contains the amino acid sequence shown in SEQ ID NO: 44 and the light chain variable region contains the amino acid sequence shown in SEQ ID NO: 43.

**21.** The antibody-drug conjugate of claims 1 to 18, wherein the Ab contains:

(a) a heavy chain containing the amino acid sequence of SEQ ID NO: 2, and
(b) a light chain containing the amino acid sequence of SEQ ID NO: 1;
or
(a) a heavy chain containing the amino acid sequence of SEQ ID NO: 14, and
(b) a light chain containing the amino acid sequence of SEQ ID NO: 13;
or
(a) a heavy chain containing the amino acid sequence of SEQ ID NO: 25, and
(b) a light chain containing the amino acid sequence of SEQ ID NO: 24;
or
(a) a heavy chain containing the amino acid sequence of SEQ ID NO: 36, and
(b) a light chain containing the amino acid sequence of SEQ ID NO: 35.

**22.** The antibody-drug conjugate of claims 1 to 18, wherein the Ab is selected from trastuzumab, pertuzumab, margetuximab or HT-19 or antibody fragments thereof, or other anti-human HER2 antibodies that recognize the same epitope or competitively bind human HER2, preferably trastuzumab; or

wherein Ab is selected from sacituzumab, datopotamab, or antibody fragments thereof, or other anti-human TROP2 antibodies that recognize the same epitope or competitively bind to human TROP2, preferably sacituzumab; or

wherein Ab is selected from either zolbetuximab, osemitamab (TST001), CMG901, ASKB589, ZL-1211, or antibody fragments thereof, or other anti-human Claudin18.2 antibodies that recognize the same epitope or competitively bind to human Claudin18.2, preferably zolbetuximab; or
wherein Ab is selected from cetuximab, panitumumab, nimotuzumab, necitumuma, amivantamab, depatuxizumab, NECITUMUMAB, IZALONTAMAB, BAFISONTAMAB, Petosemtamab, PIMURUTAMAB, FUTUXIMAB and MODOTUXIMAB, or antibody fragments thereof, or other anti-human EGFR antibodies that recognize the same epitope or competitively bind to human EGFR, preferably cetuximab.

**23.** The antibody-drug conjugate of claims 1 to 22, wherein q is an integer or non-integer of 1 to 10 or a range consisting of any two values between 1 and 10, e.g. 1, 2, 3, 4, 5, 6, 7, 8, 9, 10, 1 to 2, 1 to 3, 1 to 4, 1 to 5, 1 to 6, 1 to 7, 1 to 8, 1 to 9, 1 to 10, 2 to 10, 2 to 8, 2 to 6, 2 to 4, 4 to 6, 4 to 8, 4-10, 6 to 8 or 6 to 10; preferably q is an average DAR value of 1 to 8 or a range consisting of any two values between 1 and 8, e.g. about1, 2, 3, 4, 5, 6, 7, 8, 1 to 2, 1 to 3, 1 to 4, 1 to 5, 1 to 6, 1 to 7, 1 to 8, 2 to 8, 2 to 6, 2 to 4, 4 to 6, 4 to 8, or 6 to 8; more preferably q represents an average DAR of about 3, an average DAR of about 6, and an average DAR of about 8.

**24.** A CDN-linker unit conjugated unit, which has the following formula:

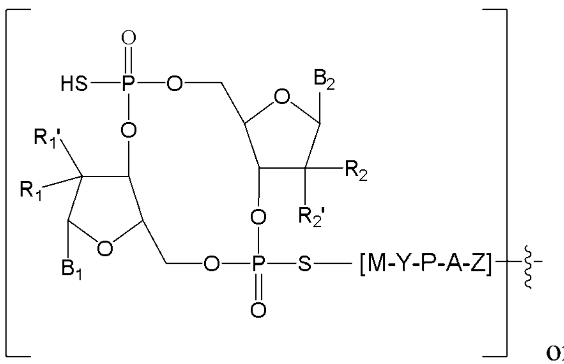
or
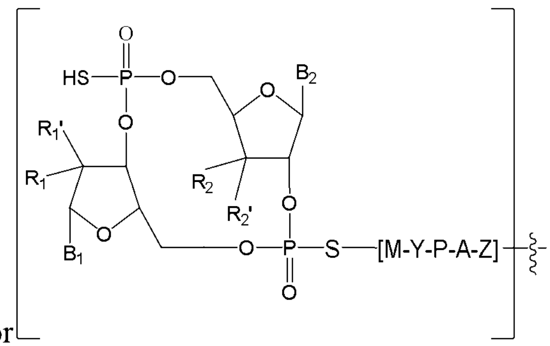

(IV-a) (IV-b)

preferably has the following formula:

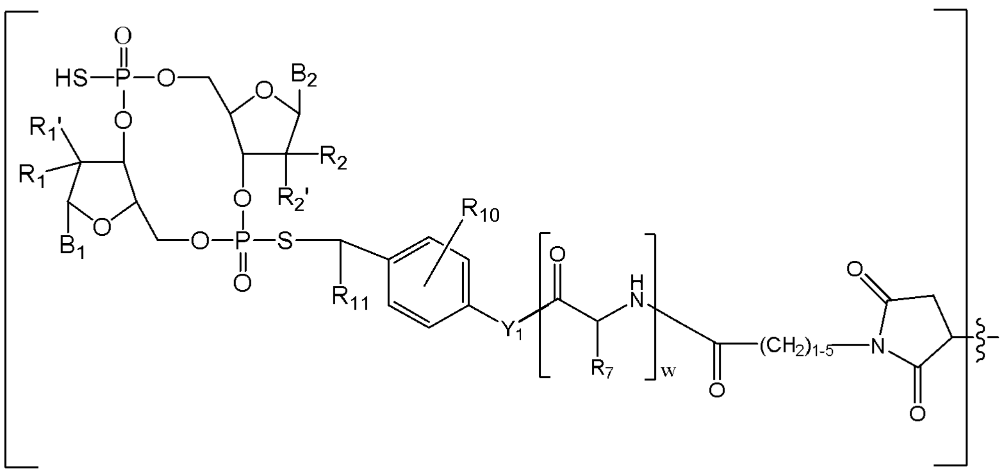
(IV-a-1)

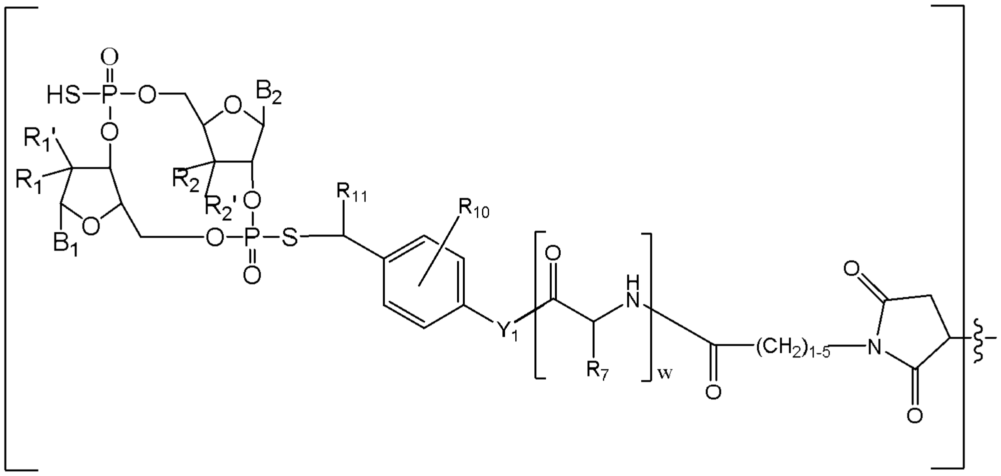

(IV-b-1)

more preferably has the following formula:

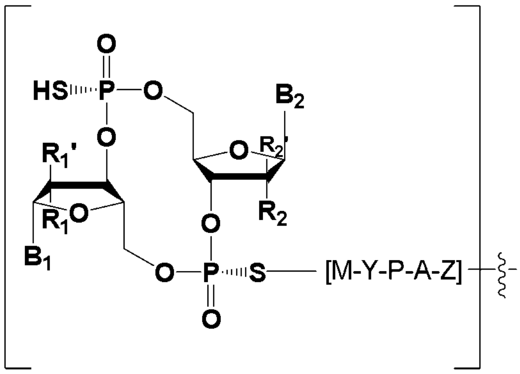

or

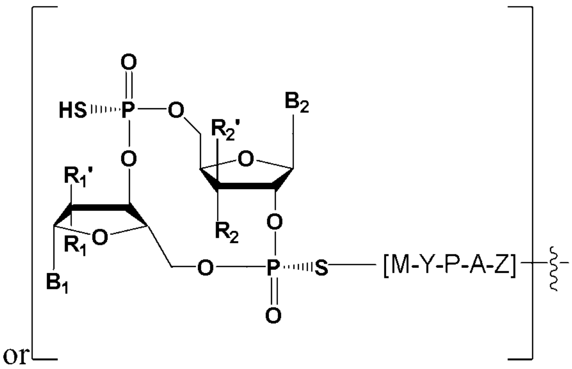

(IV-a ')

(IV-b ')

most preferably has the following formula:

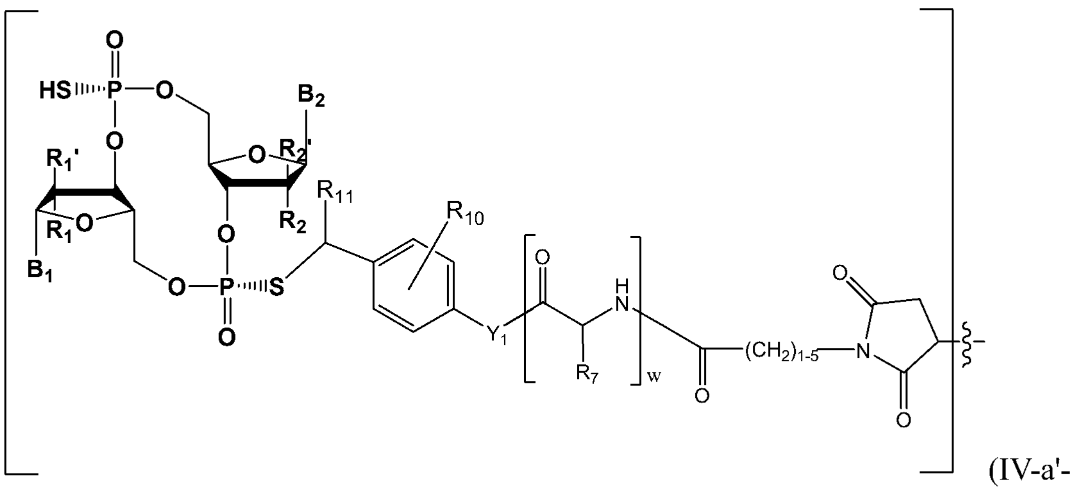

(IV-a'-1)

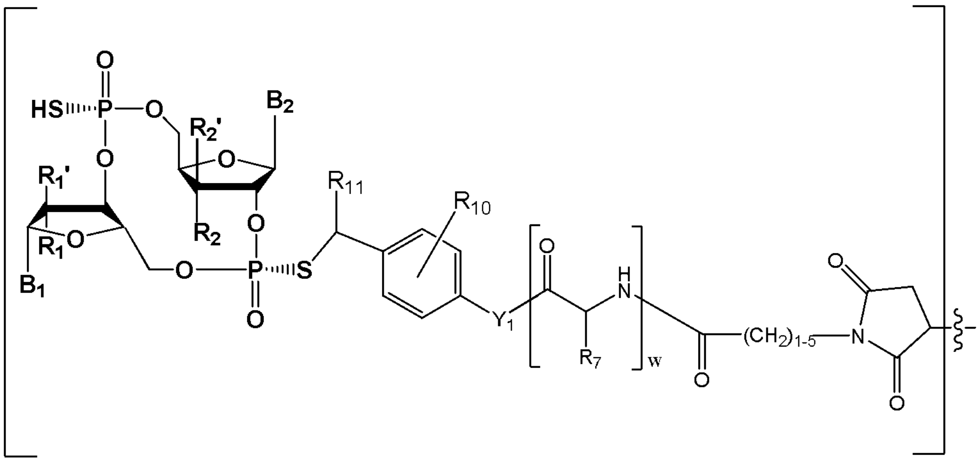

(IV-b'-1)

wherein the wavy line represents the connection point with Ab; $B_1$, $B_2$, $R_1$, $R_1$', $R_2$, $R_2$', M, Y, P, A, Z, $R_7$, $R_{10}$, $R_{11}$, $Y_1$, w are defined accordingly in claims 1 to 17, respectively.

**25.** The antibody-drug conjugate of claim 1, which has the following formula

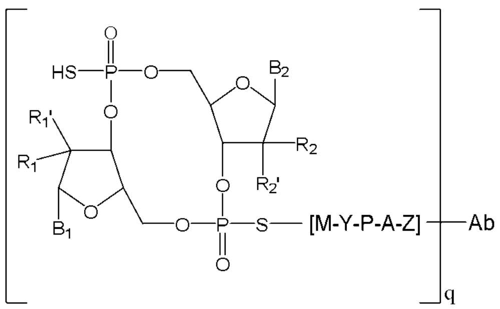

or

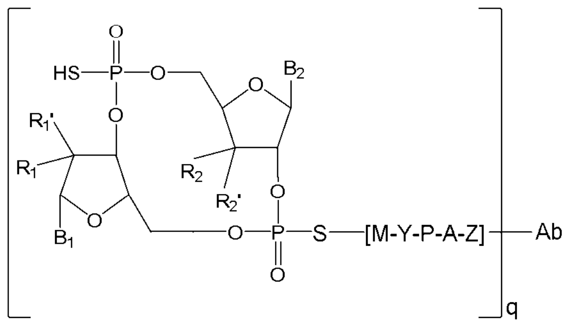

(X1-a) (X1-b)

preferably has the following formula:

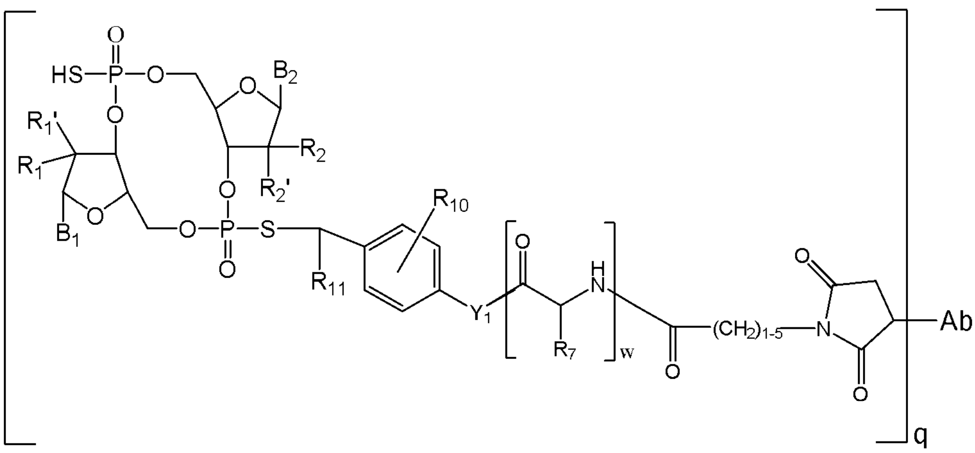

(X1-a-1)

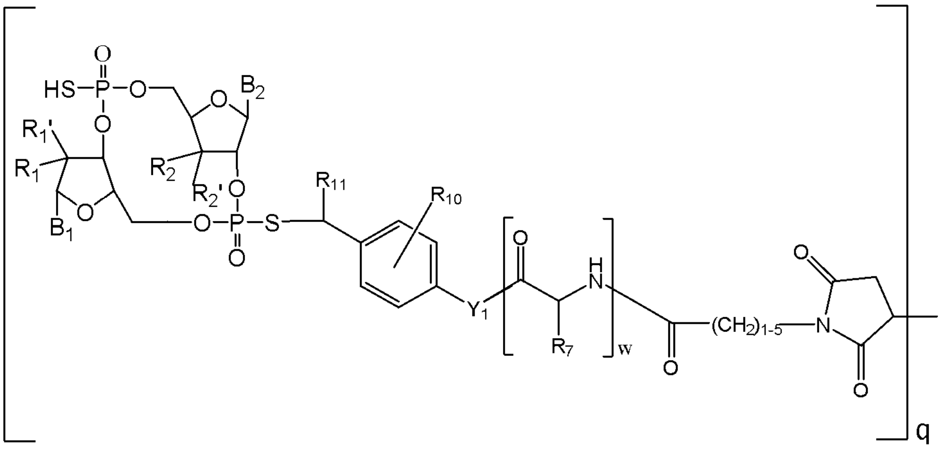

(X1-b-1)

wherein $B_1$, $B_2$, $R_1$, $R_1$', $R_2$, $R_2$', M, Y, P, A, Z, $R_7$, $R_{10}$, $R_{11}$, $Y_1$, w are defined accordingly in claims 1 to 17, Ab is defined as in any one of claims 18-22 and q is defined as in claim 23.

**26.** The antibody-drug conjugate of claim 25, wherein

$B_1$ is adenine

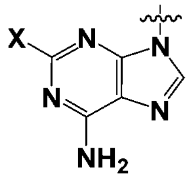

optionally substituted with X, wherein X is selected from H, Cl, F or $-NHC_{1-6}$ alkyl; or guanine

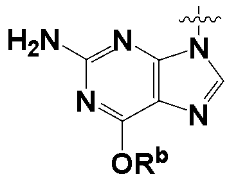

optionally substituted with $R^b$, wherein $R^b$ is selected from $-C_{1-6}$ alkyl; $B_2$ is guanine

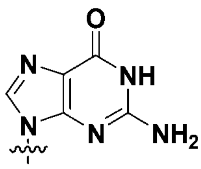

;

$R_1$ and $R_1$' are both H, or one is H and the other is F, or one is H and the other is OH;
$R_2$ and $R_2$' are both H, or one is H and the other is F, or one is H and the other is OH;
$Y_1$ is selected from -O- or -NH-;
$R_{10}$ is selected from H, $-NO_2$, $-NH_2$ or $-CF_3$;
$R_{11}$ is selected from H or $C_{1-6}$ alkyl;
$R_7$ is independently hydrogen, methyl, isopropyl, isobutyl, sec-butyl, benzyl, $-CH_2CONH_2$ or - $(CH_2)_3NHCONH_2$;
w is an integer from 2 to 8;
Ab is an antibody or antigen-binding fragment thereof that specifically binds HER2, Trop-2, or Claudin18.2; and
q is an average DAR value selected from 1 to 8 or a range consisting of any two values between 1 and 8;
preferably, $B_1$ is

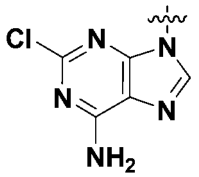,
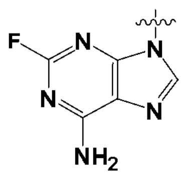,
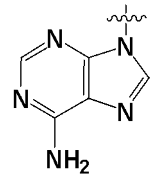,
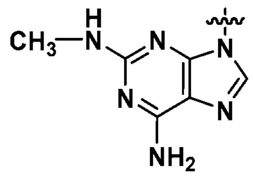

and

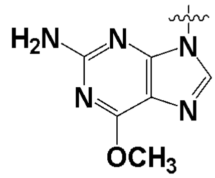,

preferably

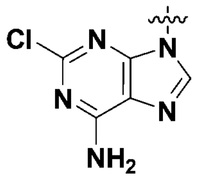,
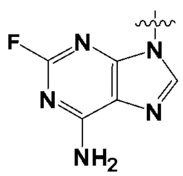,
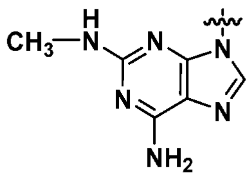;

$B_2$ is guanine

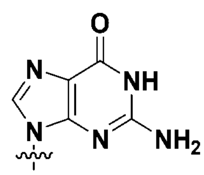;

$R_1$ and $R_1$' are both H, or one is H and the other is F, or one is H and the other is OH; one of $R_2$ and $R_2$' is H and the other is OH; $Y_1$ is -NH; $R_{10}$ and $R_{11}$ are both H; $R_7$ is independently hydrogen, methyl, isopropyl, benzyl, $-CH_2CONH_2$, or $-(CH_2)_3NHCONH_2$; w is an integer of 2 to 4; Ab is trastuzumab, sacituzumab, zolbetuximab, or cetuximab; and q represents an average DAR of about 3, an average DAR of about 6, or an average DAR of about 8.

**27.** The antibody-drug conjugate of claim 1, which has the following formula

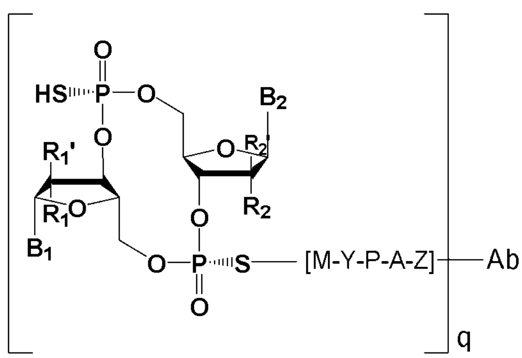
or
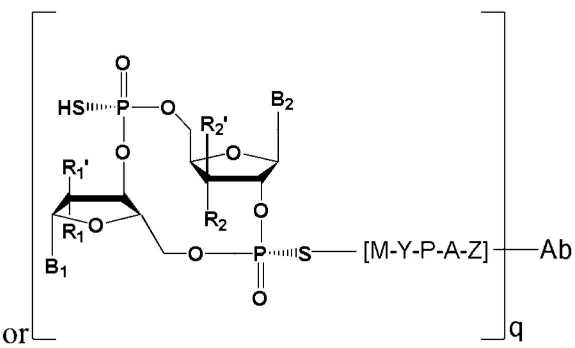

(X1-a') (X1-b')

preferably has the following formula:

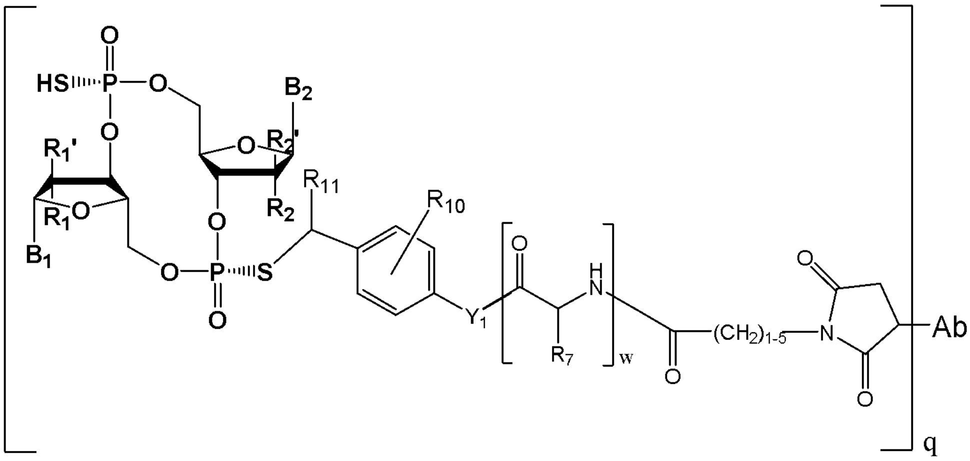

(X1-a'-1)

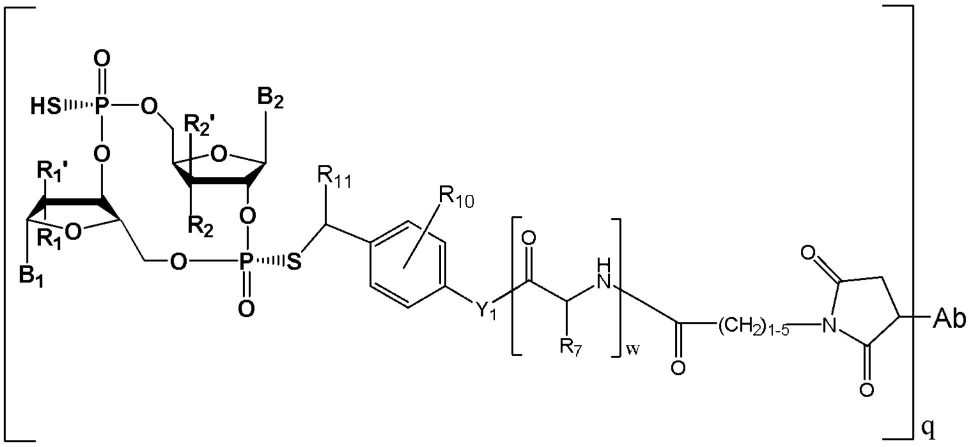

(X1-b'-1)

wherein $B_1$, $B_2$, $R_1$, $R_1$', $R_2$, $R_2$', M, Y, P, A, Z, $R_7$, $R_{10}$, $R_{11}$, $Y_1$, w are defined accordingly in claims 1 to 17, Ab is defined as in claims 18-22 and q is defined as in claim 23.

**28.** The antibody-drug conjugate of claim 27, wherein

$B_1$ is adenine

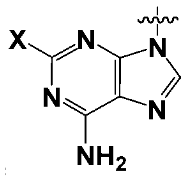

optionally substituted with X, wherein X is selected from H, Cl, F or -$NHC_{1-6}$ alkyl; or guanine

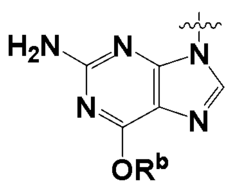

optionally substituted with $R^b$, wherein $R^b$ is selected from -$C_{1-6}$ alkyl; $B_2$ is guanine

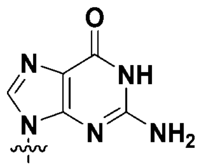

;

$R_1$ and $R_1$' are both H, or wherein $R_1$' is H and $R_1$ is F, or wherein $R_1$' is H and $R_1$ is OH; $R_2$ and $R_2$' are both H, or wherein $R_2$' is H and $R_2$ is F, or wherein $R_2$' is H and $R_2$ is OH;
$Y_1$ is selected from -O- or -NH-;
$R_{10}$ is selected from H, $-NO_2$, $-NH_2$ or $-CF_3$;
$R_{11}$ is selected from H or $C_{1-6}$ alkyl;
$R_7$ is independently hydrogen, methyl, isopropyl, isobutyl, sec-butyl, benzyl, $-CH_2CONH_2$ or $-(CH_2)_3NHCONH_2$;
w is an integer from 2 to 8;
Ab is an antibody or antigen-binding fragment thereof that specifically binds HER2, Trop-2, EGFR, or Claudin18.2; and
q is an average DAR value selected from 1 to 8 or a range consisting of any two values between 1 and 8;
preferably, $B_1$ is

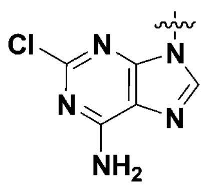 , 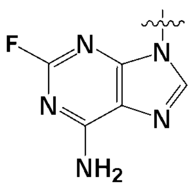 , 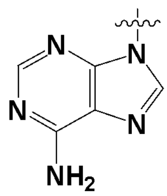 , 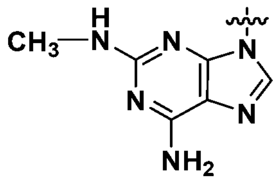

and

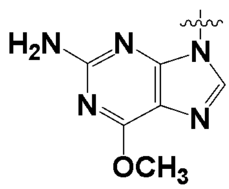 ,

preferably

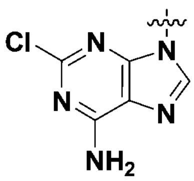 , 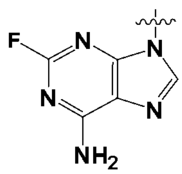 , 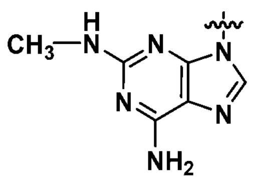 ;

$B_2$ is guanine

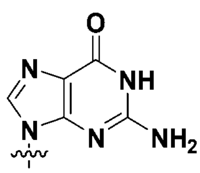

;

$R_1$ and $R_1$' are both H, or wherein $R_1$' is H and $R_1$ is F, or wherein $R_1$' is H and $R_1$ is OH; $R_2$ is OH and $R_2$' is H; $Y_1$ is -NH; $R_{10}$ and $R_{11}$ are both H, $R_7$ is independently hydrogen, methyl, isopropyl, benzyl, $-CH_2CONH_2$ or $-(CH_2)_3NHCONH_2$, and w is an integer from 2 to 4; Ab is trastuzumab, sacituzumab, zolbetuximab, or cetuximab; and q represents an average DAR of about 3, an average DAR of about 6, or an average DAR of about 8.

**29.** a drug-antibody conjugate, which is selected from:

| | |
|---|---|
| ADC-1 | 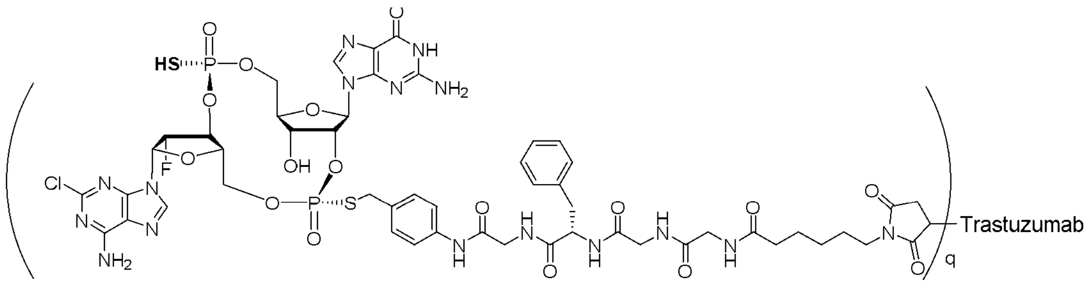 |
| ADC-2 | 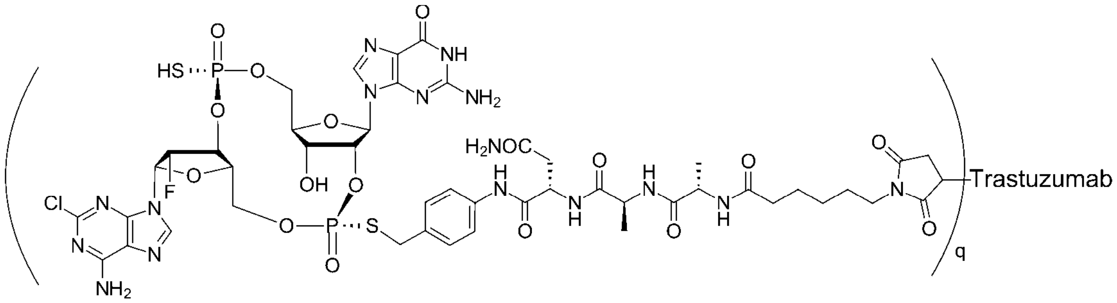 |
| ADC-3 | 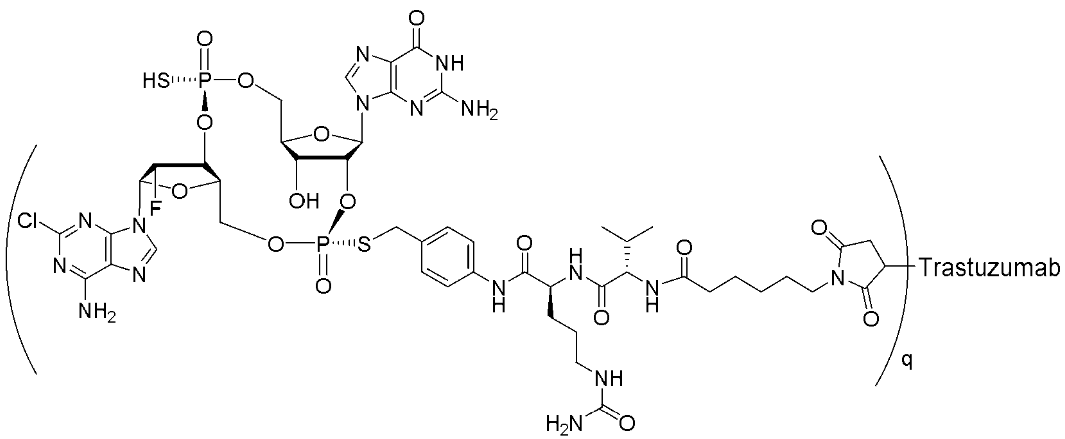 |

(continued)

| | |
|---|---|
| ADC-4 | 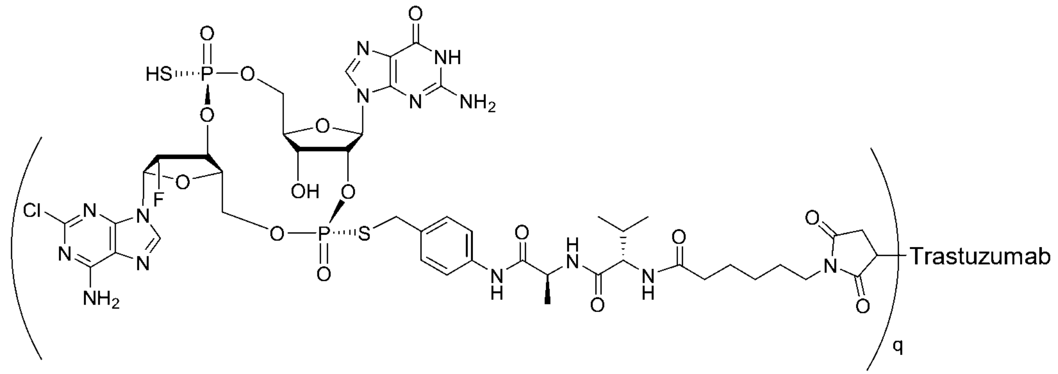 |
| ADC-5 | 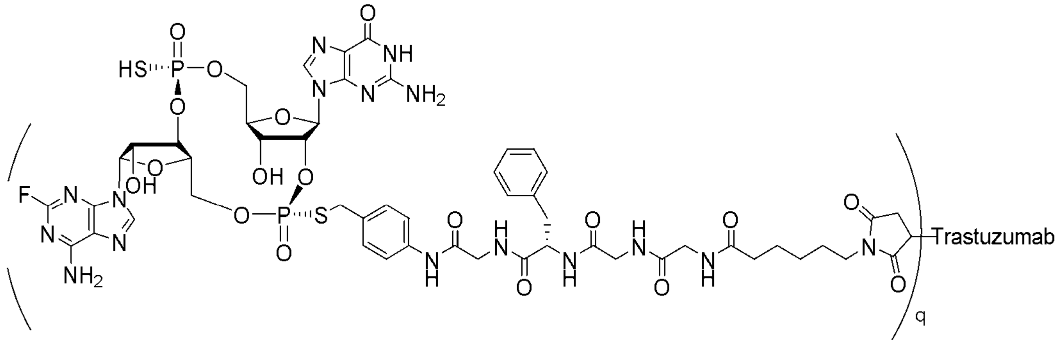 |
| ADC-6 | 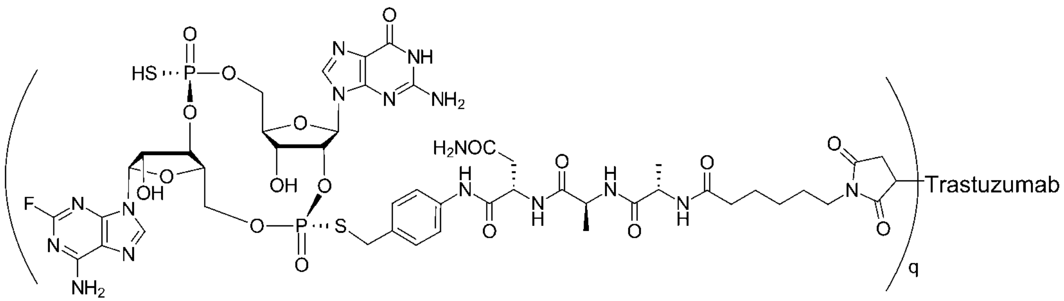 |

(continued)

| | |
|---|---|
| ADC-7 | 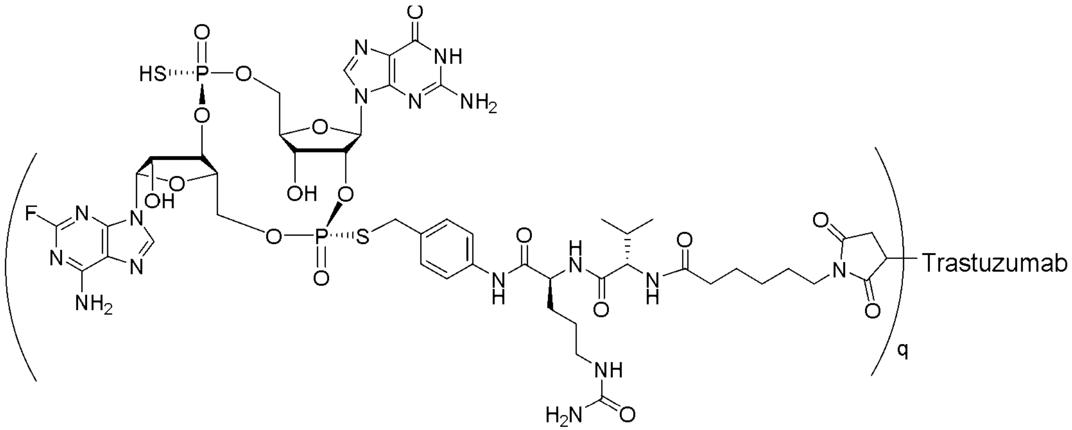 |
| ADC-8 | 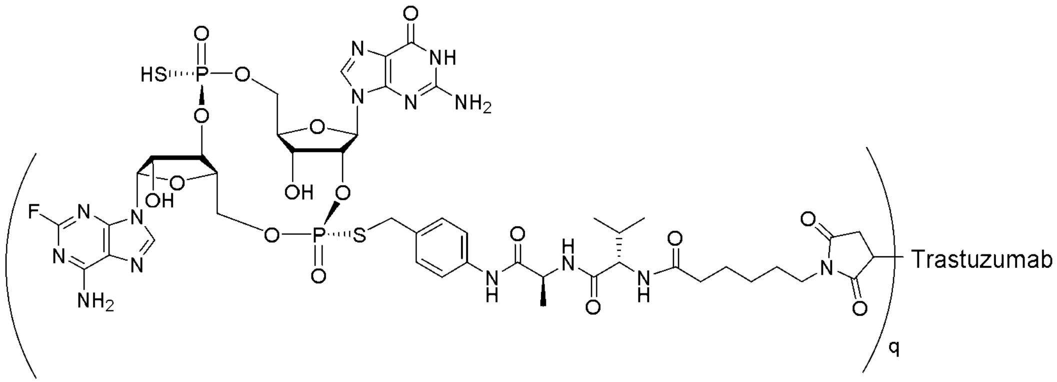 |

(continued)

| | |
|---|---|
| ADC-9 | 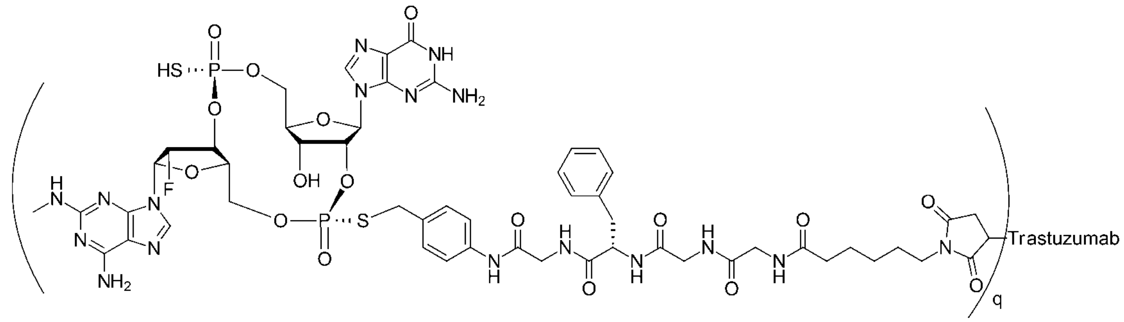 |
| ADC-10 | 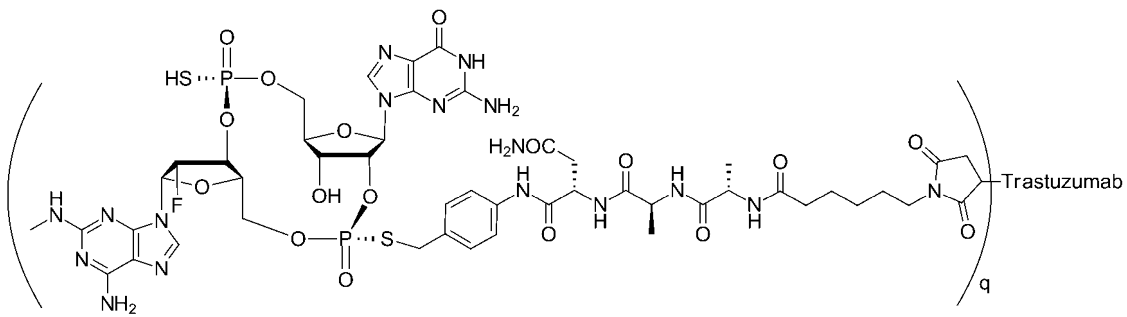 |
| ADC-11 | 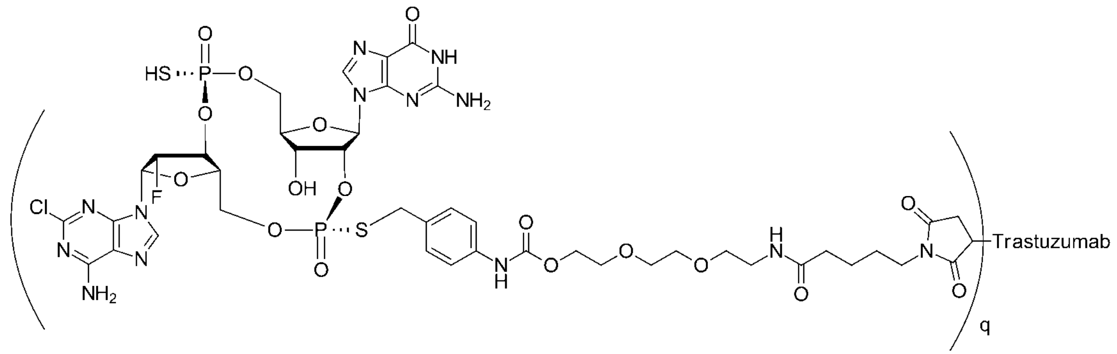 |

(continued)

| | |
| --- | --- |
| ADC-12 | 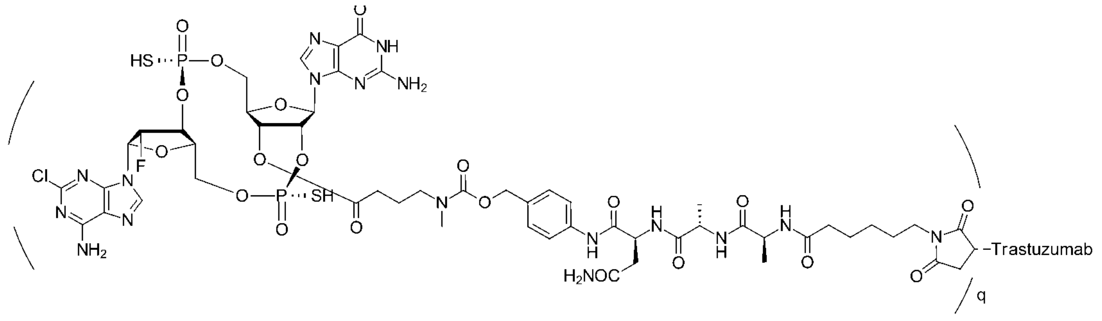 |
| ADC-13 | 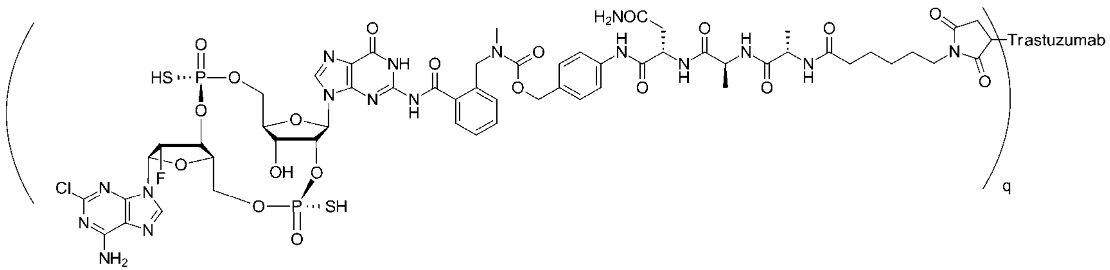 |
| ADC-15 | 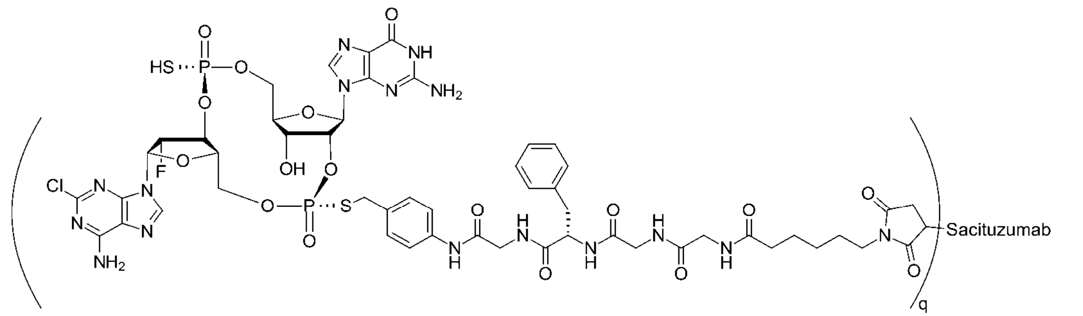 |

(continued)

| | |
|---|---|
| ADC-16 | 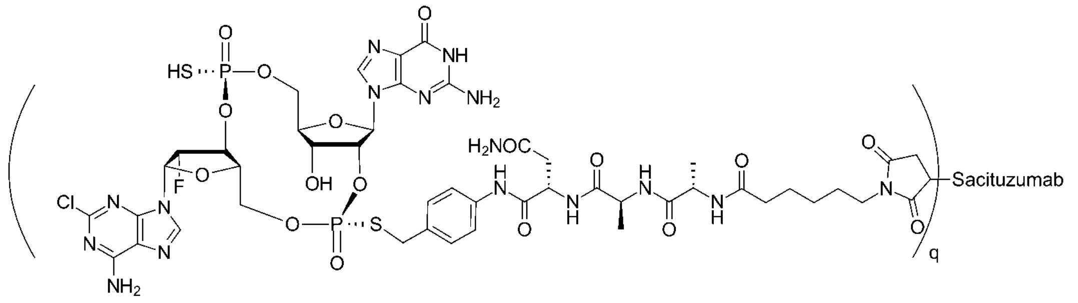 |
| ADC-17 | 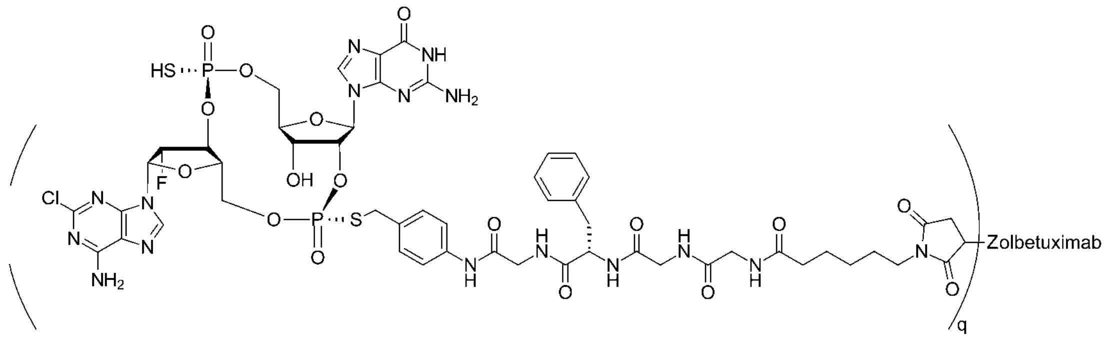 |
| ADC-18 | 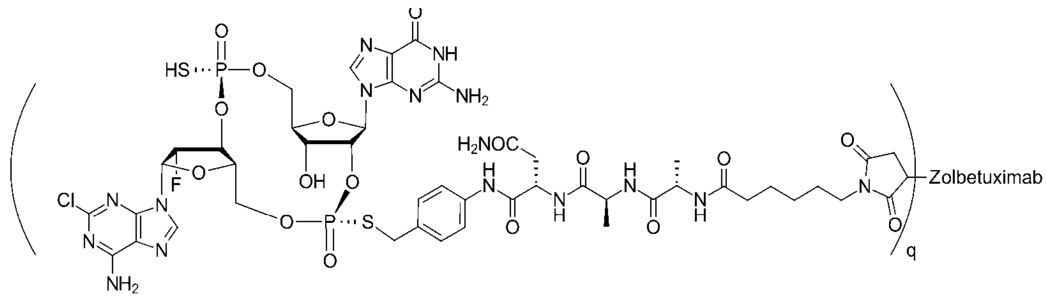 |

(continued)

| | |
|---|---|
| ADC-19 | 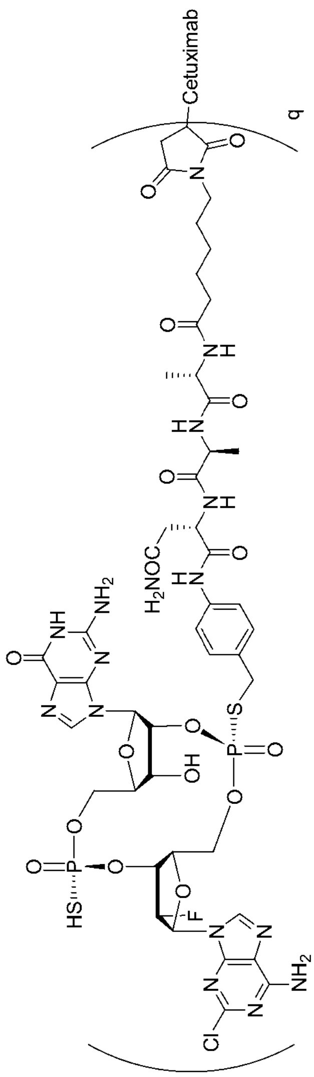 |

stereoisomers, tautomers, stable isotopic variants, pharmaceutically acceptable salts or solvates thereof, wherein q represents an average DAR value of about 1 to 8, e.g. about 2, 3, 4, 5, 6, 7, 8, 9, 10, about 1 to 2, 1 to 3, 1 to 4, 1 to 5, 1 to 6, 1 to 7, 1 to 8, 1 to 9, 1 to 10, 2 to 10, 2 to 8, 2 to 6, 2 to 4, 4 to 6, 4 to 8, 4 to 10, 6 to 8 or 6 to 10; preferably q represents an average DAR of about 3, about 6 or about 8.

**30.** A pharmaceutical composition comprising a drug-antibody conjugate of any one of claims 1-23 and 25-29 and one or more pharmaceutically acceptable excipients.

**31.** A pharmaceutical composition of claim 30, which is administered intravenously, intratumorally, subcutaneously, intramuscularly, orally, intranasally, intrathecally, transdermally or topically, preferably intravenously, intraperitoneally, subcutaneously or intramuscularly.

**32.** A method of inducing, stimulating, or assisting an immune response in an individual, including administering to the individual a drug-antibody conjugate of any one of claims 1-23 and 25-29, or a pharmaceutically acceptable salt or solvate thereof, or a pharmaceutical composition of claims 30 or 31.

**33.** A method for treating or preventing diseases associated with or mediated by immune response, specifically diseases associated with or mediated by STING, including inflammatory, allergic or autoimmune diseases, infectious diseases, or cancer, including administering a drug-antibody conjugate of any one of claims 1-23 and 25-29 or a pharmaceutically acceptable salt or solvate thereof or a pharmaceutical composition of claim 30 or 31 to a subject in need.

**34.** Use of a drug-antibody conjugate of any one of claims 1-23 and 25-29 or a pharmaceutically acceptable salt or solvate thereof or a pharmaceutical composition of claim 29 or 30 in the prevention or treatment of diseases associated with or mediated by STING, and more specifically in the treatment or prevention of inflammatory, allergic or autoimmune diseases, infectious diseases or hyperproliferative diseases, especially tumors or viral infections.

**35.** The use of a drug-antibody conjugate of any one of claims 1-23 and 25-28 or a pharmaceutically acceptable salt or solvate thereof or a pharmaceutical composition of claim 29 or 30 in the preparation of a medicament for the prevention or treatment of diseases associated with or mediated by STING, more specifically inflammatory, allergic or autoimmune diseases, infectious diseases or hyperproliferative diseases, especially tumors or viral infections.

**36.** The use of claims 34 or 35, wherein the hyperproliferative disease refers to a tumor or cancer, which is selected from brain cancer, skin cancer, bladder cancer, ovarian cancer, breast cancer, gastric cancer, pancreatic cancer, prostate cancer, colon cancer, blood cancer, lung and bone cancer, for exampleneuroblastoma, intestinal cancer such as rectal cancer, colon cancer, familial adenomatous polyposis carcinoma and hereditary nonlymphocytic colorectal cancer, esophageal cancer, lip cancer, laryngeal cancer, nasopharyngeal carcinoma, oral cancer, salivary gland cancer, peritoneal cancer, soft tissue sarcoma, urothelial carcinoma, hidradenocarcinoma, gastric cancer, adenocarcinoma, medullary thyroid cancer, papillary thyroid cancer, renal carcinoma, renal parenchymal carcinoma, ovarian cancer, cervical cancer, uterine corpus cancer, endometrial cancer, pancreatic cancer, prostate cancer, testicular cancer, breast cancer (including HER2-negative breast cancer), urinary cancer, melanoma, brain tumors such as glioblastoma, astrocytoma, meningioma, medulloepithelioma and peripheral neuroectodermal tumor, Hodgkin lymphoma, non-Hodgkin lymphoma, Burkitt's lymphoma, acute lymphoblastic leukemia (ALL), chronic lymphocytic leukemia (CLL), chronic myelogenous leukemia (CLL), and lymphocytic carcinoma, acute myelogenous leukemia (AML), myelogenous leukemia (chronic myelogenous leukemia (CML), adult T-cell lymphoma, diffuse lymphoma (DLBCL), hepatoma, multiple myeloma, seminoma, osteosarcoma, chondrosarcoma, anal canal cancer, adrenocortical carcinoma, chordoma, fallopian tube cancer, gastrointestinal stromal tumor, myeloproliferative disorder, mesothelioma, biliary tract cancer, Ewing's sarcoma, and other rare tumor types, as well as recurrent forms of the above tumors; preferably selected from small cell lung cancer, non-small cell lung cancer, colorectal cancer, liver cancer, breast cancer, ovarian cancer, gastric cancer, prostate cancer, melanoma, renal cell carcinoma, head and neck cancer, pancreatic cancer, Hodgkin's lymphoma, leukemia, or bladder cancer.

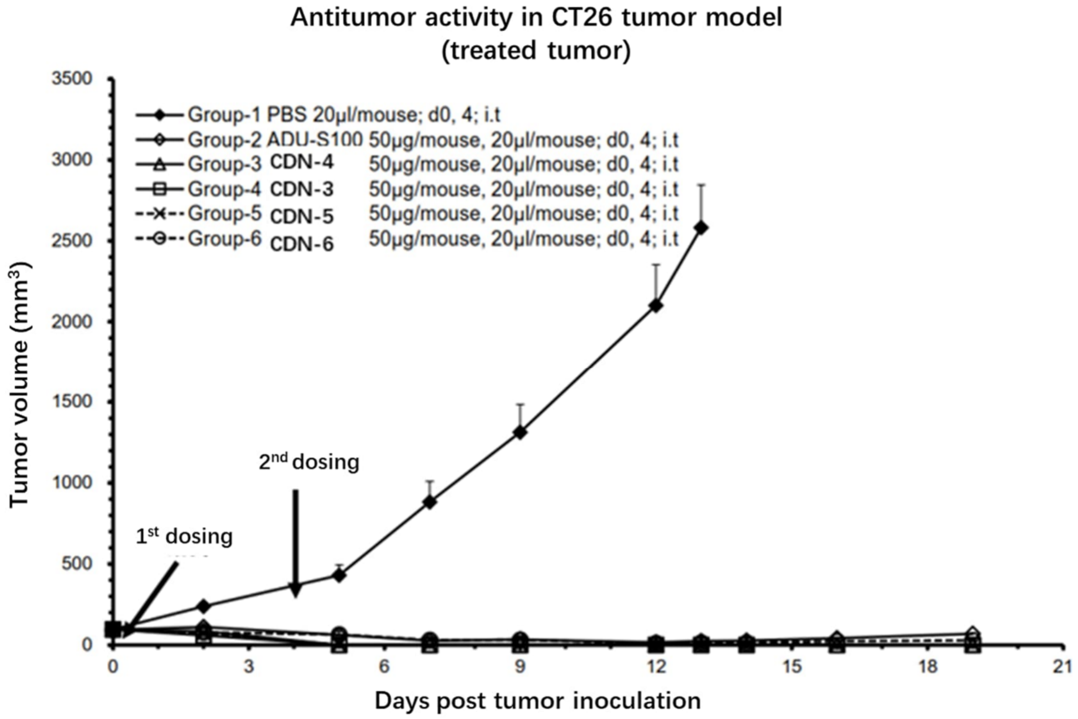
Antitumor activity in CT26 tumor model
(treated tumor)
Group-1 PBS 20µl/mouse; d0, 4; i.t
Group-2 ADU-S100 50µg/mouse, 20µl/mouse; d0, 4; i.t
Group-3 CDN-4 50µg/mouse, 20µl/mouse; d0, 4; i.t
Group-4 CDN-3 50µg/mouse, 20µl/mouse; d0, 4; i.t
Group-5 CDN-5 50µg/mouse, 20µl/mouse; d0, 4; i.t
Group-6 CDN-6 50µg/mouse, 20µl/mouse; d0, 4; i.t
2nd dosing
1st dosing
Tumor volume (mm3)
Days post tumor inoculation

FIG. 1A

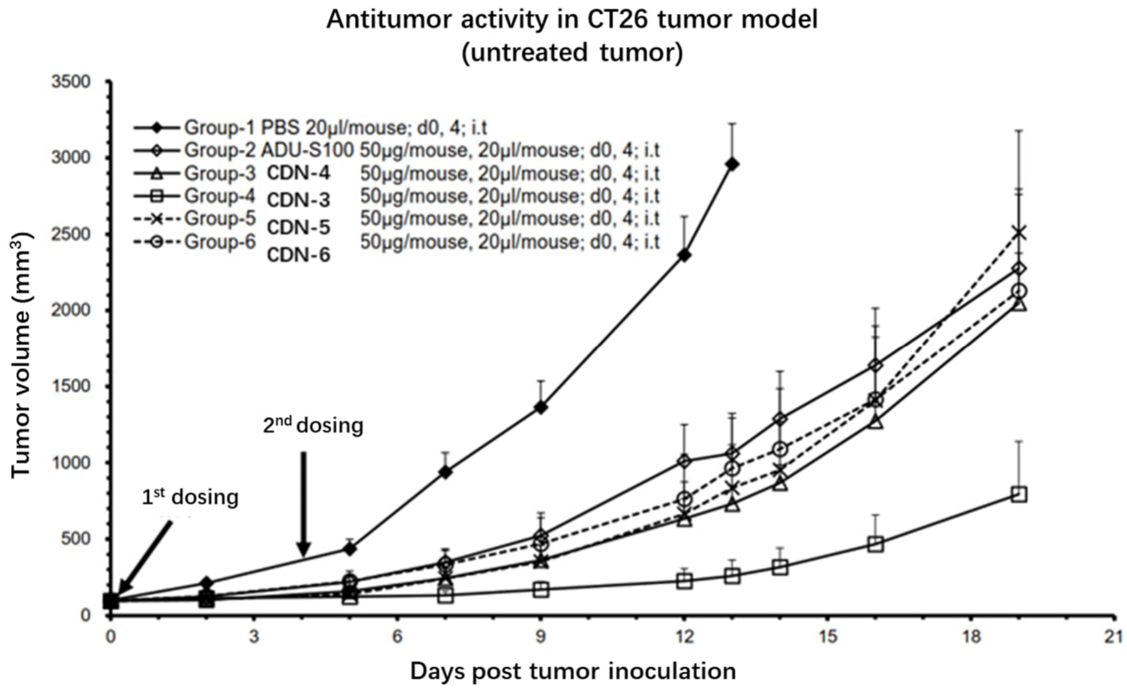
Antitumor activity in CT26 tumor model
(untreated tumor)
Group-1 PBS 20µl/mouse; d0, 4; i.t
Group-2 ADU-S100 50µg/mouse, 20µl/mouse; d0, 4; i.t
Group-3 CDN-4 50µg/mouse, 20µl/mouse; d0, 4; i.t
Group-4 CDN-3 50µg/mouse, 20µl/mouse; d0, 4; i.t
Group-5 CDN-5 50µg/mouse, 20µl/mouse; d0, 4; i.t
Group-6 CDN-6 50µg/mouse, 20µl/mouse; d0, 4; i.t
2nd dosing
1st dosing
Tumor volume (mm3)
Days post tumor inoculation

FIG. 1B

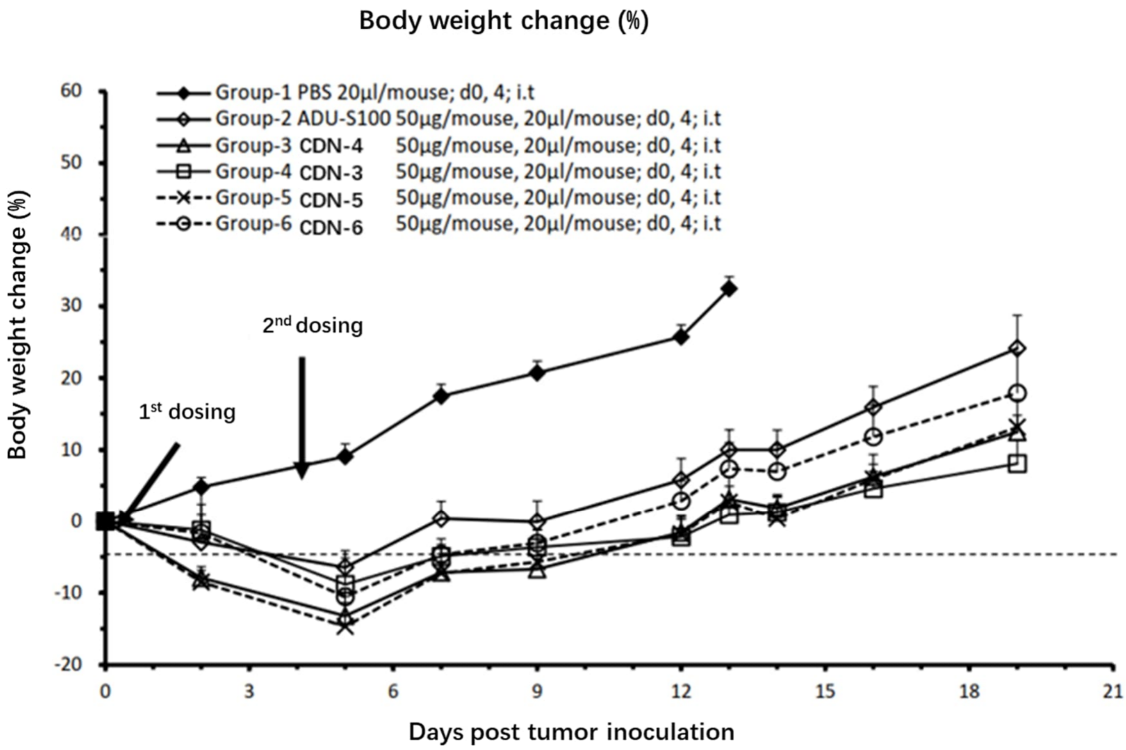
Body weight change (%)
Group-1 PBS 20µl/mouse; d0, 4; i.t
Group-2 ADU-S100 50µg/mouse, 20µl/mouse; d0, 4; i.t
Group-3 CDN-4 50µg/mouse, 20µl/mouse; d0, 4; i.t
Group-4 CDN-3 50µg/mouse, 20µl/mouse; d0, 4; i.t
Group-5 CDN-5 50µg/mouse, 20µl/mouse; d0, 4; i.t
Group-6 CDN-6 50µg/mouse, 20µl/mouse; d0, 4; i.t
Body weight change (%)
2nd dosing
1st dosing
Days post tumor inoculation

FIG. 1C

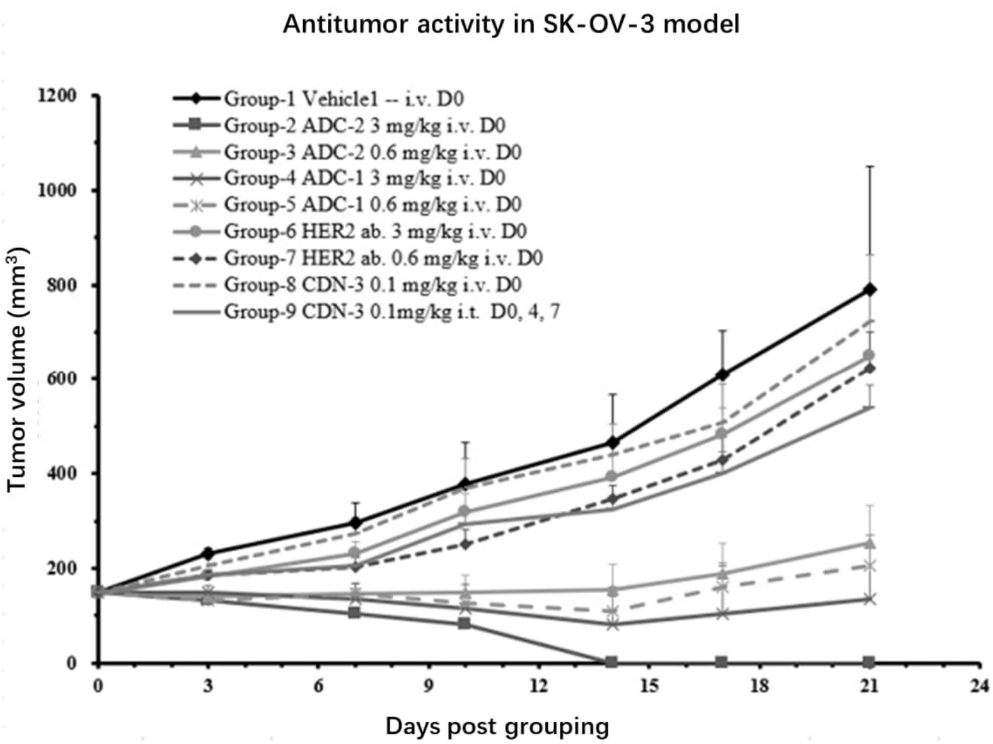
Antitumor activity in SK-OV-3 model
Group-1 Vehicle1 -- i.v. D0
Group-2 ADC-2 3 mg/kg i.v. D0
Group-3 ADC-2 0.6 mg/kg i.v. D0
Group-4 ADC-1 3 mg/kg i.v. D0
Group-5 ADC-1 0.6 mg/kg i.v. D0
Group-6 HER2 ab. 3 mg/kg i.v. D0
Group-7 HER2 ab. 0.6 mg/kg i.v. D0
Group-8 CDN-3 0.1 mg/kg i.v. D0
Group-9 CDN-3 0.1mg/kg i.t. D0, 4, 7
Tumor volume (mm3)
Days post grouping

FIG. 2

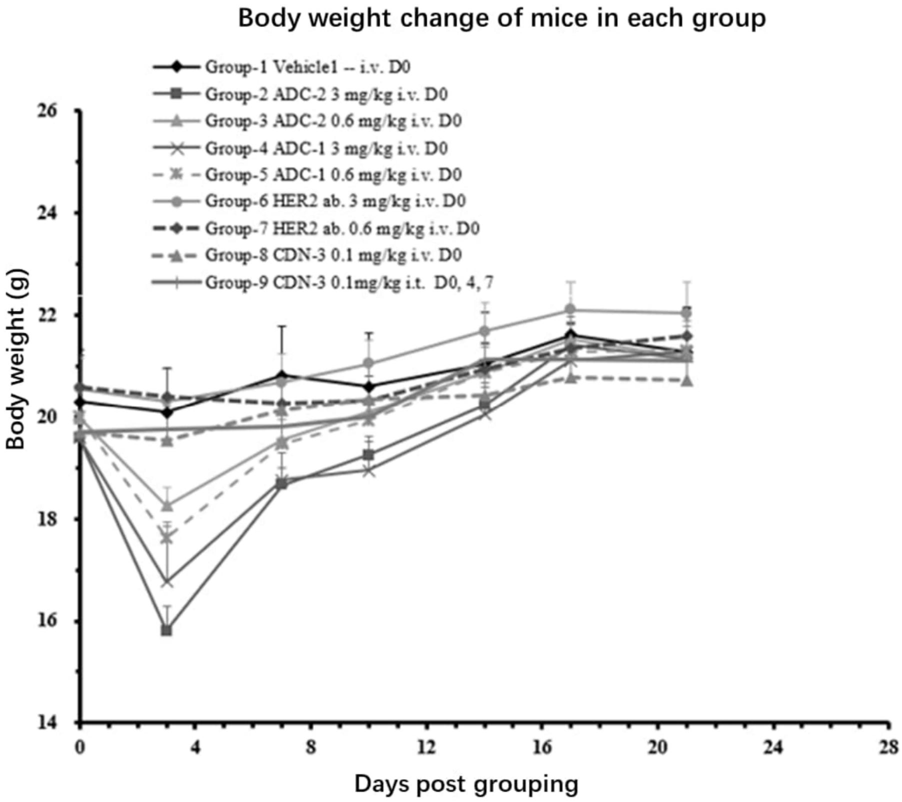
Body weight change of mice in each group
Group-1 Vehicle1 -- i.v. D0
Group-2 ADC-2 3 mg/kg i.v. D0
Group-3 ADC-2 0.6 mg/kg i.v. D0
Group-4 ADC-1 3 mg/kg i.v. D0
Group-5 ADC-1 0.6 mg/kg i.v. D0
Group-6 HER2 ab. 3 mg/kg i.v. D0
Group-7 HER2 ab. 0.6 mg/kg i.v. D0
Group-8 CDN-3 0.1 mg/kg i.v. D0
Group-9 CDN-3 0.1mg/kg i.t. D0, 4, 7
Body weight (g)
26
24
22
20
18
16
14
0
4
8
12
16
20
24
28
Days post grouping

FIG. 3

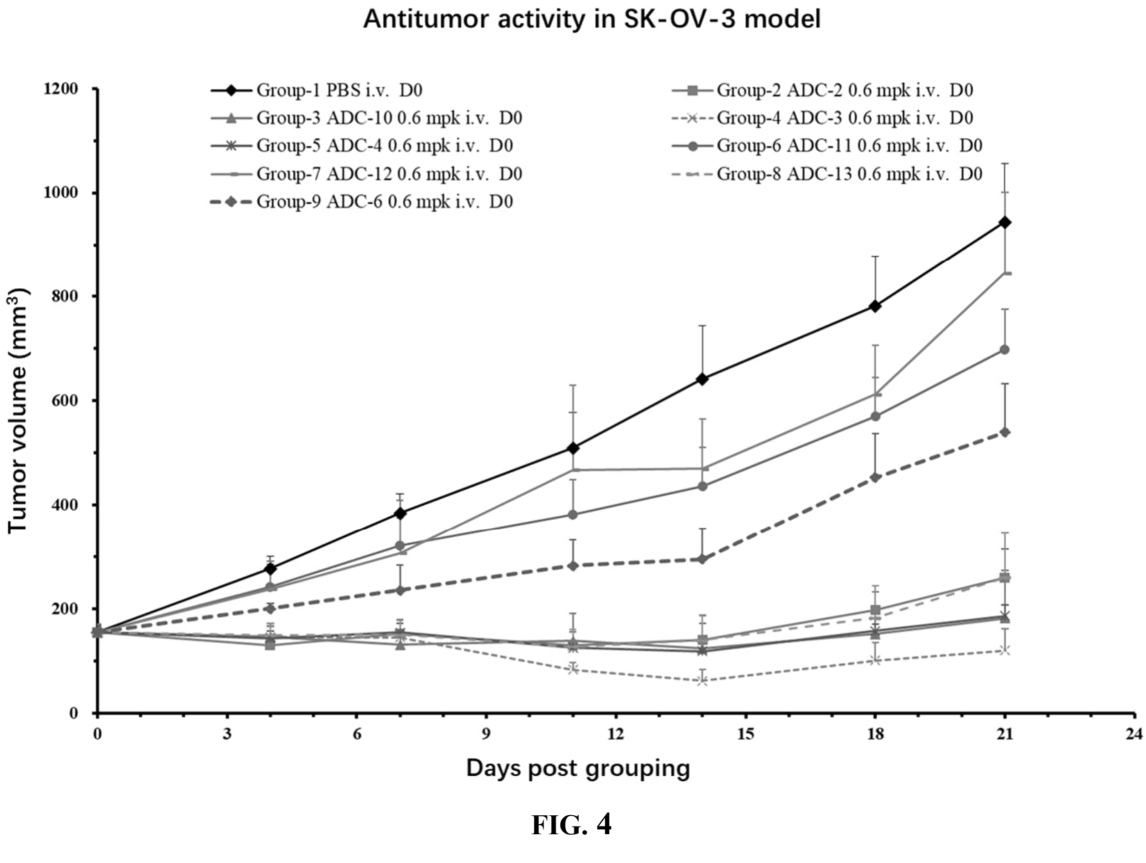
Antitumor activity in SK-OV-3 model
Group-1 PBS i.v. D0
Group-2 ADC-2 0.6 mpk i.v. D0
Group-3 ADC-10 0.6 mpk i.v. D0
Group-4 ADC-3 0.6 mpk i.v. D0
Group-5 ADC-4 0.6 mpk i.v. D0
Group-6 ADC-11 0.6 mpk i.v. D0
Group-7 ADC-12 0.6 mpk i.v. D0
Group-8 ADC-13 0.6 mpk i.v. D0
Group-9 ADC-6 0.6 mpk i.v. D0
Tumor volume (mm³)
1200
1000
800
600
400
200
0
0
3
6
9
12
15
18
21
24
Days post grouping

FIG. 4

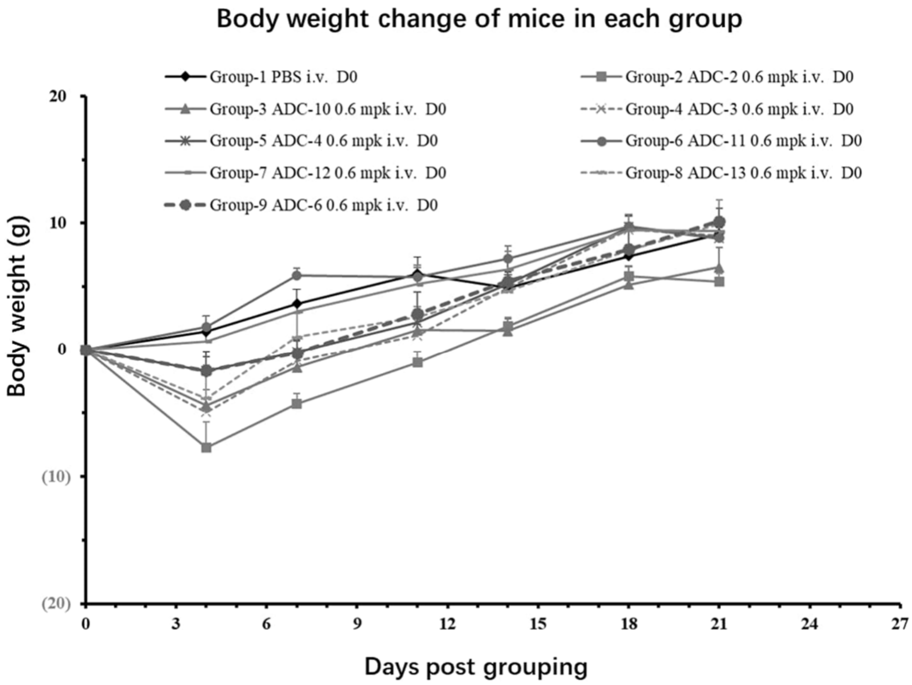
Body weight change of mice in each group
Group-1 PBS i.v. D0
Group-2 ADC-2 0.6 mpk i.v. D0
Group-3 ADC-10 0.6 mpk i.v. D0
Group-4 ADC-3 0.6 mpk i.v. D0
Group-5 ADC-4 0.6 mpk i.v. D0
Group-6 ADC-11 0.6 mpk i.v. D0
Group-7 ADC-12 0.6 mpk i.v. D0
Group-8 ADC-13 0.6 mpk i.v. D0
Group-9 ADC-6 0.6 mpk i.v. D0
Body weight (g)
20
10
0
(10)
(20)
0
3
6
9
12
15
18
21
24
27
Days post grouping

FIG. 5

**INTERNATIONAL SEARCH REPORT**

International application No. **PCT/CN2024/072762**

**A. CLASSIFICATION OF SUBJECT MATTER**

A61K47/68(2017.01)i; A61K31/7084(2006.01)i; A61K35/00(2006.01)i

According to International Patent Classification (IPC) or to both national classification and IPC

**B. FIELDS SEARCHED**

Minimum documentation searched (classification system followed by classification symbols)

IPC: A61K, A61P, C07K, C12N

Documentation searched other than minimum documentation to the extent that such documents are included in the fields searched

Electronic data base consulted during the international search (name of data base and, where practicable, search terms used)

Databases: DWPI; CNABS; CNTXT; ENTXTC; WPABSC; VEN; CNKI; PUBMED; STN: keywords: 偶联物, 缀合物, 环状二核苷酸, CDN, 核苷, STING激动剂, 腺嘌呤, 鸟嘌呤, 抗体, 结构检索(式IV-a, IV-b, CDN1-12), structure search (formulae IV-a, IV-b, CDN1-12), conjugat+, cyclic dinucleotide, sting agonist, adenine, guanine, antibod+

**C. DOCUMENTS CONSIDERED TO BE RELEVANT**

| Category* | Citation of document, with indication, where appropriate, of the relevant passages | Relevant to claim No. |
|---|---|---|
| X | WO 2019129880 A1 (INVIVOGEN) 04 July 2019 (2019-07-04) claims 1-24, and description, page 16, paragraph 1, and page 119, paragraph 1 to page 122, paragraph 1 | 1-4, 6-7, 9, 12-28, 30-32, 35, 36 (in part) |
| Y | WO 2019129880 A1 (INVIVOGEN) 04 July 2019 (2019-07-04) claims 1-24, and description, page 16, paragraph 1, and page 119, paragraph 1 to page 122, paragraph 1 | 5, 8, 10-11 |
| A | WO 2019129880 A1 (INVIVOGEN) 04 July 2019 (2019-07-04) claims 1-24, and description, page 16, paragraph 1, and page 119, paragraph 1 to page 122, paragraph 1 | 29 |
| Y | WO 2022083584 A1 (TYLIGAND BIOSCIENCE (SHANGHAI) LIMITED) 28 April 2022 (2022-04-28) claims 1-30 | 5, 8, 10-11 |
| A | CN 110799218 A (NOVARTIS AG; ADURO BIOTECH, INC.) 14 February 2020 (2020-02-14) claims 38-50 | 1-32, 35, 36 (in part) |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **22 April 2024** | **30 April 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/072762**

**C. DOCUMENTS CONSIDERED TO BE RELEVANT**

| Category* | Citation of document, with indication, where appropriate, of the relevant passages | Relevant to claim No. |
|---|---|---|
| A | CN 114786729 A (IMMUNOSENSOR THERAPEUTICS, INC.; BOARD OF REGENTS, THE UNIVERSITY OF TEXAS SYSTEM) 22 July 2022 (2022-07-22)<br>abstract, and claims 1-111 | 1-32, 35, 36 (in part) |
| A | CN 115209921 A (DAIICHI SANKYO COMPANY, LIMITED) 18 October 2022 (2022-10-18)<br>abstract, and claims 1-52 | 1-32, 35, 36 (in part) |
| A | CN 115427051 A (BEIJING XUANYI PHARMACEUTICAL TECHNOLOGY CO., LTD.) 02 December 2022 (2022-12-02)<br>claims 68-69 | 1-32, 35, 36 (in part) |
| A | CN 110325543 A (TAKEDA PHARMACEUTICAL COMPANY LIMITED) 11 October 2019 (2019-10-11)<br>claims 1-191 | 1-32, 35, 36 (in part) |

Form PCT/ISA/210 (second sheet) (July 2022)

**INTERNATIONAL SEARCH REPORT**

International application No.
**PCT/CN2024/072762**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/072762**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **32-34**
because they relate to subject matter not required to be searched by this Authority, namely:

Claim 32 relates to a method for inducing, stimulating or assisting an immune response in an individual, the method comprising administering to an individual a drug-antibody conjugate or a pharmaceutically acceptable salt or solvate or pharmaceutical composition thereof. A method for treatment of diseases cannot be excluded therefrom. Therefore, said claim belongs to the cases set forth in PCT Rule 39.1(iv) for which a search is not required. The search therefor is carried out with regard to the use of a conjugate or composition corresponding to claim 32 in the preparation of a corresponding drug.

Claims 33-34 and 36 (in part) relate to the use of a drug-antibody conjugate or a pharmaceutically acceptable salt or solvate or pharmaceutical composition thereof in the prevention or treatment of a disease, and therefore belong to the cases set out in PCT Rule 39.1(iv) for which a search is not required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/072762**

| Patent document cited in search report | Publication date (day/month/year) | Patent family member(s) | Publication date (day/month/year) |
|---|---|---|---|
| WO 2019129880 A1 | 04 July 2019 | US 2021353760 A1 | 18 November 2021 |
| | | US 2022339287 A9 | 27 October 2022 |
| | | US 2023116913 A2 | 13 April 2023 |
| | | US 11730817 B2 | 22 August 2023 |
| | | EP 3505188 A1 | 03 July 2019 |
| | | EP 3731874 A1 | 04 November 2020 |
| WO 2022083584 A1 | 28 April 2022 | CA 3198956 A1 | 28 April 2022 |
| | | US 2024059729 A1 | 22 February 2024 |
| | | EP 4242218 A1 | 13 September 2023 |
| | | AU 2021363267 A1 | 22 June 2023 |
| | | KR 20230107586 A | 17 July 2023 |
| | | JP 2023546278 A | 01 November 2023 |
| | | TW 202227099 A | 16 July 2022 |
| CN 110799218 A | 14 February 2020 | JP 2020517700 A | 18 June 2020 |
| | | EP 3615080 A1 | 04 March 2020 |
| | | WO 2018200812 A1 | 01 November 2018 |
| | | AR 113224 A1 | 19 February 2020 |
| CN 114786729 A | 22 July 2022 | WO 2021016204 A1 | 28 January 2021 |
| | | AU 2020315802 A1 | 24 February 2022 |
| | | KR 20220054794 A | 03 May 2022 |
| | | TW 202116358 A | 01 May 2021 |
| | | US 2021290774 A1 | 23 September 2021 |
| | | US 11213592 B2 | 04 January 2022 |
| | | IL 289866 A | 01 March 2022 |
| | | CA 3147890 A1 | 28 January 2021 |
| | | BR 112022000854 A2 | 08 March 2022 |
| | | US 2022105198 A1 | 07 April 2022 |
| | | US 2021015941 A1 | 21 January 2021 |
| | | US 11033635 B2 | 15 June 2021 |
| | | EP 3999118 A1 | 25 May 2022 |
| | | MX 2022000815 A | 03 May 2022 |
| | | ZA 202201988 B | 25 October 2023 |
| | | JP 2022541517 A | 26 September 2022 |
| CN 115209921 A | 18 October 2022 | JPWO 2021177438 A1 | 10 September 2021 |
| | | TW 202140044 A | 01 November 2021 |
| | | AU 2021230226 A1 | 29 September 2022 |
| | | WO 2021177438 A1 | 10 September 2021 |
| | | CO 2022013971 A2 | 31 October 2022 |
| | | IL 296124 A | 01 November 2022 |
| | | BR 112022015283 A2 | 20 September 2022 |
| | | MX 2022009597 A | 02 September 2022 |
| | | US 2023330248 A1 | 19 October 2023 |
| | | KR 20220151630 A | 15 November 2022 |
| | | EP 4115909 A1 | 11 January 2023 |
| | | CA 3168368 A1 | 10 September 2021 |
| CN 115427051 A | 02 December 2022 | WO 2021178818 A2 | 10 September 2021 |
| | | WO 2021178818 A3 | 28 October 2021 |
| | | KR 20220150356 A | 10 November 2022 |
| | | EP 4114417 A2 | 11 January 2023 |

Form PCT/ISA/210 (patent family annex) (July 2022)

**INTERNATIONAL SEARCH REPORT**
**Information on patent family members**

International application No.
**PCT/CN2024/072762**

| Patent document cited in search report | Publication date (day/month/year) | Patent family member(s) | Publication date (day/month/year) |
|---|---|---|---|
| | | AU 2021231864 A1 | 08 September 2022 |
| | | US 2024066133 A1 | 29 February 2024 |
| | | JP 2023516965 A | 21 April 2023 |
| | | TW 202144011 A | 01 December 2021 |
| | | MX 2022010884 A | 02 December 2022 |
| | | CA 3169880 A1 | 10 September 2021 |
| CN 110325543 A | 11 October 2019 | CO 2019007032 A2 | 21 October 2019 |
| | | IL 267020 A | 31 July 2019 |
| | | IL 267020 B | 01 January 2022 |
| | | BR 112019011120 A2 | 01 October 2019 |
| | | SI 3512861 T1 | 31 August 2021 |
| | | CL 2019001485 A1 | 10 January 2020 |
| | | US 2024066047 A1 | 29 February 2024 |
| | | WO 2018100558 A2 | 07 June 2018 |
| | | WO 2018100558 A3 | 19 July 2018 |
| | | WO 2018100558 A9 | 09 May 2019 |
| | | WO 2018100558 A8 | 20 June 2019 |
| | | MX 2019006397 A | 14 August 2019 |
| | | ZA 201904311 B | 28 April 2022 |
| | | EP 3512861 A2 | 24 July 2019 |
| | | EP 3512861 B1 | 17 February 2021 |
| | | US 2021106607 A1 | 15 April 2021 |
| | | US 11666594 B2 | 06 June 2023 |
| | | JP 2020055860 A | 09 April 2020 |
| | | JP 7229907 B2 | 28 February 2023 |
| | | LT 3512861 T | 25 May 2021 |
| | | JP 6633255 B1 | 22 January 2020 |
| | | JP 2020504085 A | 06 February 2020 |
| | | MA 46245 A | 24 July 2019 |
| | | US 2019192549 A1 | 27 June 2019 |
| | | US 10980825 B2 | 20 April 2021 |
| | | US 2021137962 A9 | 13 May 2021 |
| | | HUE 054450 T2 | 28 September 2021 |
| | | HRP 20210726 T1 | 01 October 2021 |
| | | PE 20210255 A1 | 10 February 2021 |
| | | RS 61818 B1 | 30 June 2021 |
| | | ECSP 19046507 A | 30 September 2019 |
| | | MY 190460 A | 21 April 2022 |
| | | ES 2866948 T3 | 20 October 2021 |
| | | AU 2017370004 A1 | 11 July 2019 |
| | | AU 2017370004 B2 | 10 March 2022 |
| | | PT 3512861 T | 29 April 2021 |
| | | JOP 20170192 A1 | 30 January 2019 |
| | | CA 3045762 A1 | 07 June 2018 |
| | | EP 3868384 A1 | 25 August 2021 |
| | | KR 20190113768 A | 08 October 2019 |
| | | KR 102640802 B1 | 26 February 2024 |
| | | CY 1124196 T1 | 27 May 2022 |
| | | PH 12019501222 A1 | 02 December 2019 |
| | | DK 3512861 T3 | 03 May 2021 |

Form PCT/ISA/210 (patent family annex) (July 2022)

**INTERNATIONAL SEARCH REPORT**
**Information on patent family members**

International application No.
**PCT/CN2024/072762**

| Patent document cited in search report | Publication date (day/month/year) | Patent family member(s) | | | Publication date (day/month/year) |
|---|---|---|---|---|---|
| | | UA | 126390 | C2 | 28 September 2022 |
| | | PL | 3512861 | T3 | 20 September 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023100744909
- CN 2023115755751
- CN 2024100467436
- WO 2022083584 A
- US 20070274985 A1
- EP 404097 A
- WO 199301161 A
- US 5821337 A
- US 7560111 B
- US 6054297 A
- US 20100119511 A
- US 20110117097 A
- US 20090202546 A
- US 20060088523 A
- US 20060018899 A
- US 20110177095 A
- US 20090285837 A
- US 20110159014 A
- US 20090187007 A
- US 20110217305 A
- US 10179171 B2
- WO 2010089782 A1
- US 20210393792 A1
- WO 2008144891 A
- WO 2011145744 A
- WO 2011155579 A
- WO 2013077458 A
- WO 2003074566 A
- WO 2011068845 A
- WO 2013068946 A
- US 20230270870 A1
- WO 2007059997 A
- WO 2016165762 A
- WO 2007059997 A1
- CN 107667118 A
- WO 2016166122 A
- US 11555070 B2
- WO 2020135674 A
- WO 2018006882 A
- CN 109762067
- WO 2019242505 A
- WO 2020038404 A
- WO 2020043044 A
- WO 2020063988 A
- WO 2020082209 A
- WO 2020018852 A
- WO 2020023679 A
- WO 2020135201 A
- WO 2020139956 A
- WO 2020025792 A
- WO 2020160560 A
- CN 111808194
- WO 2020200196 A
- WO 2007092453 A
- WO 2023040941 A1
- WO 2022271722 A1
- WO 2022159576 A1
- WO 2022128716 A1
- WO 2022105878 A1
- WO 2021247798 A1
- WO 2022104697 A1
- WO 2021066869 A1
- WO 2020233534 A1
- WO 2020130125 A1
- WO 2019046858 A1
- WO 2019046859 A1
- WO 2019035630 A2
- WO 2019035630 A3
- WO 2018098035 A1
- WO 2017214233 A1
- WO 2017214282 A1
- WO 2017214301 A1
- WO 2017161206 A1
- WO 2017139623 A1
- WO 2017136581 A1
- WO 2017076492 A1
- WO 2017060322 A3
- WO 2017025458 A1
- WO 2017008169 A1
- WO 2016065456 A1
- WO 2015143382 A1
- WO 2014143765 A1
- WO 2014143765 A8
- WO 2014152199 A1
- WO 2014094355 A1
- WO 2012143495 A2
- WO 2012143495 A3
- WO 2022083584 A1

**Non-patent literature cited in the description**


- **HUDSON et al.** *Nat.Med.*, 2003, vol. 9, 129-134
- **HOLLINGER et al.** *PNAS USA*, 1993, vol. 90, 6444-6448
- Antibody Drug Research and Application. People's Medical Publishing House, 2013
- Fundamental Immunology. Raven Press, 1993
- **KABAT et al.** Sequences of Proteins of Immunological Interest. Public Health Service, National Institutes of Health, 1991
- **GESTUR VIDARSSON et al.** *IgG subclasses and allotypes: from structure to effector functions*, 20 October 2014
- **KNAPPIK et al.** *J Mol Biol*, 2000, vol. 296, 57-86
- **ZHAO et al.** *Nucleosides, Nucleotides and Nucleic Acids*, 2009, vol. 289, 352-378
- **KNOUSE et al.** *Science*, 2018, vol. 361, 1234
- **COLIGAN et al.** Current Protocols in Protein Science. John Wiley & Sons, 2002, vol. 2
- **TREISH I** ; **SCHWARTZ R** ; **LINDLEY C**. Pharmacology and therapeutic use of trastuzumab in breast cancer. *Am J Health Syst Pharm.*, 15 November 2000, vol. 57 (22), 2063-76
- **CHO et al.** *Nature*, 2003, vol. 421, 756-760
- **HUDIS CA**. *N Engl J Med.*, 2007, vol. 357 (1), 39-51
- **FRANKLIN et al.** *Cancer Cell*, 2004, vol. 5, 317-328
- **AGUS et al.** *J Clin Oncol.*, 07 February 2005, vol. 23 (11), 2534-43
- **BERGSTROM D. A. et al.** *Cancer Res.*, 2015, vol. 75, LB-231
- **YING WEN et al.** A literature review of the promising future of TROP2: a potential drug therapy target. *Ann Transl Med.*, December 2022, vol. 10 (24), 1403
- **DAISUKE OKAJIMA et al.** Datopotamab Deruxtecan, a Novel TROP2-directed Antibody-drug Conjugate, Demonstrates Potent Antitumor Activity by Efficient Drug Delivery to Tumor Cells. *Mol Cancer Ther*, 2021, vol. 20 (12), 2329-2340
- **DAISUKE KYUNO et al.** Claudin-18.2 as a therapeutic target in cancers: cumulative findings from basic research and clinical trials. *Tissue Barriers.*, 2022, vol. 10 (1), 1967080
- **JINXIA CHEN**. Targeting CLDN18.2 in cancers of the gastrointestinal tract: New drugs and new indications. *Front Oncol.*, 2023, vol. 13, 1132319
- **JINFENG YU et al.** Antibody-Drug Conjugates Targeting the Human Epidermal Growth Factor Receptor Family in Cancers. *Front Mol Biosci.*, 2022, vol. 9, 847835
- **GENNARO A.R. et al.** Remington: The Science and Practice of Pharmacy. Lippincott, Williams & Wilkins, 2000
- **E. L. ELIEL** ; **S. H. WILEN** ; **L. N. MANDER**. Stereochemistry of Organic Compounds. Wiley-Interscience, 1994
- *CHEMICAL ABSTRACTS*, 2245335-71-9
- *CHEMICAL ABSTRACTS*, 2245335-70-8
- *CHEMICAL ABSTRACTS*, 2632342-05-1
- *CHEMICAL ABSTRACTS*, 1638970-44-1
- *CHEMICAL ABSTRACTS*, 159857-80-4
- *CHEMICAL ABSTRACTS*, 1870916-87-2
- *Journal of the American Chemical Society*, 2015, vol. 137 (15), 4924-4927
- *CHEMICAL ABSTRACTS*, 1638750-95-4
- *CHEMICAL ABSTRACTS*, 180288-69-1
- *CHEMICAL ABSTRACTS*, 1796566-95-4
- *CHEMICAL ABSTRACTS*, 1496553-00-4
- *CHEMICAL ABSTRACTS*, 205923-56-4